# EUROPEAN PATENT APPLICATION

(11) **EP 4 582 426 A1**
(43) Date of publication of application: **09.07.2025**
(21) Application number: 23867257.0
(22) Date of filing: 01.09.2023
(51) Int. Cl.: C07D 471/22, C07D 487/12, C07D 513/22, C07D 519/00, A61K 31/429, A61K 31/5025, A61P 35/00

(54) **PAN-KRAS INHIBITOR COMPOUND**

(30) Priority: 19.09.2022 CN 202211154665
(71) Applicant: Adlai Nortye Biopharma Co., Ltd., Yuhang District Hangzhou Zhejiang 311121 (CN)
(72) Inventor: CHEN, Yufeng, Hangzhou, Zhejiang 311121 (CN); LV, Meng, Hangzhou, Zhejiang 311121 (CN); LIU, Canfeng, Hangzhou, Zhejiang 311121 (CN); CHENG, Wanli, Hangzhou, Zhejiang 311121 (CN); LI, Feifan, Hangzhou, Zhejiang 311121 (CN); YANG, Han, Hangzhou, Zhejiang 311121 (CN); CHEN, Kaixuan, Hangzhou, Zhejiang 311121 (CN); LIU, Shuaishuai, Hangzhou, Zhejiang 311121 (CN); HE, Nanhai, Hangzhou, Zhejiang 311121 (CN)
(74) Representative: Office Freylinger
(86) International application number: PCT/CN2023/116437
(87) International publication number: WO 2024/060966

(57) **Abstract**

The present invention relates to a pan-KRAS inhibitor compound represented by formula (I) and a pharmaceutical composition containing the compound, and the use of compound of formula (I) for preventing and/or treating cancer, tumor, inflammatory disease, autoimmune disease or immune-mediated disease.

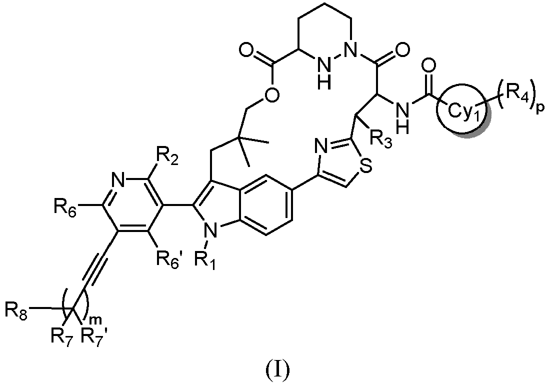

## Description

### Technical field

The present invention relates to a compound, in particular to a highly active pan-KRAS inhibitor and its use.

### Background technique

RAS is one of the most frequently mutated genes in human tumors, and its mutation occurs in about 30% of tumor patients, among which KRAS accounts for about 85% of RAS mutations. KRAS mutations exist in 88% of pancreatic cancer, 50% of colorectal adenocarcinoma, and 32% of lung adenocarcinoma. The development of targeted KRAS inhibitors has great clinical significance and value.

KRAS is a membrane-bound protein with GTPase activity, which cycles between a GDP-bound inactive conformation and a GTP-bound active conformation through nucleotide exchange, performing the function of a "molecular switch". KRAS in the GTP-bound state can activate multiple downstream signaling pathways including RAF-MEK-ERK and PI3K-AKT, and regulate life processes such as cell growth, proliferation, differentiation and apoptosis.

KRAS mutations (such as G12C, G12D, G12V, G13D, etc.) will affect the hydrolysis of GTP mediated by GTPase activating proteins (GAPs), increase KRAS in the GTP-bound activated state, and over-activate downstream signaling pathways, eventually lead to the occurrence and development of tumors. However, since the KRAS protein lacks a corresponding hydrophobic pocket suitable for drug binding, and its affinity with GTP and GDP is at the picomolar level (~20pM), it is very difficult to develop inhibitors that competitively bind to KRAS. KRAS has been considered an undruggable target for the past decades.

In May 2021, AMG510 was approved by the FDA for the treatment of locally advanced or metastatic non-small cell lung cancer carrying the KRAS^{G12C} mutation, breaking the history of KRAS being "undruggable". However, the G12C mutation only accounts for a small portion of KRAS mutations. For mutations at other sites of KRAS, there is still a lack of satisfactory and effective inhibitor compounds, and a large number of clinical needs have not been met. Therefore, the development of effective pan- KRAS inhibitor compounds are a need in the art.

### Contents of the invention

The invention provides a pan-KRAS inhibitor. Such structures are different from existing KRAS^{G12C} inhibitors that act through covalent binding, but instead mediate the formation of a ternary complex between ubiquitous intracellular chaperones (such as Cyclophilin A) and KRAS proteins. The formation of the ternary complex can block the combination of KRAS and its downstream effector molecules (such as RAF) through steric blockage, inhibit the activation of MAPK and PI3K-AKT signaling pathways, thereby inhibiting the occurrence and development of tumors, and play a role in the treatment of tumors and other diseases.

In one aspect, the present invention provides a compound having a structure of formula (I) or a pharmaceutically acceptable salt, isotopic derivative, or stereoisomer thereof: 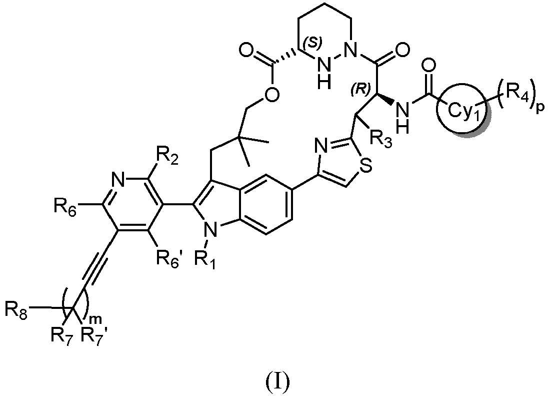 wherein:
R₁ represents C₁-C₆ alkyl, -(C₁-C₆ alkylene)-(C₃-C₈ cycloalkyl) or -(C₁-C₆ alkylene)-(3-8 membered heterocycloalkyl);
R₂ represents halogen, cyano, C₁-C₆ alkyl, -(C₀-C₆ alkylene)-(C₃-C₈ cycloalkyl), or -(C₀-C₆ alkylene)-(3-8 membered heterocycloalkyl), which may optionally be substituted by 0, 1 or 2 of the following substituents: -ORa, -SRa or - NRaRa';
R₃ represents hydrogen, -O (C₀-C₆ alkylene) Ra, -S (C₀-C₆ alkylene) Ra, -N (C₀-C₆ alkylene) Ra (C₀-C₆ alkylene) Ra', -O (C₂-C₆ alkylene) R_{L}, -S (C₂-C₆ alkylene) R_{L}, -N (C₂-C₆ alkylene) R_{L} (C₂-C₆ alkylene) R_{L}', wherein, R_{L}, R_{L}' each independently represents -ORa, -SRa, or NRaRa';
Cy₁ represents C₃-C₁₂ cycloalkyl or 3-12 membered heterocycloalkyl;
R₄ represents hydrogen, halogen, oxo, C₁-C₆ alkyl, -(C₀-C₆ alkylene) (C₃-C₆) cycloalkyl, -(C₀-C₆ alkylene) (3-8 membered) heterocycloalkyl, -(C₀-C₆ alkylene) ORa, -(C₀-C₆ alkylene) SRa, -(C₀-C₆ alkylene) NRaRa', -CORa, -(C₀-C₆ alkylene) COORa , -(C₀-C₆ alkylene) CONRaRa', -(C₀-C₆ alkylene) NRaCORa', -(C₀-C₆ alkylene) OCONRaRa', -(C₀-C₆ alkylene) NRaCONRaRa', - (C₀-C₆ alkylene) SORa, -(C₀-C₆ alkylene) S(O)₂Ra, -(C₀-C₆ alkylene) NRaS(O)₂Ra', -(C₀-C₆ alkylene) CN, -(C₀-C₆ alkylene) (C₆-C₁₀ aryl) or -(C₀-C₆ alkylene) (5-12 membered heteroaryl); wherein, R₄ on the two C atoms of Cy₁ together with the C atoms connected thereto and the atoms between the two C atoms can form a 3-8 membered ring, and the 3-8 membered ring optionally can contain 0, 1, 2 or 3 members selected from A heteroatom of N, O or S; or two R4 on the same C atom of Cy1 together with the C atom connected thereto can form a 3-8 membered ring, and the 3-8 membered ring can optionally contain 0, 1, 2 or 3 heteroatoms selected from N, O or S;
R₈ represents-Cy₂-(R₅)_{q} or-NR₉R₉', wherein,
Cy₂ represents C₃-C₁₂ cycloalkyl, 3-12 membered heterocycloalkyl, C₆-C₁₀ aryl or 5-12 membered heteroaryl;
R₅ represents hydrogen, halogen, oxo, C₁-C₆ alkyl, -(C₀-C₆ alkylene) ORa, -(C₀-C₆ alkylene) SRa, -(C₀-C₆ alkylene) NRaRa', or R₅ on the two C atoms of Cy₂ together with the C atoms connected thereto and the atoms between the two C atoms can form a 3-8 membered ring, and the 3-8 membered ring optionally can contain 0, 1, 2 or 3 heteroatoms selected from N, O or S; or two R₅ on the same C atom of Cy₂ together with the C atom connected thereto can form a 3-8 membered ring, and the 3-8 membered ring optionally may contain 0, 1, 2 or 3 heteroatoms selected from N, O or S; or at least one atom on the Cy₂ ring is substituted by S(=O)(=NRa) or S( =O)₂;
R₉ and R₉' each independently represent C₁-C₆ alkyl, C₃-C₈ cycloalkyl, 3-8 membered heterocycloalkyl, C₆-C₁₀ aryl or 5-12 membered heteroaryl which may be optionally substituted by q R5;
R₆ and R₆' each independently represent hydrogen, halogen, C₁-C₆ alkyl, C₃-C₈ cycloalkyl or -(C₀-C₆ alkylene)CN;
R₇ and R₇' each independently represent hydrogen, halogen, C₁-C₆ alkyl, C₃-C₈ cycloalkyl, 3-8 membered heterocycloalkyl; or R₇ and R₇' form a 3-8 membered ring, which may optionally contain 0, 1, 2 or 3 heteroatoms selected from N, O, S.
wherein, p and q independently represent 0, 1, 2, 3 or 4;
m represents 0, 1, 2 or 3;
Ra and Ra' independently represent hydrogen, C₁-C₆ alkyl, and C₃-C₈ cycloalkyl; wherein, if Ra and Ra' are connected to the same N atom, the Ra and Ra' can be connected to the common N atom to form a 4-8 membered ring, which optionally may contain 0, 1, 2 or 3 heteroatoms selected from N, O or S.
each of the alkyl, cycloalkyl, heterocycloalkyl and alkylene can be independently substituted by 0, 1, 2, 3, 4, 5 or 6 halogen atoms.

Furthermore, the present invention provides a compound having the structure of formula (I) as described above or a pharmaceutically acceptable salt, isotopic derivative, or stereoisomer thereof, wherein R₁ represents a C₁-C₆ alkyl, preferably a C₁ -C₃ alkyl.

Furthermore, the present invention provides a compound having the structure of formula (I) as described above or a pharmaceutically acceptable salt, isotopic derivative, or stereoisomer thereof, wherein R₂ represents C₁-C₆ alkyl, which can optionally be substituted with 0, 1 or 2 -ORa substituents. More preferably, R₂ represents 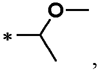 preferably 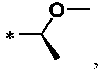 wherein, * represents the site where R₂ is linked to the linking site in formula (I).

Further, the present invention provides a compound having the structure of formula (I) as described above or a pharmaceutically acceptable salt, isotopic derivative, or stereoisomer thereof, wherein R₃ represents -O(C₁-C₆) alkyl, -O(C₀-C₆ alkylene) (C3-C8) cycloalkyl, -O(C₀-C₆ alkylene) (3-8 member) heterocycloalkyl, -O(C₂-C₆) R_{L} or hydrogen

Further, the present invention provides a compound having the structure of formula (I) as described above or a pharmaceutically acceptable salt, isotopic derivative, or stereoisomer thereof, wherein Cy**₁** represents a C₃-C₈ cycloalkyl or 3 -8 membered heterocycloalkyl.

Further, the present invention provides a compound having the structure of formula (I) as described above or a pharmaceutically acceptable salt, isotopic derivative, or stereoisomer thereof, wherein R₄ represents hydrogen, halogen, C₁-C₆ alkyl , -(C₀-C₆ alkylene) CONRaRa', -(C₀-C₆ alkylene) NRaCORa', -(C₀-C₆ alkylene) OCONRaRa', -(C₀-C₆ alkylene) CN, -(C₀-C₆ alkylene) (5-12 membered heteroaryl), or R₄ on the two C atoms of Cy₁ together with the C atom connected thereto and the atoms between the two C atoms can form a 3-8 membered ring, and the 3-8 membered ring optionally can contain 0, 1, 2 or 3 heteroatoms selected from N, O or S; or two R4 on the same C atom of Cy₁ together with the C atom connected thereto may form a 3-8 membered ring, and the 3-8 membered ring may optionally contain 0, 1, 2 or 3 heteroatoms selected from N, O or S.

Further, the present invention provides a compound having the structure of formula (I) as described above or a pharmaceutically acceptable salt, isotopic derivative, or stereoisomer thereof, wherein R₄ represents hydrogen, halogen, C₁-C₆ alkyl , -(C₀-C₆ alkylene) CONRaRa', -(C₀-C₆ alkylene) (5-12 membered heteroaryl), or R₄ on the two C atoms of Cy₁ together with the C atom connected thereto and the atoms between the two C atoms can form a 3-8 membered ring, and the 3-8 membered ring optionally can contain 0, 1, 2 or 3 heteroatoms selected from N, O or S; or two R₄ on the same C atom of Cy₁ together with the C atom connected thereto can form a 3-8 membered ring, and the 3-8 membered ring can optionally contain 0, 1, 2 or 3 heteroatoms selected from N, O or S.

Further, the present invention provides a compound having the structure of formula (I) as described above or a pharmaceutically acceptable salt, isotopic derivative, or stereoisomer thereof, wherein Cy₂ represents a 3-8 membered heterocycloalkyl or 5-12 membered heteroaryl.

Further, the present invention provides a compound having the structure of formula (I) as described above or a pharmaceutically acceptable salt, isotopic derivative, or stereoisomer thereof, wherein R₅ represents hydrogen, halogen, C₁-C₆ alkyl, -(C₀-C₆ alkylene) ORa, -(C₀-C₆ alkylene) NRaRa'; or R₅ on the two C atoms of Cy₂ together with the C atom connected thereto and the atoms between the two C atoms can form a 3-8 membered ring, and the 3-8 membered ring optionally can contain 0, 1, 2 or 3 heteroatoms selected from N, O or S; or two R5 on the same C atom of Cy2 together with the C atom connected thereto can form a 3-8 membered ring, and the 3-8 membered ring optionally can contain 0, 1, 2 or 3 heteroatoms selected from N, O or S;

Furthermore, the present invention provides a compound having the structure of formula (I) as described above or a pharmaceutically acceptable salt, isotopic derivative, or stereoisomer thereof, wherein at least one of R₉ and R₉' represents C₁-C₆ alkyl substituted by q R5.

Furthermore, the present invention provides a compound having the structure of formula (I) as described above or a pharmaceutically acceptable salt, isotopic derivative, or stereoisomer thereof, wherein R₆ and R₆' each independently represent hydrogen or C₁-C₆ alkyl; more preferably, R₆ and R₆' each independently represent hydrogen or methyl.

Furthermore, the present invention provides a compound having the structure of formula (I) as described above or a pharmaceutically acceptable salt, isotopic derivative, or stereoisomer thereof, wherein R₇ and R₇' each independently represent hydrogen, C₁-C₆ alkyl; or R₇, R₇' form a 3-8 membered ring with the C atom connected thereto, and the ring may optionally contain 0, 1, 2 or 3 heteroatoms selected from N, O, S. More preferably, R₇ represents hydrogen.

Further, the present invention provides a compound having the structure of formula (I) as described above or a pharmaceutically acceptable salt, isotopic derivative, or stereoisomer thereof, wherein m, p, and q are each independently preferably 0 , 1 or 2.

Further, the present invention provides a compound having the structure of formula (I) as described above or a pharmaceutically acceptable salt, isotopic derivative, or stereoisomer thereof, wherein the structure of -Cy₁-(R₄)p is selected from the following: 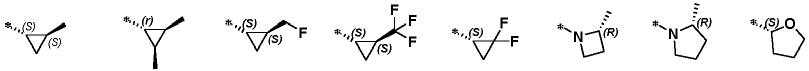 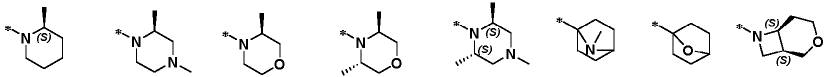 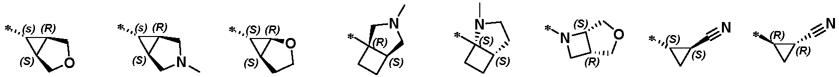 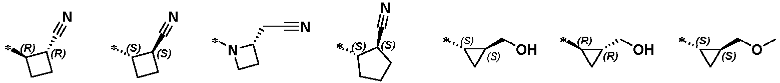 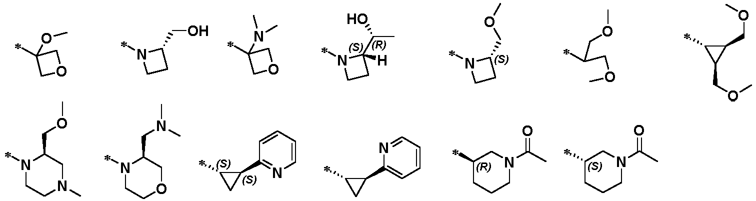 or 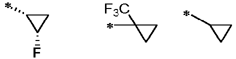 wherein, * represents the site where -Cy₁-(R₄)p is linked to the linking site in formula (I).

Further, the present invention provides a compound having the structure of formula (I) as described above or a pharmaceutically acceptable salt, isotopic derivative, or stereoisomer thereof, wherein the structure of -Cy₂-(R₅)q in formula (I) is selected from the following: 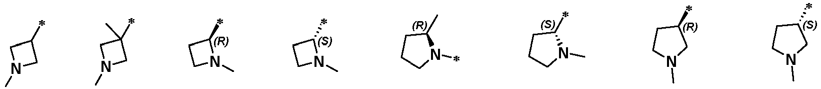 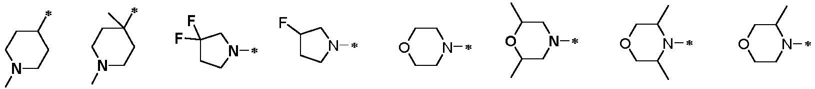 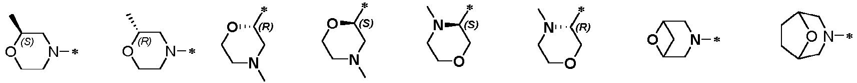 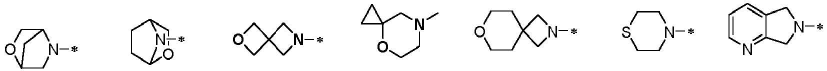 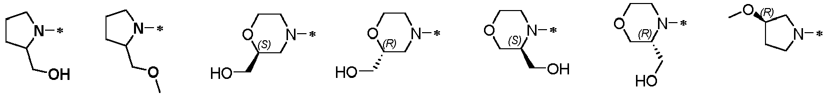 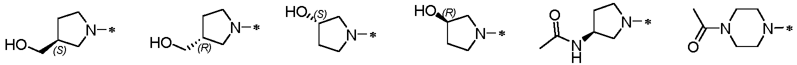 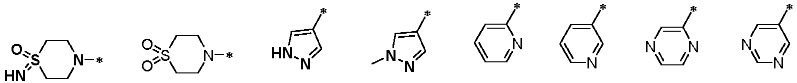 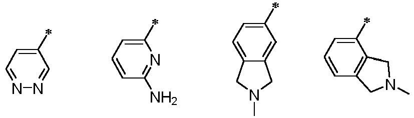 or 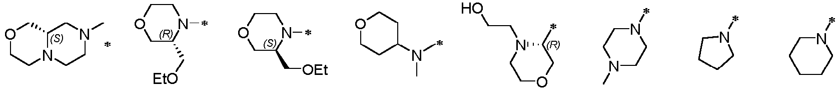 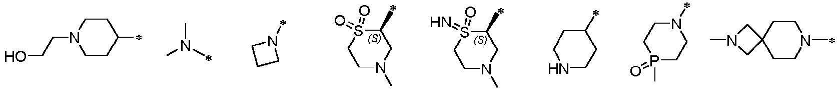 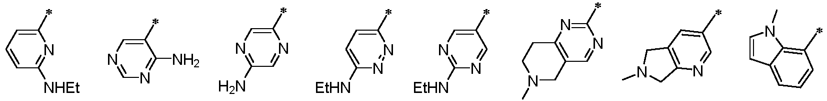 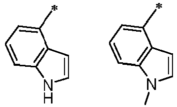 wherein, * represents the site where -Cy₂-(R₅)_{q} is linked to the linking site in formula (I).

Furthermore, the present invention provides a compound having the structure of formula (I) or a pharmaceutically acceptable salt, isotopic derivative, or stereoisomer thereof, wherein the compound of formula (I) is the compound of formula (II) structure:

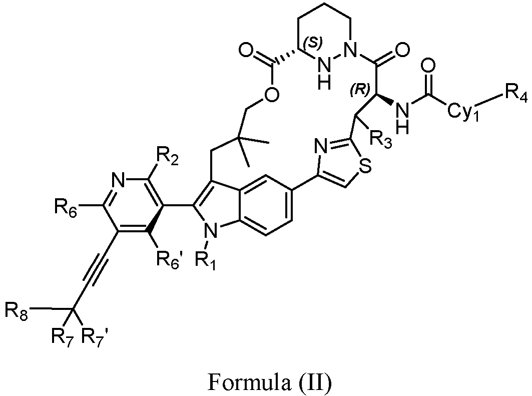

In another aspect, the present invention also provides compounds having the following structure:

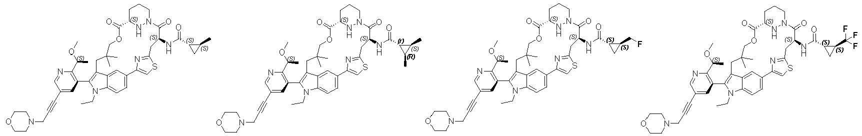

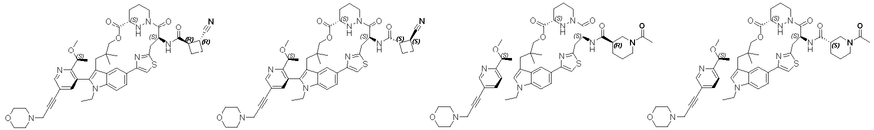

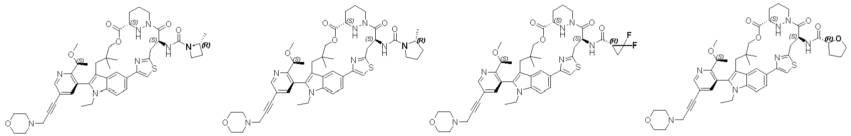

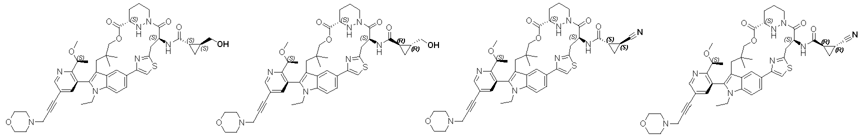

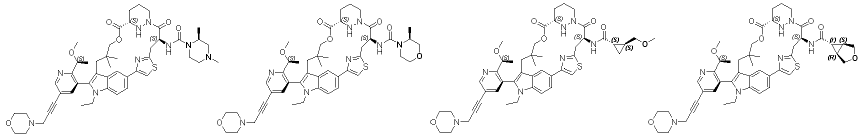

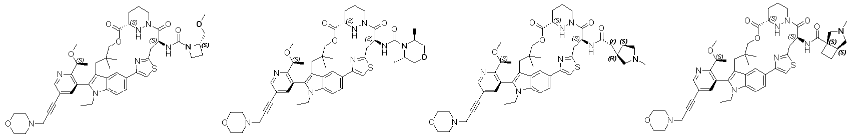

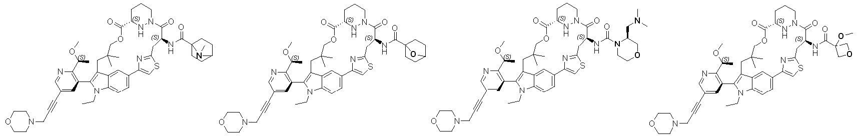

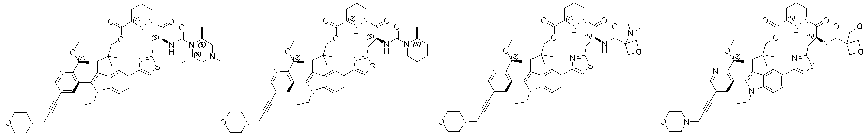

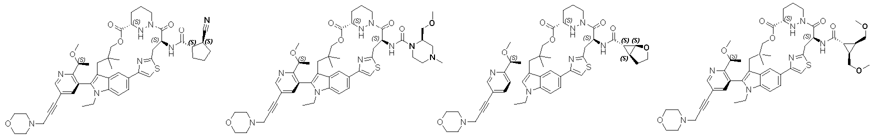

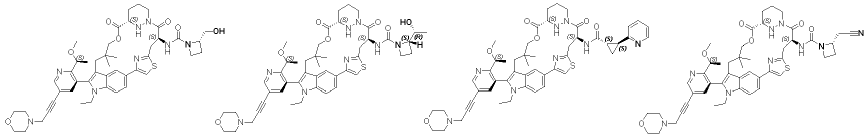

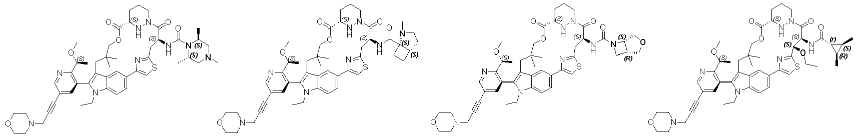

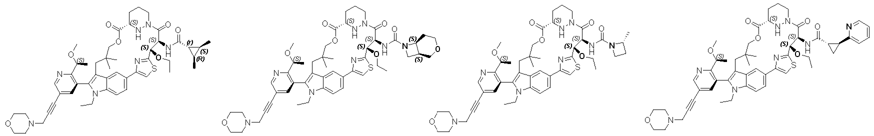

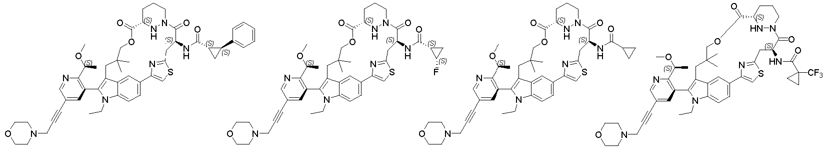

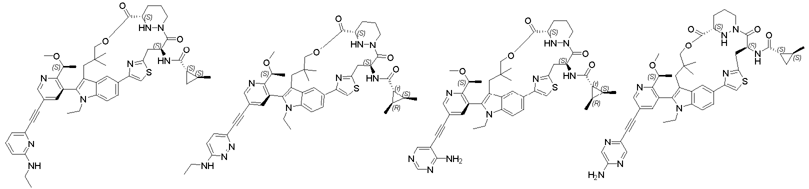

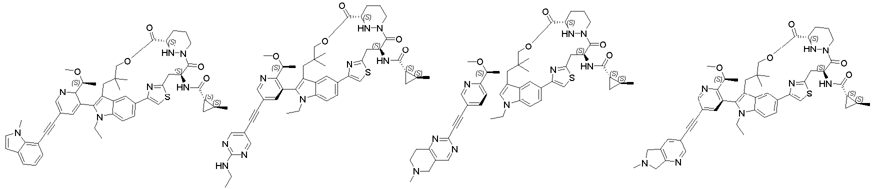

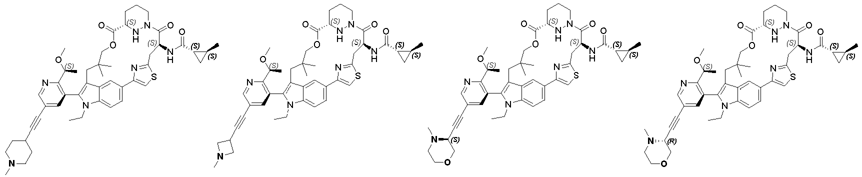

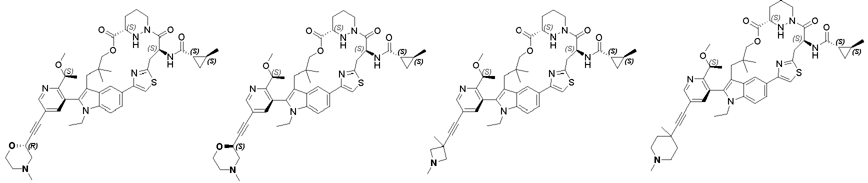

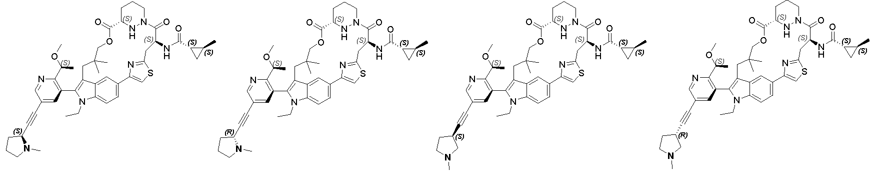

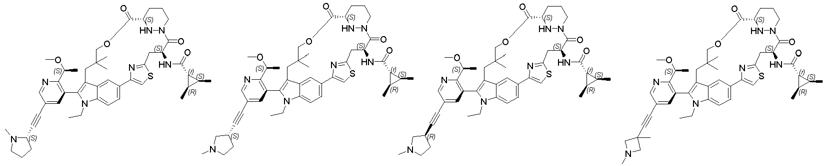

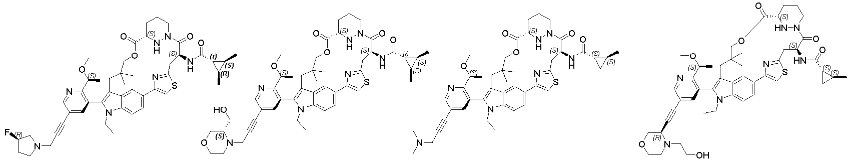

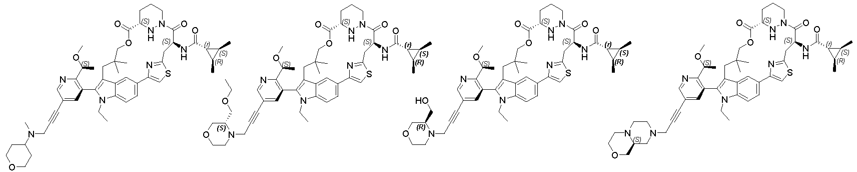

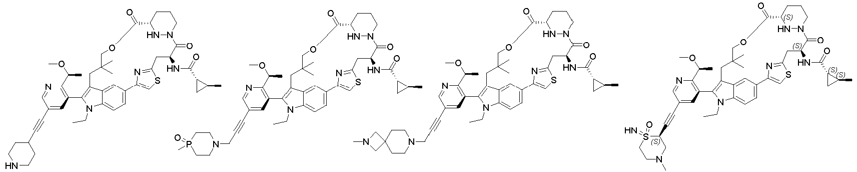

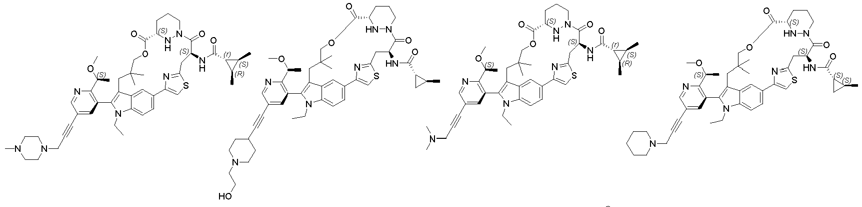

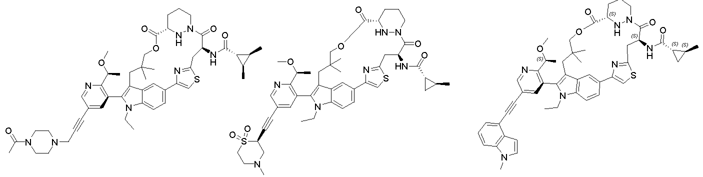

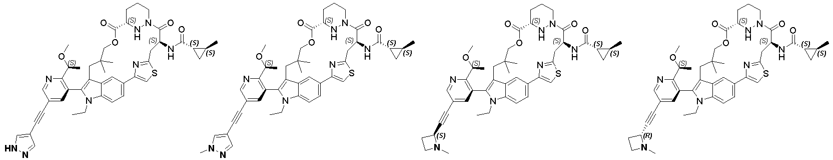

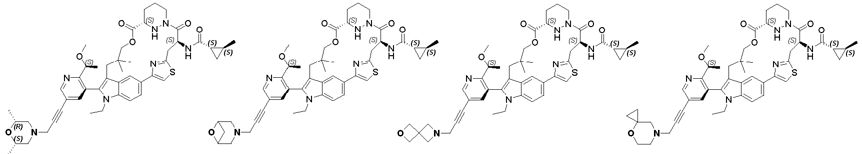

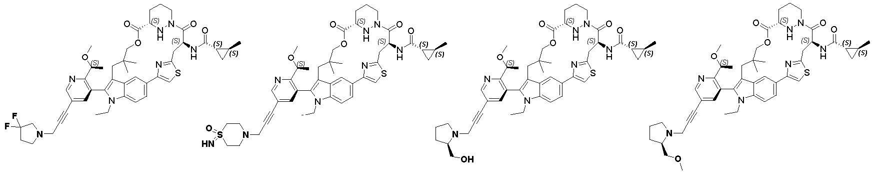

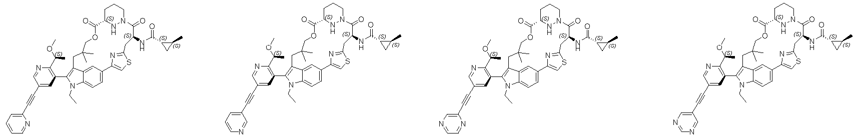

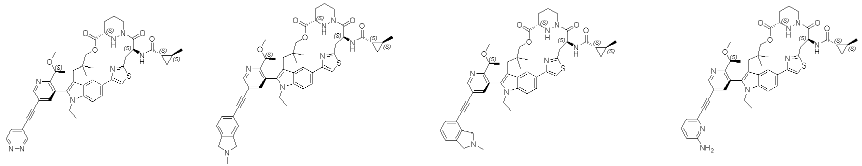

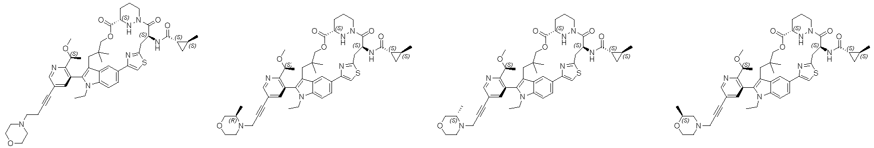

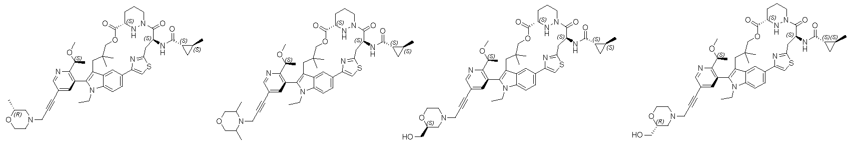

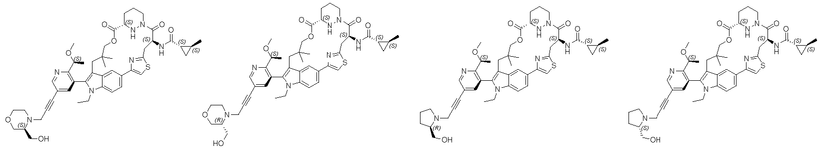

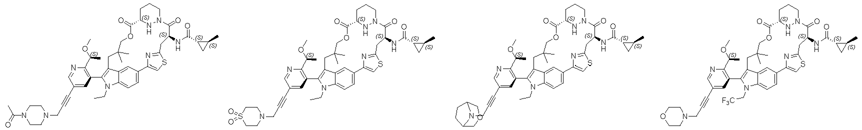

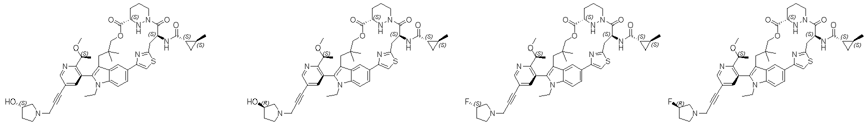

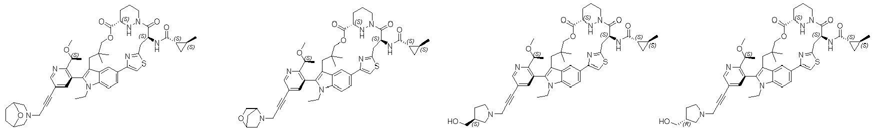

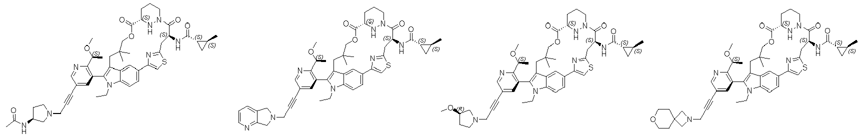

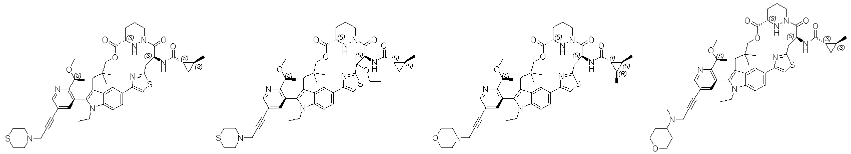

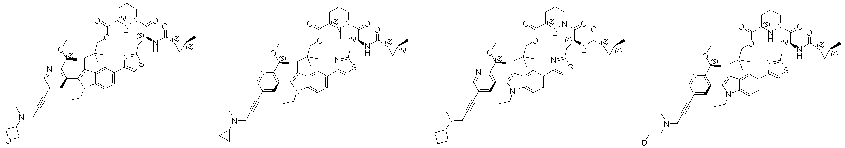

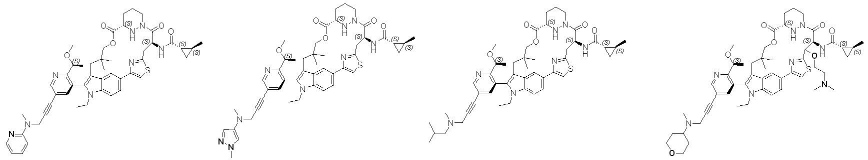

In yet another aspect, the present invention also provides a pharmaceutical composition, which includes any of the aforementioned compounds or pharmaceutically acceptable salts, isotopic derivatives, and stereoisomers thereof.

In one aspect, the present invention also provides use of the aforementioned compounds or pharmaceutically acceptable salts, isotopic derivatives, stereoisomers thereof and pharmaceutical compositions in the preparation of medicine for preventing and/or treating cancers, tumors, inflammatory diseases, autoimmune immune diseases or immune-mediated diseases.

It should be noted that, in this article, when referring to the "compound" of formula (I) and formula (II), it generally also covers its stereoisomers, diastereomers,enantiomers, racemic mixtures and isotopic derivatives.

It is well known to those skilled in the art that a compound's salt, solvate, and hydrate are alternative forms of the compound, and they can all be converted into the compound under certain conditions. When referring to the compounds of formula (I) and formula (II), generally, their pharmaceutically acceptable salts are also included, and furthermore, their solvates and hydrates are also included.

Similarly, when referring to a compound herein, its prodrugs, metabolites and nitroxides are also generally included.

The pharmaceuticall**y** acceptable salts of the present invention ma**y** be formed using, for example, the following inorganic or organic acids. "Pharmaceuticall**y** acceptable salt" means a salt which, within the scope of reasonable medical judgment, is suitable for use in contact with tissues of humans and lower animals, without undue toxicity, irritation, allergic reaction, etc., can be called a reasonable benefit / risk ratio. The salts can be prepared in situ during the final isolation and purification of the compounds of the invention, or alone b**y** reacting the free base or acid with a suitable reagent, as outlined below. For example, a free base function can be reacted with a suitable acid. Examples of pharmaceuticall**y** acceptable inorganic acid addition salts are amino acids with inorganic acids (e.g., h**y**drochloric, h**y**drobromic, phosphoric, sulfuric, and perchloric) or organic acids (e.g., acetic, oxalic, maleic, tartaric, lemon acid, succinic acid or malonic acid), or b**y** using other methods known in the art such as ion exchange. Other pharmaceuticall**y** acceptable salts include adipate, sodium alginate, ascorbate, aspartate, benzenesulfonate, benzoate, bisulfate, borate, but**y**rate, camphorate, camphorsulfonate, citrate, c**y**clopentanepropionate, digluconate, laur**y**l sulfate, ethylate, formate, fumarate, glucoheptonate, gl**y**cerin phosphate, gluconate, hernisulfate, heptanoate, hexanoate, h**y**droiodide, 2-h**y**drox**y**-ethanesulfonate, lactobionate, lactate, laurate, laur**y**l sulfate, malate, maleate, malonate, methanesulfonate, 2-naphthalenesulfonate, nicotinate, nitrate, oleate, oxalate, palmitate, pamoate, pectate, persulfate, 3-phen**y**lpropionate, phosphate, bitter salt, pivalate, propionate, stearate, succinate, sulfate, tartrate, thioc**y**anate, p-toluene sulfonate, undecanoate, valerate, etc. Representative alkali or alkaline earth metal salts include those of sodium, lithium, potassium, calcium, magnesium, and the like. Other pharmaceuticall**y** acceptable salts include, where appropriate, nontoxic ammonium salts, quaternar**y** ammonium salts, and amine cations formed with counterions, for example, halides, h**y**droxides, carbox**y**lates, sulfates, phosphates, nitrates, lower alk**y**lsulfonates and ar**y**lsulfonates.

The pharmaceutically acceptable salts of the present invention can be prepared by conventional methods, for example, by dissolving the compound of the present invention in a water-miscible organic solvent (such as acetone, methanol, ethanol and acetonitrile), adding an excess of organic acid or inorganic acid aqueous solution, so that the salt is precipitated from the resulting mixture, the solvent and remaining free acid are removed therefrom, and the precipitated salt is isolated.

The precursors or metabolites described in the present invention may be precursors or metabolites known in the art, as long as the precursors or metabolites are transformed into compounds through in vivo metabolism. For example, "prodrugs" refer to those prodrugs of the compounds of the present invention which, within the scope of sound medical judgment, are suitable for use in contact with human and lower animal tissues without undue toxicit**y**, irritation, allergic response, etc., qualified as having a reasonable benefit/risk ratio and valid for its intended use. The term "prodrug" refers to a compound that is rapidl**y** transformed in vivo to **y**ield the parent compound of the above formula, for example b**y** in vivo metabolism, or N-demeth**y**lation of a compound of the invention.

"Solvate" as used herein means a ph**y**sical association of a compound of the present invention with one or more solvent molecules, whether organic or inorganic. This ph**y**sical association includes h**y**drogen bonding. In some cases, for example, when one or more solvent molecules are incorporated into the cr**y**stal lattice of a cr**y**stalline solid, solvates will be able to be isolated. Solvent molecules in solvates ma**y** exist in regular and/or disordered arrangements. Solvates ma**y** contain stoichiometric or non-stoichiometric amounts of solvent molecules. "Solvate" encompasses both solution-phase and isolatable solvates. Exemplar**y** solvates include, but are not limited to, hydrates, ethanolates, methanolates, and isopropanolates. Solvation methods are well known in the art.

The "stereoisomerism" described in the present invention is divided into conformational isomerism and configurational isomerism, and configurational isomerism can also be divided into cis-trans isomerism and optical isomerism (that is, optical isomerism). Due to the rotation or twisting of carbon and carbon single bonds in organic molecules of a certain configuration, a stereoisomerism phenomenon in which each atom or atomic group of the molecule has a different arrangement in space, the common structures are alkanes and cycloalkanes. Such as the chair conformation and boat conformation that appear in the structure of cyclohexane. "Stereoisomer" means when a compound of the present invention contains one or more asymmetric centers and is thus available as racemates and racemic mixtures, single enantiomers, diastereomeric mixtures and single diastereomers. The compound of the present invention has an asymmetric center, and each asymmetric center can produce two optical isomers, and the scope of the present invention includes all possible optical isomers and diastereoisomer mixtures and pure or partially pure compounds . The compounds described herein may exist in tautomeric forms having different points of attachment of hydrogens by displacement of one or more double bonds. For example, a ketone and its enol form are keto-enol tautomers. Each tautomer and mixtures thereof are included in the compounds of the present invention. All enantiomers, diastereoisomers, racemates, mesoforms, cis-trans isomers, tautomers, geometric isomers, epimers of compounds of formula (I) to formula (III) and mixtures thereof are included in the scope of the present invention.

An "isotopic derivative" of the present invention refers to a molecule in which a compound is isotopically labeled. Isotopes commonl**y** used for isotopic labeling are: hydrogen isotopes, ²H and ³H; carbon isotopes: ¹¹C, ¹³C and ¹⁴C; chlorine isotopes: ³⁵Cl and ³¹Cl; fluorine isotopes: ¹⁸F; iodine isotopes: ¹²³I and ¹²⁵I; nitrogen isotopes: ¹³N and ¹⁵N ; Oxygen isotopes: ¹⁵O, ¹⁷O and ¹⁸O and sulfur isotope ³⁵S. These isotope-labeled compounds can be used to stud**y** the distribution of pharmaceutical molecules in tissues. Especiall**y** deuterium ³H and carbon ¹³C are more widel**y** used due to their easy labeling and convenient detection. Substitution of certain heav**y** isotopes, such as deuterium (²H), can enhance metabolic stabilit**y**, prolong half-life and thus achieve the purpose of reducing doses and provide therapeutic advantages. Isotopicall**y** labeled compounds are generall**y** s**y**nthesized starting from labeled starting materials and carried out in the same wa**y** as non-isotopicall**y** labeled compounds using known s**y**nthetic techniques.

The present invention also provides the use of the compound of the present invention in the preparation of medicaments for preventing and/or treating cancer, tumor, inflammatory disease, autoimmune disease or immune-mediated disease.

In addition, the present invention provides a pharmaceutical composition for preventing and/or treating cancer, tumor, inflammatory disease, autoimmune disease, neurodegenerative disease, attention-related disease or immune-mediated disease, which comprises the present invention compounds as active ingredients. The pharmaceutical composition may optionally comprise a pharmaceutically acceptable carrier.

In addition, the present invention provides a method of preventing and/or treating cancer, tumor, inflammatory disease, autoimmune disease, neurodegenerative disease, attention-related disease or immune-mediated disease, which comprises administering the compound of the invention to the mammals in need thereof.

Representative examples of inflammatory, autoimmune, and immune-mediated diseases may include, but are not limited to, arthritis, rheumatoid arthritis, spondyloarthritis, gouty arthritis, osteoarthritis, juvenile arthritis , Other Arthritis Conditions, Lupus, Systemic Lupus Erythematosus (SLE), Skin Related Disorders, Psoriasis, Eczema, Dermatitis, Atopic Dermatitis, Pain, Pulmonary Disease, Lung Inflammation, Adult Respiratory Distress Syndrome (ARDS) , pulmonary sarcoidosis, chronic pulmonary inflammatory disease, chronic obstructive pulmonary disease (COPD), cardiovascular disease, atherosclerosis, myocardial infarction, congestive heart failure, myocardial ischemia-reperfusion injury, inflammatory bowel disease, Crohn's disease, ulcerative colitis, irritable bowel syndrome, asthma, Sjogren's syndrome, autoimmune thyroid disease, urticaria (rubella), multiple sclerosis, scleroderma, organ transplant rejection, xenograft, idiopathic thrombocytopenic purpura (ITP), Parkinson's disease, Alzheimer's disease, diabetes-related diseases, inflammation, pelvic inflammatory disease, allergic rhinitis, allergic bronchitis, allergic sinusitis, leukemia, lymphoma, B Cell Lymphoma, T Cell Lymphoma, Myeloma, Acute Lymphocytic Leukemia (ALL), Chronic Lymphocytic Leukemia (CLL), Acute Myeloid Leukemia (AML), Chronic Myelogenous Leukemia (CML), Hairy Cell Leukemia, He Jie King's disease, non-Hodgkin's lymphoma, multiple myeloma, myelodysplastic syndrome (MDS), myeloproliferative neoplasm (MPN), diffuse large B-cell lymphoma, and follicular lymphoma.

Representative examples of cancer or tumor may include, but are not limited to, skin cancer, bladder cancer, ovarian cancer, breast cancer, stomach cancer, pancreatic cancer, prostate cancer, colon cancer, lung cancer, bone cancer, brain cancer, neuroblastoma, rectal cancer, colon cancer, familial adenomatous polyposis carcinoma, hereditary nonpolyposis colorectal cancer, esophagus cancer, lip cancer, larynx cancer, hypopharyngeal cancer, tongue cancer, salivary gland cancer, stomach cancer, adenocarcinoma, medullary thyroid cancer, Papillary thyroid cancer, renal cancer, renal parenchymal cancer, ovarian cancer, cervical cancer, uterine body cancer, endometrial cancer, choriocarcinoma, pancreatic cancer, prostate cancer, testicular cancer, urinary cancer, melanoma, brain tumors such as Glioblastoma, astrocytoma, meningioma, medulloblastoma and peripheral neuroectodermal tumor, Hodgkin lymphoma, non-Hodgkin lymphoma, Burkitt lymphoma, acute lymphoblastic leukemia (ALL), chronic lymphocytic leukemia (CLL), acute myeloid leukemia (AML), chronic myelogenous leukemia (CML), adult T-cell leukemia lymphoma, diffuse large B-cell lymphoma (DLBCL), hepatocellular carcinoma, gallbladder Carcinoma, bronchial carcinoma, small cell lung cancer, non-small cell lung cancer, multiple myeloma, basal cell tumor, teratoma, retinoblastoma, choroidal melanoma, seminoma, rhabdomyosarcoma, craniopharyngioma, Osteosarcoma, chondrosarcoma, sarcoma, liposarcoma, fibrosarcoma, Ewing sarcoma, or plasmacytoma.

When the compound of the present invention or a pharmaceutically acceptable salt thereof is administered in combination with another anticancer agent or immune checkpoint inhibitor for the treatment of cancer or tumors, the compound of the present invention or a pharmaceutically acceptable salt thereof can provide enhanced anticancer effects.

Representative examples of anticancer agents useful in the treatment of cancer or tumors may include, but are not limited to, cell signal transduction inhibitor, chlorambucil, melphalan, cyclophosphamide, ifosfamide, busulfan, carmustine, lomustine, streptozotocin, cisplatin, carboplatin, oxaliplatin, dacarbazine, temozolomide, procarbazine, methotrexate, fluorouracil, cytarabine, gemcitabine, Mercaptopurine, fludarabine, vinblastine, vincristine, vinorelbine, paclitaxel, docetaxel, topotecan, irinotecan, etoposide, trabectedin, dactinomycin, doxorubicin , epirubicin, daunorubicin, mitoxantrone, bleomycin, mitomycin C, ixabepilone, tamoxifen, flutamide, gonadorelin analogs, methadone progesterone, prednisone, dexamethasone, methylprednisolone, thalidomide, interferon alpha, leucovorin, sirolimus, sirolimus ester, everolimus, afatinib, alisertib, amuvatinib, apatinib, axitinib, bortezomib, bosutinib, britinib, cabozantinib, cediranib, crenolanib, kezhuotinib, dabrafenib, dabrafenib, Cotinib, danucitinib, dasatinib, dovitinib, erlotinib, foretinib, ganetespib, gefitinib, ibrutinib, icotinib, imatinib, iniparib, la Patinib, lenvatinib, linifanib, linsitinib, masitinib, momelotinib, motisanib, neratinib, nilotinib, niraparib, oprozomib, olaparib, pazopanib, pictilisib, ponatinib, quizartinib, regorafenib, rigosertib, rucaparib, ruxolitinib, saracatinib, saridegib, sorafenib, sunitinib, tiratinib, tivantinib, tivozanib, tofacitinib, trametinib, vandetanib, veliparib, vemurafenib, vimodegib, volasertib, alemtuzumab, bevacizumab, berentuzumab vedotin, catumaxumab Antibodies, cetuximab, denosumab, gemtuzumab, ipilimumab, nimotuzumab, ofatumumab, panitumumab, rituximab, tositumomab, trastuzumab, PI3K inhibitor, CSF1R inhibitor, A2A and/or A2B receptor antagonist, IDO inhibitor, anti-PD-1 antibody, anti-PD-L1 antibody, LAG3 antibody, TIM-3 antibody and an anti-CTLA-4 antibody, or any combination thereof.

When administered in combination with other therapeutic agents for the treatment of inflammatory disease, autoimmune disease and immune-mediated disease, the compounds of the present invention, or pharmaceutically acceptable salts thereof provide enhanced therapeutic effect.

Representative examples of therapeutic agents useful in the treatment of inflammatory, autoimmune, and immune-mediated diseases can include, but are not limited to, steroidal agents (e.g., prednisone, **prednisone, prednisone,** methylphenidate, cortisone, hydroxycortisone, betamethasone, dexamethasone, etc.), methotrexate, leflunomide, anti-TNFα agents (eg, etanercept, infliximab, adalib monoclonal antibody, etc.), calcineurin inhibitors (e.g., tacrolimus, pimecrolimus, etc.), and antihistamines (e.g., diphenhydramine, hydroxyzine, loratadine, ebastine, ketotifen, cetirizine, levocetirizine, fexofenadine, etc.), and at least one or more therapeutic agents selected from them can be included in the pharmaceutical composition of the present invention.

In addition, the present invention also provides a method for preventing and/or treating tumors, cancers, viral infections, organ transplant rejection, neurodegenerative diseases, attention-related diseases or autoimmune diseases, which comprises administering the compound of the invention or the pharmaceutical composition of the invention to a mammal in need thereof.

The pharmaceutical composition of the present invention can be formulated into dosage forms for oral administration or parenteral administration (including intramuscular, intravenous and subcutaneous routes, intratumoral injection) according to any of the conventional methods, such as tablets, granules, powders, capsules, syrups, emulsions, microemulsions, solutions or suspensions.

Pharmaceutical compositions of the present invention for oral administration can be prepared by mixing the active ingredient with carriers such as cellulose, calcium silicate, corn starch, lactose, sucrose, dextrose, calcium phosphate, stearic acid, stearic acid, magnesium stearate, calcium stearate, gelatin, talc, surfactant, suspending agent, emulsifier and diluent.

Examples of carriers employed in the pharmaceutical composition for injection administration of the present invention can be water, saline solution, glucose solution, glucose-like solution (glucose-like solution), alcohol, glycol, ether (for example, polyethylene glycol 400), oils, fatty acids, fatty acid esters, glycerides, surfactants, suspending agents and emulsifiers.

Other features of the invention will become apparent in the course of the description of exemplary embodiments of the invention which are given to illustrate the invention and are not intended to be limiting thereof. In the following examples the methods disclosed in the invention were used for preparation, separation and characterization.

The compounds of the present invention can be prepared in a variety of methods known to those skilled in the art of organic synthesis, the methods described below as well as synthetic methods known in the art of synthetic organic chemistry or by variations thereof known to those skilled in the art may be used for the synthesis of the compounds of the present invention. Preferred methods include, but are not limited to, those described below. Reactions are performed in solvents or solvent mixtures appropriate to the kit materials used and to the transformations effected. Those skilled in the art of organic synthesis will appreciate that the functionality present on the molecule is consistent with the proposed transitions. This sometimes requires judgment to alter the order of synthetic steps or starting materials to obtain the desired compound of the invention.

### Embodiments

### Terms

Unless otherwise stated, the terms used in the present application, including the specification and claims, are defined as follows. If not stated otherwise, conventional methods of mass spectrometry, NMR, HPLC, protein chemistry, biochemistry, recombinant DNA techniques and pharmacology are used. In this application, the use of "or" or "and" means "and/or" if not stated otherwise.

In the specification and claims, a given chemical formula or name shall encompass all stereoisomers and optical isomers thereof and racemates in which such isomers exist. Unless otherwise indicated, all chiral (enantiomers and diastereoisomers) and racemic forms are within the scope of the invention. Various geometric isomers of C=C double bonds, C=N double bonds, ring systems, etc. may also exist in the compounds, and all such stable isomers are encompassed by the present invention. The present invention describes cis- and trans- (or E- and Z-) geometric isomers of the compounds of the invention and which may be isolated as a mixture of isomers or as separated isomeric forms. The compounds of the invention may be isolated in optically active or racemic forms. All methods used to prepare the compounds of the invention and intermediates prepared therein are considered part of the invention. When preparing enantiomeric or diastereomeric products, they may be separated by customary methods, for example by chromatography or fractional crystallization. Depending on the process conditions, the end products of the invention are obtained in free (neutral) or salt form. The free forms and salts of these end products are within the scope of the present invention. A compound can be converted from one form to another, if desired. A free base or acid can be converted into a salt; a salt can be converted into the free compound or another salt; a mixture of isomeric compounds of the invention can be separated into the individual isomers. The compounds of the invention, their free forms and salts, may exist in various tautomeric forms in which the hydrogen atoms are transposed to other parts of the molecule and thus the chemical bonds between the atoms of the molecule are rearranged. It is to be understood that all tautomeric forms which may exist are included within the present invention.

Unless otherwise defined, the definitions of the substituents in the present invention are independent and not interrelated, for example (listed but not exhaustive), in one aspect, for the substituents Ra (or Ra') in different definitions of the substituents, the definitions are independent of each other. Specifically, when one definition is selected for R^{a} (or R^{a}') in one substituent, it does not mean that R^{a} (or R^{a}') has the same definition in other substituents. More specifically, for example (but not exhaustively) for NR^{a}R^{a}', when R^{a} (or R^{a}') is defined from hydrogen, it does not mean that in -C(O)-NR^{a}R^{a}', R^{a} (or R^{a}') must be hydrogen. In another aspect, when there is more than one R^{a} (or R^{a}') in a certain substituent, these R^{a} (or R^{a}') are also independent. For example, in the substituent -(CR^{a}R^{a}')m-O-(CR^{a}R^{a}')n-, when m+n is greater than or equal to 2, the m+n pieces of R^{a} (or R^{a}') are independent, and they can be have the same or different meanings.

Unless otherwise defined, when a substituent is noted as "optionally substituted", the substituent is selected from, for example, the following substituents, such as alkyl, cycloalkyl, aryl, heterocyclyl, halogen, hydroxy, alkoxy, oxo, alkanoyl, aryloxy, alkanoyloxy, amino, alkylamino, arylamino, arylalkylamino, disubstituted amino (wherein 2 amino substituents are selected from alkyl radical, aryl or arylalkyl), alkanoylamino, aroylamino, aralkanoylamino, substituted alkanoylamino, substituted arylamino, substituted aralkanoylamino, thio, alkylthio, arylthio, arylalkylthio, arylthiocarbonyl, arylalkylthiocarbonyl, alkylsulfonyl, arylsulfonyl, arylalkylsulfonyl, aminosulfonyl such as -SO2NH2, substituted sulfonylamino, nitro, cyano, carboxyl, carbamoyl such as -CONH2, substituted carbamoyl such as -CONHalkyl, -CONHaryl, -CONHarylalkyl or two optional In the case of substituents from alkyl, aryl or arylalkyl, alkoxycarbonyl, aryl, substituted aryl, guanidino, heterocyclic, e.g. indolyl, imidazolyl, furyl, thienyl , Thiazolyl, pyrrolidinyl, pyridyl, pyrimidinyl, pyrrolidinyl, piperidinyl, morpholinyl, piperazinyl, homopiperazinyl, etc. and substituted heterocyclyl.

The term "alkyl" or "alkylene" as used herein is intended to include both branched and straight chain saturated aliphatic hydrocarbon groups having the indicated number of carbon atoms. For example, "C₁-C₆ alkyl" means an alkyl group having 1 to 6 carbon atoms. Examples of alkyl groups include, but are not limited to, methyl (Me), ethyl (Et), propyl (such as n-propyl and isopropyl), butyl (such as n-butyl, isobutyl, t-butyl), and pentyl (such as n-pentyl, isopentyl, neopentyl). Herein, the alkyl group is preferably an alkyl group having 1 to 6, more preferably 1 to 4 carbon atoms.

The term "alkenyl" denotes a straight or branched chain hydrocarbon group containing one or more double bonds and generally having a length of 2 to 20 carbon atoms. For example, "C₂-C₆ alkenyl" contains two to six carbon atoms. Alkenyl groups include, but are not limited to, e.g. vinyl, propenyl, butenyl, 1-methyl-2-buten-1-yl, and the like. Herein, alkenyl is preferably C₂-C₆ alkenyl.

The term "alkynyl" denotes a straight or branched chain hydrocarbon group containing one or more triple bonds and generally having a length of 2 to 20 carbon atoms. For example, "C₂-C₆ alkynyl" contains two to six carbon atoms. Representative alkynyl groups include, but are not limited to, for example, ethynyl, 1-propynyl, 1-butynyl, and the like. Herein, the alkynyl group is preferably a C₂-C₆ alkynyl group.

The term "alkoxy" or "alkyloxy" refers to -O-alkyl. "C₁-C₆ alkoxy" (or alkyloxy) is intended to include C₁, C₂, C₃, C₄, C₅, C₆ alkoxy. Examples of alkoxy include, but are not limited to, methoxy, ethoxy, propoxy (e.g., n-propoxy and isopropoxy), and tert-butoxy. Herein, the alkoxy group is preferably an alkoxy group having 1 to 6, more preferably 1 to 4 carbon atoms. Similarly, "alkylthio" or "thiothio" denotes an alkyl group as defined above having the indicated number of carbon atoms attached through a sulfur bridge; e.g. methyl-S- and ethyl-S-.

The term "carbonyl" refers to an organic functional group (C=O) consisting of two atoms, carbon and oxygen, linked by a double bond.

The term "aryl", alone or as part of a larger moiety such as "aralkyl", "arylalkoxy" or "aryloxyalkyl", refers to a monocyclic, bicyclic or tricyclic ring system having a total of 5 to 12 ring members, wherein at least one ring in the system is aromatic and wherein each ring in the system contains 3 to 7 ring members. In certain embodiments of the invention, "aryl" refers to an aromatic ring system which includes, but is not limited to, phenyl, biphenyl, indanyl, 1-naphthyl, 2-naphthyl, and tetrahydronaphthyl. The term "aralkyl" or "arylalkyl" refers to an alkyl residue attached to an aryl ring, non-limiting examples of which include benzyl, phenethyl, and the like. A fused aryl group can be attached to another group at a suitable position on the cycloalkyl ring or aromatic ring. Dashed lines drawn from ring systems indicate that bonds may be attached to any suitable ring atom.

The term "cycloalkyl" refers to monocyclic, bicyclic or branched cyclic alkyl groups. Monocyclic cycloalkyl refers to C₃-C₁₂ cycloalkyl, including but not limited to cyclopropyl, cyclobutyl, cyclopentyl, cyclohexyl and norbornyl. Branched cycloalkyl groups such as 1-methylcyclopropyl and 2-methylcyclopropyl are included within the definition of "cycloalkyl". Bicyclic cycloalkyl groups include bridged, spiro or fused cycloalkyl groups. Herein, the cycloalkyl group is preferably a C₃-C₁₂ cycloalkyl group, more preferably a C₃-C₈ cycloalkyl group.

Similarly, the term "heterocycloalkyl" refers to a 3-12 membered ring structure in which at least one carbon atom on a cycloalkyl group is replaced by a heteroatom selected from N, O, S and P, which may be a monocyclic , bicyclic or branched ring structures. Herein, the heterocycloalkyl group is preferably a 3-12 membered heterocycloalkyl group, more preferably a 3-8 membered heterocycloalkyl group.

The term "bridged cycloalkyl" as used herein refers to polycyclic compounds sharing two or more carbon atoms. "Bbridged cycloalkyl" can be divided into bicyclic bridged ring hydrocarbons and polycyclic bridged ring hydrocarbons. The former is composed of two alicyclic rings sharing more than two carbon atoms; the latter is a bridged ring hydrocarbon composed of more than three rings.

The term "spirocycloalkyl" as used herein refers to polycyclic hydrocarbons in which the monocyclic rings share one carbon atom (called the spiro atom).

The term "bridged heterocycle" as used herein refers to a polycyclic compound sharing two or more carbon atoms, and the ring contains at least one heteroatom selected from O, N and S atoms. It can be divided into bicyclic bridged heterocycles and polycyclic bridged heterocycles.

The term "heterospirocyclyl" as used herein refers to a polycyclic hydrocarbon that shares one carbon atom (called a spiro atom) between monocyclic rings, and the ring contains at least one heteroatom selected from O, N and S atoms.

The term "substituted" as used herein means that at least one hydrogen atom is replaced by a non-hydrogen group, provided that normal valences are maintained and that the substitution results in a stable compound. A cyclic double bond, as used herein, is a double bond formed between two adjacent ring atoms (e.g., C=C, C=N or N=N).

The term "cycloalkenyl" refers to a monocyclic or bicyclic cyclic alkenyl group. Monocyclic cycloalkenyl refers to C₃-C₈ cyclic alkenyl, including but not limited to cyclopropenyl, cyclobutenyl, cyclopentenyl, cyclohexenyl and norbornenyl. Branched cycloalkenyl groups such as 1-methylcyclopropenyl and 2-methylcyclopropenyl are included within the definition of "cycloalkenyl". Bicyclic cycloalkenyl groups include bridged, spiro, or fused cycloalkenyl groups.

"Halo" or "halogen" includes fluoro, chloro, bromo and iodo. "Haloalkyl"/"haloalkylene" is intended to include branched and straight chain saturated alkyl/alkylene groups having the indicated number of carbon atoms substituted with one or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoro propyl and heptachloropropyl. Examples of haloalkyl also include "fluoroalkyl" intended to include branched and straight-chain saturated aliphatic hydrocarbon groups having the specified number of carbon atoms and substituted with 1 or more fluorine atoms. "Halocycloalkyl"/"haloheterocycloalkyl" is intended to include cycloalkyl/heterocycloalkyl having the indicated number of carbon atoms substituted with one or more halogens. In the present invention, the halogen atom is preferably fluorine or chlorine, more preferably fluorine.

"Haloalkoxy" or "haloalkyloxy" means a haloalkyl group as defined above having the indicated number of carbon atoms attached through an oxygen bridge. For example, "haloC₁-C₆ alkoxy" is intended to include C₁, C₂, C₃, C₄, C₅, C₆ haloalkoxy. Examples of haloalkoxy include, but are not limited to, trifluoromethoxy, 2,2,2-trifluoroethoxy, and pentafluoroethoxy. Similarly, "haloalkylthio" or "thiohaloalkoxy" denotes a haloalkyl group as defined above having the indicated number of carbon atoms attached through a sulfur bridge; for example trifluoromethyl-S- and pentafluoroethyl -S-.

In the present disclosure, the expression Cₓ₁-Cₓ₂ is used when referring to some substituent groups, which means that the number of carbon atoms in the substituent groups may be x1 to x2. For example, C₀-C₈ means that the group contains 0, 1, 2, 3, 4, 5, 6, 7 or 8 carbon atoms, C₁-C₈ means that the group contains 1, 2, 3, 4, 5 , 6, 7 or 8 carbon atoms, C₂-C₈ means that the group contains 2, 3, 4, 5, 6, 7 or 8 carbon atoms, C₃-C₈ means that the group contains 3, 4, 5 , 6, 7 or 8 carbon atoms, C₄-C₈ means that the group contains 4, 5, 6, 7 or 8 carbon atoms, C₀-C₆ means that the group contains 0, 1, 2, 3, 4 , 5 or 6 carbon atoms, C₁-C₆ means that the group contains 1, 2, 3, 4, 5 or 6 carbon atoms, C₂-C₆ means that the group contains 2, 3, 4, 5 or 6 carbon atoms, C₃-C₆ means that the group contains 3, 4, 5 or 6 carbon atoms.

In this disclosure, the expression "x1-x2 membered ring" is used when referring to cyclic groups such as aryl, heteroaryl, cycloalkyl, and heterocycloalkyl, which means that the number of the ring atoms of the group can be x1 to x2. For example, the 3-12 membered cyclic group may be a 3, 4, 5, 6, 7, 8, 9, 10, 11 or 12 membered ring, and the number of ring atoms may be 3, 4, 5, 6, 7, 8, 9, 10, 11 or 12; 3-6-membered ring means that the cyclic group can be 3, 4, 5 or 6-membered ring, and the number of ring atoms can be 3, 4, 5 or 6 ; 3-8 membered ring means that the cyclic group can be 3, 4, 5, 6, 7 or 8-membered ring, and the number of ring atoms can be 3, 4, 5, 6, 7 or 8; 3-9 membered ring means that the cyclic group can be a 3, 4, 5, 6, 7, 8 or 9-membered ring, and the number of ring atoms can be 3, 4, 5, 6, 7, 8 or 9; 4-7 membered ring means that the cyclic group can be a 4, 5, 6 or 7-membered ring, and the number of ring atoms can be 4, 5, 6 or 7; 5-8-membered ring means that the cyclic group can be 5, 6, 7 or 8-membered ring, the number of ring atoms can be 5, 6, 7 or 8; 5-12 membered ring means that the ring group can be 5, 6, 7, 8, 9, 10, 11 or 12-membered ring, the number of ring atoms can be 5, 6, 7, 8, 9, 10, 11 or 12; 6-12 membered ring means that the ring group can be 6, 7, 8, 9, 10, 11- or 12-membered rings, the number of ring atoms may be 6, 7, 8, 9, 10, 11 or 12. The ring atoms may be carbon atoms or heteroatoms, for example selected from N, O and S. When the ring is heterocyclic, the heterocyclic ring may contain 1, 2, 3, 4, 5, 6, 7, 8, 9, 10 or more ring heteroatoms, for example selected from N, O and S of heteroatoms.

In the present disclosure, the one or more halogens may each be independently selected from fluorine, chlorine, bromine and iodine.

The term "heteroaryl" means a stable 5-membered, 6-membered, or 7-membered aromatic monocyclic ring or aromatic bicyclic ring or a 7-membered, 8-membered, 9-membered, 10-membered, 11-membered, 12-membered aromatic polycyclic heterocycle, which is fully unsaturated or partially unsaturated, and contains carbon atoms and 1, 2, 3 or 4 heteroatoms independently selected from N, O and S; and includes cycloalkane or heterocycloalkane with a structure in which aromatic rings such as benzene rings or heteroaromatic rings such as pyridine are condensed. The position of the structure as a substituent may be located on a cycloalkane, a heterocycloalkane, an aromatic ring or a heteroaryl ring. Nitrogen and sulfur heteroatoms can be optionally oxidized. The nitrogen atom is substituted or unsubstituted (i.e. N or NR, where R is H or another substituent if defined). A heterocycle can be attached to its pendant group at any heteroatom or carbon atom that results in a stable structure. The heterocyclyl groups described herein may be substituted on carbon or nitrogen atoms if the resulting compound is stable. The nitrogen in the heterocycle can optionally be quaternized. Preferably, when the total number of S and O atoms in the heterocycle exceeds 1, then these heteroatoms are not adjacent to each other. Preferably, the total number of S and O atoms in the heterocycle is not greater than one. When the term "heterocycle" is used, it is intended to include heteroaryl. Examples of heteroaryl groups include, but are not limited to, acridinyl, azetidinyl, aziocinyl, benzimidazolyl, benzofuryl, benzothiofuranyl, benzothienyl, benzooxa azolyl, benzoxazolyl, benzothiazolyl, benzotriazolyl, benzotetrazolyl, benzisoxazolyl, benzisothiazolyl, benzimidazolyl, carbazolyl, 4aH-carbazolyl, carbolinyl, chromanyl, chromenyl, cinnolinyl, decahydroquinolyl, 2H,6H-1,5,2-dithiazinyl, dihydrofuro[2, 3-b] tetrahydrofuryl, furyl, furanyl, imidazolidinyl, imidazolinyl, imidazolyl, 1H-indazolyl, imidazopyridyl, indolenyl, indolinyl, Indolazinyl, indolyl, 3H-indolyl, isatinoyl, isobenzofuryl, isochromanyl, isoindazolyl, isoindolinyl, isoindolyl, isoindolyl quinolinyl, isothiazolyl, isothiazolopyridyl, isoxazolyl, isoxazolopyridyl, methylenedioxyphenyl, morpholinyl, diazanaphthyl, octahydroisoquinoline oxadiazolyl, 1,2,3-oxadiazolyl, 1,2,4-oxadiazolyl, 1,2,5-oxadiazolyl, 1,3,4-oxadiazolyl , oxazolidinyl, oxazolyl, oxazolopyridyl, oxazolidinyl, diazaphenyl, oxindolyl, pyrimidinyl, phenanthridinyl, phenanthrolinyl, phenazinyl , phenothiazinyl, phenoxathiyl, phenoxazinyl, phthalazinyl, piperazinyl, piperidinyl, piperidinyl, 4-piperidinyl, piperonyl, pteridinyl, purinyl, pyranyl, pyrazinyl, pyrazolidinyl, pyrazolinyl, pyrazolopyridyl, pyrazolyl, pyridazinyl, pyridoxazolyl, pyridimidazolyl, pyridothiazolyl, pyridyl , pyrimidinyl, pyrrolidinyl, pyrrolinyl, 2-pyrrolidonyl, 2H-pyrrolyl, pyrrolyl, quinazolinyl, quinolinyl, 4H-quinazinyl, quinoxalinyl, quinuclidinyl, tetrazolyl, tetrahydrofuryl, tetrahydroisoquinolyl, tetrahydroquinolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthryl, thiazolyl, thienyl, thiazolopyridyl, thienothiazolyl, thienooxa Azolyl, thienoimidazolyl, thienyl, triazinyl, 1,2,3-triazolyl, 1,2,4-triazolyl, 1,2,5-triazolyl, 1,3,4-triazolyl and xanthenyl, quinolinyl, isoquinolyl, phthalazinyl, quinazolinyl, indolyl, isoindolyl, indolinyl, 1H-indazolyl, benzo Imidazolyl, 1,2,3,4-tetrahydroquinolinyl, 1,2,3,4-tetrahydroisoquinolinyl, 5,6,7,8-tetrahydro-quinolinyl, 2,3 -Dihydro-benzofuryl, chromanyl, 1,2,3,4-tetrahydro-quinoxalinyl and 1,2,3,4-tetrahydro-quinazolinyl. The term "heteroaryl" may also include biaryl structures formed by the above-defined "aryl" and a monocyclic "heteroaryl", such as but not limited to "-phenyl bipyridyl-", "- "phenyl bipyrimidyl", "-pyridyl biphenyl", "-pyridyl bipyrimidyl-", "-pyrimidyl biphenyl-"; wherein the present invention also includes condensed ring and spiro compound containing, for example, the above-mentioned heterocycles.

Where nitrogen atoms (e.g. amines) are present on compounds of the invention, these nitrogen atoms can be converted to N-oxides by treatment with oxidizing agents (e.g. mCPBA and/or hydrogen peroxide) to obtain other compounds of the invention. Accordingly, both shown and claimed nitrogen atoms are considered to cover both the shown nitrogen and its N-oxides to obtain the derivatives of the present invention.

When any variable occurs more than one time in any composition or formula of a compound, its definition on each occurrence is independent of its definition at every other occurrence. Thus, for example, if a group is shown to be substituted with 0-3 R, then said group may be optionally substituted with up to three R groups, and R at each occurrence is independently selected from the definition of R. Also, combinations of substituents and/or variables are permissible only if such combinations result in stable compounds.

The term "patient" as used herein refers to an organism being treated by the methods of the present invention. Such organisms preferably include, but are not limited to, mammals (e.g., murine, ape, monkey, equine, bovine, porcine, canine, feline, etc.) and most preferably refer to humans.

The term "effective amount" as used herein means an amount of a drug or agent (i.e., a compound of the invention) that will elicit a biological or medical response in a tissue, system, animal or human being sought, e.g., by a researcher or clinician. Furthermore, the term "therapeutically effective amount" means an amount which results in improved treatment, cure, prevention or alleviation of a disease, disorder or side effect, or a reduction in the rate of disease or disorder progression. An effective amount may be given in one or more administrations, applications or doses and is not intended to be limited by a particular formulation or route of administration. The term also includes within its scope amounts effective to enhance normal physiological function.

As used herein, the term "treating" includes any effect that results in amelioration of a condition, disease, disorder, etc., such as alleviation, reduction, regulation, amelioration or elimination, or amelioration of the symptoms thereof.

The term "pharmaceutically acceptable" is used herein to refer to those compounds, substances, compositions and/or dosage forms: within the scope of sound medical judgment, they are suitable for use in contact with human and animal tissues without excessive toxicity, irritation, allergic reactions, and/or other problems or complications, commensurate with a reasonable benefit/risk ratio.

The phrase "pharmaceutically acceptable carrier" as used herein means a pharmaceutical substance, composition or vehicle, such as a liquid or solid filler, diluent, excipient, manufacturing aid (e.g. lubricant, talc, magnesium stearate, calcium stearate, or zinc stearate, or stearic acid) or solvent-encapsulated substances involved in the carrying or transport of a subject compound from one organ or body part to another. Each carrier must be "acceptable" in the sense of being compatible with the other ingredients of the formulation and not injurious to the patient.

The term "pharmaceutical composition" means a composition comprising a compound of the present invention together with at least one other pharmaceutically acceptable carrier. "Pharmaceutically acceptable carrier" means a medium generally accepted in the art for delivering a biologically active agent to an animal, particularly a mammal, including (i.e.) an adjuvant, excipient or vehicle, such as a diluent , preservatives, fillers, flow regulators, disintegrants, wetting agents, emulsifiers, suspending agents, sweeteners, flavoring agents, fragrances, antibacterial agents, antifungal agents, lubricants and dispersants, which depends on the mode of administration and the nature of the dosage form.

### Certain pharmaceutical and medical terms

The term "acceptable", as used herein, means that a formulation ingredient or active ingredient does not have an undue adverse effect on health for the general purpose of treatment.

The term "cancer", as used herein, refers to an abnormal growth of cells that cannot be controlled and, under certain conditions, is capable of metastasizing (spreading). Cancers of this type include, but are not limited to, solid tumors (e.g., bladder, bowel, brain, chest, uterus, heart, kidney, lung, lymphoid tissue (lymphoma), ovary, pancreas or other endocrine organs (e.g., thyroid), prostate , skin (melanoma), or blood cancer (such as non-leukemic leukemia).

The term "administration in combination" or similar terms, as used herein, refers to the administration of several selected therapeutic agents to a patient, in the same or different modes of administration at the same or different times.

The term "enhancing" or "capable of enhancing", as used herein, means that a desired result is capable of being increased or prolonged, either in potency or duration. Thus, in relation to enhancing the therapeutic effect of a drug, the term "capable of potentiating" refers to the ability of the drug to increase or prolong its potency or duration in the system. As used herein, "potency value" refers to the ability to maximize the enhancement of another therapeutic drug in an ideal system.

The term "immune disease" refers to a disease or condition of an adverse or deleterious reaction to an endogenous or exogenous antigen. The result is usually dysfunction of the cells, or destruction thereof and dysfunction, or destruction of organs or tissues that may produce immune symptoms.

The terms "kit" and "product packaging" are synonymous.

The term "subject" or "patient" includes mammals and non-mammals. Mammals include, but are not limited to, mammals: humans, non-human primates such as orangutans, apes, and monkeys; agricultural animals such as cattle, horses, goats, sheep, and pigs; domestic animals such as rabbits and dogs; experimental animals include rodents, such as rats, mice and guinea pigs. Non-mammalian animals include, but are not limited to, birds, fish, and the like. In a preferred aspect, the selected mammal is a human.

The term "treatment", "course of treatment" or "therapy" as used herein includes alleviating, suppressing or ameliorating the symptoms or conditions of a disease; inhibiting the development of complications; ameliorating or preventing the underlying metabolic syndrome; inhibiting the development of diseases or symptoms, such as controlling the development of a disease or condition; alleviating a disease or a symptom; causing a disease or a symptom to regress; alleviating a complication caused by a disease or a symptom, or preventing and/or treating a sign caused by a disease or a symptom.

As used herein, a certain compound or pharmaceutical composition, after administration, can improve a certain disease, symptom or condition, especially improve its severity, delay the onset, slow down the progression of the disease, or reduce the duration of the disease. Circumstances that may be attributable to or related to the administration, whether fixed or episodic, continuous or intermittent.

Suitable routes of administration include, but are not limited to, oral, intravenous, rectal, aerosol, parenteral, ocular, pulmonary, transdermal, vaginal, ear canal , nasal administration and topical administration. In addition, by way of example only, parenteral administration includes intramuscular injection, subcutaneous injection, intravenous injection, intramedullary injection, intraventricular injection, intraperitoneal injection, intralymphatic injection, and intranasal injection.

In one aspect, the compounds described herein are administered locally rather than systemically. In certain embodiments, the depot formulation is administered by implantation (e.g., subcutaneously or intramuscularly) or by intramuscular injection. Furthermore, in another specific embodiment, the drug is administered via a targeted drug delivery system. For example, liposomes coated with organ-specific antibodies. In such embodiments, the liposomes are selectively directed to and taken up by specific organs.

### Pharmaceutical Composition and Dosage

The present invention also provides pharmaceutical compositions comprising a therapeutically effective amount of one or more compounds of the present invention formulated together with one or more pharmaceutically acceptable carriers (additives) and/or diluents, and optionally one or more of the other therapeutic agents described above. The compounds of the present invention may be administered for any of the above uses by any suitable means, for example orally, such as tablets, pills, powders, granules, elixirs, tinctures, suspensions (including nanosuspensions, microsuspensions, spray-dried dispersions), syrups and emulsions; sublingually; buccally; parenterally, such as by subcutaneous, intravenous, intramuscular or intrasternal injection or infusion techniques (e.g., as sterile injectable aqueous or nonaqueous solutions or suspensions liquid form); nasally, including to the nasal membranes, such as by inhalation spray; topically, such as in the form of a cream or ointment; or rectally, such as in the form of a suppository; or by intratumoral injection. They can be administered alone, but generally will be administered using a pharmaceutical carrier selected on the basis of the chosen route of administration and standard pharmaceutical practice.

Pharmaceutical carriers include aqueous and non-aqueous liquid media and various solid and semisolid pharmaceutical carriers. Such carriers may include many different ingredients and additives besides the active agent, which are included in the formulation for various reasons known to those skilled in the art, such as stabilizers, binders, and the like. A description of suitable pharmaceutical carriers and the factors involved in the selection of the carrier can be found in several readily available sources, such as Allen L.VJr. et al. Remington: The Science and Practice of PharmaCy1 (2 Volumes), 22nd Edition (2012), Pharmaceutical Press.

Dosage regimens for the compounds of the present invention will of course vary depending on known factors such as the pharmacodynamic properties of the particular agent and its mode and route of administration; the species, age, sex, health, medical condition and weight of the recipient; nature and extent of symptoms; type of concomitant therapy; frequency of therapy; route of administration, patient's renal and hepatic function, and desired effects. As a general guide, the daily oral dosage of each active ingredient should be from about 0.001 mg/day to about 10-5000 mg/day, preferably from about 0.01 mg/day to about 1000 mg/day, and most preferably From about 0.1 mg/day to about 250 mg/day. The most preferred dose intravenously will be about 0.01 mg/kg/minute to about 10 mg/kg/minute during a constant rate infusion. The compounds of the present invention may be administered in a single daily dose, or the total daily dose may be administered in divided doses of two, three or four times daily.

The compounds are usually formulated with a suitable pharmaceutical diluent, excipient or carrier (herein collectively referred to as drug carriers) in the form of mixtures for administration.

Dosage forms (pharmaceutical compositions) suitable for administration may contain from about 1 milligram to about 2000 milligrams of active ingredient per dosage unit. In these pharmaceutical compositions, the active ingredient will generally be present in an amount of about 0.1-95% by weight, based on the total weight of the composition.

Included within the scope of the present invention are pharmaceutical compositions comprising (alone or in combination with a pharmaceutical carrier) a therapeutically effective amount of at least one compound of the present invention as an active ingredient. Optionally, compounds of the invention may be used alone, in combination with other compounds of the invention, or in combination with one or more other therapeutic agents (e.g., anticancer agents or other pharmaceutically active substances).

Irrespective of the chosen route of administration, the compounds of the invention (which may be used in suitably hydrated form) and/or the pharmaceutical compositions of the invention are formulated into pharmaceutical dosage forms by conventional methods known to those skilled in the art.

The actual dosage levels of the active ingredients in the pharmaceutical compositions of the present invention can be varied to obtain an amount of the active ingredient that is effective in achieving the desired therapeutic response, composition, and mode of administration for a particular patient without being toxic to the patient.

The selected dosage level will depend on a variety of factors, including the activity of the particular compound of the invention employed, or its ester, salt or amide; the route of administration; the time of administration; the rate of excretion of the particular compound employed; and the rate and extent of absorption; duration of treatment; other drugs, compounds and/or substances used in combination with the particular compound used; factors well known in the medical art such as age, sex, weight, condition, general health and prior medical history of the patient being treated.

A physician or veterinarian having ordinary skill in the art can determine and prescribe the effective amount of the pharmaceutical composition required. In general, a suitable daily dose of a compound of the invention will be that amount of the compound at the lowest dose effective to produce a therapeutic effect. Such effective dosage will generally depend on the factors mentioned above. Typically, oral, intravenous, intracerebroventricular and subcutaneous doses of the compounds of this invention to patients range from about 0.01 to about 50 mg/kg body weight/day. If desired, an effective daily dose of the active compound may be administered in two, three, four, five, six or more sub-doses at appropriate intervals throughout the day, optionally in unit dosage form. In certain aspects of the invention, the administration is once daily.

Although the compounds of the present invention may be administered alone, it is preferred to administer the compounds in the form of pharmaceutical formulations (compositions).

### Kit/Product Packaging

Kits/product packaging are also described herein for use in the treatment of the above indications. These kits may consist of transporters, packs, or container boxes, which may be divided into compartments to accommodate one or more types of containers, such as vials, test tubes, and the like, with each container containing a single component in the method described above. Suitable containers include bottles, vials, syringes, test tubes and the like. Containers are made of acceptable materials such as glass or plastic.

For example, a container may contain one or more compounds described herein, either as a pharmaceutical compound or in admixture with other ingredients described herein. The container can have a sterile outlet (e.g., the container can be an IV bag or bottle, the stopper of which can be pierced by a hypodermic needle). Such a kit may contain a compound, together with instructions for use, labels or instructions for the methods described herein.

A typical kit may include one or more containers, each containing one or more materials (such as reagents, concentrated stock solutions, and/or or equipment). These materials include, but are not limited to, buffers, diluents, filters, needles, syringes, transporters, bags, containers, bottles and/or test tubes, accompanied by a list of contents and/or instructions for use, and inner packaging also accompanied by instructions. Instructions for the entire set are to be included.

Labels can be displayed on or closely associated with the container. The appearance of the label on the container means that the label letters, numbers or other features are pasted, molded, or engraved on the container; the label can also appear in the container box or shipping box containing various containers, such as in the product insert. A label may be used to indicate a specific therapeutic use of the contents. The label may also bear instructions for use of the contents, such as described in the methods above.

All features described in this specification (including any stated claims, abstract and drawings), and/or all steps involved in any method or process, may exist in any combination, unless certain features or steps are mutually exclusive in the same combination.

The above-mentioned features mentioned in the present invention, or the features mentioned in the embodiments can be combined arbitrarily. All the features disclosed in the specification of this case can be used in combination with any combination, and each feature disclosed in the specification can be replaced by any alternative feature that can provide the same, equivalent or similar purpose. Therefore, unless otherwise stated, the disclosed features are only general examples of equivalent or similar features.

Below in conjunction with specific embodiment, further illustrate the present invention. It should be understood that these examples are only used to illustrate the present invention and are not intended to limit the scope of the present invention. For the experimental methods without specific conditions indicated in the following examples, usually follow the conventional conditions or the conditions suggested by the manufacturer. All percentages, ratios, **ratios,** or parts are by weight unless otherwise indicated.

The unit of weight volume percentage in the present invention is well known to those skilled in the art, for example, it refers to the weight (g) of solute in 100 ml of solution. Unless otherwise defined, all professional and scientific terms used herein have the same meanings as are familiar to those skilled in the art. In addition, any methods and materials similar or equivalent to those described can be applied to the method of the present invention. The preferred implementation methods and materials described herein are for demonstration purposes only.

### Example

### General process

When the preparation route is not included, the raw materials and reagents used in the present invention are known products, which can be synthesized according to methods known in the art, or can be obtained by purchasing commercially available products. All commercially available reagents were used without further purification.

Room temperature means 20-30°C.

There is no special description in the reaction examples, and the reactions are all carried out under a nitrogen atmosphere. The nitrogen atmosphere refers to a nitrogen balloon of about 1 L connected to the reaction flask.

The hydrogenation reaction is usually vacuumized and filled with hydrogen, and the operation is repeated 3 times. The hydrogen atmosphere means that the reaction bottle is connected with a hydrogen balloon of about 1L.

Microwave reactions use the Biotage^{®} Initiator+ Microwave Reactor.

The structures of the compounds of the present invention were determined by nuclear magnetic resonance (NMR) and mass spectroscopy (MS). NMR shifts (δ) are given in units of 10⁻⁶ (ppm). The determination of NMR is to use (Bruker AscendTM 500 type) nuclear magnetic analyzer, the measurement solvent is deuterated dimethyl sulfoxide (DMSO-d6), deuterated chloroform (CDCl3), deuterated methanol (CD3OD), and the internal standard is tetramethylsilane (TMS). The following abbreviations are used for the multiplicity of NMR signals: s = singlet, brs = broad singlet, d = doublet, t = triplet, m = multiplet. Coupling constants are listed as J values, measured in Hz.

As to reverse-phase preparative chromatography, a Thermo (UltiMate 3000) reverse-phase preparative chromatograph was used. The flash column chromatography uses Agela (FS-9200T) automatic column passing machine, and the silica gel prepacked column uses Santai SEPAFLASH^{®} prepacked column. yantai Huanghai HSGF254 or Qingdao GF254 silica gel plates are used for thin-layer chromatography silica gel plates, and the specifications used for thin-layer chromatography separation and purification products are 0.4mm to 0.5mm.

LC-MS analysis method is as follows:
1) Mass spectrometry method: Thermo Fisher MSQ PLUS mass spectrometer, ESI source, positive ion mode. Ion source parameter settings: drying gas temperature is 350 °C; drying gas flow rate is 10 L/min; MS Range: 120-1000.
2) Liquid phase conditions: Chromatographic column: Waters XBridge (3.5µm, 50mm×4.6mm); mobile phase A is an aqueous solution containing 0.1% ammonium bicarbonate, mobile phase B is an acetonitrile solution, and perform linear gradient elution in the following table 1; Flow rate: 2 mL/min; column temperature: 30°C; UV detection wavelength: 214nm, 254nm, 280nm; injection volume 2µL.

**Table 1. Gradient elution conditions**

| Time/min | Mobile phase A/% | Mobile phase B/% |
|---|---|---|
| 0 | 95 | 5 |
| 1.4 | 5 | 95 |
| 2.8 | 5 | 95 |
| 2.820 | 95 | 5 |
| 3 | 95 | 5 |

HPLC analysis method is as follows:
Chromatographic column: Waters XBridge phenyl (3.5µm, 150mm×4.6mm); mobile phase A is aqueous solution containing 0.1% ammonium bicarbonate, mobile phase B is acetonitrile solution, and linear gradient elution is carried out in the following table 2; flow rate: 1 mL/ min; column temperature: 30°C; UV detection wavelength: 214nm, 254nm, 280nm; injection volume 2µL.

**Table 2. Gradient elution conditions**

| Time/min | Mobile phase A/% | Mobile phase B/% |
|---|---|---|
| 0 | 95 | 5 |
| 15 | 5 | 95 |
| 20 | 5 | 95 |
| 20.2 | 95 | 5 |
| 24 | 95 | 5 |

The synthetic method of some intermediates in the invention is as follows:

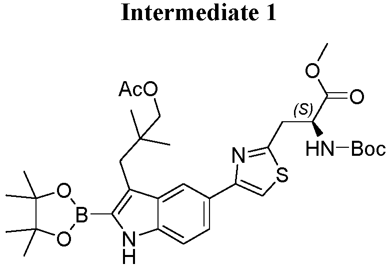

Intermediate 1 was prepared by the following steps:

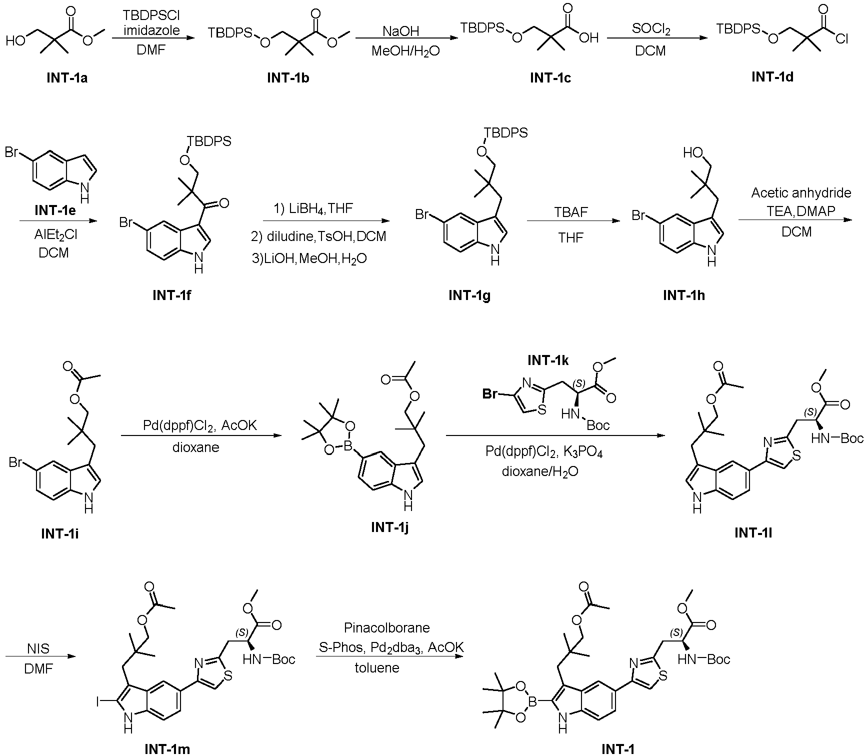

Step 1: 2.2-dimethyl-3-hydroxypropionate methyl ester **INT-1a** (100 g, 757 mmol) was dissolved in 1L N,N-dimethylformamide, imidazole (128.8 g, 1.89 mol) was added, stirred to dissolve, and tert-butyldiphenylchlorosilane (229 g, 832 mmol) was added dropwise at room temperature. After the addition was complete, stirring was continued for 4 hours. After the reaction was complete, the reaction solution was poured into 3L of ice water, the suspension was extracted with ethyl acetate (1L*2), the organic phase was washed with water three times, and then concentrated under reduced pressure to obtain **INT-1b** as a colorless oil. It was directly used in the next step without purification. ESI-MS (m/z): 371.2 [M+H]⁺.

Step 2: Add the raffinate **INT-1b** obtained in the previous step into 2L of methanol, add 360 g of 33% aqueous sodium hydroxide solution, and stir at room temperature for 17 hours. After the reaction was complete, add 1L of water, remove methanol under reduced pressure, extract the residue with petroleum ether (1L*5), adjust the pH value of the aqueous phase to 4~5 with hydrochloric acid after extraction, continue to stir for 30 minutes, filter with suction, and dry to obtain white solid **INT-1c** (269 g, yield 90%). ESI-MS (m/z): 357.8 [M+H]⁺.

Step 3: **INT-1c** (130 g, 365 mmol) was dissolved in 500 mL of dichloromethane, thionyl chloride (130.1 g, 1.09 mol, 79.4 mL) was added at room temperature, stirred at 60 °C for 3 hours. After the reaction was complete, dichloromethane and remaining thionyl chloride were removed under reduced pressure to obtain **INT-1d** as a light yellow oil, without purification, 200 mL of dichloromethane was added for use.

Step 4: **INT-1e** (64.8 g, 331 mmol) was dissolved in 400 mL of dichloromethane, 198 mL of diethylaluminum chloride solution (2M in hexanes) was added dropwise at 0 °C. During the dropping process, the temperature was controlled to be no more than 5 ° C. Stir the solution for 30 minutes after the dropwise addition, and add the obtained **INT-1d** dichloromethane solution dropwise into the reaction flask. During the dropping process the temperature was controlled to be no more than 10 °C. After the dropping was complete, continue the stirring for 2 hours. After the reaction was complete, the reaction solution was poured into 1L of ice water, stirred for 30 minutes, concentrated under reduced pressure to remove dichloromethane. The residue was extracted with ethyl acetate (1L*2), washed with water, and the organic phase was spin evaporated to obtain a brown oil. The oil was added to 2L of petroleum ether/ethyl acetate=10/1 mixed solution, the solid was precipitated by stirring, and filtered by suction to obtain **INT-1f** as a yellow solid (139 g, yield 78%). ESI-MS (m/z): 534.8 [M+H]⁺.

Step 5: **INT-1f** (100 g, 187 mmol) was dissolved in 500 mL tetrahydrofuran, lithium borohydride (12.2 g, 561 mmol) was added, and stirred overnight at 60 °C. After the raw materials disappeared, the reaction solution was added to 200 mL of ice water to quench, extracted with ethyl acetate (500 mL*3), and the organic phase was washed with water, dried, concentrated under reduced pressure, and the residue was dissolved in 500 mL of dichloromethane, then 2,6-dimethyl- Diethyl 1,4-dihydro-3,5-pyridinedicarboxylate (28.4 g, 112 mmol) and p-toluenesulfonic acid (21.4 g, 112.2 mmol) were added thereto, and the reaction solution was stirred at room temperature for 3 hours. After the reaction was complete, the reaction solution was concentrated under reduced pressure to remove methylene chloride, and the residue was dissolved in 500 mL of methanol, 14% lithium hydroxide aqueous solution (100 mL) prepared in advance was added, and the solution was stirred at room temperature for 3 hours, and filtered with suction to obtain a yellow solid **INT-1g** (84 g, collected rate of 86.3%). ESI-MS (m/z): 520.2 [M+H]⁺.

Step 6: **INT-1g** (50 g, 96 mmol) was dissolved in 250 mL tetrahydrofuran, tetrabutylammonium fluoride (1M in THF, 197 mL) was added, stirred overnight at 60°C. After the reaction was complete, the reaction solution was added to 300 mL of water, extracted with ethyl acetate (200 mL*3), washed with water, and concentrated under reduced pressure to obtain a brown oil. Dissolve the obtained residue in 40 mL of methanol, add 20 mL of water, wash the mixed solution with petroleum ether (40 mL*5), concentrate under reduced pressure to remove methanol, and extract the residue with ethyl acetate (50 mL*2). The organic phase was washed with water and dried to obtain **INT-1h** (25 g, yield 90.4%) as a pale yellow oil. ESI-MS (m/z): 282.8 [M+H]⁺.

Step 7: Dissolve compound **INT-1h** (22 g, 77 mmol) in 100 mL of dichloromethane. Add 4-dimethylaminopyridine (467 mg, 3.82 mmol), triethylamine (23.2 g, 230 mmol), and add acetic anhydride (7.9 g, 77 mmol) dropwise at 0 °C. After the dropwise addition, the temperature was raised naturally, and the solution was stirred overnight. After the reaction was complete, the reaction solution was washed with water, dried, and concentrated to obtain a brown oil, which was purified by silica gel column chromatography (petroleum ether/ethyl acetate=4/1) to obtain a light yellow oil **INT-1i** (22.5 g, yield 90.7%). ESI-MS (m/z): 324.2 [M+H]⁺.

Step 8: Compound **INT-1i** (40 g, 123.4 mmol) was dissolved in dioxane (400 mL), potassium acetate (30.3 g, 308.4 mmol), [1,1'-bis(diphenyl Phosphine)ferrocene]palladium dichloride (10 g, 12.3 mmol), pinacol diborate (78.3 g, 308.4 mmol) were added to react at 90 °C for 3 hours under nitrogen protection, LCMS monitored the complete reaction of the raw materials, and the reaction solution was directly concentrated under reduced pressure. The residue was dissolved in ethyl acetate (300 mL), washed with water and brine, and the organic phase was purified by silica gel column chromatography to obtain white solid compound **INT-1j** (35 g, yield 76.4%). ESI-MS (m/z): 372.5 [M+H]⁺.

Step 9: Dissolve compound INT-1j (35 g, 94.3 mmol) and compound INT-1k (37.9 g, 104 mmol) in dioxane (300 mL) and water (30 mL), add potassium phosphate (50 g, 236 mmol) and [1,1'-bis(diphenylphosphino)ferrocene]palladium dichloride (6.89 g, 9.43 mmol) to react overnight at 90 °C under nitrogen protection. LCMS monitored the complete reaction of the raw material, the reaction solution was directly concentrated under reduced pressure, the residue was dissolved in ethyl acetate (300 mL), washed with water and brine, and the organic phase was purified by silica gel column chromatography to obtain yellow oily compound **INT-11** (28 g, yield 56.1%). ESI-MS (m/z): 530.7 [M+H]⁺.

Step 10: Dissolve compound **INT-11** (28 g, 52.9 mmol) in N,N-dimethylformamide (280 mL), add N-iodosuccinimide (11.9 g, 52.9 mmol) to react at 50°C for 2 hours. LCMS monitored the complete reaction of the raw materials, and the reaction solution was poured into water (800 mL), extracted with ethyl acetate (200 mL*2). The organic phase was washed with saturated brine, dried, filtered, and purified through a silica gel column chromatography to give a yellow solid compound **INT-1m** (22 g, yield 63.5%). ESI-MS (m/z): 656.6 [M+H]⁺.

Step 11: Compound TNT-1m (5 g, 7.63 mmol), 2-dicyclohexylphosphine-2',6'-dimethoxy-biphenyl (939.4 mg, 2.29 mmol), tris(dimethoxy Benzylacetone) dipalladium (838.1 mg, 0.915 mmol), potassium acetate (2.6 g, 26.7 mmol) were dissolved in toluene (100 mL), and pinacol borane (4.9 g, 38.1 mmol) was added under nitrogen protection, after the addition was complete, the reaction was carried out at 50 °C for 5 hours under the protection of nitrogen. LCMS monitored the complete reaction of the raw materials. The reaction solution was filtered and purified by silica gel column chromatography to obtain a yellow oily compound **INT-1** (4.5 g, yield 90%). ESI-MS (m/z): 656.5 [M+H]⁺.

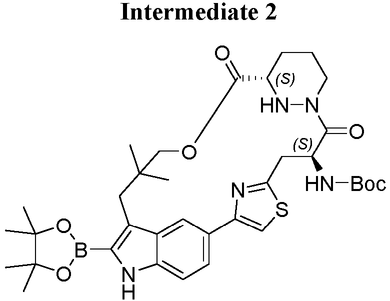

Intermediate 2 was prepared by the following steps:

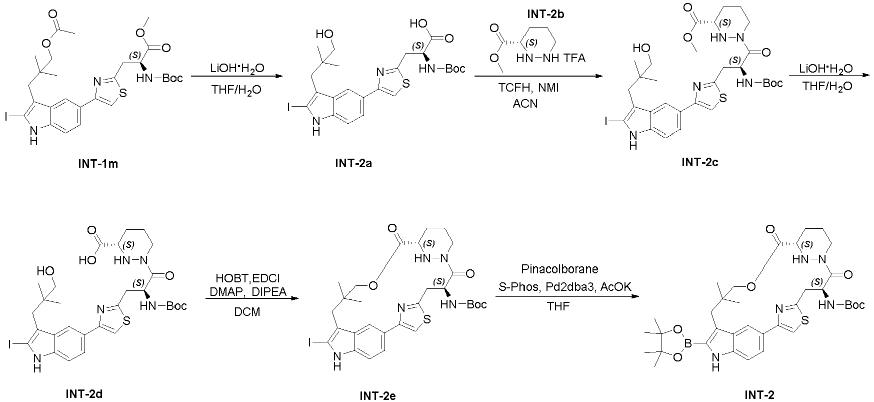

Step 1: Compound **INT-1m** (12 g, 18.3 mmol) was dissolved in tetrahydrofuran (120 mL) and water (20 mL), lithium hydroxide monohydrate (3.84 g, 91.5 mmol) was added to react overnight at room temperature, LCMS monitored the complete reaction of raw materials, the reaction solution was directly concentrated under reduced pressure, the residue was dissolved in water (100 mL), the pH was adjusted to 4~5 with 4M hydrochloric acid, the solution was extracted with dichloromethane (100 mL*3), the organic phase was washed with water, and washed with salt water, dried over anhydrous sodium sulfate, filtered, and concentrated to obtain white solid compound **INT-2a** (10.6 g, yield 96.6%). ESI-MS (m/z): 600.7 [M+H]⁺.

Step 2: Compound INT-2a (9.5 g, 15.9 mmol) and compound INT-2b (11.7 g, 31.7 mmol) were dissolved in acetonitrile (190 mL), N,N,N',N '-Tetramethylchloroformamidine hexafluorophosphate (6.67 g, 23.8 mmol) and 1-methylimidazole (6.51 g, 79.2 mmol) were added to react at 0 °C for 1 hour, and the reaction of the raw materials was monitored by LCMS to be complete. The reaction solution was poured into water (200 mL), extracted with dichloromethane (100 mL*3), the organic phase was washed with water, and purified by silica gel column chromatography to obtain compound **INT-2c** (9.6 g, yield 83.5%) as a yellow solid. ESI-MS (m/z): 726.3 [M+H]⁺.

Step 3: Dissolve compound **INT-2c** (9.6 g, 13.2 mmol) in tetrahydrofuran (100 mL) and water (10 mL), add lithium hydroxide monohydrate (1.39 g, 33.1 mmol), and react at room temperature for 4 hours. LCMS monitored the complete reaction of the raw materials. The reaction solution was directly concentrated under reduced pressure, the residue was dissolved in water (100 mL), the pH was adjusted to 4~5 with 4M hydrochloric acid, and a white solid precipitated. After being filtered, washed with water, and dried a white solid compound **INT- 2d** (8.3 g, 88.2% yield) was obtained. ESI-MS (m/z): 712.6 [M+H]⁺.

Step 4: Dissolve compound INT-2d (3.5 g, 4.9 mmol), 1-hydroxybenzotriazole (1.99 g, 14.8 mmol), 4-dimethylaminopyridine (1.8 g, 14. mmol) in dichloromethane (170 mL), N,N-diisopropylethylamine (6 mL, 34.4 mmol) was added at 0°C, then 1-(3-dimethylaminopropyl)-3-ethylcarbodieneamine hydrochloride (4.71 g, 24.6 mmol) was added to react overnight at room temperature. LCMS monitored the complete reaction of the raw materials. The reaction solution was washed with saturated ammonium chloride aqueous solution, dried over sodium sulfate, and purified by silica gel column chromatography to obtain a yellow solid compound **INT-2e** ( 2 g, yield 58.6%). ESI-MS (m/z): 694.6 [M+H]⁺.

Step 5: Compound **INT-2e** (500 mg, 0.721 mmol), 2-dicyclohexylphosphine-2',6'-dimethyl-biphenyl (88.8 mg, 0.216 mmol), tris(dibenzylidene acetone) dipalladium (79 mg, 0.086 mmol), potassium acetate (247 mg, 2.52 mmol) were dissolved in tetrahydrofuran (20 mL), and pinacolborane (461 mg, 3.6 mmol) was added under nitrogen protection. After the addition was complete, the reaction solution was allowed to react at 50 °C for 3 hours under the protection of nitrogen. LCMS monitored the complete reaction of the raw materials. The reaction solution was filtered and purified by silica gel column chromatography to obtain a yellow solid compound **INT-2** (400 mg, yield 80%). ESI-MS (m/z): 694.6 [M+H]⁺.

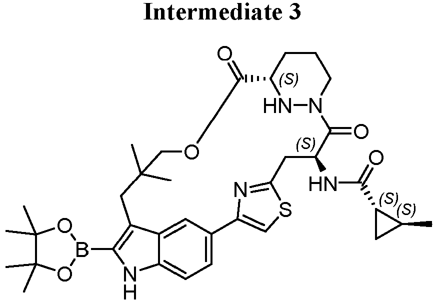

Intermediate 3 was prepared by the following steps:

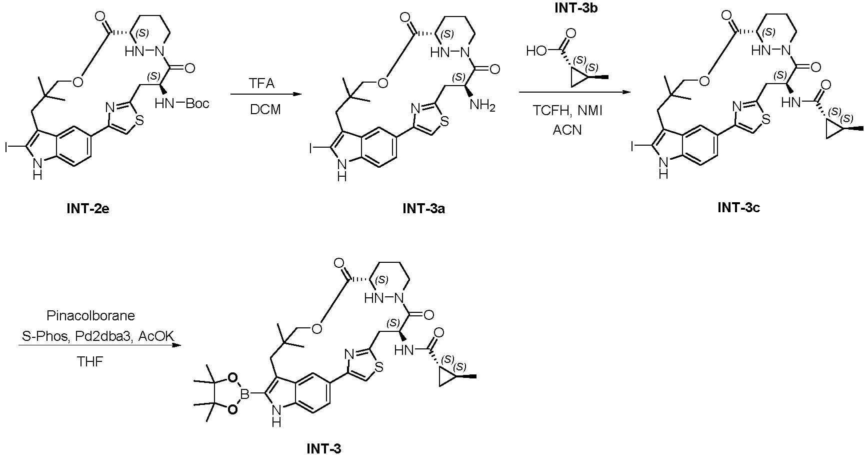

Step 1: Dissolve compound **INT-2e** (1.7 g, 2.45 mmol) in dichloromethane (20 mL), add trifluoroacetic acid (5 mL), and react at room temperature for 2 hours. LCMS monitored that the reaction of the raw materials was complete, and the reaction solution was directly concentrated under reduced pressure, the residue was dissolved in DCM (50 mL), washed twice with saturated NaHCO₃ aqueous solution, the organic phase was washed with water, dried over sodium sulfate, filtered, and concentrated to obtain compound **INT-3a** (1.3 g, yield 89.4%) as a yellow solid. ESI-MS (m/z): 594.7 [M+H]⁺.

Step 2: Compound INT-3a (1.3 g, 2.19 mmol) and compound INT-3b (0.24 g, 2.41 mmol) were dissolved in acetonitrile (30 mL), N,N,N',N '-tetramethylchloroformamidine hexafluorophosphate (922 mg, 3.29 mmol), and 1-methylimidazole (414 mg, 5.04 mmol) were added to react at 0 °C for 1 hour, LCMS monitored the complete reaction of the raw materials, and the reaction solution was poured into water (50 mL), extracted with dichloromethane (50 mL*3). Wash the organic phase with water, mix the sample and pass through the column for purification to obtain white solid compound **INT-3c** (1.3 g, yield 87.9%). ESI-MS (m/z): 675.7 [M+H]⁺.

Step 3: Compound **INT-3c** (1.1 g, 1.63 mmol), 2-dicyclohexylphosphine-2',6'-dimethyl-biphenyl (200.5 mg, 0.188 mmol), tris(dibenzylidene acetone) dipalladium (179 mg, 0.195 mmol), potassium acetate (559 mg, 5.7 mmol) were dissolved in toluene (30 mL), and pinacol borane (1.04 g, 8.14 mmol) was added under nitrogen protection. The solution was allowed to react at 50 °C for 3 hours under nitrogen protection after the addition was complete. LCMS monitored the complete reaction of the raw materials, the reaction solution was filtered, and purified by silica gel column chromatography to obtain a yellow solid compound **INT-3** (990 mg, yield 90%). ESI-MS (m/z): 676.9 [M+H]⁺.

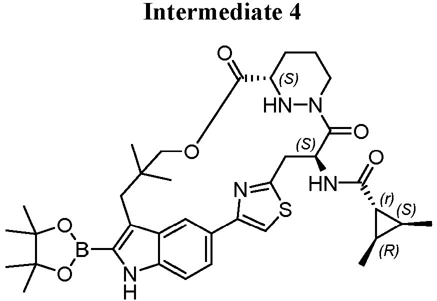

Intermediate 4 was prepared by the following steps:

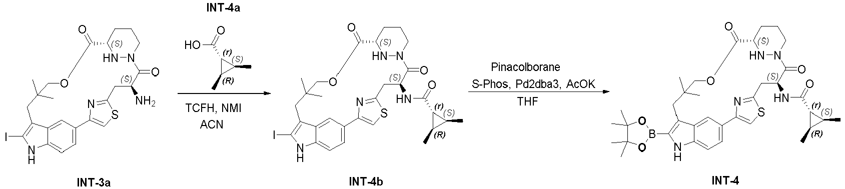

Step 1: Compound **INT-3a** (2.2 g, 3.71 mmol) and compound **INT-4a** (0.47 g, 4.08 mmol) was dissolvd in dichloromethane (50 mL), N,N,N' at 0 °C , N'-tetramethylchloroformamidine hexafluorophosphate (1.56 g, 5.56 mmol), and 1-methylimidazole (0.70 g, 8.53 mmol) were added to react at 0 °C for 1 hour. LCMS monitored the complete reaction of the raw materials, and the reaction solution was poured into water (50 mL), extracted with dichloromethane (50 mL*3), the organic phase was washed with water, and the sample was mixed and purified by column to obtain white solid compound **INT-4b** (2.3 g, yield 90.0%). ESI-MS (m/z): 690.2 [M+H]⁺.

Step 2: Compound **INT-4b** (2.1 g, 3.05 mmol), 2-dicyclohexylphosphine-2',6'-dimethyl-biphenyl (375 mg, 0.91 mmol), tris(dibenzylidene acetone) dipalladium (334.6 mg, 0.365 mmol), potassium acetate (1.05 g, 10.7 mmol) were dissolved in toluene (30 mL), and pinacolborane (1.95 g, 15.2 mmol) was added under nitrogen protection. After the addition was complete, the reaction system was allowed react at 50 °C for 3 hours under the protection of nitrogen. LCMS monitored the complete reaction of the raw materials. The reaction solution was filtered and purified by silica gel column chromatography to obtain a yellow solid compound **INT-4** (1.8 g, yield 85.7%). ESI-MS (m/z): 690.3 [M+H]⁺.

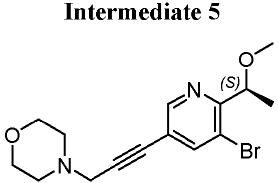

Intermediate 5 was prepared by the following steps:

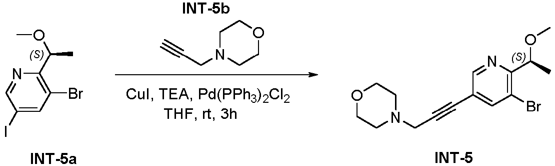

Step 1: Dissolve (S)-3-bromo-5-iodo-2-(1-methoxyethyl)pyridine **INT-5a** (2.0 g, 5.85 mmol) in tetrahydrofuran (20 mL), and add cuprous iodide (111.4 mg, 0.585 mmol), bistriphenylphosphinepalladium dichloride (410.5 mg, 0.585 mmol), triethylamine (1.18 g, 11.7 mmol), and 4-propyne-1-morpholine **INT -5b** (878.5 mg, 7.02 mmol). The reaction mixture was stirred at room temperature for 3 hours under nitrogen protection. After the reaction was complete, the reaction solution was concentrated under reduced pressure, and the residue was purified by silica gel column chromatography (dichloromethane/ethyl acetate = 1/1) to obtain light yellow oily compound **INT-5** (1.8 g, yield 90.7%). ESI-MS (m/z): 339.4 [M+H]⁺.

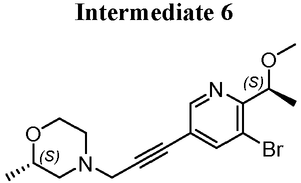

Intermediate 6 was prepared by the following steps:

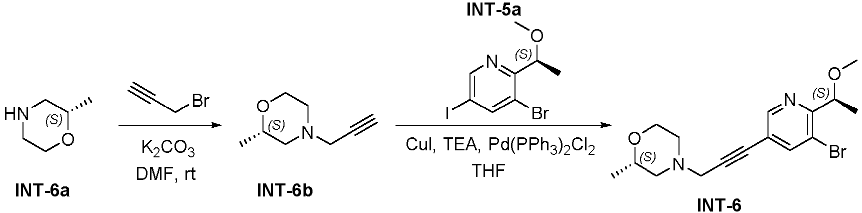

Step 1: (S)-2-methylmorpholine hydrochloride INT-6a (1.00 g, 9.27 mmol), 3-bromopropyne (1.04 g, 8.72 mmol), potassium carbonate (3.01 g, 21.8 mmol) were dissolved in N,N-dimethylformamide (15 mL), and the reaction solution was stirred overnight at room temperature. After the reaction was complete, the reaction solution was extracted with ethyl acetate, and the organic phase was dried and concentrated to obtain a crude product. The crude product was purified by column chromatography (petroleum ether/ethyl acetate = 1/1) to obtain light yellow oily compound **INT-6b** (450 mg, yield 44.5%). ESI-MS (m/z): 140.3 [M+H]⁺.

Step 2: **INT-6b** (269 mg, 1.93 mmol), **INT-5a** (600 mg, 1.75 mmol), cuprous iodide (33.4 mg, 0.18 mmol), triethylamine (355.1 mg, 3.51 mmol), bistriphenylpalladium dichloride (123.0 mg, 0.18 mmol) were dissolved in tetrahydrofuran (10 mL), and the reaction solution was stirred overnight at room temperature. After the reaction was complete, the reaction solution was filtered, and the filtrate was concentrated to obtain a crude product. The crude product was purified by column chromatography (petroleum ether/ethyl acetate = 1/1) to obtain light yellow oily compound **INT-6** (360 mg, yield 58.10%). ESI-MS (m/z): 353.5 [M+H]⁺.

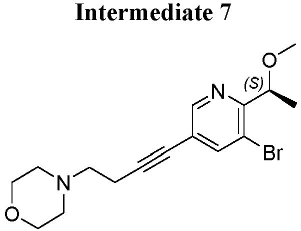

Intermediate 7 was prepared by the following steps:

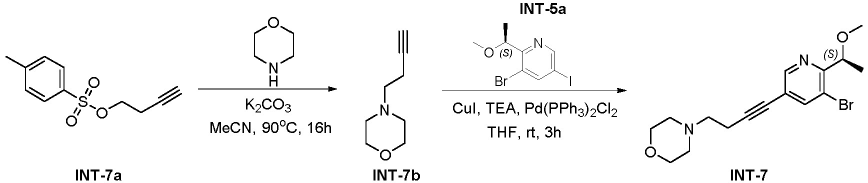

Step 1: Compound **INT-7a** (1 g, 4.46 mmol) was dissolved in acetonitrile (10 mL), and morpholine (1.16 g, 13.3 mmol) and potassium carbonate (1.23 g, 8.92 mmol) were added in sequence. The reaction mixture was stirred at 90 °C for 16 hours under nitrogen protection. After the reaction was complete, the reaction solution was filtered with diatomaceous earth, the filtrate was washed twice with ammonium chloride aqueous solution and then concentrated to obtain a yellow oily compound **INT-7b** (450 mg, yield 72.6%). ESI-MS (m/z): 140.1 [M+H]⁺.

Step 2: Dissolve (S)-3-bromo-5-iodo-2-(1-methoxyethyl)pyridine **INT-5a** (920 mg, 2.69 mmol) in tetrahydrofuran (20 mL), and add cuprous iodide (51 mg, 0.27 mmol), bistriphenylphosphine palladium dichloride (188 mg, 0.27 mmol), triethylamine (540 mg, 5.34 mmol) and **INT-7b** (450 mg, 3.24 mmol) . The reaction mixture was stirred at room temperature for 3 hours under nitrogen protection. After the reaction was complete, the reaction solution was concentrated under reduced pressure, and the residue was purified by silica gel column chromatography (dichloromethane/ethyl acetate = 1/1) to obtain light yellow oily compound **INT-7** (850 mg, yield 89.2%). ESI-MS (m/z): 353.6 [M+H]⁺.

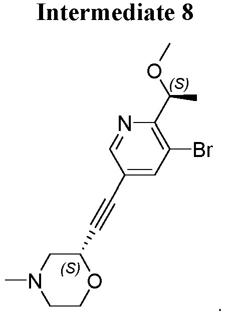

Intermediate 8 was prepared by the following steps:

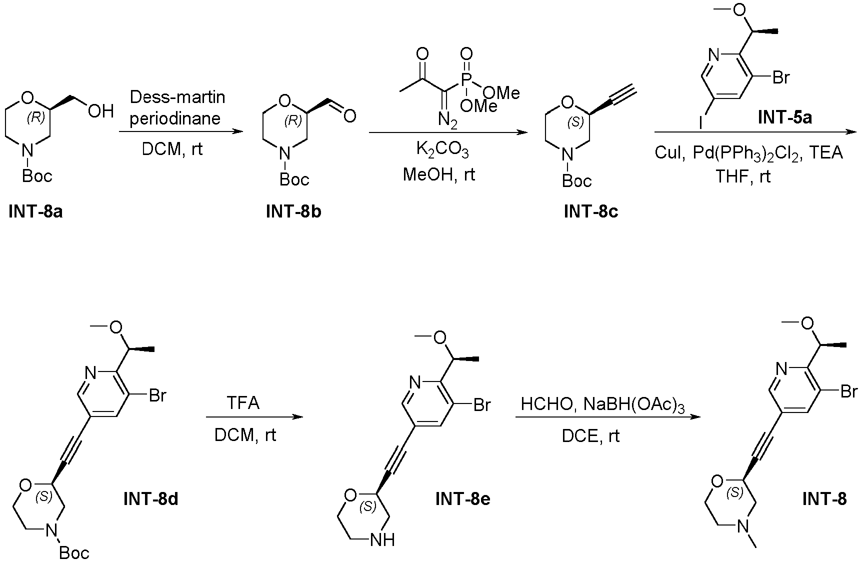

Step 1: Add Dess Martin reagent (5.08 g, 12.0 mmol) to a solution of **INT-8a** (2.0 g, 9.21 mmol) in dichloromethane (40 mL) at 0 °C. After the reaction solution was warmed to room temperature, stirring was continued for 5 hours. After the reaction was complete, saturated aqueous sodium bicarbonate and sodium thiosulfate were added to quench the reaction, the reaction solution was extracted with dichloromethane, the organic phases were combined and washed with saturated brine, dried over anhydrous sodium sulfate, and the organic phase was concentrated to obtain the crude product **INT-8b.**

Step 2: The above crude product **INT-8b** and potassium carbonate (3.81 g, 27.60 mmol) were added to methanol (40 mL), and (1-diazo-2-oxopropyl) dimethyl phosphonate (3.53 g, 18.40 mmol) was added dropwise thereto at 0 °C. After the reaction solution was stirred at room temperature for 4 hours, the reaction was monitored by TLC to be complete. The reaction was quenched by adding water, the reaction solution was extracted with ethyl acetate, and the combined organic phases were washed with saturated brine, dried over anhydrous sodium sulfate, then the organic phase was concentrated and purified by silica gel column chromatography (petroleum ether: ethyl acetate = 6:1) to obtain colorless transparent solid **INT-8c** (1.20 g, two-step yield 62.0%). ESI-MS (m/z): 212.3 [M+H]⁺.

Step 3: Under nitrogen atmosphere, add (S)-3-bromo-5-iodo-2-(1-methoxyethyl)pyridine **INT-5a** (300 mg, 0.88 mmol) and cuprous iodide (17 mg, 0.088 mmol), bistriphenylphosphine palladium dichloride (62 mg, 0.088 mmol), triethylamine (178 mg, 1.75 mmol), compound **INT-8c** (222 mg, 1.05 mmol) and tetrahydrofuran ( 10 mL) successively into the reaction flask. After the reaction solution was stirred at room temperature for 4 hours, the reaction was monitored by LCMS to be complete. The reaction solution was concentrated under reduced pressure and purified by silica gel column chromatography (petroleum ether/ethyl acetate=3/1) to obtain a pale yellow oily liquid **INT-8d.** ESI-MS (m/z): 425.7 [M+H]⁺.

Step 4: The above product **INT-8d** was dissolved in dichloromethane (6 mL), and trifluoroacetic acid (2 mL) was added dropwise thereto. After the reaction solution was stirred at room temperature for 30 minutes, the reaction was monitored by LCMS to be complete. The reaction solution was concentrated under reduced pressure to obtain compound **INT-8e.** ESI-MS (m/z): 325.5 [M+H]⁺.

Step 5: Dissolve the above product **INT-8e** in 1,2-dichloroethane (10 mL), add formaldehyde aqueous solution (213 mg, 2.63 mmol, 37%) dropwise thereto, react for 30 minutes, then add triacetyl sodium oxyborohydride (1.11 g, 5.26 mmol). The reaction solution was stirred at room temperature for 30 minutes, and LCMS monitored the completion of the reaction. The reaction was quenched by adding saturated aqueous sodium bicarbonate solution, the reaction solution was extracted with dichloromethane and methanol, the organic phases were combined and washed with saturated brine, dried over anhydrous sodium sulfate, then the organic phase was concentrated and purified by silica gel column chromatography (dichloromethane:methanol=10: 1) to obtain **INT-8** (269 mg, 90.5% yield in three steps) as light yellow oily liquid. ESI-MS (m/z): 339.6 [M+H]⁺.

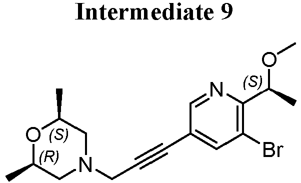

Intermediate 9 was prepared by the following steps:

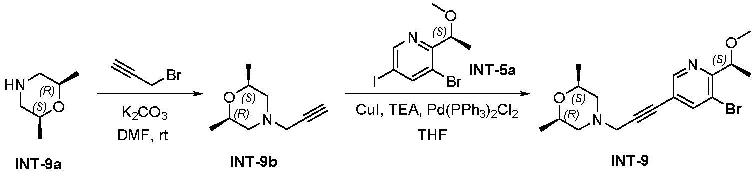

Step 1: cis-2,6-dimethylmorpholine **INT-9a** (1.06 g, 9.20 mmol), 3-bromopropyne (1.09 g, 9.20 mmol), cesium carbonate (3.82 g, 27.6 mmol) were dissolved in N, N-dimethylformamide (10 mL), and the reaction solution was stirred at room temperature overnight. After the reaction was complete, the reaction solution was extracted with ethyl acetate, and the organic phase was dried and concentrated to obtain a crude product. The crude product was purified by column chromatography (petroleum ether/ethyl acetate = 1/1) to obtain light yellow oily compound **INT-9b** (600 mg, yield 42.6%). ESI-MS (m/z): 154.2 [M+H]⁺.

Step 2: **INT-9b** (296 mg, 1.93 mmol), **INT-5a** (600 mg, 1.75 mmol), cuprous iodide (33.4 mg, 0.18 mmol), triethylamine (355.1 mg, 3.51 mmol), bistriphenylpalladium dichloride (123.0 mg, 0.18 mmol) were dissolved in tetrahydrofuran (10 mL), and the reaction solution was stirred overnight at room temperature. After the reaction was complete, the reaction solution was filtered, and the filtrate was concentrated and spin-dried to obtain a crude product. The crude product was purified by column chromatography (petroleum ether/ethyl acetate=1/1) to obtain light yellow oily compound **INT-9** (600 mg, yield 93.1%). ESI-MS (m/z): 367.3 [M+H]⁺.

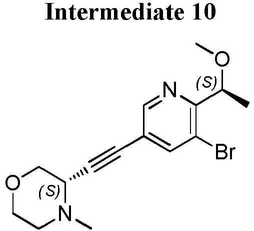

Intermediate 10 was prepared by the following steps:

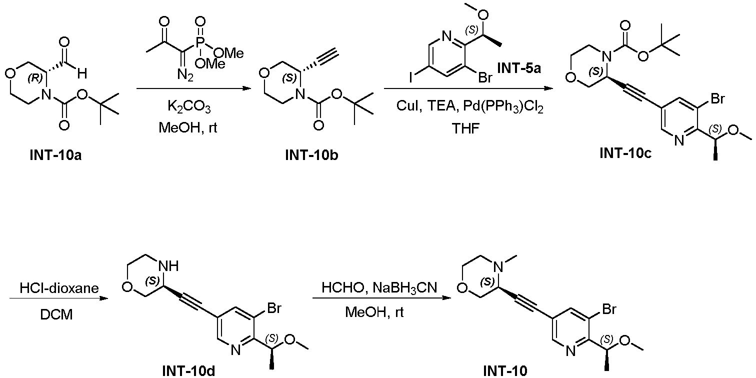

Step 1: (R)-3-formylmorpholine-4-carboxylate tert-butyl ester INT-10a (4.06 g, 18.9 mmol) and (1-diazo-2-oxopropyl) dimethyl phosphonate (5.44 g, 28.3 mmol) were dissolved in methanol (30 mL), and potassium carbonate was added to the above reaction solution at room temperature. The reaction solution was stirred overnight at room temperature. After the reaction was complete, the reaction solution was extracted with ethyl acetate, and the organic phase was dried and concentrated to obtain a crude product. The crude product was purified by column chromatography (petroleum ether/ethyl acetate = 3/1) to obtain light yellow oily compound **INT-10b** (3.2 g, yield 80.3%). ESI-MS (m/z): 212.4 [M+H]⁺.

Step 2: **INT-10b** (710.4 mg, 3.36 mmol), **INT-5a** (1.00 g, 2.92 mmol), triethylamine (592 mg, 5.58 mmol), cuprous iodide (55.7 mg, 0.29 mmol), bistriphenylpalladium dichloride (205.3 mg, 0.29 mmol) were dissolved in tetrahydrofuran (10 mL), and the reaction solution was stirred overnight at room temperature. After the reaction was complete, the reaction solution was filtered, and the filtrate was concentrated to obtain a crude product. The crude product was purified by column chromatography (dichloromethane/methanol = 10/1) to obtain light yellow solid compound **INT-10c** (1.05 g, yield 84.4%). ESI-MS (m/z): 425.5 [M+H]⁺.

Step 3: Dissolve INT-10c (1.20 g, 2.82 mmol) in dichloromethane (15 mL), and add hydrochloric acid-dioxane (7.05 mL, 4M) to the above reaction solution. The reaction was stirred at room temperature for 4 hours. After the reaction was complete, a saturated aqueous sodium bicarbonate solution was added to the reaction solution. The organic phases were combined, dried and concentrated to obtain light yellow solid compound **INT-10d** (918 mg, yield 100.00%). ESI-MS (m/z): 325.5 [M+H]⁺.

Step 4: **INT-10d** (917.06 mg, 2.82 mmol), aqueous formaldehyde (288.2 mg, 8.46 mmol), and sodium cyanoborohydride (532 mg, 8.46 mmol) were dissolved in methanol (10 mL), and the reaction solution was stirred overnight at room temperature. After the reaction was complete, a saturated aqueous sodium bicarbonate solution was added to the reaction solution, followed by extraction with dichloromethane. The combined organic phases were dried and concentrated to obtain a crude product, which was purified by column chromatography (dichloromethane/methanol = 10/1) to obtain light yellow oily compound **INT-10** (650 mg, yield 68.0%). ESI-MS (m/z): 339.2 [M+H]⁺.

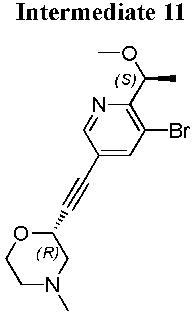

Intermediate 11 was prepared by the following steps:

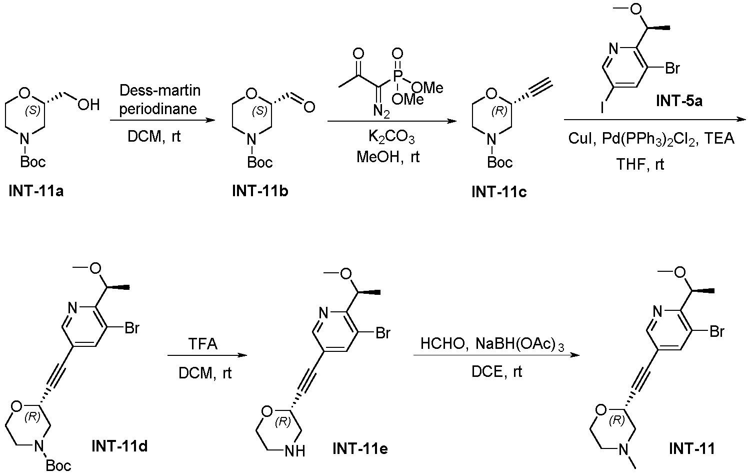

Step 1: Add Dess Martin reagent (5.08 g, 12.0 mmol) to a solution of **INT-11a** (2.0 g, 9.21 mmol) in dichloromethane (40 mL) at 0 °C. After the reaction solution was warmed to room temperature, stirring was continued for 5 hours. After the reaction was complete, saturated aqueous sodium bicarbonate and sodium thiosulfate were added to quench the reaction. The reaction solution was extracted with dichloromethane, and the organic phases were combined and washed with saturated brine, dried over anhydrous sodium sulfate, and the organic phase was concentrated to obtain the crude product **INT-11b.**

Step 2: The above crude product **INT-11b** and potassium carbonate (3.81 g, 27.60 mmol) were added to methanol (40 mL), and (1-diazo-2-oxo Dimethyl phosphonate (3.53 g, 18.4 mmol) was added thereto at 0 °C. After the reaction solution was stirred at room temperature for 16 hours, the reaction was monitored by TLC to be complete. The reaction was quenched by adding water, the reaction solution was extracted with ethyl acetate, the organic phases were combined and washed with saturated brine, dried over anhydrous sodium sulfate, and the organic phase was concentrated and purified by silica gel column chromatography (petroleum ether: ethyl acetate = 6/1) to obtain colorless transparent solid **INT-11c** (1.38 g, 70.8% yield in two steps). ESI-MS (m/z): 212.2 [M+H]⁺.

Step 3: Under nitrogen atmosphere, add (S)-3-bromo-5-iodo-2-(1-methoxyethyl)pyridine **INT-5a** (500 mg, 1.46 mmol) and cuprous iodide (28 mg, 0.146 mmol), bistriphenylphosphine palladium dichloride (103 mg, 0.146 mmol), triethylamine (296 mg, 2.92 mmol), compound **INT-11c** (402 mg, 1.90 mmol) and tetrahydrofuran (10 mL) successively into the reaction flask. After the reaction solution was stirred at room temperature for 16 hours, the reaction was monitored by LCMS to be complete. The reaction solution was concentrated under reduced pressure and purified by silica gel column chromatography (petroleum ether/ethyl acetate=3/1) to obtain a pale yellow oily liquid **Int-11d.** ESI-MS (m/z): 425.6 [M+H]⁺.

Step 4: The above product **Int-11d** was dissolved in dichloromethane (9 mL), and trifluoroacetic acid (3 mL) was added dropwise thereto. After the reaction solution was stirred at room temperature for 30 minutes, the reaction was monitored by LCMS to complete. The reaction solution was concentrated under reduced pressure to obtain compound **Int-11e.** ESI-MS (m/z): 325.3 [M+H]⁺.

Step 5: Dissolve the above product **Int-11e** in 1,2-dichloroethane (10 mL), add formaldehyde aqueous solution (356 mg, 4.38 mmol, 37%) dropwise thereto, react for 30 minutes, then add triacetyl sodium oxyborohydride (1.86 g, 8.76 mmol). The reaction solution was stirred at room temperature for 30 minutes, and LCMS monitored the completion of the reaction. The reaction was quenched by adding saturated aqueous sodium bicarbonate solution, the reaction solution was extracted with dichloromethane and methanol, the organic phases were combined and washed with saturated brine, dried over anhydrous sodium sulfate, and the organic phase was concentrated and purified by silica gel column chromatography (dichloromethane/methanol=10/ 1) to obtain **Int-11** (481 mg, three-step yield 97.1%) as light yellow oily liquid. ESI-MS (m/z): 339.4 [M+H]⁺.

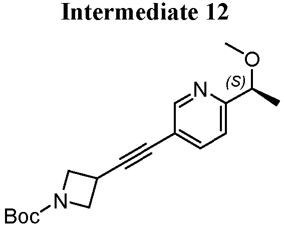

Intermediate 12 was prepared by the following steps:

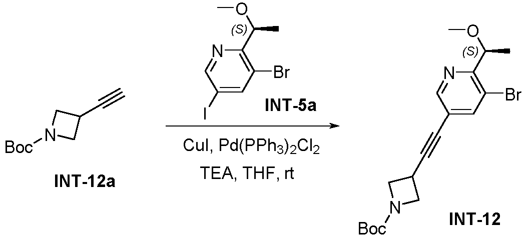

Compound **INT-12** can be obtained by replacing **INT-5b** in intermediate 5 with **INT-12a** and using similar methods and reaction steps. ESI-MS (m/z): 395.5 [M+H]⁺.

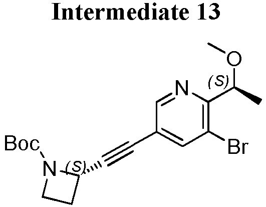

Intermediate 13 was prepared by the following steps:

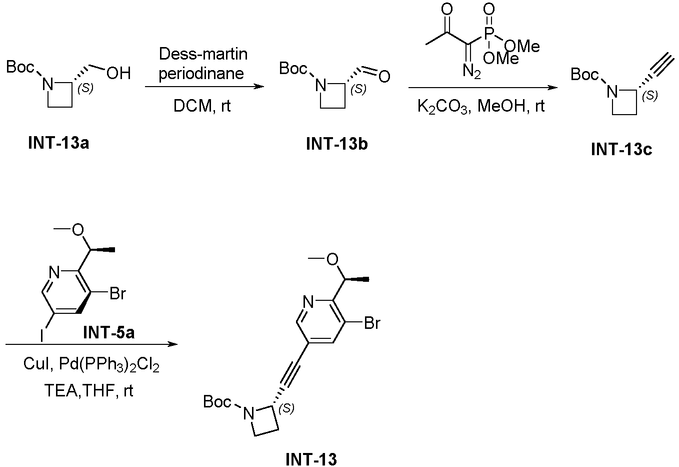

Compound **INT-13** can be obtained by replacing **INT-8a** in intermediate 8 with **INT-13a** and using similar methods and reaction steps. ESI-MS (m/z): 395.5 [M+H]⁺.

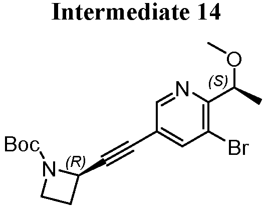

Intermediate 14 was prepared by the following steps:

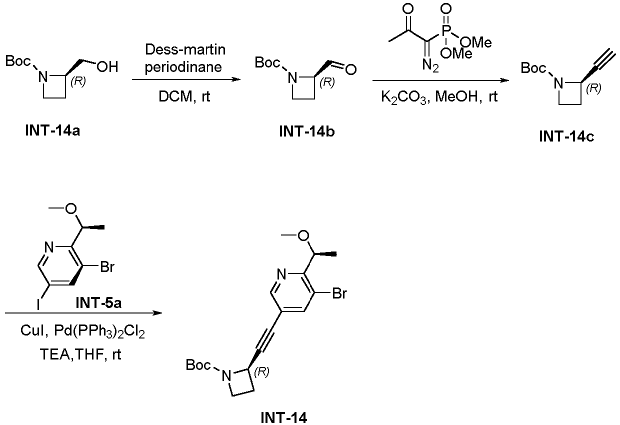

Compound **INT-14** can be obtained by replacing **INT-8a** in intermediate 8 with **INT-14a** and using similar methods and reaction steps. ESI-MS (m/z): 395.5 [M+H]⁺.

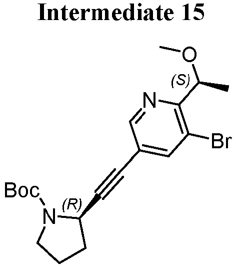

Intermediate 15 was prepared by the following steps:

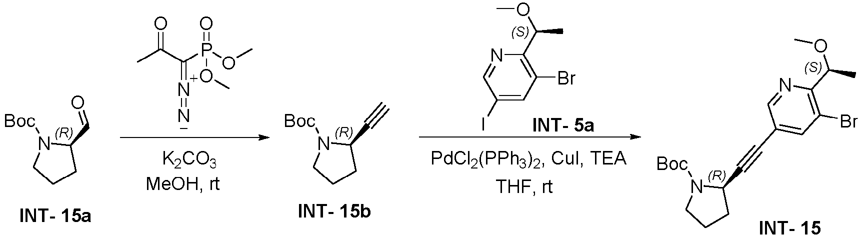

Step 1: Dissolve compound **INT-15a** (600 mg, 3 mmol) in methanol (5 mL), add potassium carbonate (1.25 g, 9 mmol) and (1-diazo-2-oxopropyl) dimethyl phosphonate (1.16 g, 6 mmol) under room temperature. The reaction solution was stirred at room temperature for 12 h. TLC detected that the reaction was complete. Saturated brine was added to the reaction system, and the reaction solution was extracted with dichloromethane, the organic phases were combined, washed with saturated brine, dried over anhydrous sodium sulfate, filtered and concentrated to obtain the crude product of compound INT-15b (587 mg, yield 99%).

Step 2: Dissolve compound **INT-15b** (570 mg, 2.92 mmol) and compound **INT-5a** (1 g, 2.92 mmol) in tetrahydrofuran (8 mL), add bistriphenylphosphine palladium dichloride (204 mg , 0.29 mmol), cuprous iodide (56 mg, 0.29 mmol) and triethylamine (591 mg, 5.85 mmol). The reaction system was stirred at room temperature for 8 h after nitrogen replacement. LCMS detected that the reaction was complete. Saturated brine was added to the reaction system, and the reaction solution was extracted with ethyl acetate, the organic phases were combined, washed with saturated brine, dried over anhydrous sodium sulfate, concentrated by filtration, and the residue was purified by silica gel column chromatography (petroleum ether/ethyl acetate=5/ 1) to obtain **INT-15** (1.08 g, yield 90%) as a yellow oily liquid. ESI-MS (m/z): 409.6 [M+H]⁺.

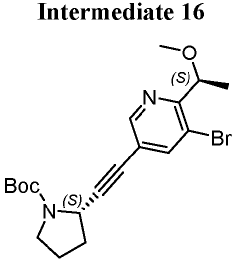

Intermediate 16 was prepared by the following steps:

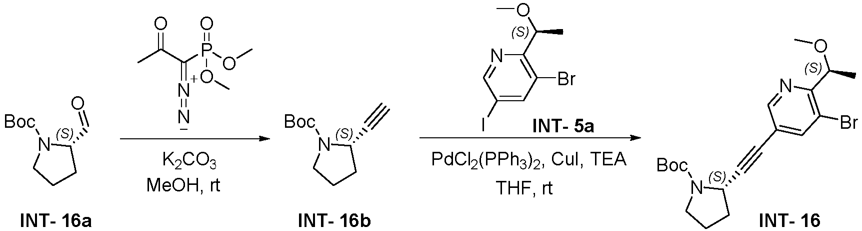

The **INT-15a** in the intermediate **INT-15** was replaced by **INT-16a,** and the compound **INT-16** can be obtained by a similar method and reaction steps. ESI-MS (m/z): 409.5 [M+H]⁺.

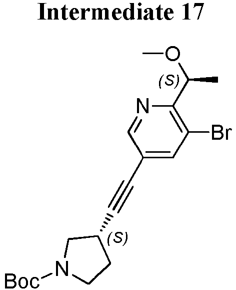

Intermediate 17 was prepared by the following steps:

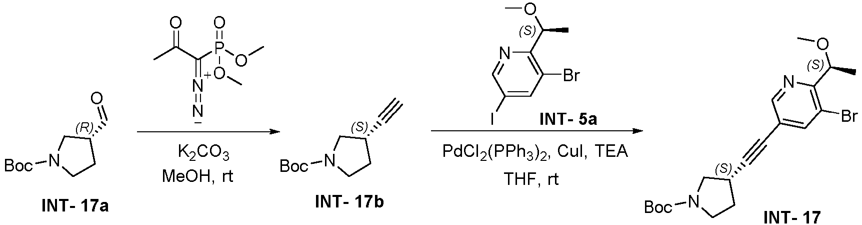

The **INT-15a** in the intermediate **INT-15** was replaced by **INT-17a,** and the compound **INT-17** can be obtained by a similar method and reaction steps. ESI-MS (m/z): 409.3 [M+H]⁺.

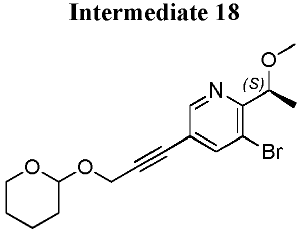

Intermediate 18 was prepared by the following steps:

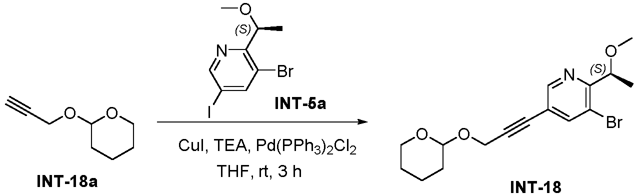

The **INT-5b** in the intermediate **INT-5** was replaced by **INT-18a,** and the compound **INT-18** can be obtained by a similar method and reaction steps. ESI-MS (m/z): 354.3 [M+H]⁺.

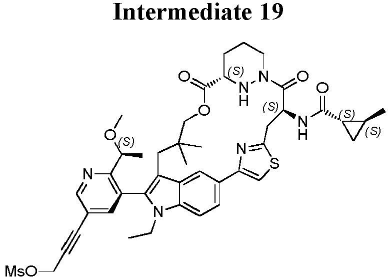

Intermediate 19 was prepared by the following steps:

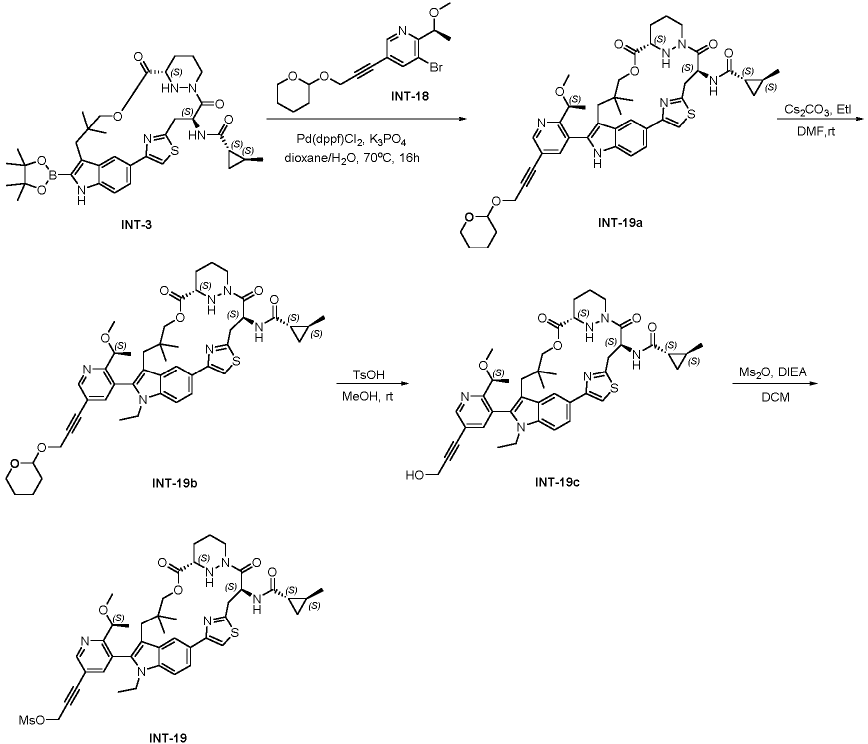

Step 1: Dissolve compound **INT-3** (300 mg, 0.44 mmol) in a mixed solution of 1,4-dioxane (5 mL) and water (1 mL), and add **INT-18** (170 mg, 0.48 mmol), [1,1'-bis(diphenylphosphino)ferrocene]palladium dichloride (32 mg, 0.04 mmol) and potassium phosphate (188 mg, 0.88 mmol) successively. The reaction mixture was stirred at 70°C for 16 hours under nitrogen protection. After the reaction was complete, the reaction solution was filtered with celite, and the concentrated residue was purified by silica gel column chromatography (dichloromethane/methanol = 20/1) to obtain light yellow oily compound **INT-19a** (260 mg, yield 71.2%) . ESI-MS (m/z): 823.1 [M+H]⁺.

Step 2: Dissolve compound **INT-19a** (260 mg, 0.31 mmol) in N,N-dimethylformamide (4 mL), add cesium carbonate (205 mg, 0.63 mmol) and ethyl iodide ( 145 mg, 0.93 mmol). The reaction mixture was stirred at room temperature for 16 hours. After the reaction was complete, water (20 mL) was added to the reaction system, the reaction solution was extracted with ethyl acetate (30 mL*2), the organic phases were combined, washed with saturated brine, dried over anhydrous sodium sulfate, filtered and concentrated to obtain a light yellow solid compound **INT-19b** (110 mg, yield 41.0%). ESI-MS (m/z): 851.2 [M+H]⁺.

Step 3: Compound **INT-19b** (110 mg, 0.13 mmol) was dissolved in methanol (3 mL), and p-toluenesulfonic acid monohydrate (123 mg, 0.65 mmol) was added thereto. The reaction mixture was stirred at room temperature for 3 hours. After the reaction was complete, water (30 mL) was added to the reaction system, the reaction solution was extracted with ethyl acetate (30 mL*2), the organic phases were combined, washed with saturated brine, dried over anhydrous sodium sulfate, filtered and concentrated to obtain a light yellow solid compound **INT-19c** (80 mg, yield 80.8%). ESI-MS (m/z): 767.5 [M+H]⁺.

Step 4: Dissolve compound **INT-19c** (80 mg, 0.10 mmol) in dichloromethane (4 mL), add methanesulfonic anhydride (54 mg, 0.31 mmol) and diisopropylethylamine (68 mg, 0.53 mmol). The reaction mixture was stirred at room temperature for 2 hours. After the reaction was complete, dichloromethane (30 mL) was added to the reaction system, the reaction solution was washed with water (15 mL*2) and saturated brine, the organic phase was dried over anhydrous sodium sulfate, filtered and concentrated to obtain light yellow solid compound **INT-19** (70 mg, yield 79.5%). ESI-MS (m/z): 845.5 [M+H]⁺.

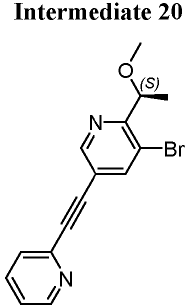

Intermediate 20 was prepared by the following steps:

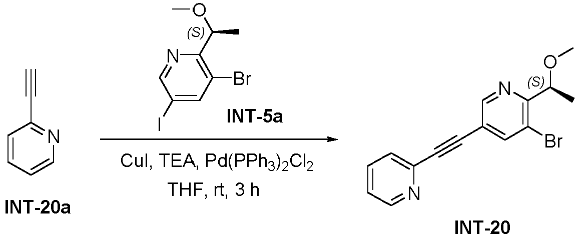

The **INT-5b** in the intermediate **INT-5** was replaced by **INT-20a,** and the compound **INT-20** can be obtained by a similar method and reaction steps. ESI-MS (m/z): 317.3 [M+H]⁺.

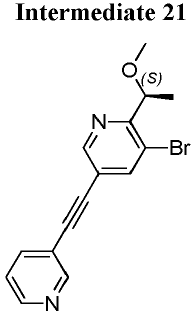

Intermediate 21 was prepared by the following steps:

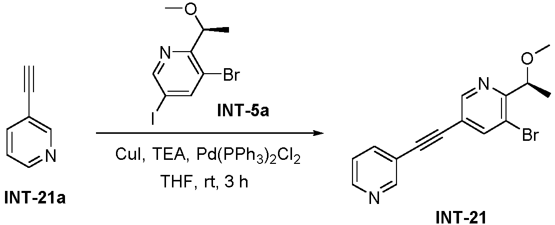

The compound **INT-21** can be obtained by using **INT-21a** to replace **INT-5b** in the intermediate **INT-5,** and using similar methods and reaction steps. ESI-MS (m/z): 317.3 [M+H]⁺.

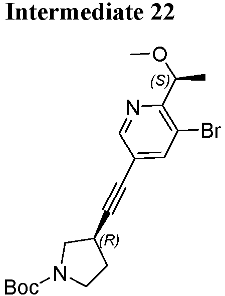

By replacing **INT-15a** in the intermediate **INT-15** with (S)-tert-butyl 3-formylpyrrolidine-1-carboxylate, compound **INT-22** can be obtained using similar methods and reaction steps. ESI-MS (m/z): 409.5 [M+H]⁺.

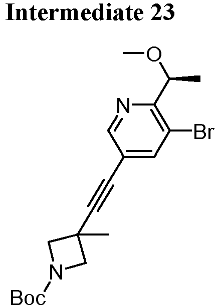

Intermediate 23 was prepared by the following steps:

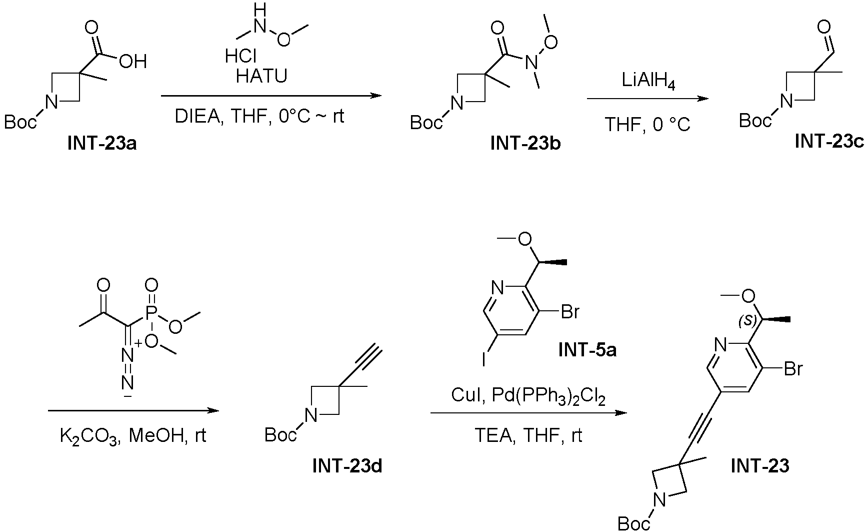

Step 1: Add 2-(7-Azobenzotriazole)-N,N,N',N'-tetramethyluronium hexafluorophosphate (2.12 g, 5.58 mmol) to a solution **of INT-23a** (1.0 g, 4.65 mmol) and N,N-diisopropylethylamine (13.8 g, 13.9 mmol) in tetrahydrofuran (15 mL) at 0°C. After stirring at the same temperature for 1.5 hours, methoxymethylamine hydrochloride (0.50 g, 5.11 mmol) was added to the reaction solution. After the mixture was heated to room temperature and stirred for 16 hours, a saturated aqueous ammonium chloride solution was added to quench the reaction, and the mixture was extracted with ethyl acetate. The organic phases were combined and washed with saturated brine and dried over anhydrous sodium sulfate. The organic phase was concentrated and purified by silica gel column chromatography (petroleum ether: ethyl acetate = 3/1) to obtain a colorless oily liquid **INT-23b** (1.14 g, yield 95.0%). ESI-MS (m/z): 259.5 [M+H]⁺.

Step 2: Under nitrogen atmosphere at 0°C, lithium aluminum tetrahydride (0.18 g, 4.85 mmol) was added in batches to a tetrahydrofuran solution (10 mL) of compound **INT-23b** (1.14 g, 4.41 mmol). After stirring at this temperature for 1 hour, the reaction was completed as monitored by LCMS. Water was added to quench the reaction, and the mixture was extracted with ethyl acetate. The organic phases were combined and washed with saturated brine, dried over anhydrous sodium sulfate, and concentrated to obtain a colorless oily liquid **INT-23c,** which was used directly in the next step without purification.

Step 3: The crude product **INT-23c** and potassium carbonate (1.22 g, 8.82 mmol) were added to methanol (10 mL), and dimethyl (1-diazo-2-oxopropyl)phosphonate (1.70 g, 8.82 mmol) was added dropwise at 0°C. The reaction solution was stirred at room temperature for 16 hours and the reaction was completed as monitored by TLC. Water was added to quench the reaction, and the product was extracted with ethyl acetate. The organic phases were combined and washed with saturated brine and dried over anhydrous sodium sulfate. The organic phase was concentrated and purified by silica gel column chromatography (petroleum ether:ethyl acetate = 6/1) to obtain a colorless oily liquid **INT-23d** (368 mg, yield 42.7%). ESI-MS (m/z): 196.4 [M+H]⁺.

Step 4: Under nitrogen atmosphere, (S)-3-bromo-5-iodo-2-(1-methoxyethyl)pyridine **INT-5a** (300 mg, 0.88 mmol), cuprous iodide (17 mg, 0.088 mmol), bistriphenylphosphine palladium dichloride (62 mg, 0.088 mmol), triethylamine (178 mg, 1.75 mmol), compound **INT-23d** (188 mg, 0.96 mmol) and tetrahydrofuran (2 mL) were added to the reaction bottle in sequence. The reaction solution was stirred at room temperature for 16 hours, and the reaction was completed as monitored by LCMS. The reaction solution was concentrated under reduced pressure and purified by silica gel column chromatography (petroleum ether/ethyl acetate = 3/1) to obtain a light yellow oily liquid **INT-23** (278 mg, two-step yield 77.4%). ESI-MS (m/z): 409.5 [M+H]⁺.

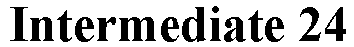

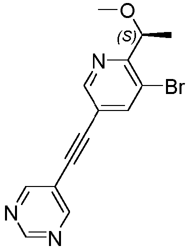

By replacing **INT-5b** in intermediate 5 with 5-ethynylpyrimidine, compound **INT-24** can be obtained using similar methods and reaction steps. ESI-MS (m/z): 318.4 [M+H]⁺.

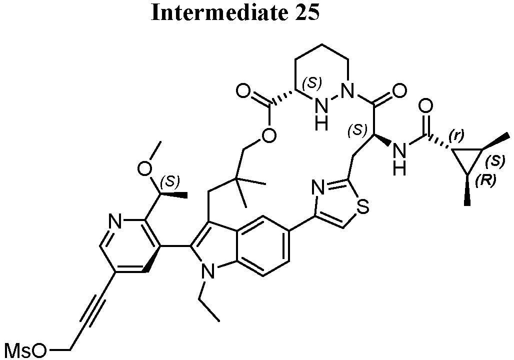

By replacing **INT-3** in the synthesis step of intermediate **INT-19** with INT-4, compound **INT-25** can be obtained using similar methods and reaction steps. ESI-MS (m/z): 859.7 [M+H]⁺.

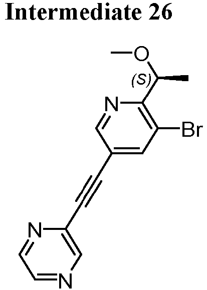

By replacing **INT-5b** in intermediate 5 with 2-ethynylpyrazine, compound **INT-26** can be obtained using similar methods and reaction steps. ESI-MS (m/z): 318.4 [M+H]⁺.

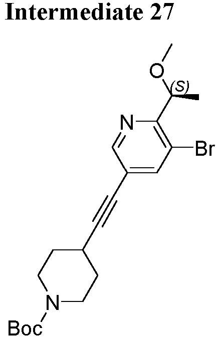

By replacing **INT-5b** in the intermediate **INT-5** with 1-Boc-4-ethynylpiperidine, compound **INT-27** can be obtained using similar methods and reaction steps. ESI-MS (m/z): 423.1 [M+H]⁺.

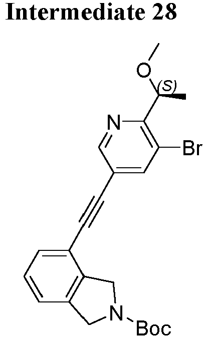

Intermediate 28 was prepared by the following steps:

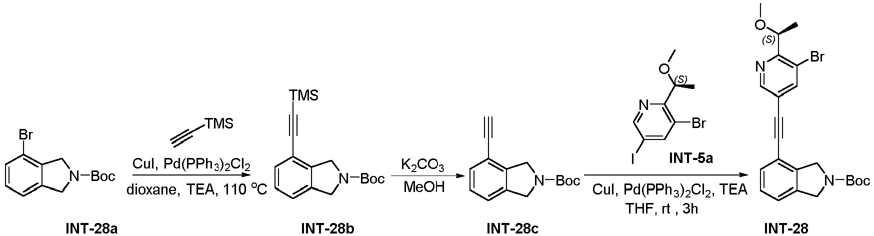

Step 1: Dissolve **INT-28a** (250 mg, 0.84 mmol), trimethylsilyl acetylene (412 mg, 4.19 mmol), cuprous iodide (16 mg, 0.084 mmol), and bistriphenylpalladium dichloride (59 mg, 0.084 mmol) in 1, 4-dioxane (1.5 mL) and triethylamine (1.5 mL). Stir the reaction solution at 110 °C overnight. After the reaction is completed, the reaction solution is filtered, and the filtrate is concentrated and spin-dried to obtain a crude product. The crude product was purified by column chromatography (petroleum ether/ethyl acetate = 20/1) to obtain a light yellow solid compound **INT-28b** (240 mg, yield 90.7%). ESI-MS (m/z): 316.3 [M+H]⁺.

Step 2: **INT-28b** (240 mg, 0.76 mmol) was dissolved in methanol (5 mL), potassium carbonate (526 mg, 3.8 mmol) was added at room temperature, and the reaction solution was stirred at room temperature for 4 h. After the reaction was completed, water (30 mL) was added, extracted with ethyl acetate (30 mL*2), the organic phases were combined, dried over anhydrous sodium sulfate, filtered, and the filtrate was concentrated and spin-dried to obtain a crude product. The crude product was purified by column chromatography (petroleum ether/ethyl acetate = 20/1) to obtain a light yellow solid compound **INT-28c** (170 mg, yield 91.9%). ESI-MS (m/z): 244.3 [M+H]⁺.

Step 3: Dissolve **INT-28c** (170 mg, 0.7 mmol), **INT-5a** (239 mg, 0.7 mmol), cuprous iodide (13 mg, 0.07 mmol), triethylamine (212 mg, 2.1 mmol), and bistriphenylpalladium dichloride (49 mg, 0.07 mmol) in tetrahydrofuran (5 mL), and stir the reaction solution at room temperature overnight. After the reaction is completed, the reaction solution is filtered, and the filtrate is concentrated and spin-dried to obtain a crude product. The crude product was purified by column chromatography (petroleum ether/ethyl acetate = 3/1) to obtain a light yellow oily compound **INT-28** (220 mg, yield 68.8%). ESI-MS (m/z): 457.3 [M+H]⁺.

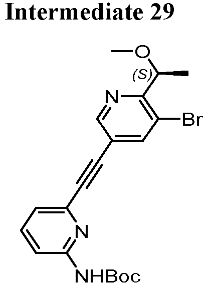

By replacing **INT-28a** in intermediate **INT-28** with 6-bromo-2-tert-butoxycarbonylaminopyridine, compound **INT-29** can be obtained using similar methods and reaction steps. ESI-MS (m/z): 432.3 [M+H]⁺.

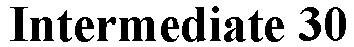

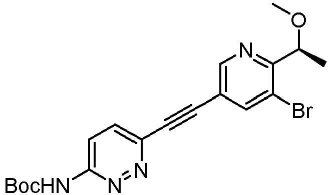

By replacing INT-28a in intermediate INT-28 with tert-butyl (6-iodopyridazin-3-yl)carbamate, compound **INT-30** can be obtained using similar methods and reaction steps. ESI-MS (m/z): 433.3 [M+H]⁺.

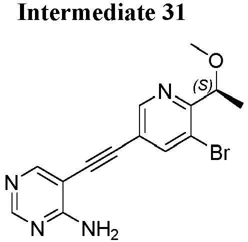

By replacing **INT-28a** in intermediate **28** with 4-amino-5-bromopyrimidine, compound **INT-31** can be obtained using similar methods and reaction steps. ESI-MS (m/z): 333.3 [M+H]⁺.

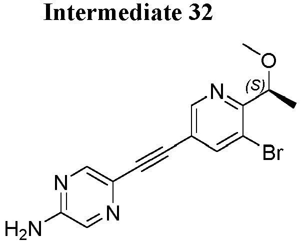

By replacing **INT-28a** in intermediate **28** with 2-amino-5-bromopyrazine, compound **INT-32** can be obtained using similar methods and reaction steps. ESI-MS (m/z): 333.3 [M+H]⁺.

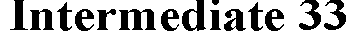

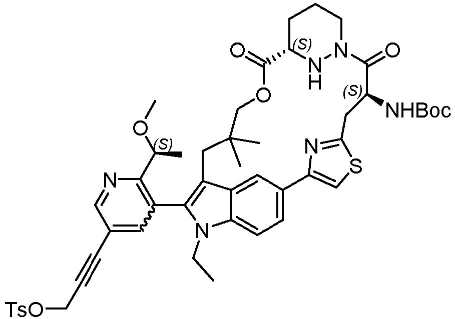

Intermediate 33 was prepared by the following steps:

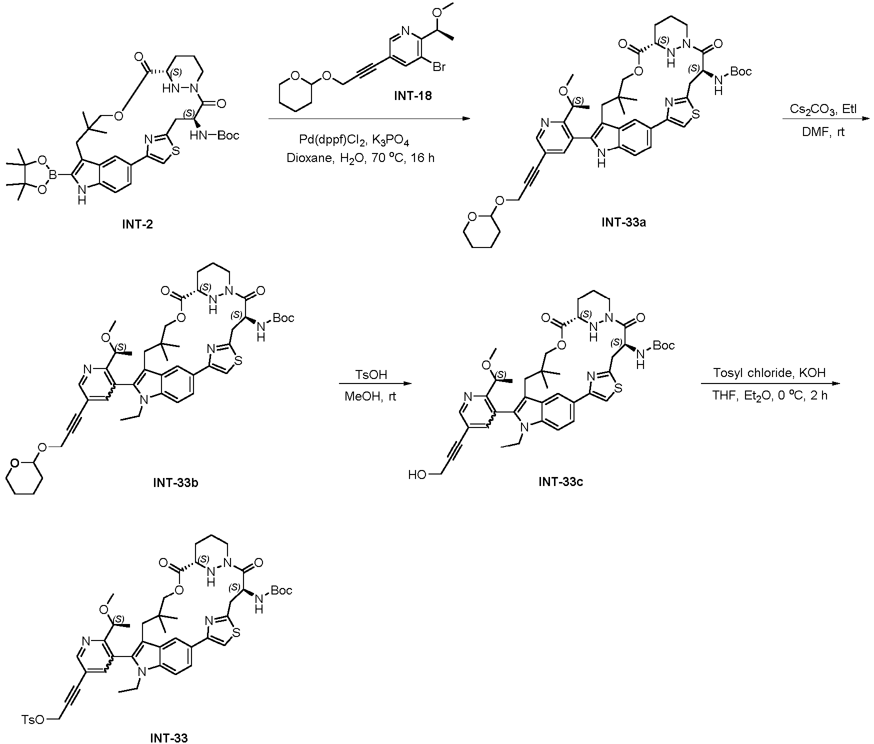

Step 1: Dissolve compound **INT-2** (300 mg, 0.43 mmol) in a mixed solution of 1,4-dioxane (5 mL) and water (1 mL), and add **INT-18** (170 mg, 0.48 mmol), [1,1'-bis(diphenylphosphino)ferrocene]palladium dichloride (32 mg, 0.04 mmol) and potassium phosphate (188 mg, 0.88 mmol) in sequence. The reaction mixture was stirred at 70°C for 16 hours under nitrogen protection. After the reaction was completed, the reaction solution was filtered through celite, and the concentrated residue was purified by silica gel column chromatography (dichloromethane/methanol = 20/1) to obtain a light yellow oil solid **INT-33a** (210 mg, yield 57%). ESI-MS (m/z): 841.6 [M+H]⁺.

Step 2: Compound **INT-33a** (200 mg, 0.28 mmol) was dissolved in N,N-dimethylformamide (3 mL), and cesium carbonate (232 mg, 0.71 mmol) and iodoethane (185 mg, 1.19 mmol) were added thereto. The reaction mixture was stirred at room temperature for 16 hours. After the reaction was complete, water (20 mL) was added to the reaction system, and the mixture was extracted with ethyl acetate (30 mL*2). The organic phases were combined and washed with saturated brine, dried over anhydrous sodium sulfate, filtered and concentrated to obtain a light yellow solid **INT-33b** (200 mg, yield 95%). ESI-MS (m/z): 869.8 [M+H]⁺.

Step 3: Compound **INT-33b** (200 mg, 0.23 mmol) was dissolved in methanol (3 mL), and p-toluenesulfonic acid monohydrate (175 mg, 0.92 mmol) was added thereto. The reaction mixture was stirred at room temperature for 3 hours. After the reaction was complete, water (30 mL) was added to the reaction system, and the mixture was extracted with ethyl acetate (30 mL*2). The organic phases were combined and washed with saturated brine, dried over anhydrous sodium sulfate, filtered and concentrated to obtain a yellow oily compound **INT-33c** (160 mg, yield 89%). ESI-MS (m/z): 785.6 [M+H]⁺.

Step 4: Compound **INT-33c** (140 mg, 0.18 mmol) was dissolved in tetrahydrofuran (3 mL) and diethyl ether (3 mL), and p-toluenesulfonyl chloride (68 mg, 0.36 mmol) and potassium hydroxide (20 mg, 0.36 mmol) were added thereto. The reaction mixture was stirred at 0°C for 2 hours. After the reaction was complete, dichloromethane (30 mL) was added to the reaction system, and the mixture was washed with water (15 mL*2) and saturated brine. The organic phase was dried over anhydrous sodium sulfate, filtered and concentrated. The residue was purified by preparative thin layer chromatography (dichloromethane/methanol = 20/1) to obtain **INT-33** (110 mg, yield 66%). ESI-MS (m/z): 939.9 [M+H]⁺.

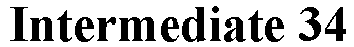

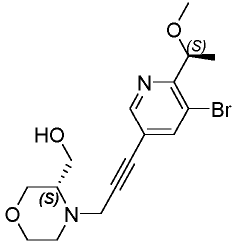

Intermediate 34 was prepared by the following steps:

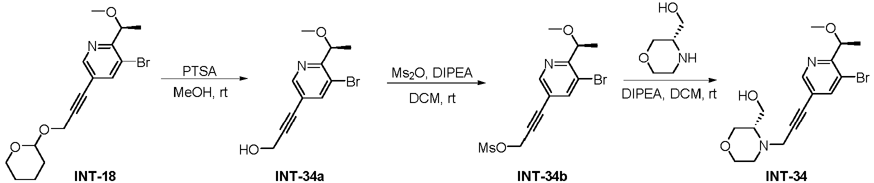

Step 1: Compound **INT-18** (500 mg, 1.41 mmol) was dissolved in methanol (5 mL), and p-toluenesulfonic acid monohydrate (537 mg, 2.82 mmol) was added thereto. The reaction mixture was stirred at room temperature for 3 hours. After the reaction was complete, water (30 mL) was added to the reaction system, and the mixture was extracted with ethyl acetate (30 mL*2). The organic phases were combined and washed with saturated brine, dried over anhydrous sodium sulfate, filtered and concentrated to obtain a light yellow solid compound **INT-34a** (210 mg, yield 55%). ESI-MS (m/z): 270.3 [M+H]⁺.

Step 2: Compound **INT-34a** (350 mg, 1.30 mmol) was dissolved in dichloromethane (5 mL), and methanesulfonic anhydride (1.13 g, 6.48 mmol) and diisopropylethylamine (1.34 g, 10.37 mmol) were added thereto. The reaction mixture was stirred at room temperature for 2 hours. After the reaction was complete, water (30 mL) was added to the reaction system, and the mixture was extracted with dichloromethane (30 mL*2). The organic phases were combined, dried over anhydrous sodium sulfate, filtered and concentrated to obtain a light yellow solid compound **INT-34b** (330 mg, yield 73%). ESI-MS (m/z): 348.2 [M+H]⁺.

Step 3: Compound **INT-34b** (200 mg, 0.58 mmol) and (S)-3-hydroxymethylmorpholine (87 mg, 0.75 mmol) were dissolved in dichloromethane (5 mL), and N,N-diisopropylethylamine (148 mg, 1.15 mmol) was added thereto. The reaction mixture was stirred at room temperature for 4 hours. After the reaction was complete, water (30 mL) was added to the reaction system, extracted with dichloromethane (30 mL*2), the organic phases were combined, washed with saturated brine, dried over anhydrous sodium sulfate, filtered and concentrated, and the residue was purified by silica gel column chromatography (dichloromethane/methanol = 10/1) to obtain colorless oily liquid **INT-34** (180 mg, yield 85%). ESI-MS (m/z): 369.3 [M+H]⁺.

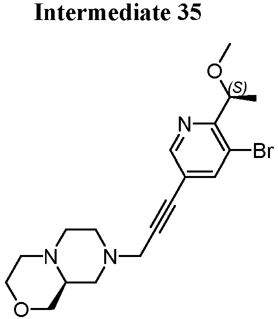

By replacing the (S)-3-hydroxymethylmorpholine in the intermediate **INT-34** with (S)-octahydropyrazine[2,1-c][1,4]oxazine dihydrochloride, compound **INT-35** can be obtained using similar methods and reaction steps. ESI-MS (m/z):
394.5[M+H]⁺.

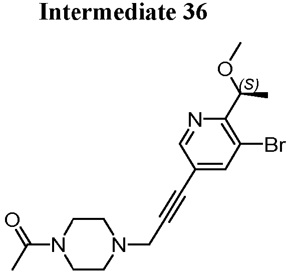

By replacing the (S)-3-hydroxymethylmorpholine in the intermediate **INT-34** with 1-acetylpiperazine, compound INT-36 can be obtained using similar methods and reaction steps. ESI-MS (m/z): 380.4 [M+H]⁺.

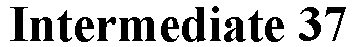

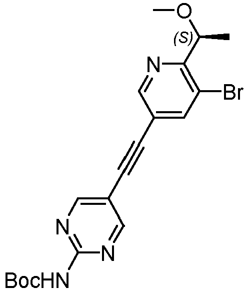

By replacing **INT-28a** in intermediate **INT-28** with N-Boc-2-amino-5-bromopyrimidine, compound **INT-37** can be obtained using similar methods and reaction steps. ESI-MS (m/z): 433.4 [M+H]⁺.

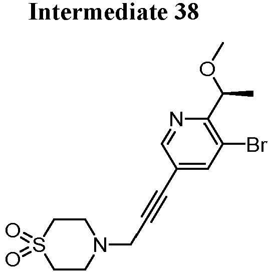

By replacing **INT-5b** in the intermediate **INT-5** with 4-propargylthiomorpholine-1,1-dioxide, compound **INT-38** can be obtained using similar methods and reaction steps. ESI-MS (m/z): 387.5 [M+H]⁺.

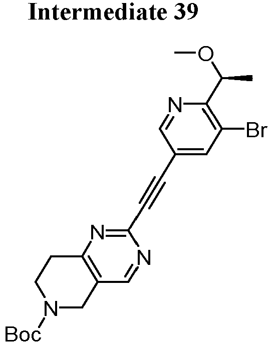

By replacing **INT-28a** in intermediate **INT-28** with tert-butyl 2-chloro-7,8-dihydropyrido[4,3-d]pyrimidine-6(5H)-carboxylate, compound **INT-39** can be obtained using similar methods and reaction steps. ESI-MS (m/z): 473.3 [M+H]⁺.

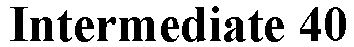

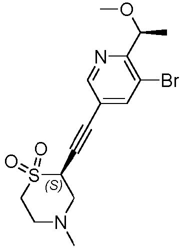

Intermediate 40 was prepared by the following steps:

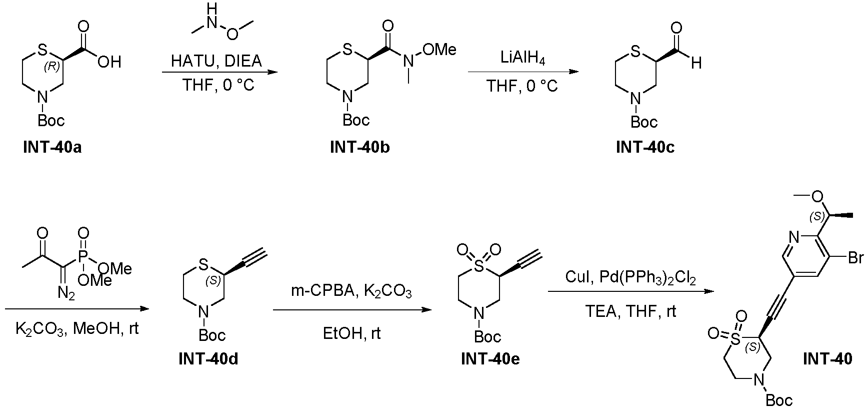

Step 1: At 0 °C, add 2-(7-azobenzotriazole)-N,N,N',N'-tetramethyluronium hexafluorophosphate (1.48 g, 3.88 mmol) to a solution of **INT-40a** (800 mg, 3.23 mmol) and N,N-diisopropylethylamine (1.25 g, 9.70 mmol) in tetrahydrofuran (10 mL). After stirring at the same temperature for 1.5 hours, methoxymethylamine hydrochloride (347 mg, 3.56 mmol) was added to the reaction solution. After the mixture was heated to room temperature and stirred for 16 hours, a saturated aqueous ammonium chloride solution was added to quench the reaction, and the mixture was extracted with ethyl acetate. The organic phases were combined and washed with saturated brine and dried over anhydrous sodium sulfate. The organic phase was concentrated and purified by silica gel column chromatography (petroleum ether: ethyl acetate = 3/1) to obtain a colorless oily liquid **INT-40b** (696 mg, yield 74.1%). ESI-MS (m/z): 291.5 [M+H]⁺.

Step 2: Under nitrogen atmosphere and 0 °C, lithium aluminum tetrahydride (211 mg, 5.56 mmol) was added in batches to a tetrahydrofuran solution (15 mL) of compound **INT-40b** (1.47 g, 5.06 mmol). After stirring at this temperature for 3 hour, the reaction was completed as monitored by LCMS. Water was added to quench the reaction, and the mixture was extracted with ethyl acetate. The organic phases were combined and washed with saturated brine, dried over anhydrous sodium sulfate, and the organic phase was concentrated. After concentration of the organic phase, the crude product of compound **INT-40c** was obtained, which was directly used in the next reaction without purification.

Step 3: Add the crude product **INT-40c** and potassium carbonate (1.40 g, 10.1 mmol) into methanol (15 mL), and add dimethyl (1-diazo-2-oxopropyl)phosphonate (1.94 g, 10.1 mmol) dropwise at 0 °C. The reaction solution was stirred at room temperature for 16 hours and the reaction was completed as monitored by TLC. Water was added to quench the reaction, and the product was extracted with ethyl acetate. The organic phases were combined and washed with saturated brine and dried over anhydrous sodium sulfate. The organic phase was concentrated and purified by silica gel column chromatography (petroleum ether:ethyl acetate = 6:1) to obtain a colorless, transparent oily compound **INT-40d** (767 mg, two-step yield 66.7%).

Step 4: m-Chloroperbenzoic acid (399 mg, 2.31 mmol) and potassium carbonate (319 mg, 2.31 mmol) were added sequentially to a solution of **INT-40d** (150 mg, 0.66 mmol) in ethanol (6 mL). The reaction solution was stirred at room temperature for 8 hours. The reaction was completed after monitoring by TLC. Saturated sodium bicarbonate and sodium thiosulfate aqueous solutions were added to quench the reaction. The mixture was extracted with ethyl acetate. The organic phases were combined and washed with saturated brine and dried over anhydrous sodium sulfate. The organic phase was concentrated to obtain a crude product of compound **INT-40e,** which was used directly in the next step without purification.

Step 5: Under nitrogen atmosphere, (S)-3-bromo-5-iodo-2-(1-methoxyethyl)pyridine **INT-5a** (196 mg, 0.57 mmol), cuprous iodide (11 mg, 0.057 mmol), bistriphenylphosphine palladium dichloride (40 mg, 0.057 mmol), and triethylamine (116 mg, 1.15 mmol) were added to a solution of the crude product **INT-40e** in tetrahydrofuran (5 mL) in sequence. The reaction solution was stirred at room temperature for 16 hours, and the reaction was completed as monitored by LCMS. The reaction solution was concentrated under reduced pressure and purified by silica gel column chromatography (petroleum ether/ethyl acetate = 2/1) to obtain a yellow solid **INT-40** (227 mg, yield 83.7%). ESI-MS (m/z): 473.3 [M+H]⁺.

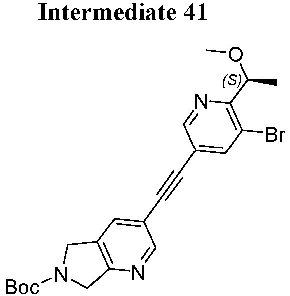

By replacing **INT-28a** in intermediate **INT-28** with tert-butyl-3-bromo-5H,6H, 7H-pyrrolo[3,4-b]pyridine-6-carboxylate, compound **INT-41** can be obtained using similar methods and reaction steps. ESI-MS (m/z): 458.4 [M+H]⁺.

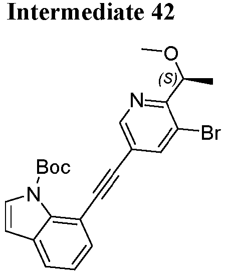

By replacing **INT-28a** in intermediate **INT-28** with 1-BOC-7-bromoindole, compound INT-42 can be obtained using similar methods and reaction steps. ESI-MS (m/z): 455.3 [M+H]⁺.

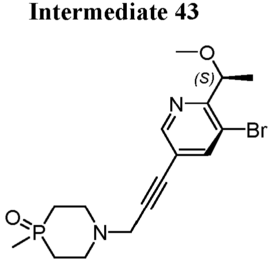

Intermediate 43 was prepared by the following steps:
By replacing the (S)-3-hydroxymethylmorpholine in the intermediate **INT-34** with 4-methyl-1,4-azaphosphane 4-oxide, compound **INT-43** can be obtained using similar methods and reaction steps. ESI-MS (m/z): 386.2 [M+H]⁺.

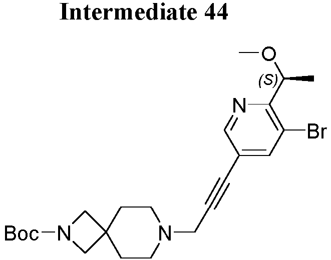

By replacing the (S)-3-hydroxymethylmorpholine in the intermediate **INT-34** with 2-tert-butoxycarbonyl-2,7-diazaspiro[3.5]nonane hydrochloride, compound **INT-44** can be obtained using similar methods and reaction steps. ESI-MS (m/z): 478.4 [M+H]⁺.

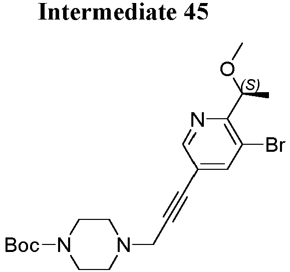

By replacing the (S)-3-hydroxymethylmorpholine in the intermediate **INT-34** with 1-tert-butyloxycarbonylpiperazine, compound **INT-45** can be obtained using similar methods and reaction steps. ESI-MS (m/z): 437.4 [M+H]⁺. 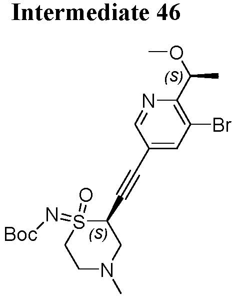 Intermediate 46 was prepared by the following steps:

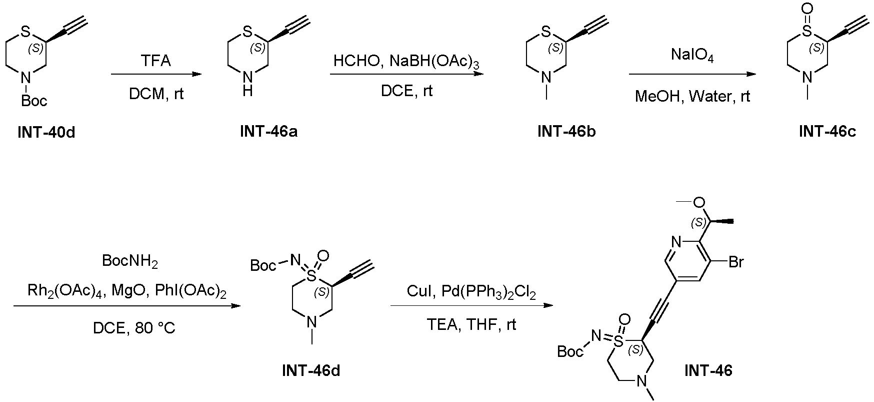

Step 1: Compound **INT-40d** (485 mg, 2.31 mmol) was dissolved in dichloromethane (5 mL), and trifluoroacetic acid (1.5 mL) was added dropwise thereto. The reaction solution was stirred at room temperature for 30 minutes. The reaction mixture was concentrated under reduced pressure to obtain compound **INT-46a.** ESI-MS (m/z): 128.3 [M+H]⁺.

Step 2: The above product **INT-46a** was dissolved in 1,2-dichloroethane (7 mL), and formaldehyde aqueous solution (519 mg, 6.39 mmol, 37% w/w) was added dropwise thereto. After reacting for 20 minutes, sodium triacetoxyborohydride (1.81 g, 8.52 mmol) was added. The reaction solution was stirred at room temperature for 30 minutes, and the reaction was completed after monitoring by LCMS. Saturated aqueous sodium bicarbonate solution was added to quench the reaction, and the product was extracted with ethyl acetate. The organic phases were combined and washed with saturated brine and dried over anhydrous sodium sulfate. The organic phase was concentrated and purified by silica gel column chromatography (dichloromethane/methanol = 20/1) to obtain a colorless oily liquid **INT-46b** (211 mg, two-step yield 70.1%). ESI-MS (m/z): 142.1 [M+H]⁺.

Step 3: Sodium periodate (479 mg, 2.24 mmol) was added to a mixed solution of **INT-46b** (211 mg, 1.49 mmol) in methanol (6 mL) and water (2 mL). The reaction solution was stirred at room temperature for 6 hours. The reaction was completed as monitored by TLC, and a sodium thiosulfate aqueous solution was added to quench the reaction. The reaction was extracted with ethyl acetate, and the organic phases were combined and washed with saturated brine and dried over anhydrous sodium sulfate. The organic phase was concentrated and purified by silica gel column chromatography (dichloromethane/methanol = 15/1) to obtain a colorless oily liquid **INT-46c** (171 mg, yield 72.8%). ESI-MS (m/z): 158.3 [M+H]⁺.

Step 4: Add tert-butyl carbamate (255 mg, 2.18 mmol), rhodium acetate (15 mg, 0.054 mmol), magnesium oxide (175 mg, 4.35 mmol), iodobenzene acetate (525 mg, 1.63 mmol), compound **INT-46c** (171 mg, 1.09 mmol) and 1,2-dichloroethane (5 mL) into the reaction flask. The reaction solution was stirred at 80°C for 48 hours. The reaction solution was filtered through diatomaceous earth, and the filtrate was extracted with a mixture of dichloromethane and methanol. The organic phases were combined and washed with saturated brine and dried over anhydrous sodium sulfate. The organic phase was concentrated and purified by silica gel column chromatography (dichloromethane/methanol = 15/1) to obtain compound **INT-46d** (219 mg, yield 73.9%). ESI-MS (m/z): 273.4 [M+H]⁺.

Step 5: (S)-3-bromo-5-iodo-2-(1-methoxyethyl)pyridine **INT-5a** (212 mg, 0.62 mmol), cuprous iodide (12 mg, 0.062 mmol), bistriphenylphosphine palladium dichloride (44 mg, 0.062 mmol), triethylamine (125 mg, 1.24 mmol), compound **INT-46d** (219 mg, 0.80 mmol) and tetrahydrofuran (5 mL) were added to the reaction bottle in sequence. After nitrogen was replaced in the reaction solution, the mixture was stirred at room temperature for 16 hours. The reaction was completed as monitored by LCMS. The reaction solution was concentrated under reduced pressure and purified by silica gel column chromatography (dichloromethane/methanol = 20/1) to obtain yellow solid **INT-46** (179 mg, yield 59.5%). ESI-MS (m/z): 486.3 [M+H]⁺.

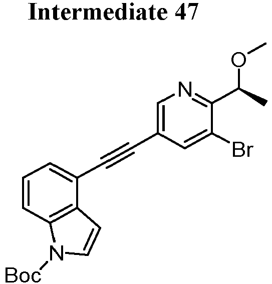

By replacing **INT-28a** in intermediate **INT-28** with 1-BOC-4-bromoindole, compound **INT-47** can be obtained using similar methods and reaction steps. ESI-MS (m/z): 455.3[M+H]⁺. 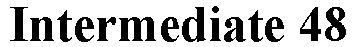 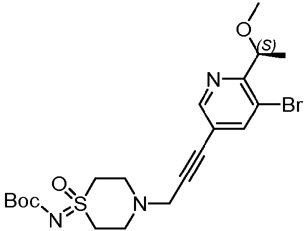 Intermediate 48 was prepared by the following steps:

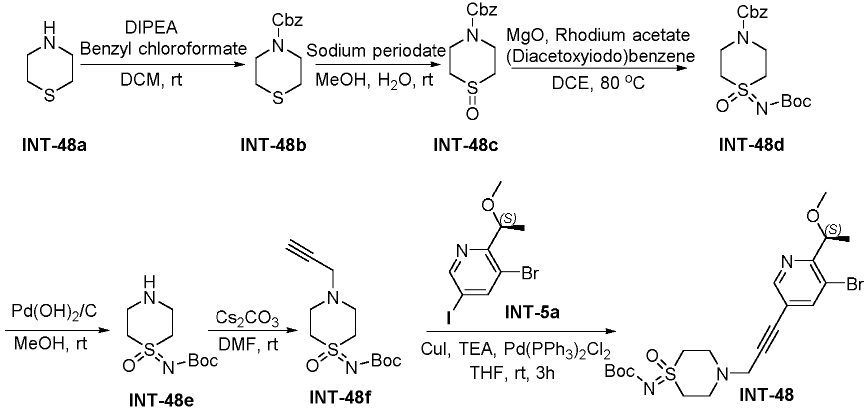

Step 1: Dissolve thiomorpholine **INT-48a** (4.0 g, 38.8 mmol), benzyl chloroformate (7.94 g, 46.5 mmol), and N,N-diisopropylethylamine (15.0 g, 116 mmol) in dichloromethane (40 mL), and stir the reaction solution at room temperature overnight. After the reaction was completed, the reaction solution was extracted with dichloromethane, and the organic phase was dried and concentrated to obtain a crude product. The crude product was purified by column chromatography (petroleum ether/ethyl acetate = 1/1) to obtain a light yellow oily compound **INT-48b** (9.0 g, yield 97.8%). ESI-MS (m/z): 238.3 [M+H]⁺.

Step 2: Dissolve compound **INT-48b** (1.0 g, 38.8 mmol) in a mixed solution of methanol (10 mL) and water (5 mL), add sodium periodate (1.17 g, 5.5 mmol) under ice bath, and stir the reaction solution at room temperature overnight. After the reaction was completed, the reaction solution was extracted with ethyl acetate, and the organic phase was dried and concentrated to obtain a crude product **INT-48c** (900 mg, yield 84.3%). The crude product was used directly in the next reaction without purification. ESI-MS (m/z): 254.3 [M+H]⁺.

Step 3: Dissolve compound INT-48c (850 mg, 3.36 mmol) in 1,2-dichloroethane (15 mL), add tert-butyl carbamate (1.97 g, 16.8 mmol), rhodium acetate (94 mg, 0.34 mmol), magnesium oxide (541 mg, 13.4 mmol) and iodophenyl diacetic acid (3.24 g, 10.1 mmol) at room temperature, and stir the reaction solution at 80 °C overnight. After the reaction was completed, the reaction solution was concentrated. The crude product was purified by column chromatography (dichloromethane/methanol = 10/1) to obtain a light yellow solid compound **INT-48d** (1.0 g, yield 80.9%). ESI-MS (m/z): 369.3 [M+H]⁺.

Step 4: Dissolve compound INT-48d (850 mg, 2.31 mmol) in methanol (10 mL) and add palladium hydroxide/carbon (85 mg, 10% w/w). The reaction solution was stirred at room temperature overnight under a hydrogen atmosphere. After the reaction was completed, the mixture was filtered through celite and the filtrate was concentrated to obtain a light yellow oily compound **INT-48e** (410 mg, yield 75.9%). ESI-MS (m/z): 235.3 [M+H]⁺.

Step 5: Dissolve compound **INT-48e** (150 mg, 0.64 mmol) in N,N-dimethylformamide (3 mL), add cesium carbonate (626 mg, 1.92 mmol) and 3-bromopropyne (114 mg, 0.96 mmol) at room temperature, and stir the reaction solution at room temperature overnight. After the reaction was completed, the reaction solution was extracted with ethyl acetate, and the organic phase was dried and concentrated to obtain a crude product. The crude product was purified by column chromatography (petroleum ether/ethyl acetate = 1/1) to obtain a light yellow solid compound **INT-48f** (135 mg, yield 77.4%). ESI-MS (m/z): 273.3 [M+H]⁺.

Step 6: Dissolve compound **INT-48f** (140 mg, 0.51 mmol) in tetrahydrofuran (5 mL), add **INT-5a** (176 mg, 0.51 mmol), cuprous iodide (10 mg, 0.051 mmol), bistriphenylphosphine palladium dichloride (36 mg, 0.051 mmol) and triethylamine (104 mg, 1.03 mmol) at room temperature, and stir the reaction solution at room temperature overnight. After the reaction was completed, the reaction solution was concentrated. The crude product was purified by column chromatography (dichloromethane/methanol = 10/1) to obtain a light yellow solid compound **INT-48** (60 mg, yield 24.0%). ESI-MS (m/z): 486.5 [M+H]⁺.

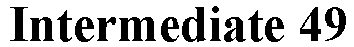

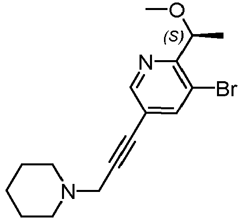

By replacing **INT-6a** in the intermediate **INT-6** with piperidine, compound INT-49 can be obtained using similar methods and reaction steps. ESI-MS (m/z): 337.4 [M+H]⁺.

The synthetic method of embodiment compound among the present invention is as follows:

### Example 1

### (1S,2S)-N-((63S,4S,Z)-11-ethyl-12-(2-((S)-1-methoxyethyl)-5-(3-morpholinoprop-1-yn-1-yl )pyridin-3-yl)-10,10-dimethyl-5,7-dioxo-61,62,63,64,65,66-hexahydro-11H-8-oxa-2(4,2)-thiazola-1 (5,3)-indola-6(1,3)-pyridazinacyclonecaphane-4-yl)-2-methylcyclopropane-1-carboxamide

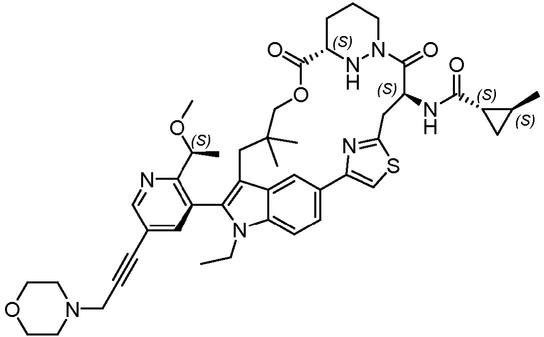

Example 1 is prepared by the following steps:

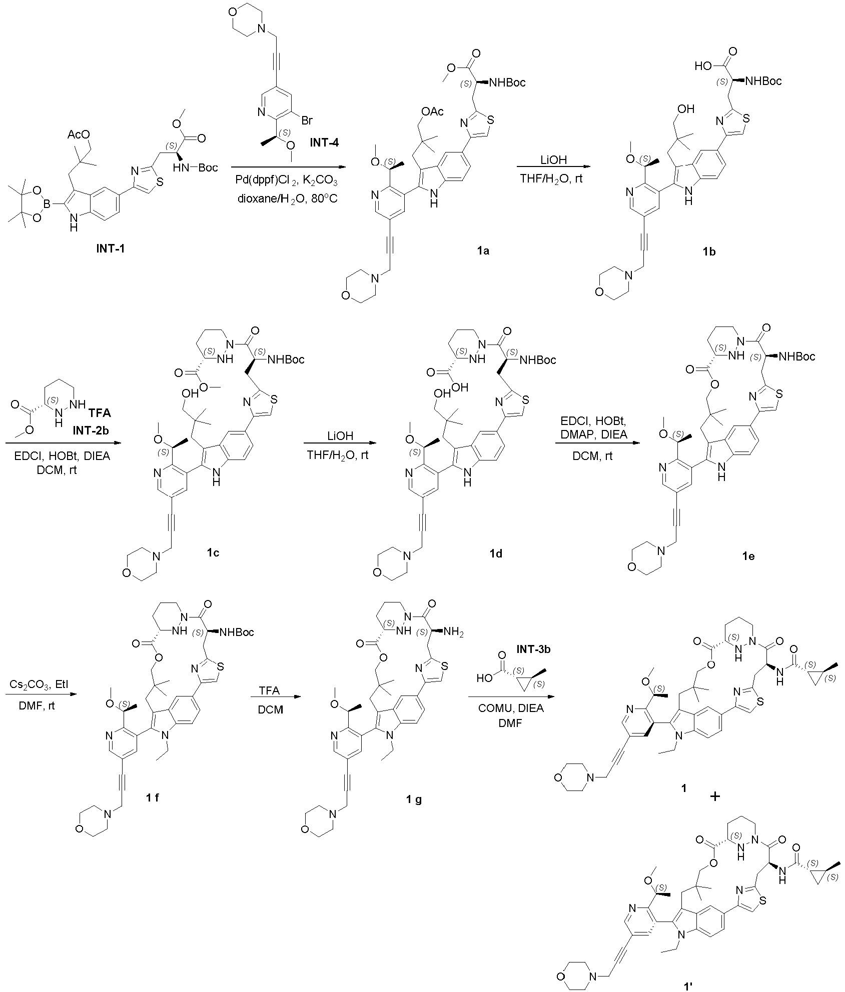

Step 1: Dissolve compound **INT-4** (600 mg, 1.77 mmol) in a mixed solution of 1,4-dioxane (10 mL) and water (1 mL), and successively add **INT-1** (1.16 g, 1.77 mmol), [1,1'-bis(diphenylphosphino)ferrocene]palladium dichloride (129.3 mg, 0.177 mmol) and potassium carbonate (1.18 g, 4.42 mmol). The reaction mixture was stirred at 80 °C for 16 hours under the protection of nitrogen. After the reaction was complete, the reaction solution was filtered with celite and concentrated to obtain the black crude compound **1a** (1.39 g, yield 100%), which was directly used in the next reaction without purification. ESI-MS (m/z): 788.1 [M+H]⁺.

Step 2: the crude compound **1a** (1.39 g, 1.77 mmol) was dissolved in a mixed solution of tetrahydrofuran (3 mL) and water (3 mL), and lithium hydroxide (211.2 mg, 8.82 mmol) was added thereto. The reaction mixture was stirred at room temperature for 3 hours. After the reaction was complete, adjust the pH of the reaction solution to 6 with 6N hydrochloric acid solution, the reaction solution was extracted with ethyl acetate (40 mL*2), the organic phases were combined, washed with saturated brine, dried over anhydrous sodium sulfate, and concentrated by filtration. The residue was purified by silica gel column chromatography (dichloromethane/methanol = 30/1) to obtain compound **1b** as a pale yellow solid (1.0 g, yield 77.3%). ESI-MS (m/z): 732.1 [M+H]⁺.

Step 3: Compound **1b** (1.0 g, 1.37 mmol) was dissolved in dichloromethane (20 mL), and **INT-2b** (1.01 g, 2.73 mmol, TFA salt), 1-(3-dimethylamino propyl)-3-ethylcarbodiimide hydrochloride (523.9 mg, 2.73 mmol), 1-hydroxybenzotriazole (369.2 mg, 2.73 mmol) and N,N diisopropylethylamine (882.9 mg , 6.83 mmol) were added thereto. The reaction mixture was stirred at room temperature for 16 hours. After the reaction was complete, water (50 mL) was added to the reaction system, the reaction system was extracted with dichloromethane (50 mL*2), the organic phases were combined, washed with saturated brine, dried over anhydrous sodium sulfate, concentrated by filtration, and the residue was purified by silica gel column chromatography (dichloromethane/methanol = 30/1) to obtain compound **1c** (700 mg, yield 59.7%) as a pale yellow solid. ESI-MS (m/z): 858.2 [M+H]⁺.

Step 4: Compound 1c (700 mg, 0.815 mmol) was dissolved in a mixed solution of tetrahydrofuran (3 mL) and water (3 mL), and lithium hydroxide (195.4 mg, 8.16 mmol) was added thereto. The reaction mixture was stirred at room temperature for 4 hours. After the reaction was complete, adjust the pH of the reaction solution to 6 with 6N hydrochloric acid solution. The reaction solution was extracted with ethyl acetate (40mL*2), the organic phases were combined, washed with saturated brine, dried over anhydrous sodium sulfate, filtered and concentrated to obtain a light yellow solid compound **1d** (640 mg, yield 93.0%). ESI-MS (m/z): 844.2 [M+H]⁺.

Step 5: Dissolve compound 1d (370 mg, 0.44 mmol) in dichloromethane (8 mL), add thereto 1-(3-dimethylaminopropyl)-3-ethylcarbodiimide hydrochloride (840.4 mg, 4.4 mmol), 1-hydroxybenzotriazole (296.2 mg, 2.2 mmol), 4-dimethylaminopyridine (267.8 mg, 2.2 mmol) and N,N diisopropylethylamine (849.8 mg , 6.6 mmol). The reaction mixture was stirred at room temperature for 16 hours. After the reaction was complete, water (30 mL) was added to the reaction system. The reaction solution was extracted with dichloromethane (30 mL*2), the organic phases were combined, washed with saturated brine, dried over anhydrous sodium sulfate, concentrated by filtration, and the residue was purified by silica gel column chromatography (dichloromethane/methanol = 30/1) to obtain compound **1e** as a pale yellow solid (120 mg, yield 33.1%). ESI-MS (m/z): 826.2 [M+H]⁺.

Step 6: Dissolve compound **1e** (150 mg, 0.18 mmol) in N,N-dimethylformamide (3 mL), add cesium carbonate (118.3 mg, 0.36 mmol) and iodoethane (42.5 mg, 0.27 mmol). The reaction mixture was stirred at room temperature for 16 hours. After the reaction was complete, water (30 mL) was added to the reaction system, extracted with ethyl acetate (30 mL*2), the organic phases were combined, washed with saturated brine, dried over anhydrous sodium sulfate, filtered and concentrated to obtain a light yellow solid compound 1f (150 mg, yield 96.7%). ESI-MS (m/z): 854.2 [M+H]⁺.

Step 7: Compound **1f** (120 mg, 0.14 mmol) was dissolved in dichloromethane (2 mL), and trifluoroacetic acid (0.5 mL) was added thereto. The reaction mixture was stirred at room temperature for 2 hours. After the reaction was complete, add saturated sodium bicarbonate solution (30 mL) to the reaction system under ice bath. The reaction solution was extracted with dichloromethane (30 mL*2), the organic phases were combined, washed with saturated brine, and dried over anhydrous sodium sulfate, filtered and concentrated to obtain light yellow solid compound **1g** (100 mg, yield 94.4%). ESI-MS (m/z): 754.2 [M+H]⁺.

Step 8: Dissolve compound **1g** (70 mg, 0.093 mmol) in N,N-dimethylformamide (3 mL), add thereto (1S,2S)-2-methylcyclopropanecarboxylic acid **INT- 3b** (18.6 mg, 0.186 mmol), diisopropylethylamine (36.0 mg, 0.278 mmol) and (1-Cyano-2-ethoxy-2-oxoethylidenaminooxy)dimethylamino-morpholino-carbenium hexafluorophosphate (79.5 mg, 0.186 mmol). The reaction mixture was stirred under ice bath for 1 hour. After the reaction was complete, water (20 mL) was added to the reaction system. The reaction solution was extracted with ethyl acetate (30 mL*2), the organic phases were combined, washed with saturated brine, dried over anhydrous sodium sulfate, concentrated by filtration, and the residue was purified by preparative liquid chromatography to obtain white solid compound **1** (15 mg, yield 19.3%) and epimer 1' (20 mg, yield 25.8%). The absolute configurations drawn by the two compounds are based on empirical assumptions. In the existing analytical methods, 1 is a compound with relatively low polarity and relatively long retention time in LC-MS and HPLC, and 1' is compound with relatively high polarity and relatively short retention time in LC-MS and HPLC.

### Compound 1:

ESI-MS (m/z): 836.5 [M+H]⁺; LC-MS retention time RT=1.80 min. HPLC retention time RT=11.63 min.

¹H NMR (500 MHz, DMSO-d6) δ 8.81 (d, J = 2.0 Hz, 1H), 8.54 - 8.48 (m, 2H), 7.86 (d, J = 2.0 Hz, 1H), 7.81 (s, 1H) , 7.77 - 7.73 (m, 1H), 7.58 (m, 1H), 5.56 (t, J = 9.0 Hz, 1H), 5.10 - 5.04 (m, 1H), 4.36 - 4.15 (m, 4H), 4.14 - 4.01 (m, 1H), 3.61 (t, J = 5.0 Hz, 4H), 3.59 - 3.53 (m, 4H), 3.32 - 3.29 (m, 1H), 3.25 (s, 3H), 3.18 - 3.11 (m, 1H ), 3.00 - 2.94 (m, 1H), 2.79 - 2.73 (m, 1H), 2.56 - 2.52 (m, 4H), 2.40 - 2.35 (m, 1H), 2.11 - 2.06 (m, 1H), 1.85 - 1.73 (m, 2H), 1.57 - 1.47 (m, 2H), 1.35 (d, J = 6.0 Hz, 3H), 1.11 - 1.03 (m, 4H), 0.91 (s, 3H), 0.90 - 0.85 (m, 4H), 0.59 - 0.53 (m, 1H), 0.34 (s, 3H).

### Compound 1':

ESI-MS (m/z): 836.5 [M+H]⁺; LC-MS retention time RT=1.77 min. HPLC retention time RT=11.27 min.

¹H NMR (500 MHz, DMSO-d6) δ 8.83 (d, J = 2.0 Hz, 1H), 8.56 - 8.51 (m, 2H), 8.00 (d, J = 2.0 Hz, 1H), 7.81 (s, 1H) , 7.77 - 7.71 (m,1H), 7.57 - 7.52 (m, 1H), 5.55 (t, J = 9.0 Hz, 1H), 5.10 - 5.04 (m, 1H), 4.28 - 4.17 (m, 2H), 4.00 - 3.91 (m, 2H), 3.88 - 3.79 (m, 1H), 3.69 - 3.53 (m, 9H), 3.18 - 3.13 (m, 1H), 3.10 (s, 3H), 3.07 - 3.02 (m, 1H) , 2.81 - 2.74 (m, 1H), 2.57 - 2.52 (m, 4H), 2.35 - 2.29 (m, 1H), 2.16 - 2.07 (m, 1H), 1.84 - 1.76 (m, 2H), 1.56 - 1.48 (m, 2H), 1.22 (d, J = 6.0 Hz, 3H), 1.11 (t, J = 7.0 Hz, 3H), 1.08 - 1.05 (m, 4H), 0.98 - 0.92 (m, 3H), 0.90 - 0.85 (m, 1H), 0.58 - 0.54 (m, 1H), 0.50 (s, 3H).

### Example 2

### (1r,2R,3S)-N-((63S,4S,Z)-11-ethyl-12-(2-((S)-1-methoxyethyl)-5-(3-morpholinoprop-1-yn-1 -yl)pyridin-3-yl)-10,10-dimethyl-5,7-dioxo-61,62,63,64,65,66-hexahydro-11H-8-oxa-2(4,2)-thiazola -1(5,3)-indola-6(1,3)-pyridazinacycloundecaphane-4-yl)-2,3-dimethylcyclopropane-1-carboxamide

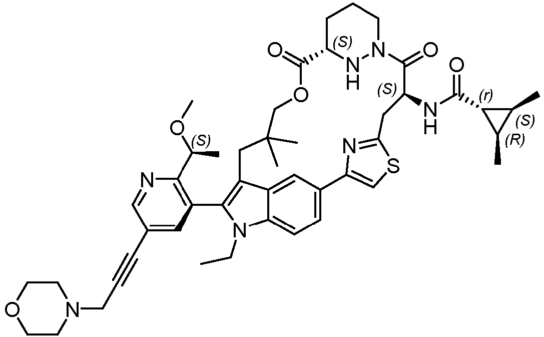

Example 2 is prepared by the following steps:

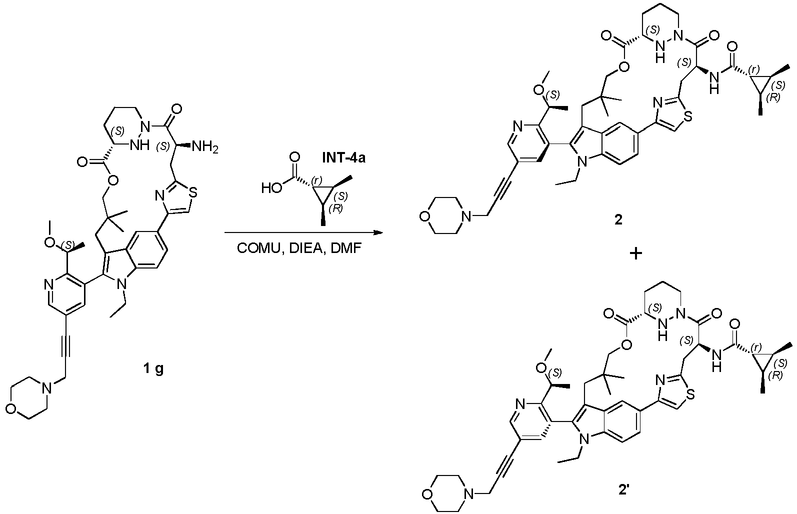

Step 1: Dissolve compound **1g** (30 mg, 0.04 mmol) in N,N-dimethylformamide (3 mL), add (2R,3S)-2,3-dimethylcyclopropanecarboxylic acid **2a** (9.1 mg, 0.08 mmol), N,N diisopropylethylamine (15.4 mg, 0.12 mmol) and (1-Cyano-2-ethoxy-2-oxoethylidenaminooxy)dimethylamino-morpholino-carbenium hexafluorophosphate (34.1 mg, 0.08 mmol). The reaction mixture was stirred under ice bath for 1 hour. After the reaction was complete, water (20 mL) was added to the reaction system. The reaction solution was extracted with ethyl acetate (30 mL*2), the organic phases were combined, washed with saturated brine, dried over anhydrous sodium sulfate, concentrated by filtration, and the residue was purified by preparative liquid chromatography to obtain white solid compound **2** (10 mg, yield 29.6%) and epimer 2' (15 mg, yield 44.4%). The absolute configurations drawn by the two compounds are based on empirical assumptions. In the existing analytical methods, 2 is a compound with relatively low polarity and relatively long retention time in LC-MS and HPLC, and 2' is compound with relatively high polarity and relatively short retention time in LC-MS and HPLC.

### Compound 2:

ESI-MS (m/z): 850.6 [M+H]⁺; LC-MS retention time RT=1.86 min. HPLC retention time RT=12.10 min.

¹H NMR (500 MHz, DMSO-d₆) δ 8.81 (d, J = 2.0 Hz, 1H), 8.51 - 8.49 (m, 1H), 8.39 (d, J = 9.0 Hz, 1H), 7.86 (d, J = 2.0 Hz, 1H), 7.82 (s, 1H), 7.77 - 7.73 (m, 1H), 7.58 (d, J = 9.0 Hz, 1H), 5.56 (t, J = 9.0 Hz, 1H), 5.10 - 5.04 (m, 1H), 4.36 - 4.04 (m, 5H), 3.63 - 3.59 (m, 4H), 3.59 - 3.55 (m, 4H), 3.31 - 3.29 (m, 1H), 3.25 (s, 3H), 3.18 - 3.12 (m, 1H), 2.99 - 2.92 (m, 1H), 2.79 - 2.72 (m, 1H), 2.56 - 2.52 (m, 4H), 2.42 - 2.36 (m, 1H), 2.11 - 2.05 (m, 1H ), 1.83 - 1.70 (m, 2H), 1.56 - 1.47 (m, 1H), 1.35 (d, J = 6.0 Hz, 3H), 1.26 - 1.13 (m, 4H), 1.10 - 1.04 (m, 5H), 0.91 (s, 3H), 0.88 (t, J = 7.0 Hz, 3H), 0.34 (s, 3H).

### Compound 2':

ESI-MS (m/z): 850.6 [M+H]⁺; LC-MS retention time RT=1.83 min. HPLC retention time RT=11.76 min.

¹H NMR (500 MHz, DMSO-d₆) δ 8.82 (d, J = 2.0 Hz, 1H), 8.54 - 8.51 (m, 1H), 8.41 (d, J = 9.0 Hz, 1H), 8.00 (d, J = 2.0 Hz, 1H), 7.82 (s, 1H), 7.76 - 7.71 (m, 1H), 7.54 (d, J = 9.0 Hz, 1H), 5.53 (t, J = 9.0 Hz, 1H), 5.07 - 5.02 (m, 1H), 4.26 - 4.18 (m, 2H), 4.00 - 3.91 (m, 2H), 3.87 - 3.79 (m, 1H), 3.69 - 3.65 (m, 1H), 3.61 (t, J = 5.0 Hz, 4H), 3.59 - 3.52 (m, 3H), 3.31 - 3.29 (m, 1H), 3.19 - 3.12 (m, 1H), 3.09 (s, 3H), 3.06 - 3.02 (m, 1H), 2.81 - 2.73 (m, 1H), 2.56 - 2.52 (m, 4H), 2.34 - 2.30 (m, 1H), 2.15 - 2.06 (m, 1H), 1.84 - 1.75 (m, 2H), 1.57 - 1.47 (m, 1H), 1.25 - 1.20 (m, 4H), 1.20 - 1.15 (m, 2H), 1.13 - 1.08 (m, 6H), 1.08 - 1.05 (m, 3H), 0.93 (s, 3H), 0.49 (s, 3H).

### Example 3

### (1S,25)-N-((6385,4S,72)-12-(2-((S)-1-methoxyethyl)-5-(3-morpholinoprop-1-yn-1-yl)pyridin-3 -yl)-10,10-dimethyl-5,7-dioxo-11-(2,2,2-trifluoroethyl)-61,62,63,64,65,66-hexahydro-11H-8-oxa-2(4,2)-thiazola-1(5,3)-indola-6(1,3)-pyridazinacyclonecaphane-4-yl)-2-methylcyclopropane-1-carboxamide

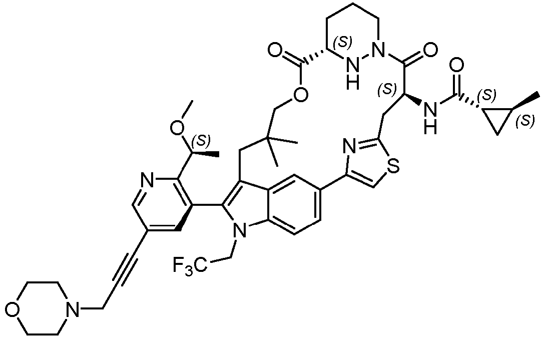

Example 3 is prepared by the following steps:

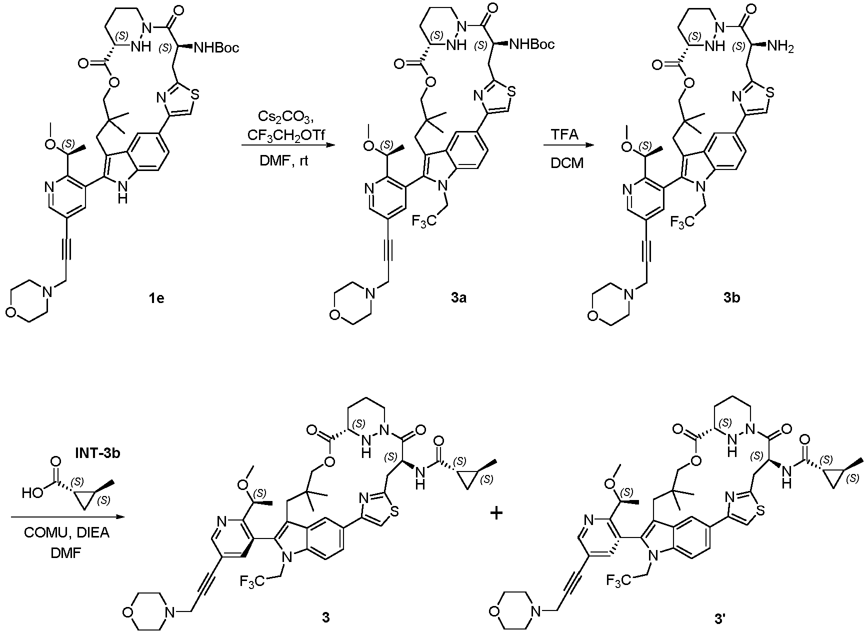

Step 1: Compound **1g** (30 mg, 0.036 mmol) was dissolved in N,N-dimethylformamide (2 mL), and cesium carbonate (23.7 mg, 0.072 mmol) and 2,2,2-trifluoroethyl trifluoromethanesulfonate (12.6 mg, 0.054 mmol) were added thereto. The reaction mixture was stirred at room temperature for 16 hours. After the reaction was complete, water (30 mL) was added to the reaction system. The reaction solution was extracted with ethyl acetate (30 mL*2), the organic phases were combined, washed with saturated brine, dried over anhydrous sodium sulfate, concentrated by filtration, and the residue was purified by preparative thin-layer chromatography (DCM:MeOH=30/1) to obtain light yellow solid compound **3a** (10 mg, yield 30.2%). ESI-MS (m/z): 908.2 [M+H]⁺.

Step 2: Compound **3a** (10 mg, 0.011 mmol) was dissolved in dichloromethane (2 mL), and trifluoroacetic acid (0.5 mL) was added thereto. The reaction mixture was stirred at room temperature for 2 hours. After the reaction was complete, saturated sodium bicarbonate solution (30 mL) was added to the reaction system under ice bath. The reaction system was extracted with dichloromethane (30 mL*2), and the organic phases were combined, washed with saturated brine, and dried over anhydrous sodium sulfate, filtered and concentrated to obtain light yellow solid compound **3b** (7 mg, yield 78.7%). ESI-MS (m/z): 808.2 [M+H]⁺.

Step 3: Compound **3b** (7 mg, 0.011 mmol) was dissolved in N,N-dimethylformamide (2 mL), and (1S,2S)-2-methylcyclopropanecarboxylic acid **INT-3b** (2.2 mg, 0.022 mmol), N,N-diisopropylethylamine (4.2 mg, 0.033 mmol) and (1-Cyano-2-ethoxy-2-oxoethylidenaminooxy)dimethylamino-morpholino-carbenium hexafluorophosphate (9.4 mg, 0.022 mmol) were added thereto. The reaction mixture was stirred under ice bath for 1 hour. After the reaction was complete, water (20 mL) was added to the reaction system, the reaction system was extracted with ethyl acetate (30 mL*2), and the organic phases were combined, washed with saturated brine, dried over anhydrous sodium sulfate, concentrated by filtration, and the residue was purified by preparative liquid chromatography to obtain white solid compound **3** (0.25 mg, yield 4.5%) and epimer 3' (0.25 mg, yield 4.5%). The absolute configurations drawn by the two compounds are based on empirical assumptions. In the existing analytical methods, 3 is a compound with relatively low polarity and relatively long retention time in LC-MS and HPLC, and 3' is compound with relatively high polarity and relatively short retention time in LC-MS and HPLC.

### Compound 3:

ESI-MS (m/z): 890.2 [M+H]⁺; LC-MS retention time RT=1.84 min. HPLC retention time RT=13.37 min.

¹H NMR (500 MHz, DMSO-d6) δ 8.85 - 8.82 (m, 1H), 8.55 - 8.50 (m, 2H), 7.87 (s, 1H), 7.86 - 7.81 (m, 2H), 7.79 - 7.75 (m, 1H), 5.63 - 5.51 (m, 2H), 5.09 - 5.04 (m, 1H), 4.89 - 4.81 (m, 1H), 4.27 - 4.20 (m, 3H), 3.63 - 3.56 (m, 8H), 3.30 - 3.27 (m, 1H), 3.19 - 3.11 (m, 2H), 3.06 - 3.00 (m, 1H), 2.79 - 2.72 (m, 1H), 2.55 - 2.52 (m, 2H), 2.40 - 2.36 (m, 1H), 2.12 - 2.08 (m, 1H), 2.03 - 1.97 (m, 1H), 1.82 - 1.78 (m, 2H), 1.54 - 1.49 (m, 2H), 1.36 (d, J = 6.0 Hz, 3H), 1.27 - 1.19 (m, 2H), 1.10 - 1.05 (m, 4H), 0.94 (s, 3H), 0.89 - 0.84 (m, 2H), 0.58 - 0.53 (m, 1H), 0.29 (s, 3H).

### Compound 3':

ESI-MS (m/z): 890.2 [M+H]⁺; LC-MS retention time RT=1.80 min. HPLC retention time RT=12.95 min.

¹H NMR (500 MHz, DMSO-d₆) δ 8.82 (d, J = 2.0 Hz, 1H), 8.55 - 8.51 (m, 2H), 7.95 (d, J = 2.0 Hz, 1H), 7.87 (s, 1H) , 7.84 - 7.80 (m, 1H), 7.71 - 7.67 (m, 1H), 5.54 (t, J = 9.0 Hz, 1H), 5.07 - 5.02 (m, 1H), 4.98 - 4.79 (m, 2H), 4.27 - 4.19 (m, 2H), 3.90 (q, J = 6.0 Hz, 1H), 3.68 - 3.65 (m, 1H), 3.64 - 3.59 (m, 4H), 3.58 (s, 2H), 3.53 - 3.47 (m, 1H), 3.19 - 3.12 (m, 1H), 3.09 (s, 3H), 3.04 (d, J = 14.5 Hz, 1H), 2.80 - 2.73 (m, 1H), 2.56 - 2.52 (m, 4H), 2.47 - 2.42 (m, 1H), 2.16 - 2.09 (m, 1H), 1.84 - 1.77 (m, 2H), 1.54 - 1.49 (m, 2H), 1.24 (d, J = 6.0 Hz, 3H), 1.09 - 1.06 (m, 4H), 0.93 (s, 3H), 0.90 - 0.83 (m, 2H), 0.58 - 0.54 (m, 1H), 0.46 (s, 3H).

### Example 4

### (1S,2S)-N-((63S,4S,Z)-11-ethyl-12-(2-((S)-1-methoxyethyl)-5-(3-((S)-2-methylmorpholino) prop-1-yn-1-yl)pyridin-3-yl)-10,10-dimethyl-5,7-dioxo-61,62,63,64,65,66-hexahydro-11H-8-oxa-2 (4,2)-thiazola-1(5,3)-indola-6(1,3)-pyridazinacyclonecaphane-4-yl)-2-methylcyclopropane-1-carboxamide

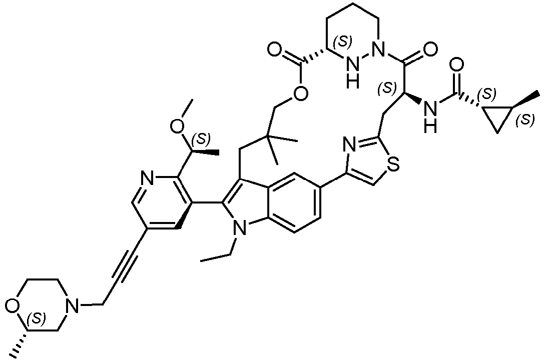

Example 4 is prepared by the following steps:

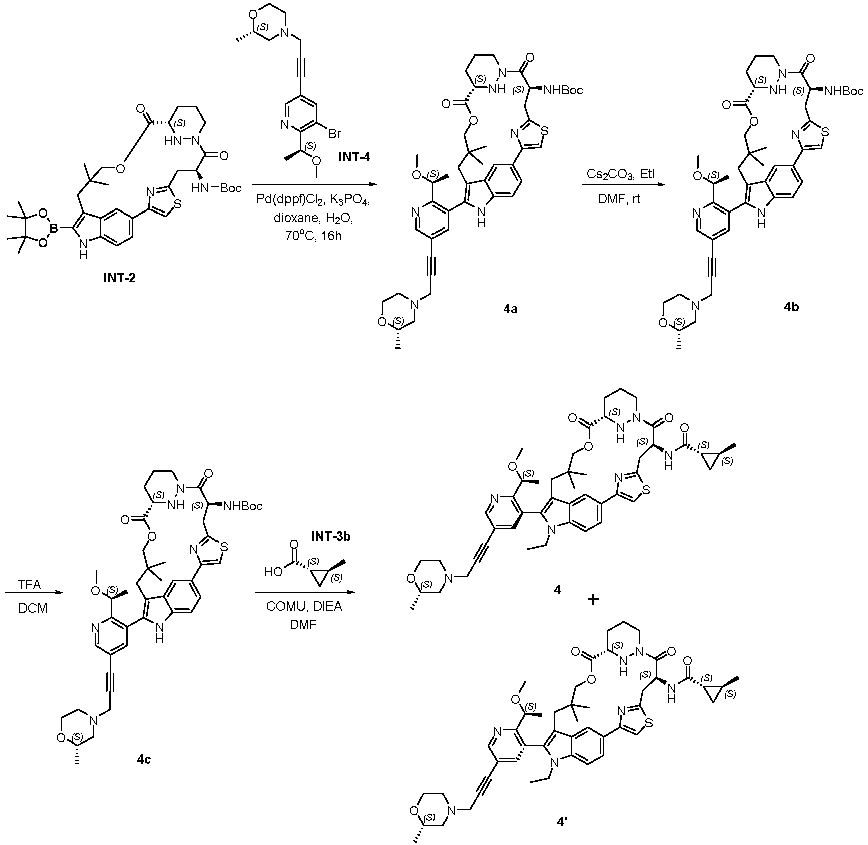

Step 1: Dissolve compound INT-2 (102.1 mg, 0.15 mmol) in a mixed solution of 1,4-dioxane (3 mL) and water (0.3 mL), and add successively **INT-6** (40 mg, 0.11 mmol), [1,1'-bis(diphenylphosphino)ferrocene]palladium dichloride (8.3 mg, 0.011 mmol) and potassium phosphate (72.1 mg, 0.33 mmol). The reaction mixture was stirred at 70 °C for 16 hours under nitrogen protection. After the reaction was complete, water (20 mL) was added to the reaction system, the reaction system was extracted with ethyl acetate (30 mL*2), the organic phases were combined, washed with saturated brine, dried over anhydrous sodium sulfate, concentrated by filtration, and the residue was purified by preparative thin-layer chromatography (dichloromethane/methanol=20:1) to obtain light yellow solid compound **4a** (60 mg, yield 63.1%). ESI-MS (m/z): 840.2 [M+H]⁺.

Step 2: Compound **4a** (60 mg, 0.071 mmol) was dissolved in N,N-dimethylformamide (2 mL), and cesium carbonate (46.3 mg, 0.142 mmol) and iodoethane (16.6 mg , 0.106 mmol) were added thereto. The reaction mixture was stirred at room temperature for 16 hours. After the reaction was complete, water (30 mL) was added to the reaction system, the reaction system was extracted with ethyl acetate (30 mL*2), the organic phases were combined, washed with saturated brine, dried over anhydrous sodium sulfate, concentrated by filtration, and the residue was purified by preparative thin-layer chromatography (dichloromethane/methanol=30/1) to obtain light yellow solid compound **4b** (50 mg, yield 81.2%). ESI-MS (m/z): 868.6 [M+H]⁺.

Step 3: Compound **4b** (50 mg, 0.058 mmol) was dissolved in dichloromethane (2 mL), and trifluoroacetic acid (0.5 mL) was added thereto. The reaction mixture was stirred at room temperature for 2 hours. After the reaction was complete, saturated sodium bicarbonate solution (30 mL) was added to the reaction system under ice bath. The reaction system was extracted with dichloromethane (30 mL*2), and the organic phases were combined, washed with saturated brine, and dried over anhydrous sodium sulfate, filtered and concentrated to obtain light yellow solid compound **4c** (40 mg, yield 90.4%). ESI-MS (m/z): 768.4 [M+H]⁺.

Step 4: Dissolve compound **4c** (40 mg, 0.052 mmol) in N,N-dimethylformamide (2 mL), add thereto (1S,2S)-2-methylcyclopropanecarboxylic acid **INT-3b** (10.4 mg, 0.104 mmol), N,N-Diisopropylethylamine (20.2 mg, 0.156 mmol) and (1-Cyano-2-ethoxy-2-oxoethylidenaminooxy)dimethylamino-morpholino-carbenium hexafluorophosphate (55.8 mg, 0.104 mmol). The reaction mixture was stirred under ice bath for 1 hour. After the reaction was complete, water (20 mL) was added to the reaction system, the reaction system was extracted with ethyl acetate (30 mL*2), the organic phases were combined, washed with saturated brine, dried over anhydrous sodium sulfate, concentrated by filtration, and the residue was purified by preparative liquid chromatography to obtain white solid compound **4** (6 mg, yield 13.6%) and epimer 4' (9 mg, yield 20.3%). The absolute configurations drawn by the two compounds are based on empirical assumptions. In the existing analytical methods, 4 is a compound with relatively low polarity and relatively long retention time in LC-MS and HPLC, and 4' is compound with relatively high polarity and relatively short retention time in LC-MS and HPLC.

### Compound 4:

ESI-MS (m/z): 850.4 [M+H]⁺; LC-MS retention time RT=1.88 min. HPLC retention time RT=13.62 min.

¹H NMR (500 MHz, DMSO-d₆) δ 8.81 (d, J = 2.0 Hz, 1H), 8.53 - 8.48 (m, 2H), 7.86 (d, J = 2.0 Hz, 1H), 7.81 (s, 1H) , 7.77 - 7.73 (m, 1H), 7.58 (d, J = 8.5 Hz, 1H), 5.56 (t, J = 9.0 Hz, 1H), 5.10 - 5.06 (m, 1H), 4.36 - 4.13 (m, 4H), 4.12 - 4.02 (m, 1H), 3.80 - 3.75 (m, 1H), 3.61 - 3.48 (m, 6H), 3.25 (s, 3H), 3.18 - 3.11 (m, 1H), 2.99 - 2.94 (m, 1H), 2.81 - 2.70 (m, 3H), 2.40 - 2.36 (m, 1H), 2.30 - 2.24 (m, 1H), 2.11 - 2.05 (m, 1H), 2.00 - 1.94 (m, 2H), 1.83 - 1.75 (m, 2H), 1.53 - 1.47 (m, 2H), 1.35 (d, J = 6.0 Hz, 3H), 1.08 - 1.04 (m, 7H), 0.92 - 0.85 (m, 8H), 0.57 - 0.53 (m, 1H), 0.34 (s, 3H).

### Compound 4':

ESI-MS (m/z): 850.4 [M+H]⁺; LC-MS retention time RT=1.85 min. HPLC retention time RT=13.25 min.

¹H NMR (500 MHz, DMSO-d₆) δ 8.82 (d, J = 2.0 Hz, 1H), 8.56 - 8.49 (m, 2H), 7.99 (d, J = 2.0 Hz, 1H), 7.81 (s, 1H) , 7.76 - 7.72 (m, 1H), 7.54 (d, J = 8.5 Hz, 1H), 5.55 (t, J = 9.0 Hz, 1H), 5.07 - 5.02 (m, 1H), 4.28 - 4.17 (m, 2H), 4.00 - 3.91 (m, 2H), 3.87 - 3.75 (m, 2H), 3.70 - 3.64 (m, 1H), 3.60 - 3.48 (m, 4H), 3.20 - 3.02 (m, 5H), 2.81 - 2.68 (m, 3H), 2.35 - 2.24 (m, 2H), 2.15 - 2.09 (m, 1H), 2.03 - 1.93 (m, 2H), 1.84 - 1.75 (m, 2H), 1.56 - 1.47 (m, 2H), 1.42 - 1.38 (m, 1H), 1.27 - 1.19 (m, 7H), 1.14 - 1.05 (m, 6H), 0.93 (s, 3H), 0.89 - 0.82 (m, 2H), 0.59 - 0.53 (m, 1H), 0.50 (s, 2H).

### Example 5

### (1S,2S)-N-((63S,4S,Z)-11-ethyl-12-(2-((S)-1-methoxyethyl)-5-(4-morpholinobut-1-yn-1-yl )pyridin-3-yl)-10,10-dimethyl-5,7-dioxo-61,62,63,64,65,66-hexahydro-11H-8-oxa-2(4,2)-thiazola-1 (5,3)-indola-6(1,3)-pyridazinacyclonecaphane-4-yl)-2-methylcyclopropane-1-carboxamide

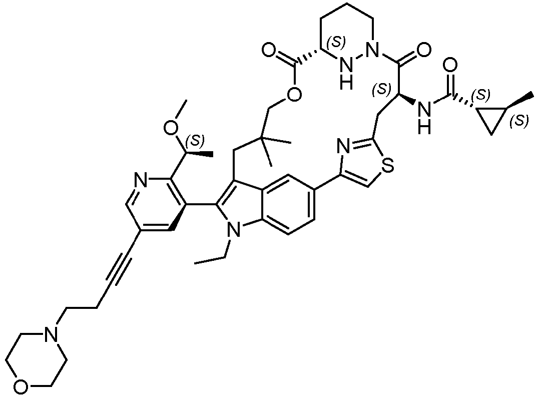

Example 5 is prepared by the following steps:

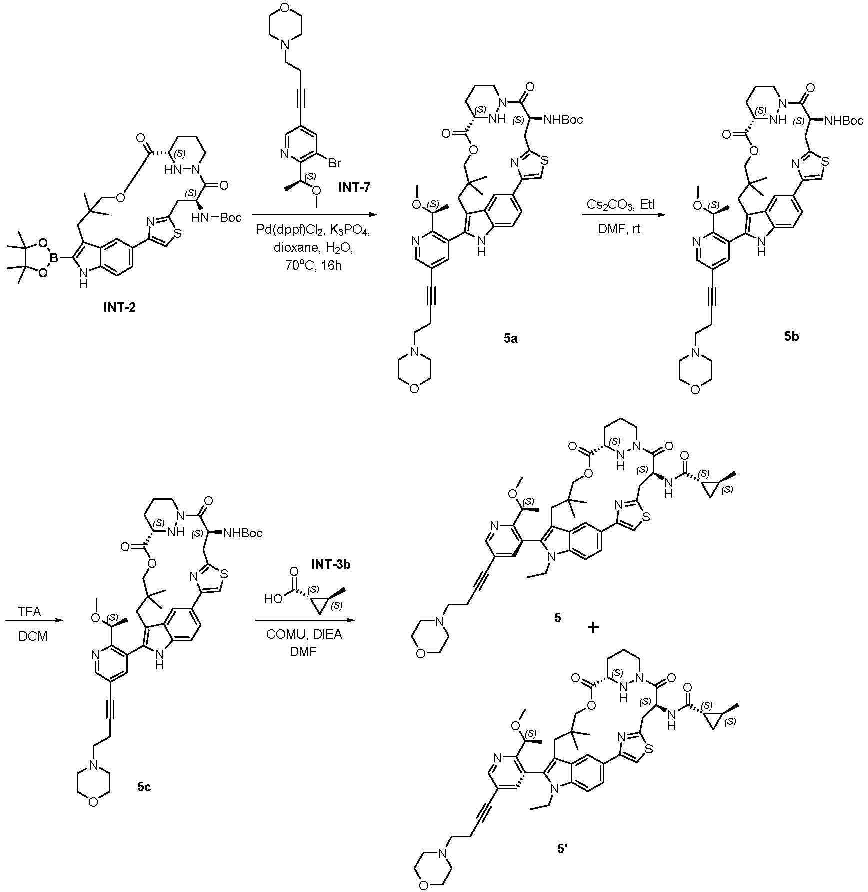

Step 1: Dissolve compound INT-2 (100 mg, 0.14 mmol) in a mixed solution of 1,4-dioxane (5 mL) and water (1 mL), and add **INT-7** (51 mg, 0.14 mmol), [1,1'-bis(diphenylphosphino)ferrocene]palladium dichloride (10 mg, 0.01 mmol) and potassium phosphate (60 mg, 0.28 mmol) successively. The reaction mixture was stirred at 70 °C for 16 hours under nitrogen protection. After the reaction was complete, the reaction solution was filtered with celite, and the concentrated residue was purified by silica gel column chromatography (dichloromethane/methanol = 20/1) to obtain light yellow oily compound **5a** (65 mg, yield 53.7%). ESI-MS (m/z): 840.1 [M+H]⁺;

Step 2: Compound **5a** (60 mg, 0.07 mmol) was dissolved in N,N-dimethylformamide (2 mL), and cesium carbonate (46 mg, 0.14 mmol) and iodoethane (22 mg , 0.14 mmol) were added thereto. The reaction mixture was stirred at room temperature for 16 hours. After the reaction was complete, water (15 mL) was added to the reaction system, the reaction system was extracted with ethyl acetate (20 mL*2), the organic phases were combined, washed with saturated brine, dried over anhydrous sodium sulfate, filtered and concentrated to obtain a light yellow solid Compound **5b** (25 mg, yield 40.3%). ESI-MS (m/z): 868.2 [M+H]⁺;

Step 3: Compound **5b** (25 mg, 0.03 mmol) was dissolved in dichloromethane (2 mL), and trifluoroacetic acid (0.5 mL) was added thereto. The reaction mixture was stirred at room temperature for 2 hours. After the reaction was complete, saturated sodium bicarbonate solution (20 mL) was added to the reaction system under ice bath. The reaction system was extracted with dichloromethane (20 mL*2), and the organic phases were combined, washed with saturated brine, and dried over anhydrous sodium sulfate, filtered and concentrated to obtain light yellow solid compound 5c (20 mg, yield 90.9%). ESI-MS (m/z): 768.2 [M+H]⁺;

Step 4: Dissolve compound **5c** (20 mg, 0.03 mmol) in N,N-dimethylformamide (1 mL), add thereto (1S,2S)-2-methylcyclopropanecarboxylic acid **INT-3b** (5 mg, 0.05 mmol), N,N-diisopropylethylamine (10 mg, 0.07 mmol) and (1-Cyano-2-ethoxy-2-oxoethylidenaminooxy)dimethylamino-morpholino-carbenium hexafluorophosphate (11 mg, 0.03 mmol). The reaction mixture was stirred under ice bath for 1 hour. After the reaction was complete, water (20 mL) was added to the reaction system, the reaction system was extracted with ethyl acetate (20 mL*2), the organic phases were combined, washed with saturated brine, dried over anhydrous sodium sulfate, concentrated by filtration, and the residue was purified by preparative liquid chromatography to obtain white solid compound **5** (5 mg, yield 22.7%) and epimer 5' (7 mg, yield 31.8%). The absolute configurations drawn by the two compounds are based on empirical assumptions. In the existing analytical methods, 5 is a compound with relatively low polarity and relatively long retention time in LC-MS and HPLC, and 5' is compound with relatively high polarity and relatively short retention time in LC-MS and HPLC.

### Compound 5:

ESI-MS (m/z): 850.6 [M+H]⁺; LC-MS retention time RT=1.89 min. HPLC retention time RT=13.37 min.

¹H NMR (500 MHz, DMSO-d₆) δ 8.75 (d, J = 2.1 Hz, 1H), 8.55 - 8.48 (m, 2H), 7.81 (s, 1H), 7.78 - 7.73 (m, 2H), 7.58 (d, J = 8.7 Hz, 1H), 5.56 (t, J = 9.1 Hz, 1H), 5.10 - 5.06 (m, 1H), 4.37 - 4.04 (m, 5H), 3.60 - 3.54 (m, 6H), 3.25 (s, 3H), 3.18 - 3.11 (m, 1H), 2.98 - 2.94 (m, 1H), 2.80 - 2.72 (m, 1H), 2.69 - 2.57 (m, 4H), 2.44 (s, 4H), 2.39 - 2.35 (m, 1H), 2.11 - 2.07 (m, 1H), 1.83 - 1.76 (m, 2H), 1.53- 1.46 (m, 2H), 1.34 (d, J = 6.0 Hz, 3H), 1.07 (s, 3H), 0.91 (s, 3H), 0.87 (t, J = 7.0 Hz, 3H), 0.57 - 0.53 (m, 1H), 0.33 (s, 3H).

### Compound 5':

ESI-MS (m/z): 850.6 [M+H]⁺; LC-MS retention time RT=1.83 min. HPLC retention time RT=13.01 min.

¹H NMR (500 MHz, DMSO-d₆) δ 8.76 (d, J = 2.1 Hz, 1H), 8.56 - 8.50 (m, 2H), 7.91 (d, J = 2.2 Hz, 1H), 7.81 (s, 1H) , 7.74 (dd, J = 8.7, 1.7 Hz, 1H), 7.54 (d, J = 8.7 Hz, 1H), 5.54 (t, J = 9.2 Hz, 1H), 5.10 - 5.06 (m, 1H), 4.27 - 4.15 (m, 2H), 3.97 - 3.88 (m, 2H), 3.84 - 3.77 (m, 1H), 3.70 - 3.66 (m, 1H), 3.59 - 3.55 (m, 5H), 3.18 - 3.14 (m, 1H ), 3.08 (s, 3H), 3.07 - 3.02 (m, 1H), 2.80 - 2.73 (m, 1H), 2.68 - 2.57 (m, 5H), 2.47 - 2.42 (m, 4H), 2.34 - 2.28 (m, 1H), 2.15 - 2.10 (m, 1H), 1.84 - 1.77 (m, 2H), 1.56 - 1.47 (m, 2H), 1.21 (d, J = 6.2 Hz, 3H), 1.10 (t, J = 7.2 Hz, 3H), 1.07 (s, 3H), 0.93 (s, 3H), 0.90 - 0.84 (m, 2H), 0.55 (d, J = 6.1 Hz, 1H), 0.49 (s, 3H).

### Example 6

### (1S,2S)-N-((63S,4S,Z)-11-ethyl-12-(2-((S)-1-methoxyethyl)-5-(((S)-4-methylmorpholin-2- yl)ethynyl)pyridin-3-yl)-10,10-dimethyl-5,7-dioxo-61,62,63,64,65,66-hexahydro-11H-8-oxa-2(4,2)- Thiazola-1(5,3)-indola-6(1,3)-pyridazinacyclonecaphane-4-yl)-2-methylcyclopropane-1-carboxamide

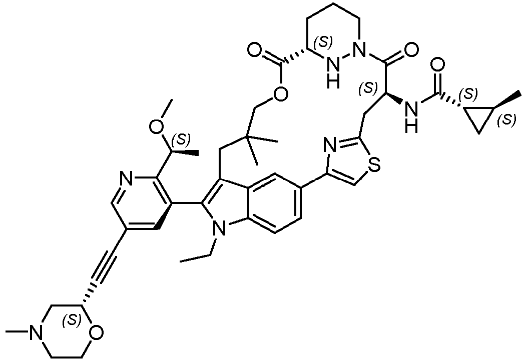

Example 6 is prepared by the following steps:

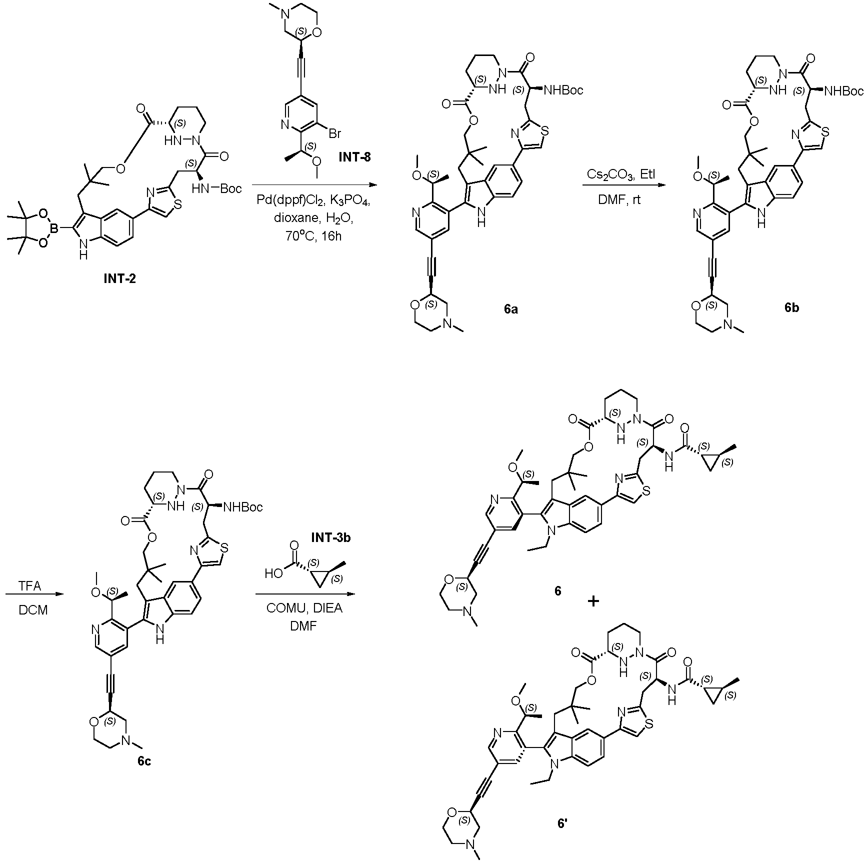

Step 1: Dissolve compound INT-2 (119.6 mg, 0.172 mmol) in a mixed solution of 1,4-dioxane (3 mL) and water (0.3 mL), and add successively **INT-8** (45 mg, 0.133 mmol), [1,1'-bis(diphenylphosphino)ferrocene]palladium dichloride (9.7 mg, 0.013 mmol) and potassium phosphate (84.5 mg, 0.399 mmol). The reaction mixture was stirred at 70 °C for 16 hours under nitrogen protection. After the reaction was complete, water (20 mL) was added to the reaction system, the reaction system was extracted with ethyl acetate (30 mL*2), the organic phases were combined, washed with saturated brine, dried over anhydrous sodium sulfate, concentrated by filtration, and the residue was purified by preparative thin-layer chromatography to obtain compound **6a** (65 mg, yield 59.3%) as a pale yellow solid. ESI-MS (m/z): 826.2 [M+H]⁺.

Step 2: Compound **6a** (65 mg, 0.079 mmol) was dissolved in N,N-dimethylformamide (2 mL), and cesium carbonate (51.3 mg, 0.157 mmol) and iodoethane (18.4 mg, 0.118 mmol) were added thereto. The reaction mixture was stirred at room temperature for 16 hours. After the reaction was complete, water (30 mL) was added to the reaction system, the reaction system was extracted with ethyl acetate (30 mL*2), the organic phases were combined, washed with saturated brine, dried over anhydrous sodium sulfate, concentrated by filtration, and the residue was purified by preparative thin-layer chromatography (dichloromethane/methanol=30/1) to obtain light yellow solid compound **6b** (50 mg, yield 74.4%). ESI-MS (m/z): 854.4 [M+H]⁺.

Step 3: Compound **6b** (50 mg, 0.059 mmol) was dissolved in dichloromethane (2 mL), and trifluoroacetic acid (0.5 mL) was added thereto. The reaction mixture was stirred at room temperature for 2 hours. After the reaction was complete, saturated sodium bicarbonate solution (30 mL) was added to the reaction system under ice bath. The reaction system was extracted with dichloromethane (30 mL*2), and the organic phases were combined, washed with saturated brine, and dried over anhydrous sodium sulfate, filtered and concentrated to obtain light yellow solid compound **6c** (40 mg, yield 90.6%). ESI-MS (m/z): 754.4 [M+H]⁺.

Step 4: Dissolve compound **6c** (40 mg, 0.053 mmol) in N,N-dimethylformamide (2 mL), add thereto (1S,2S)-2-methylcyclopropanecarboxylic acid **INT-3b** (10.6 mg, 0.106 mmol), N,N-Diisopropylethylamine (20.6 mg, 0.159 mmol) and (1-Cyano-2-ethoxy-2-oxoethylidenaminooxy)dimethylamino-morpholino-carbenium hexafluorophosphate (45.4 mg, 0.106 mmol). The reaction mixture was stirred under ice bath for 1 hour. After the reaction was complete, water (20 mL) was added to the reaction system, the reaction system was extracted with ethyl acetate (30 mL*2), the organic phases were combined, washed with saturated brine, dried over anhydrous sodium sulfate, concentrated by filtration, and the residue was purified by preparative liquid chromatography to obtain white solid compound **6** (8 mg, yield 18.0%) and epimer 6' (10 mg, yield 22.5%). The absolute configurations drawn by the two compounds are based on empirical assumptions. In the existing analytical methods, 6 is a compound with relatively low polarity and relatively long retention time in LC-MS and HPLC, and 6' is compound with relatively high polarity and relatively short retention time in LC-MS and HPLC.

### Compound 6:

ESI-MS (m/z): 836.4 [M+H]⁺; LC-MS retention time RT=1.80 min. HPLC retention time RT=13.34 min.

¹H NMR (500 MHz, DMSO-d₆) δ 8.81 (d, J = 2.0 Hz, 1H), 8.54 - 8.48 (m, 2H), 7.86 (d, J = 2.0 Hz, 1H), 7.81 (s, 1H) , 7.77 - 7.74 (m, 1H), 7.58 (d, J = 8.5 Hz, 1H), 5.56 (t, J = 9.0 Hz, 1H), 5.10 - 5.05 (m, 1H), 4.63 - 4.57 (m, 1H ), 4.37 - 4.16 (m, 4H), 4.11 - 4.04 (m, 1H), 3.90 - 3.83 (m, 1H), 3.61 - 3.54 (m, 3H), 3.25 (s, 3H), 3.18 - 3.11 (m, 1H), 3.00 - 2.93 (m, 1H), 2.80 - 2.72 (m, 2H), 2.48 - 2.44 (m, 1H), 2.40 - 2.36 (m, 1H), 2.34 - 2.28 (m, 1H), 2.22 - 2.17 (m, 4H), 2.11 - 2.04 (m, 1H), 1.83 - 1.75 (m, 2H), 1.53 - 1.47 (m, 2H), 1.35 (d, J = 6.0 Hz, 3H), 1.10 - 1.04 (m, 5H), 0.92 (s, 3H), 0.90 - 0.85 (m, 4H), 0.57 - 0.52 (m, 1H), 0.33 (s, 3H).

### Compound 6':

ESI-MS (m/z): 836.4 [M+H]⁺; LC-MS retention time RT=1.76 min. HPLC retention time RT=12.94 min.

¹H NMR (500 MHz, DMSO-d₆) δ 8.82 (d, J = 2.0 Hz, 1H), 8.56 - 8.50 (m, 2H), 8.00 (d, J = 2.0 Hz, 1H), 7.81 (s, 1H) , 7.76 - 7.71 (m, 1H), 7.54 (d, J = 8.5 Hz, 1H), 5.54 (t, J = 9.0 Hz, 1H), 5.07 - 5.02 (m, 1H), 4.64 - 4.58 (m, 1H ), 4.28 - 4.18 (m, 2H), 3.98 - 3.91 (m, 2H), 3.88 - 3.79 (m, 2H), 3.71 - 3.64 (m, 1H), 3.62 - 3.50 (m, 2H), 3.31 - 3.27 (m, 1H), 3.18 - 3.12 (m, 1H), 3.09 (s, 3H), 3.07 - 3.02 (m, 1H), 2.80 - 2.74 (m, 2H), 2.2.48 - 2.2.43 (m, 1H), 2.34 - 2.26 (m, 2H), 2.21 (s, 3H), 2.19 - 2.09 (m, 2H), 1.85 - 1.75 (m, 2H), 1.54 - 1.48 (m, 2H), 1.21 (d, *J* = 6.0 Hz, 3H), 1.13 - 1.06 (m, 7H), 0.93 (s, 3H), 0.90 - 0.86 (m, 1H), 0.58 - 0.54 (m, 1H), 0.49 (s, 3H).

### Example 7

### (1S,2S)-N-((63S,4S,Z)-11-ethyl-12-(2-((S)-1-methoxyethyl)-5-(((R)-4-methylmorpholin-3- yl)ethynyl)pyridin-3-yl)-10,10-dimethyl-5,7-dioxo-61,62,63,64,65,66-hexahydro-11H-8-oxa-2(4,2)- Thiazola-1(5,3)-indola-6(1,3)-pyridazinacyclonecaphane-4-yl)-2-methylcyclopropane-1-carboxamide

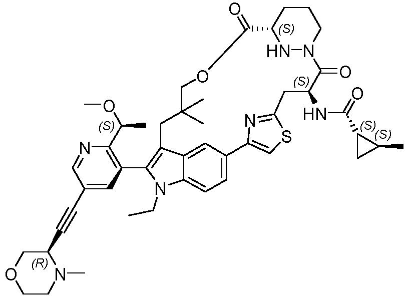

Example 7 is prepared by the following steps:

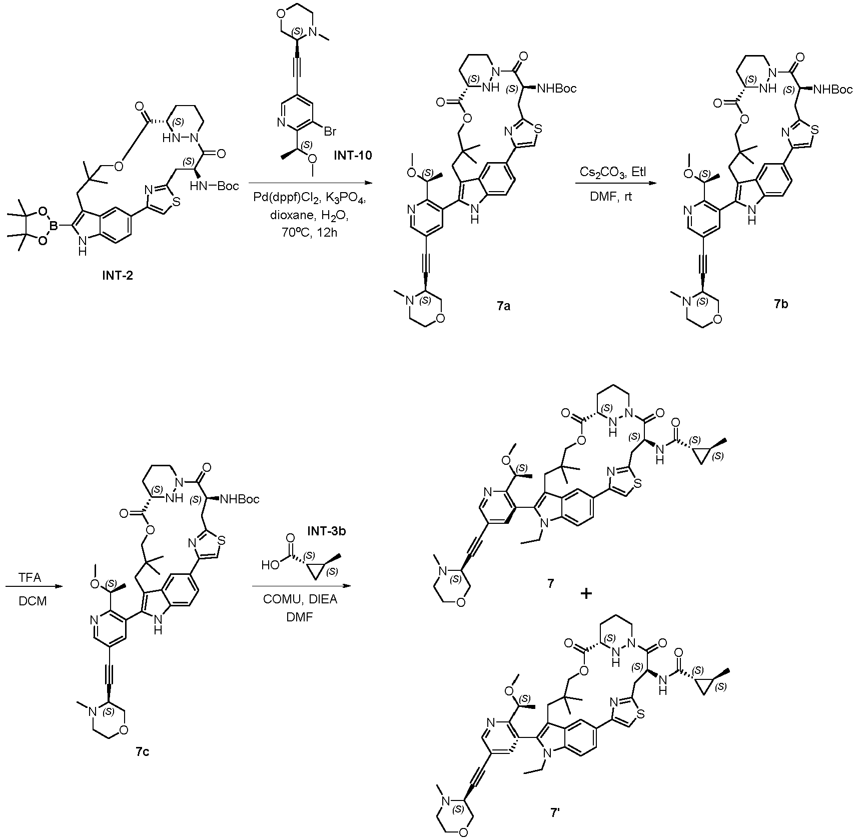

Step 1: Dissolve intermediate INT-2 (98 mg, 0.14 mmol) and intermediate INT-10 (40 mg, 0.12 mmol) in a mixed solvent of 1,4-dioxane (2 mL) and water (0.2 mL), and add [1,1'-bis(diphenylphosphino)ferrocene]palladium dichloride and potassium phosphate (75 mg, 0.35 mmol). The reaction system was replaced with nitrogen and then heated to 70 °C and stirred for 12 hours. After the reaction solution was cooled to room temperature, the reaction solution was filtered through celite, and the filtrate was concentrated. The residue was purified by preparative thin-layer chromatography (dichloromethane/methanol=30/1) to obtain compound **7a** (92 mg, yield 94%). ESI-MS (m/z): 826.5 [M+H]⁺.

Step 2: Compound 7a (92 mg, 0.11 mmol) was dissolved in N,N-dimethylformamide (2 mL), cesium carbonate (73 mg, 0.22 mmol) was added, subsequently ethyl iodide (35 mg , 0.22 mmol) was added dropwise to the reaction solution, and the reaction solution was stirred at 50 °C for 6 hours. After the reaction solution was cooled to room temperature, saturated brine (20 mL) was added to the reaction system. The reaction solution was extracted with ethyl acetate (30 mL*2), the organic phases were combined, washed with saturated brine, dried over anhydrous sodium sulfate, filtered and concentrated to obtain the crude product of compound **7b** (80 mg, yield 84%). ESI-MS (m/z): 854.5 [M+H]⁺.

Step 3: Dissolve compound 7b (80 mg, 0.09 mmol) in dichloromethane (1 mL), cool the reaction solution to 0 °C, then add trifluoroacetic acid (53 mg, 0.47 mmol) dropwise to the reaction solution . The reaction continued to be stirred at 0 °C for 1 hour. The reaction solution was quenched with saturated aqueous sodium bicarbonate solution, extracted with dichloromethane, separated, the organic phase was dried over anhydrous sodium sulfate, filtered and concentrated to obtain the crude product of compound **7c** (70 mg, yield 99%). ESI-MS (m/z): 754.4 [M+H]⁺.

Step 4: Dissolve compound 7c (70 mg, 0.09 mmol) and intermediate **INT- 3** (18 mg, 0.19 mmol) in N,N-dimethylformamide (3 mL), add N,N-diisopropylethylamine (36 mg, 0.28 mmol) and (1-Cyano-2-ethoxy-2-oxoethylidenaminooxy)dimethylamino-morpholino-carbenium hexafluorophosphate (36 mg, 0.19 mmol). The reaction solution was stirred at 0 °C for 30 minutes. Saturated brine (20 mL) was added to the reaction system, the reaction system was extracted with ethyl acetate (30 mL*2), the organic phases were combined, washed with saturated brine, dried over anhydrous sodium sulfate, filtered and concentrated, and the residue was purified by preparative liquid phase chromatography to obtain white solid compound **7** (7 mg, yield 9%) and epimer 7' (10 mg, yield 12.9%). The absolute configurations drawn by the two compounds are based on empirical assumptions. In the existing analytical methods, 7 is a compound with relatively low polarity and relatively long retention time in LC-MS and HPLC, and 7' is compound with relatively high polarity and relatively short retention time in LC-MS and HPLC.

### Compound 7:

ESI-MS (m/z): 836.6 [M+H]⁺. LC-MS retention time RT=2.09 min. HPLC retention time RT=13.10 min.

¹H NMR (500 MHz, DMSO) δ 8.81 (d, J = 2.1 Hz, 1H), 8.51 (dd, J = 9.3, 5.0 Hz, 2H), 7.85 (d, J = 1.9 Hz, 1H), 7.81 (s, 1H), 7.75 (dd, J = 8.6, 1.5 Hz, 1H), 7.58 (d, J = 8.7 Hz, 1H), 5.56 (t, J = 9.3 Hz, 1H), 5.09 - 5.05 (m, 1H), 4.35 - 4.16 (m, 4H), 4.10 - 4.05 (m, 1H), 3.79 - 3.75 (m, 1H), 3.72 - 3.56 (m, 5H), 3.51 (s, 1H), 3.31 (s, 2H), 3.25 (s, 3H), 3.17 - 3.08 (m, 2H), 3.00 - 2.95 (m, 1H), 2.79 - 2.72 (m, 1H), 2.68 - 2.62 (m, 1H), 2.40 - 2.33 (m, 4H), 2.32 - 2.27 (m, 1H), 2.11 - 2.07 (m, 1H), 1.83 - 1.74 (m, 2H), 1.54 - 1.46 (m, 2H), 1.35 (d, *J* = 6.1 Hz, 3H), 1.23 (s, 1H), 1.06 (s, 3H), 0.92 (s, 3H), 0.87 (t, *J* = 7.0 Hz, 3H), 0.55 (dd, *J* = 7.7, 5.5 Hz, 1H), 0.34 (s, 3H).

### Compound 7':

ESI-MS (m/z): 836.6 [M+H]⁺. LC-MS retention time RT=2.06 min. HPLC retention time RT=12.76 min.

¹H NMR (500 MHz, DMSO) δ 8.84 - 8.80 (m, 1H), 8.53 (d, J = 9.2 Hz, 2H), 7.99 (d, J = 1.5 Hz, 1H), 7.81 (s, 1H), 7.74 (dd, J = 8.7, 1.5 Hz, 1H), 7.54 (d, J = 8.7 Hz, 1H), 5.54 (t, J = 9.3 Hz, 1H), 5.07 - 5.02 (m, 1H), 4.25 - 4.20 (m, 1H), 3.98 - 3.92 (m, 1H), 3.86 - 3.82 (m, 1H), 3.78 - 3.72 (m, 1H), 3.70 - 3.65 (m, 3H), 3.63 - 3.59 (m, 1H), 3.57 - 3.49 (m, 2H), 3.31 (s, 2H), 3.18 - 3.12 (m, 1H), 3.09 (s, 3H), 3.07 - 3.02 (m, 1H), 2.79 - 5.75 (m, 1H), 2.68 - 2.61 (m, 1H), 2.33 (d, *J* = 4.1 Hz, 3H), 2.32 - 2.25 (m, 2H), 2.14 - 2.09 (m, 1H), 1.85 - 1.75 (m, 2H), 1.51 (dt, *J* = 8.0, 6.9 Hz, 2H), 1.21 (d, *J* = 6.3 Hz, 3H), 1.11 (t, *J* = 7.1 Hz, 3H), 1.07 (s, 4H), 0.93 (s, 3H), 0.90 - 0.85 (m, 1H), 0.56 (dd, *J* = 7.7, 5.4 Hz, 1H), 0.50 (s, 3H).

### Example 8

### (1S,2S)-N-((63S,4S,Z)-11-ethyl-12-(2-((S)-1-methoxyethyl)-5-(((R)-4-methylmorpholin-2- yl)ethynyl)pyridin-3-yl)-10,10-dimethyl-5,7-dioxo-61,62,63,64,65,66-hexahydro-11H-8-oxa-2(4,2)- Thiazola-1(5,3)-indola-6(1,3)-pyridazinacyclonecaphane-4-yl)-2-methylcyclopropane-1-carboxamide

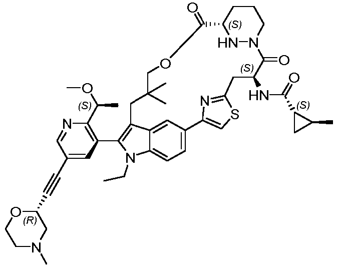

Example 8 is prepared by the following steps:

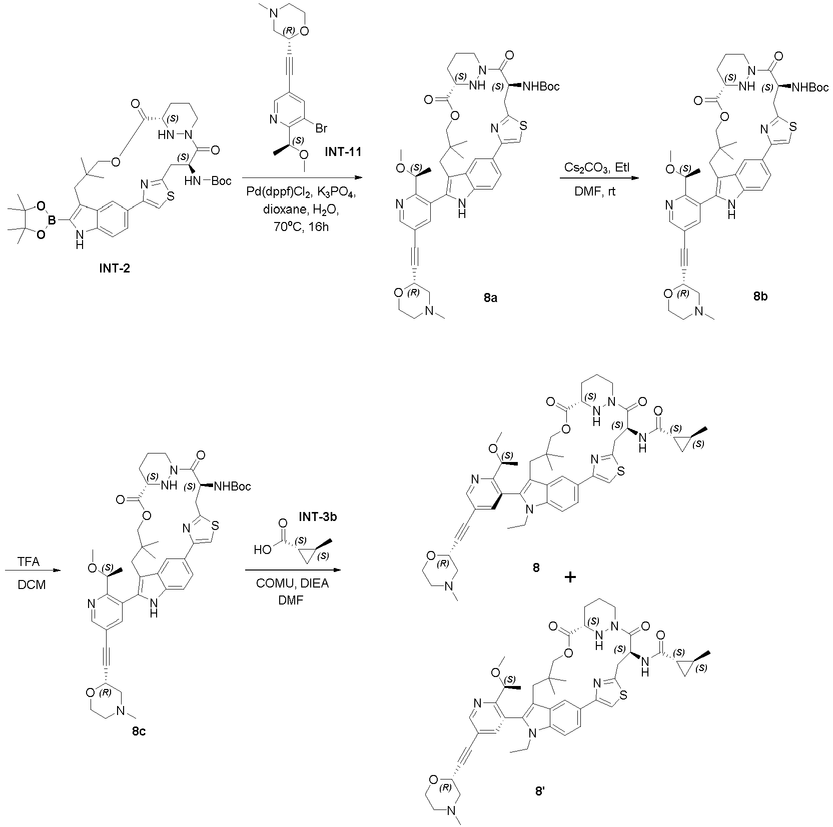

Step 1: Dissolve intermediate **INT-2** (98 mg, 0.14 mmol) and intermediate **INT-11** (40 mg, 0.12 mmol) in a mixed solvent of 1,4-dioxane (2 mL) and water (0.2 mL), and add [1,1'-bis(diphenylphosphino)ferrocene]palladium dichloride (9 mg, 0.01 mmol) and potassium phosphate (75 mg, 0.35 mmol). The reaction system was replaced with nitrogen and then heated to 70 °C and stirred for 12 hours. After the reaction solution was cooled to room temperature, the reaction solution was filtered through celite, and the filtrate was concentrated. The residue was purified by preparative thin-layer chromatography (dichloromethane/methanol=30/1) to obtain compound **8a** (37 mg, yield 38%). ESI-MS (m/z): 826.5 [M+H]⁺.

Step 2: Compound **8a** (37 mg, 0.05 mmol) was dissolved in N,N-dimethylformamide (2 mL), cesium carbonate (29 mg, 0.09 mmol) was added, then ethyl iodide (14 mg, 0.09 mmol) was added dropwise to the reaction solution, and the reaction solution was stirred at 50 °C for 6 hours. After the reaction solution was cooled to room temperature, saturated brine (20 mL) was added to the reaction system, the reaction solution was extracted with ethyl acetate (30 mL*2), the organic phases were combined, washed with saturated brine, dried over anhydrous sodium sulfate, filtered and concentrated, to obtain the crude product of compound **8b** (30 mg, yield 78%). ESI-MS (m/z): 854.3 [M+H]⁺.

Step 3: Dissolve compound **8b** (30 mg, 0.04 mmol) in dichloromethane (1 mL), cool the reaction solution to 0 °C, then add trifluoroacetic acid (20 mg, 0.18 mmol) dropwise to the reaction solution. The reaction continued to be stirred at 0 °C for 1 hour. The reaction solution was quenched with saturated aqueous sodium bicarbonate, extracted with dichloromethane, separated, the organic phase was dried over anhydrous sodium sulfate, filtered and concentrated to obtain the crude product of compound **8c** (26 mg, yield 99%). ESI-MS (m/z): 754.5 [M+H]⁺.

Step 4: Dissolve compound **8c** (26 mg, 0.04 mmol) and intermediate **INT-3** (7 mg, 0.07 mmol) in N,N-dimethylformamide (2 mL), add N,N-di Isopropylethylamine (13 mg, 0.10 mmol) and (1-Cyano-2-ethoxy-2-oxoethylidenaminooxy)dimethylamino-morpholino-carbenium hexafluorophosphate (14 mg, 0.07 mmol), The reaction solution was stirred at 0°C for 30 minutes. Saturated brine (20 mL) was added to the reaction system, the reaction system was extracted with ethyl acetate (30 mL*2), the organic phases were combined, washed with saturated brine, dried over anhydrous sodium sulfate, filtered and concentrated, and the residue was purified by preparative liquid phase chromatography to obtain white solid compound **8** (3 mg, yield 10%) and epimer 8' (5 mg, yield 17.8%). The absolute configurations drawn by the two compounds are based on empirical assumptions. In the existing analytical methods, 8 is a compound with relatively low polarity and relatively long retention time in LC-MS and HPLC, and 8' is compound with relatively high polarity and relatively short retention time in LC-MS and HPLC.

### Compound 8:

ESI-MS (m/z): 836.2 [M+H]⁺. LC-MS retention time RT=1.81 min. HPLC retention time RT=13.43 min.

¹H NMR (500 MHz, DMSO) δ 8.81 (d, J = 1.9 Hz, 1H), 8.55 - 8.50 (m, 2H), 7.85 (d, J = 1.9 Hz, 1H), 7.81 (s, 1H), 7.75 (d, J = 8.7 Hz, 1H), 7.58 (d, J = 8.6 Hz, 1H), 5.56 (t, J = 9.1 Hz, 1H), 5.09 - 5.05 (m, 1H), 4.60 (dd, J = 8.0, 2.5 Hz, 1H), 4.38 - 4.15 (m, 5H), 4.10 - 4.04 (m, 1H), 3.88 - 3.83 (m, 1H), 3.61 - 3.54 (m, 3H), 3.32 - 3.31 (m, 2H), 3.25 (s, 3H), 3.17 - 3.08 (m, 2H), 2.99 - 2.95 (m, 1H), 2.80 - 2.72 (m, 2H), 2.40 - 2.35 (m, 1H), 2.32 - 2.28 (m, 1H), 2.20 (s, 3H), 2.11 - 2.05 (m, 1H), 1.81 - 1.76 (m, 2H), 1.53 - 1.46 (m, 2H), 1.35 (d, *J =* 6.0 Hz, 3H), 1.23 (s, 1H), 1.06 (s, 3H), 0.91 (s, 3H), 0.87 (t, *J =* 7.1 Hz, 3H), 0.55 (dd, *J =* 7.5, 5.4 Hz, 1H), 0.33 (s, 3H).

### Compound 8':

ESI-MS (m/z): 836.2 [M+H]⁺. LC-MS retention time RT=1.78 min. HPLC retention time RT=13.01 min.

¹H NMR (500 MHz, DMSO) δ 8.82 (d, J = 2.1 Hz, 1H), 8.53 (d, J = 9.2 Hz, 2H), 8.00 (d, J = 2.1 Hz, 1H), 7.81 (s, 1H), 7.74 (dd, J = 8.7, 1.5 Hz, 1H), 7.54 (d, J = 8.7 Hz, 1H), 5.54 (t, J = 9.4 Hz, 1H), 5.07 - 5.02 (m, 1H), 4.60 (dd, J = 8.1, 2.8 Hz, 1H), 4.25 - 4.20 (m, 2H), 4.00 - 3.90 (m, 2H), 3.88 - 3.79 (m, 2H), 3.68 - 3.62 (m, 1H), 3.60 - 3.57 (m, 1H), 3.58 - 3.50 (m, 1H), 3.31 (d, J = 1.8 Hz, 2H), 3.18 - 3.14 (m, 1H), 3.09 (s, 3H), 3.08 - 3.02 (m, 1H), 2.78 - 2.73 (m, 2H), 2.50 - 3.45 (m, 1H), 2.32 - 2.25 (m, 2H), 2.20 (d, J = 8.0 Hz, 3H), 2.19 - 2.06 (m, 2H), 1.83 - 1.77 (m, 2H), 1.56 - 1.47 (m, 2H), 1.21 (d, J = 6.3 Hz, 3H), 1.12 - 1.17 (m, 6H), 0.93 (s, 3H), 0.89 - 0.85 (m, 1H), 0.56 (dd, J = 7.6, 5.5 Hz, 1H), 0.49 (s, 3H).

### Example 9

### (1S,2S)-N-((63S,4S,Z)-12-(5-(3-((2S,6R)-2,6-dimethylmorpholino)prop-1-yn-1-yl)-2 -((S)-1-methoxyethyl)pyridin-3-yl)-11-ethyl-10,10-dimethyl-5,7-dioxo-61,62,63,64,65,66-hexahydro-11H-8-oxa-2(4,2)-thiazola-1(5,3)-indola-6(1,3)-pyridazinacyclonecaphane-4-yl)-2-methylcyclopropane-1-carboxamide

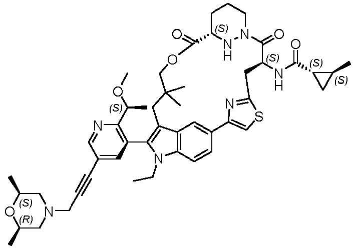

Example 9 is prepared by the following steps:

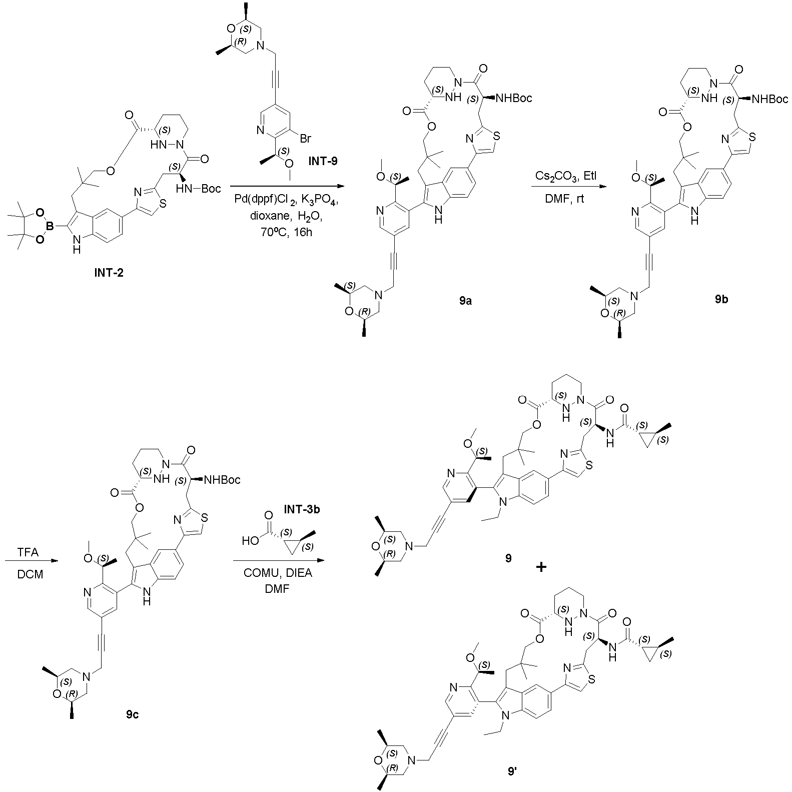

Step 1: **INT-3** (85.0 mg, 0.12 mmol), **INT-9** (30 mg, 0.08 mmol), 1,1-bis(diphenylphosphine) dioxonium dichloride palladium (6.0 mg, 0.008 mmol), potassium phosphate (52.0 mg, 0.25 mmol) were dissolved in a mixed solution of 1,4-dioxane (5 mL) and water (1 mL), and the reaction solution was stirred overnight at 70 °C under a nitrogen atmosphere. After the reaction was complete, the reaction solution was filtered, and the filtrate was concentrated to obtain a crude product. The crude product was purified by column chromatography (dichloromethane/methanol = 10/1) to obtain brown oily compound **9a** (69.76 mg, yield 100.00%). ESI-MS (m/z): 854.3 [M+H]⁺;

Step 2: Dissolve compound **9a** (60.00 mg, 0.07 mmol), cesium carbonate (45.78 mg, 0.14 mmol), iodoethane (21.91 mg, 0.14 mmol) in N,N-dimethylformamide (2 mL), the reaction solution was stirred overnight at room temperature. After the reaction, the reaction solution was extracted with ethyl acetate. Then the organic phases were combined, dried and concentrated to obtain light yellow oily compound **9b** (61.97 mg, yield 100.00%). ESI-MS (m/z): 882.5 [M+H]⁺;

Step 3: Compound **9b** (60 mg, 0.07 mmol) was dissolved in dichloromethane (2 mL), trifluoroacetic acid (77.55 mg, 0.68 mmol) was added to the above reaction solution, and the reaction solution was stirred overnight at room temperature. After the reaction was complete, saturated aqueous sodium bicarbonate solution was added to the reaction solution. The neutralized reaction solution was extracted with dichloromethane, and the combined organic phases were dried and concentrated to obtain compound **9c** (53.19 mg, yield 100.00%). ESI-MS (m/z): 782.5 [M+H]⁺;

Step 4: Compound **9c** (60 mg, 0.08 mmol), **INT-3b** (15.4 mg, 0.15 mmol), N-diisopropylethylamine (49.6 mg, 0.38 mmol) were dissolved in N,N-dimethylformamide (2 mL), and (1-Cyano-2-ethoxy-2-oxoethylidenaminooxy)dimethylamino-morpholino-carbenium hexafluorophosphate (65.7 mg, 0.15 mmol) was added to the reaction solution under 0°C. The reaction solution reacted at 0 °C for 2 hours. After the reaction was complete, the reaction solution was extracted with ethyl acetate, the combined organic phases were dried and concentrated to obtain the crude product, which was purified by preparative liquid chromatography to obtain compound 9 (4 mg, yield 6.0%) and epimer 9' (6 mg, yield 9.0%). The absolute configurations drawn by the two compounds are based on empirical assumptions. In the existing analytical methods, 9 is a compound with relatively low polarity and relatively long retention time in LC-MS and HPLC, and 9' is compound with relatively high polarity and relatively short retention time in LC-MS and HPLC.

### Compound 9:

ESI-MS (m/z): 864.6 [M+H]⁺; LC-MS retention time RT = 1.95 min. HPLC retention time RT = 14.28 min.

¹H NMR (500 MHz, DMSO-d₆) δ 8.81 (d, J = 2.0 Hz, 1H), 8.53-8.49 (m, 2H), 7.85 (d, J = 2.5 Hz, 1H), 7.81 (s, 1H), 7.76-7.74 (m, 1H), 7.59-7.57 (m, 1H), 5.56 (t, J = 9.5 Hz, 1H), 5.10 - 5.05 (m, 1H), 4.40-4.15 (m, 5H), 4.12 -4.05 (m, 1H), 3.62-3.55 (m, 7H), 3.25 (s, 3H), 3.17-3.12 (m, 1H), 2.98-2.95 (m, 1H), 2.78-2.75 (m, 3H), 2.40 - 2.35 (m, 1H), 2.09-2.07 (m, 1H), 1.93 - 1.88 (m, 2H), 1.79-1.76 (m, 2H), 1.60-1.45 (m, 3H), 1.35 (d, J = 6.0 Hz, 3H), 1.24 (s, 1H), 1.07 (s, 3H), 1.06 (s, 3H), 0.91 (s, 3H), 0.90-0.85 (m, 5H), 0.56-0.52 (m , 1H), 0.34 (s, 3H).

### Compound 9':

ESI-MS (m/z): 864.6 [M+H]⁺; LC-MS retention time RT = 1.87 min. HPLC retention time RT = 13.84 min.

### Example 10

### (1S,2S)-N-((63S,4S,Z)-11-ethyl-12-(2-((S)-1-methoxyethyl)-5-(((R)-1-methylpyrrolidin-2- yl)ethynyl)pyridin-3-yl)-10,10-dimethyl-5,7-dioxo-61,62,63,64,65,66-hexahydro-11H-8-oxa-2(4,2)- Thiazola-1(5,3)-indola-6(1,3)-pyridazinacyclonecaphane-4-yl)-2-methylcyclopropane-1-carboxamide

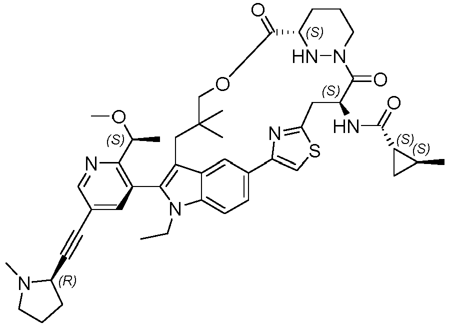

Example 10 was prepared by the following steps:

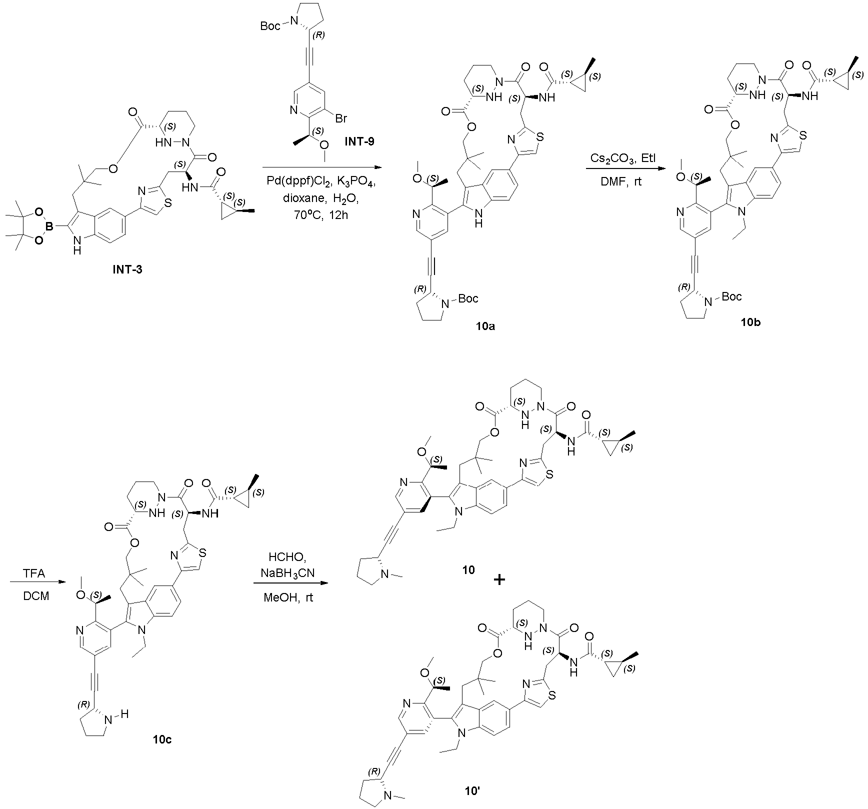

Step 1: Dissolve intermediate **Int-3** (55 mg, 0.08 mmol) and intermediate **Int-15** (30 mg, 0.08 mmol) in a mixed solvent of 1,4-dioxane (3 mL) and water (0.2 mL) , add 1,1-bis(diphenylphosphine)ironium dichloride palladium (6 mg, 0.01 mmol) and potassium phosphate (47 mg, 0.02 mmol) thereto. The reaction system was replaced with nitrogen and then heated to 70 °C and stirred for 12 hours. After the reaction solution was cooled to room temperature, the reaction solution was filtered through celite, and the filtrate was concentrated. The residue was purified by preparative thin-layer chromatography (dichloromethane/methanol=30/1) to obtain compound **10a** (62 mg, yield 96%). ESI-MS (m/z): 879.2 [M+H]⁺.

Step 2: Dissolve compound **10a** (62 mg, 0.07 mmol) in DMF (2 mL), add cesium carbonate (47 mg, 0.14 mmol), then add iodoethane (23 mg, 0.14 mmol) dropwise to the reaction solution. The reaction solution was stirred at room temperature for 6 hours. LCMS detected that the reaction was complete. Saturated brine was added to the reaction system, the reaction solution was extracted with ethyl acetate, the organic phases were combined, washed with saturated brine, dried over anhydrous sodium sulfate, filtered and concentrated to obtain the crude compound **10b** (64 mg, yield 99%). ESI-MS (m/z): 908.3 [M+H]⁺.

Step 3: Compound **10b** (64 mg, 0.07 mmol) was dissolved in dichloromethane (1 mL), and trifluoroacetic acid (41 mg, 0.36 mmol) was added dropwise to the reaction solution at 0 °C. The reaction solution was stirred at 0 °C for 1 hour. LCMS detected that the reaction was complete. Add saturated aqueous sodium bicarbonate solution to the reaction system to quench the reaction, the reaction solution was extracted with dichloromethane, the organic phases were combined, washed with saturated brine, dried over anhydrous sodium sulfate, filtered and concentrated to obtain compound **10c** (57 mg, yield 99%) crude products. ESI-MS (m/z): 807.6 [M+H]⁺.

Step 4: The crude compound **10c** (57 mg, 0.07 mmol) was dissolved in methanol (2 mL), and aqueous formaldehyde (0.05 mL) was added dropwise to the reaction solution at room temperature, and the reaction solution was stirred at room temperature for 10 min. Then sodium cyanoborohydride (14 mg, 0.21 mmol) was slowly added to the reaction solution, and the reaction solution was stirred at room temperature for 3 hours. LCMS detected that the reaction was complete. Add saturated ammonium chloride aqueous solution to the reaction system to quench the reaction. The reaction solution was extracted with dichloromethane, the organic phases were combined and concentrated, and the residue was purified by preparative liquid chromatography to obtain white solid compound **10** (3 mg, yield 5.2%) and the isomer 10' (4 mg, yield 6.9%). The absolute configurations drawn by the two compounds are based on empirical assumptions. In the existing analytical methods, 10 is a compound with relatively low polarity and relatively long retention time in LC-MS and HPLC, and 10' is compound with relatively high polarity and relatively short retention time in LC-MS and HPLC.

### ompound 10:

ESI-MS (m/z): 820.0 [M+H]⁺. LC-MS retention time RT = 1.97 min. HPLC retention time RT = 14.55 min.

¹H NMR (500 MHz, DMSO) δ 8.79 (d, J = 2.1 Hz, 1H), 8.55 - 8.47 (m, 2H), 7.82 (d, J = 2.1 Hz, 1H), 7.81 (s, 1H), 7.75 (dd, J = 8.7, 1.5 Hz, 1H), 7.58 (d, J = 8.7 Hz, 1H), 5.56 (t, J = 9.3 Hz, 1H), 5.10 - 5.05 (m, 1H), 4.36 - 4.15 (m, 4H), 4.12 - 4.03 (m, 1H), 3.57 (s, 2H), 3.37 (t, J = 6.8 Hz, 1H), 3.25 (s, 3H), 3.17 - 3.11 (m, 1H), 3.00 - 2.94 (m, 1H), 2.83 - 2.72 (m, 2H), 2.41 - 2.31 (m, 5H), 2.19 - 2.11 (m, 1H), 2.08 (d, J = 7.5 Hz, 1H), 1.94 - 1.68 (m, 6H), 1.56 - 1.46 (m, 2H), 1.35 (d, J = 6.1 Hz, 3H), 1.23 (s, 1H), 1.06 (s, 3H), 0.91 (s, 3H), 0.87 (t, J = 7.0 Hz, 4H), 0.57 - 0.52 (m, 1H), 0.34 (s, 3H).

### Compound 10':

ESI-MS (m/z): 820.0 [M+H]⁺. LC-MS retention time RT = 1.90 min. HPLC retention time RT = 13.87 min.

¹H NMR (500 MHz, DMSO) δ 8.80 (d, J = 2.1 Hz, 1H), 8.53 (dd, J = 9.3, 2.3 Hz, 2H), 7.96 (d, J = 2.1 Hz, 1H), 7.81 (s, 1H), 7.74 (dd, J = 8.7, 1.5 Hz, 1H), 7.54 (d, J = 8.7 Hz, 1H), 5.54 (t, J = 9.4 Hz, 1H), 5.07 - 5.02 (m, 1H), 4.27 - 4.18 (m, 2H), 3.99 - 3.90 (m, 2H), 3.85 - 3.80 (m, 1H), 3.70 - 3.66 (m, 1H), 3.57 - 3.52 (m, 1H), 3.42 - 3.37 (m, 1H), 3.31 (s, 2H), 3.19 - 3.01 (m, 5H), 2.80 - 2.75 (m, 2H), 2.40 - 2.28 (m, 5H), 2.18 - 2.12 (m, 2H), 1.92 - 1.69 (m, 5H), 1.57 - 1.47 (m, 2H), 1.21 (d, J = 6.3 Hz, 3H), 1.10 (t, J = 7.1 Hz, 3H), 1.07 (s, 3H), 0.93 (s, 3H), 0.90 - 0.85 (m, 1H), 0.56 (dd, J = 7.7, 5.5 Hz, 1H), 0.50 (s, 3H).

### Example 11

### (1r,2R,3S)-N-((63S,4S,Z)-11-ethyl-12-(2-((S)-1-methoxyethyl)-5-(((S)-1-methylpyrrolidin- 2-yl)ethynyl)pyridin-3-yl)-10,10-dimethyl-5,7-dioxo-61,62,63,64,65,66-hexahydro-11H-8-oxa-2(4,2 )-thiazola-1(5,3)-indola-6(1,3)-pyridazinacyclonecaphane-4-yl)-2,3-dimethylcyclopropane-1-carboxamide

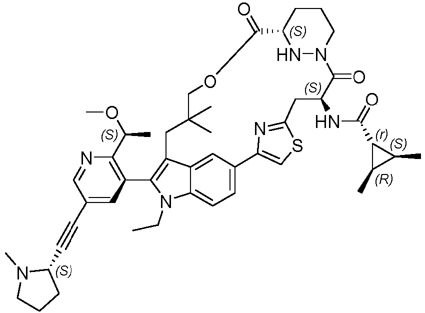

Example 11 is prepared by the following steps:

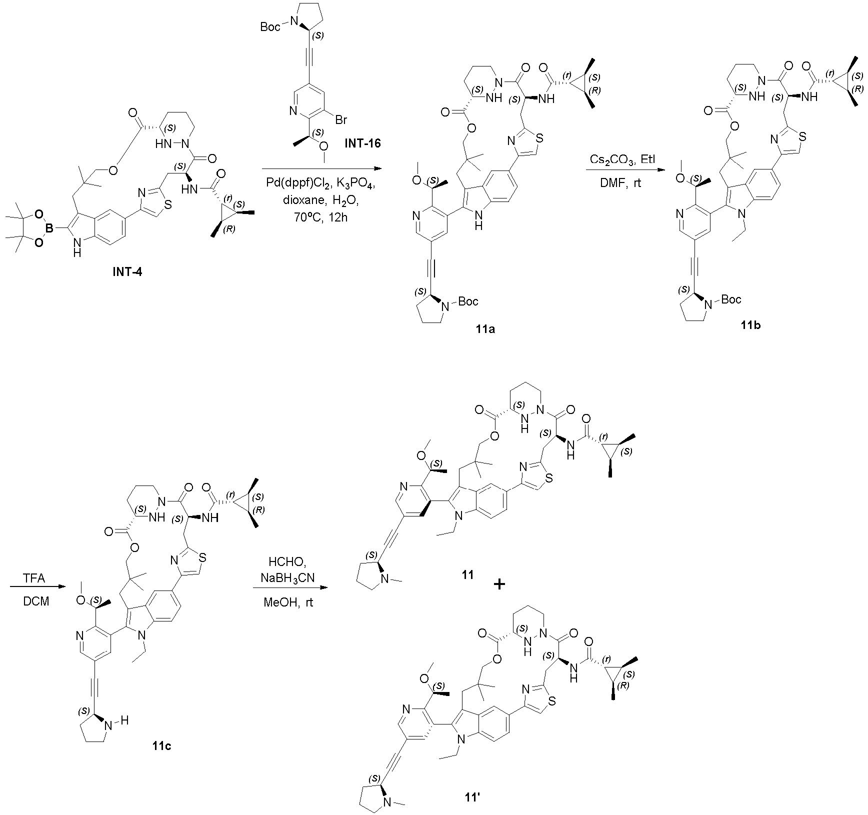

Step 1: Dissolve intermediate **Int-4** (93 mg, 0.14 mmol) and intermediate **Int-16** (50 mg, 0.12 mmol) in a mixed solvent of 1,4-dioxane (3 mL) and water (0.2 mL), add 1,1-bis(diphenylphosphine)ironium dichloride palladium (9 mg, 0.01 mmol) and potassium phosphate (78 mg, 0.37 mmol) thereto. After the system was replaced with nitrogen, it was heated to 70 °C and stirred for 12 hours. After the reaction solution was cooled to room temperature, the reaction solution was filtered through celite, and the filtrate was concentrated. The residue was purified by preparative thin-layer chromatography (dichloromethane/methanol=30/1) to obtain compound **11a** (105 mg, yield 96%). ESI-MS (m/z): 892.4 [M+H]⁺.

Step 2: Dissolve compound **11a** (105 mg, 0.12 mmol) in DMF (2 mL), add cesium carbonate (115 mg, 0.35 mmol), then add iodoethane (55 mg, 0.35 mmol) dropwise to the reaction solution. The reaction solution was stirred at room temperature for 6 hours. LCMS detected that the reaction was complete. Saturated brine was added to the reaction system, the reaction solution was extracted with ethyl acetate, the organic phases were combined, washed with saturated brine, dried over anhydrous sodium sulfate, filtered and concentrated to obtain the crude compound **11b** (108 mg, yield 99%). ESI-MS (m/z): 920.5 [M+H]⁺.

Step 3: Compound **11b** (108 mg, 0.12 mmol) was dissolved in dichloromethane (1 mL), and trifluoroacetic acid (67 mg, 0.59 mmol) was added dropwise to the reaction solution at 0 °C. The reaction solution was stirred at 0 °C for 1 hour. LCMS detected that the reaction was complete. Saturated aqueous sodium bicarbonate solution was added to the reaction system to quench the reaction, the reaction solution was extracted with dichloromethane, the organic phases were combined, washed with saturated brine, dried over anhydrous sodium sulfate, filtered and concentrated to obtain compound **11c** (96 mg, yield 99%) crude products. ESI-MS (m/z): 820.4 [M+H]⁺.

Step 4: The crude compound **11c** (96 mg, 0.12 mmol) was dissolved in methanol (2 mL), and aqueous formaldehyde (0.05 mL) was added dropwise to the reaction solution at room temperature, and the reaction solution was stirred at room temperature for 10 min. Then sodium cyanoborohydride (22 mg, 0.35 mmol) was slowly added to the reaction solution, and the reaction solution was stirred at room temperature for 3 hours. LCMS detected that the reaction was complete. Add saturated ammonium chloride aqueous solution to the reaction system to quench the reaction, extract the reaction solution with dichloromethane, combine and concentrate the organic phase, and the residue was purified by preparative liquid chromatography to obtain white solid compound **11** (3 mg, yield 3%) and epimer 11' (5 mg, yield 5%). The absolute configurations drawn by the two compounds are based on empirical assumptions. In the existing analytical methods, 11 is a compound with relatively low polarity and relatively long retention time in LC-MS and HPLC, and 11' is compound with relatively high polarity and relatively short retention time in LC-MS and HPLC.

### Compound 11:

ESI-MS (m/z): 834.5 [M+H]⁺. LC-MS retention time RT = 2.07 min. HPLC retention time RT = 15.25 min.

¹H NMR (500 MHz, DMSO) δ 8.79 (d, J = 2.0 Hz, 1H), 8.50 (s, 1H), 8.39 (d, J = 8.9 Hz, 1H), 7.82 (d, J = 2.1 Hz, 1H), 7.81 (s, 1H), 7.75 (dd, J = 8.6, 1.3 Hz, 1H), 7.58 (d, J = 8.7 Hz, 1H), 5.55 (t, J = 9.2 Hz, 1H), 5.09 - 5.02 (m, 1H), 4.37 - 4.13 (m, 5H), 4.12 - 4.02 (m, 1H), 3.57 (s, 2H), 3.24 (s, 3H), 3.18 - 3.10 (m, 1H), 2.99 - 2.92 (m, 1H), 2.82 - 2.77 (m, 1H), 2.76 - 2.71 (m, 1H), 2.36 (s, 3H), 2.35 - 2.31 (m, 1H), 2.19 - 2.12 (m, 1H), 2.10 - 2.05 (m, 1H), 1.94 - 1.72 (m, 6H), 1.55 - 1.46 (m, 1H), 1.35 (d, J = 6.1 Hz, 3H), 1.23 (s, 1H), 1.16 (t, J = 5.5 Hz, 2H), 1.08 (d, J = 5.9 Hz, 3H), 1.06 (d, J = 5.5 Hz, 3H), 0.91 (s, 3H), 0.88 (t, J = 7.1 Hz, 3H), 0.35 (s, 3H).

### Compound 11':

ESI-MS (m/z): 834.5 [M+H]⁺. LC-MS retention time RT = 2.01 min. HPLC retention time RT = 14.61 min.

### Example 12

### (1S,2S)-N-((63S,4S,Z)-11-ethyl-12-(2-((S)-1-methoxyethyl)-5-((1-methylazetidin-3-yl)ethynyl) pyridin-3-yl)-10,10-dimethyl-5,7-dioxo-61,62,63,64,65,66-hexahydro-11H-8-oxa-2(4,2)-thiazola-1(5,3)-indola-6(1,3)-pyridazinacyclonecaphane-4-yl)-2-methylcyclopropane-1-carboxamide

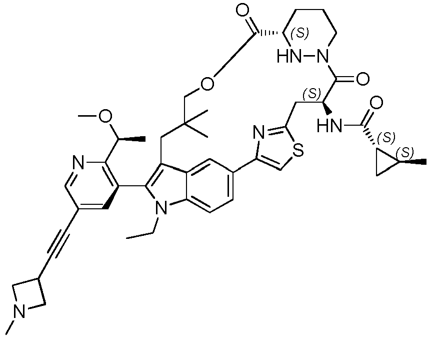

Example 12 is prepared by the following steps:

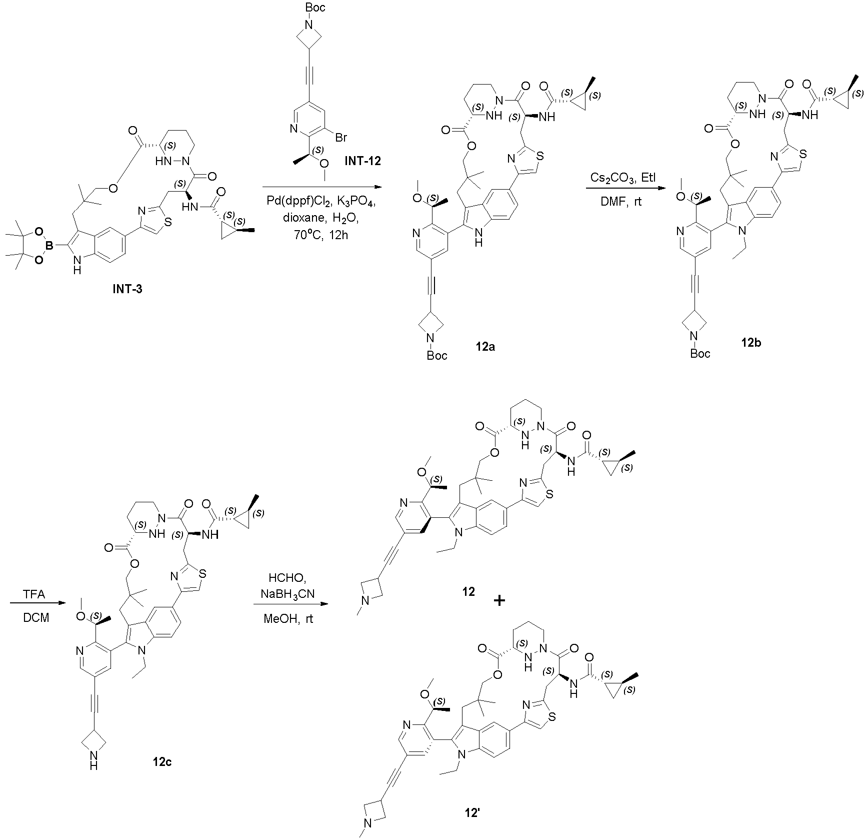

Replace **INT-15** in Example 10 with **INT-12,** and use similar methods and reaction steps to obtain compound **12** and epimer 12'. The absolute configurations drawn by the two compounds are based on empirical assumptions. In the existing analytical methods, 12 is a compound with relatively low polarity and relatively long retention time in LC-MS and HPLC, and 12' is compound with relatively high polarity and relatively short retention time in LC-MS and HPLC.

### Compound 12:

ESI-MS (m/z): 806.5 [M+H]⁺; LC-MS retention time RT = 1.88 min. HPLC retention time RT = 13.59 min.

¹H NMR (500 MHz, DMSO-d6) δ 8.77 (d, J = 2.1 Hz, 1H), 8.55 - 8.46 (m, 2H), 7.83 - 7.77 (m, 2H), 7.75 (dd, J = 8.7, 1.7 Hz, 1H), 7.58 (d, J = 8.7 Hz, 1H), 5.56 (t, J = 9.1 Hz, 1H), 5.10 - 5.05 (m, 1H), 4.36 - 4.16 (m, 4H), 4.07 (d, J = 7.5 Hz, 1H), 3.61 - 3.52 (m, 4H), 3.45 (p, J = 7.3 Hz, 1H), 3.25 (s, 3H), 3.18 - 3.06 (m, 3H), 2.99 - 2.94 (m, 1H), 2.79 - 2.72 (m, 1H), 2.40 - 2.35 (m, 1H), 2.22 (s, 3H), 2.12 - 2.05 (m, 1H), 1.84 - 1.75 (m, 2H), 1.54 - 1.46 (m, 2H), 1.35 (d, J = 6.0 Hz, 3H), 1.09 - 1.05 (m, 4H), 0.93 - 0.85 (m, 7H), 0.58 - 0.52 (m, 1H), 0.33 (s, 3H).

### Compound 12':

ESI-MS (m/z): 806.5 [M+H]⁺; LC-MS retention time RT = 1.83 min. HPLC retention time RT = 12.92 min.

¹H NMR (500 MHz, DMSO-d₆) δ 8.77 (d, J = 2.1 Hz, 1H), 8.55 - 8.46 (m, 2H), 7.83 - 7.77 (m, 2H), 7.75 (dd, J = 8.7, 1.7 Hz, 1H), 7.58 (d, J = 8.7 Hz, 1H), 5.56 (t, J = 9.1 Hz, 1H), 5.10 - 5.05 (m, 1H), 4.36 - 4.16 (m, 4H), 4.07 (d, J = 7.5 Hz, 1H), 3.61 - 3.52 (m, 4H), 3.45 (p, J = 7.3 Hz, 1H), 3.25 (s, 3H), 3.18 - 3.06 (m, 3H), 2.99 - 2.94 (m, 1H), 2.79 - 2.72 (m, 1H), 2.40 - 2.35 (m, 1H), 2.22 (s, 3H), 2.12 - 2.05 (m, 1H), 1.84 - 1.75 (m, 2H), 1.54 - 1.46 (m, 2H), 1.35 (d, J = 6.0 Hz, 3H), 1.09 - 1.05 (m, 4H), 0.93 - 0.85 (m, 7H), 0.58 - 0.52 (m, 1H), 0.33 (s, 3H).

### Example 13

### (1S,2S)-N-((63S,4S,Z)-11-ethyl-12-(2-((S)-1-methoxyethyl)-5-(((S)-1-methylazetidin-2- yl)ethynyl)pyridin-3-yl)-10,10-dimethyl-5,7-dioxo-61,62,63,64,65,66-hexahydro-11H-8-oxa-2(4,2)- Thiazola-1(5,3)-indola-6(1,3)-pyridazinacyclonecaphane-4-yl)-2-methylcyclopropane-1-carboxamide

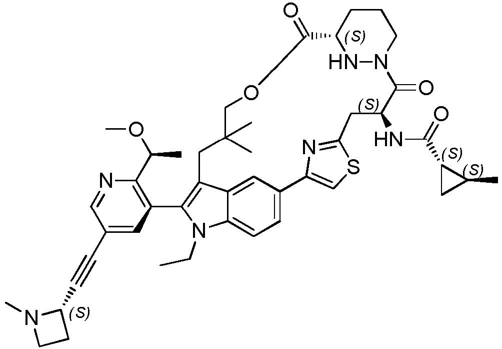

Example 13 is prepared by the following steps:

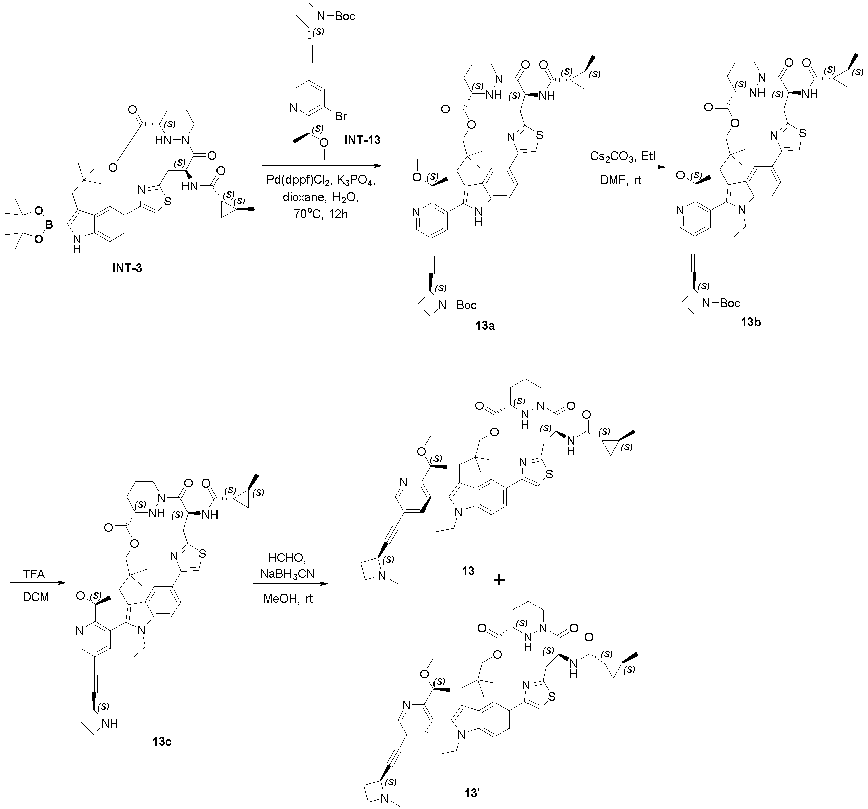

Replace **INT-15** in Example 10 with **INT-13,** and use similar methods and reaction steps to obtain compound **13** and epimer 13'. The absolute configurations drawn by the two compounds are based on empirical assumptions. In the existing analytical methods, 13 is a compound with relatively low polarity and relatively long retention time in LC-MS and HPLC, and 13' is compound with relatively high polarity and relatively short retention time in LC-MS and HPLC.

### Compound 13:

ESI-MS (m/z): 806.5 [M+H]⁺; LC-MS retention time RT = 1.88 min. HPLC retention time RT = 13.92 min.

### Compound 13':

ESI-MS (m/z): 806.5 [M+H]⁺; LC-MS retention time RT = 1.82 min. HPLC retention time RT = 13.32 min.

### Example 14

### (1,2R,3S)-N-((63S,4S,Z))-11-ethyl-12-(2-((S)-1-methoxyethyl)-5-(((S)-1-methylpyrrolidin- 3-yl)ethynyl)pyridin-3-yl)-10,10-dimethyl-5,7-dioxo-61,62,63,64,65,66-hexahydro-11H-8-oxa-2(4,2 )-thiazola-1(5,3)-indola-6(1,3)-pyridazinacyclonecaphane-4-yl)-2,3-dimethylcyclopropane-1-carboxamide

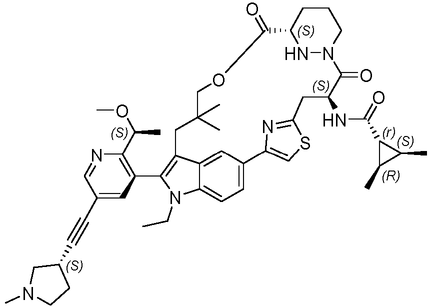

Example 14 is prepared by the following steps:

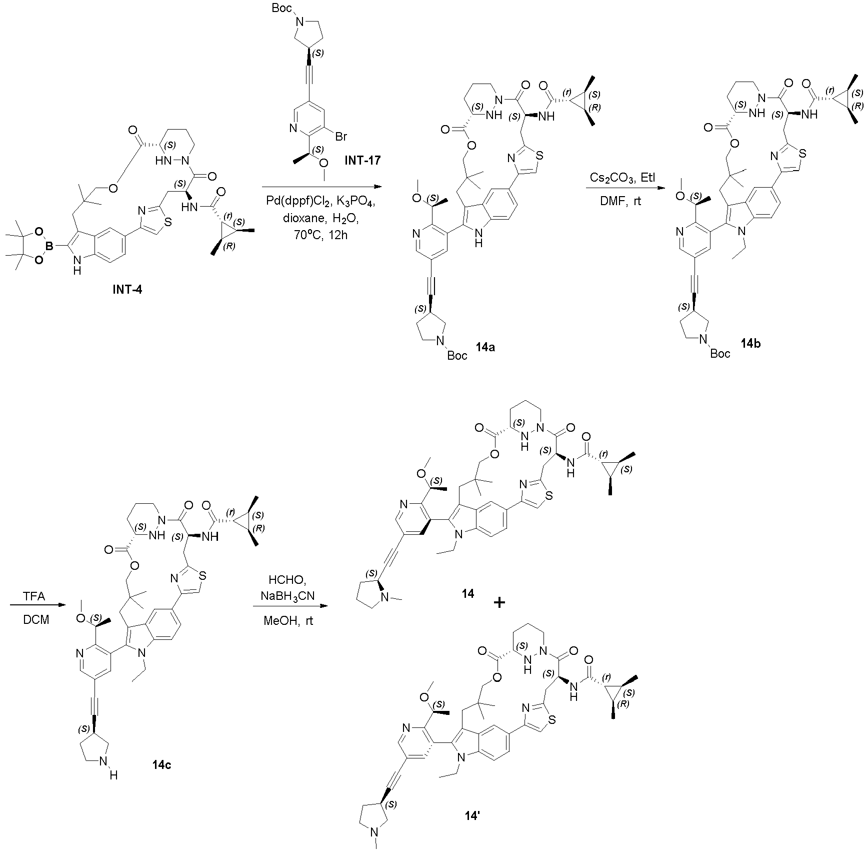

Replace **INT-15** in Example 10 with **INT-17,** and use similar methods and reaction steps to obtain compound **14** and epimer 14'. The absolute configurations drawn by the two compounds are based on empirical assumptions. In the existing analytical methods, 14 is a compound with relatively low polarity and relatively long retention time in LC-MS and HPLC, and 14' is compound with relatively high polarity and relatively short retention time in LC-MS and HPLC.

### Compound 14:

ESI-MS (m/z): 834.5 [M+H]⁺; LC-MS retention time RT = 2.02 min. HPLC retention time RT = 14.80 min.

¹H NMR (500 MHz, DMSO) δ 8.74 (d, J = 2.1 Hz, 1H), 8.49 (d, J = 1.3 Hz, 1H), 8.39 (d, J = 9.0 Hz, 1H), 7.81 (s, 1H ), 7.76 (d, J = 2.1 Hz, 1H), 7.75 (dd, J = 8.7, 1.6 Hz, 1H), 7.57 (d, J = 8.7 Hz, 1H), 5.55 (t, J = 9.4 Hz, 1H ), 5.10 - 5.05 (m, 1H), 4.35 - 4.29 (m, 1H), 4.25 (d, J = 6.1 Hz, 1H), 4.24 - 4.20 (m, 1H), 4.20 - 4.15 (m, 1H), 4.09 - 4.03 (m, 1H), 3.57 (s, 2H), 3.24 (s, 3H), 3.17 - 3.11 (m, 2H), 2.98 - 2.92 (m, 1H), 2.83 (t, J = 8.2 Hz, 1H), 2.76 - 2.72 (m, 1H), 2.48 - 2.45 (m, 1H), 2.40 - 5.35 (m, 1H), 2.26 (s, 3H), 2.24 - 2.20 (m, 1H), 2.10 - 2.05 (m, 1H), 2.03 - 1.99 (m, 1H), 1.91 - 1.83 (m, 2H), 1.80 - 1.78 (m, 1H), 1.34 (d, *J* = 6.1 Hz, 3H), 1.23 (s, 3H), 1.16 (t, *J* = 5.4 Hz, 3H), 1.08 (d, *J =* 5.9 Hz, 3H), 1.06 (d, *J* = 5.7 Hz, 3H), 0.91 (s, 3H), 0.88 (d, *J* = 7.0 Hz, 3H), 0.34 (s, 3H).

### Compound 14':

ESI-MS (m/z): 834.5 [M+H]⁺; LC-MS retention time RT = 1.95 min. HPLC retention time RT = 14.11 min.

¹H NMR (500 MHz, DMSO) δ 8.76 (d, J = 2.0 Hz, 1H), 8.52 (s, 1H), 8.40 (d, J = 9.0 Hz, 1H), 7.90 (d, J = 2.0 Hz, 1H), 7.82 (s, 1H), 7.73 (dd, J = 8.6, 1.3 Hz, 1H), 7.54 (d, J = 8.7 Hz, 1H), 5.53 (t, J = 9.3 Hz, 1H), 5.10 - 5.05 (m, 1H), 4.27 - 4.20 (m, 2H), 3.97 - 3.89 (m, 2H), 3.85 - 3.77 (m, 1H), 3.70 - 3.65 (m, 1H), 3.56 - 3.51 (m, 1H) , 3.26 - 3.21 (m, 2H), 3.19 - 3.12 (m, 2H), 3.08 (s, 3H), 3.07 - 3.02 (m, 1H), 2.86 - 2.82 (m, 1H), 2.81 - 2.71 (m, 2H), 2.56 - 2.53 (m, 1H), 2.45 (d, J = 7.2 Hz, 1H), 2.34 - 2.29 (m, 1H), 2.26 (s, 3H), 2.25 - 2.19 (m, 1H), 2.14 - 2.10 (m, 1H), 2.03 - 1.96 (m, 1H), 1.88 - 1.84 (m, 1H), 1.81 - 1.79 (m, 1H), 1.56 - 1.43 (m, 3H), 1.23 (s, 3H), 1.20 (d, *J* = 6.2 Hz, 3H), 1.18 (s, 1H), 1.11 (s, 1H), 1.09 (d, *J =* 5.7 Hz, 3H), 1.07 (d, *J=* 5.7 Hz, 3H), 0.93 (s, 3H), 0.85 (t, *J* = 6.2 Hz, 1H).

### Example 15

### (1S,2S)-N-((63S,4S,Z)-11-ethyl-12-(2-((S)-1-methoxyethyl)-5-(3-((R)-3-methylmorpholino) prop-1-yn-1-yl)pyridin-3-yl)-10,10-dimethyl-5,7-dioxo-61,62,63,64,65,66-hexahydro-11H-8-oxa-2 (4,2)-thiazola-1(5,3)-indola-6(1,3)-pyridazinacyclonecaphane-4-yl)-2-methylcyclopropane-1-carboxamide

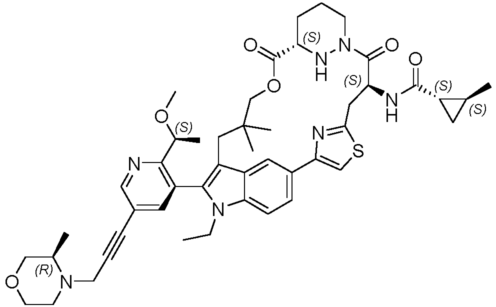

Example 15 is prepared by the following steps:

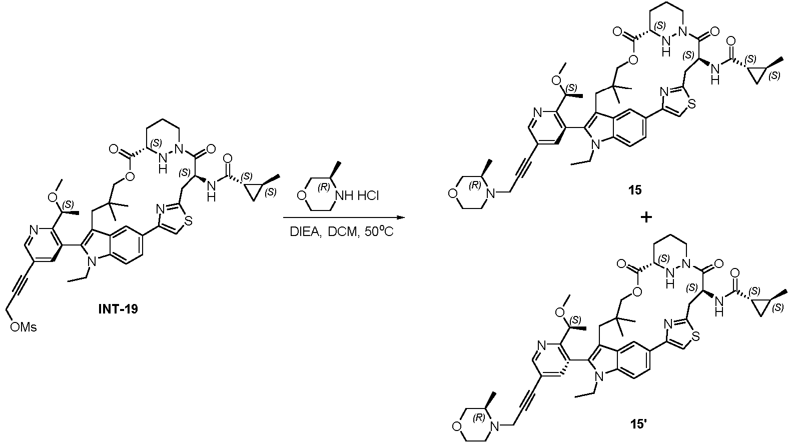

Step 1: Dissolve compound **INT-19** (20 mg, 0.02 mmol) in dichloromethane (5 mL), add thereto (R)-3 methylmorpholine hydrochloride (10 mg, 0.07 mmol) and diisopropylethylamine (18 mg, 0.14 mmol). The reaction mixture was stirred at 50 °C for 16 hours. After the reaction was complete, the reaction solution was concentrated, and the residue was purified by preparative liquid chromatography to obtain white solid compound **15** (3 mg, yield 15.0%) and epimer 15' (5 mg, yield 25.0%). The absolute configurations drawn by the two compounds are based on empirical assumptions. In the existing analytical methods, 15 is a compound with relatively low polarity and relatively long retention time in LC-MS and HPLC, and 15' is compound with relatively high polarity and relatively short retention time in LC-MS and HPLC.

### Compound 15:

ESI-MS (m/z): 850.6 [M+H]⁺; LC-MS retention time RT = 1.90 min. HPLC retention time RT=13.50 min.

¹H NMR (500 MHz, DMSO-d6) δ 8.80 (d, J = 2.1 Hz, 1H), 8.54 - 8.48 (m, 2H), 7.85 (d, J = 2.1 Hz, 1H), 7.81 (s, 1H), 7.75 (dd, J = 8.6, 1.6 Hz, 1H), 7.58 (d, J = 8.7 Hz, 1H), 5.56 (t, J = 9.1 Hz, 1H), 5.10 - 5.05 (m, 1H), 4.38 - 4.15 (m, 4H), 4.12 - 4.04 (m, 1H), 3.78 - 3.61 (m, 4H), 3.58 (s, 2H), 3.52 - 3.47 (m, 1H), 3.25 (s, 3H), 3.19 - 3.02 (m, 3H), 3.00 - 2.95 (m, 1H), 2.80 - 2.69 (m, 2H), 2.41 - 2.36 (m, 1H), 2.11 - 2.05 (m, 1H), 1.84 - 1.74 (m, 2H), 1.57 - 1.46 (m, 2H), 1.35 (d, J = 6.0 Hz, 3H), 1.07 (s, 3H), 0.94 - 0.91 (m, 6H), 0.88 (t, J = 7.0 Hz, 3H), 0.58 - 0.52 (m, 1H), 0.34 (s, 3H).

### Compound 15':

ESI-MS (m/z): 850.6 [M+H]⁺; LC-MS retention time RT = 1.86 min. HPLC retention time RT=13.14 min.

¹H NMR (500 MHz, DMSO-d6) δ 8.82 (d, J = 2.1 Hz, 1H), 8.58 - 8.48 (m, 2H), 7.99 (d, J = 2.1 Hz, 1H), 7.81 (s, 1H), 7.74 (dd, J = 8.6, 1.7 Hz, 1H), 7.54 (d, J = 8.6 Hz, 1H), 5.55 (t, J = 9.1 Hz, 1H), 5.10 - 5.05 (m, 1H), 4.28 - 4.16 (m, 2H), 4.01 - 3.89 (m, 2H), 3.87 - 3.73 (m, 3H), 3.71 - 3.60 (m, 3H), 3.57 - 3.45 (m, 2H), 3.18 - 3.12 (m, 1H ), 3.09 (s, 3H), 3.07 - 3.02 (m, 2H), 2.83 - 2.67 (m, 2H), 2.63 - 2.53 (m, 2H), 2.35 - 2.30 (m, 1H), 2.15 - 2.0 (m, 1H), 1.85 - 1.74 (m, 2H), 1.58 - 1.45 (m, 2H), 1.22 (d, J = 6.2 Hz, 3H), 1.11 (t, J = 7.1 Hz, 3H), 1.07 (s, 3H), 0.95 - 0.91 (m, 6H), 0.90 - 0.84 (m, 1H), 0.58 - 0.53 (m, 1H), 0.50 (s, 3H).

### Example 16

### (1S,2S)-N-((63S,4S,Z)-11-ethyl-12-(2-((S)-1-methoxyethyl)-5-(pyridin-2-ylethynyl)pyridin-3-yl )-10,10-dimethyl-5,7-dioxo-61,62,63,64,65,66-hexahydro-11H-8-oxa-2(4,2)-thiazola-1(5,3)- indola-6(1,3)-pyridazinacyclonecaphane-4-yl)-2-methylcyclopropane-1-carboxamide

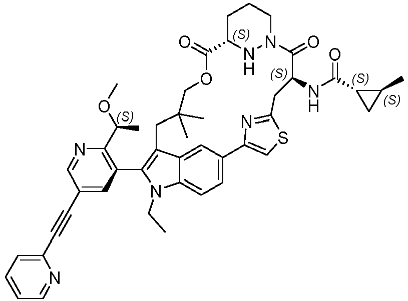

Example 16 is prepared by the following steps:

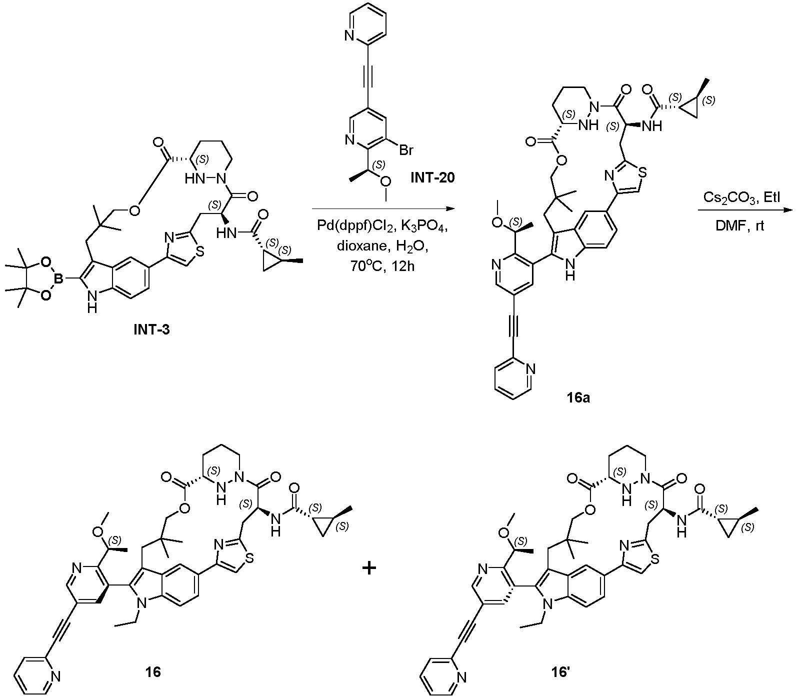

Replace INT-15 in Example 10 with INT-20, and use similar methods and reaction steps to obtain compound **16** and epimer 16'. The absolute configurations drawn by the two compounds are based on empirical assumptions. In the existing analytical methods, 16 is a compound with relatively low polarity and relatively long retention time in LC-MS and HPLC, and 16' is compound with relatively high polarity and relatively short retention time in LC-MS and HPLC.

### Compound 16:

ESI-MS (m/z): 814.5 [M+H]⁺; LC-MS retention time RT = 1.89 min. HPLC retention time RT = 14.05 min.

¹H NMR (500 MHz, DMSO-d6) δ 8.92 (dd, J = 7.1, 2.1 Hz, 1H), 8.63 - 8.54 (m, 1H), 8.50 - 8.39 (m, 2H), 7.99 (d, J = 2.1 Hz, 1H), 7.83 (tt, J = 7.7, 2.1 Hz, 1H), 7.74 (s, 1H), 7.71 - 7.65 (m, 2H), 7.53 (d, J = 8.8 Hz, 1H), 7.42 - 7.37 (m, 1H), 5.49 (t, J = 9.1 Hz, 1H), 5.10 - 5.05 (m, 1H), 4.33 - 4.21 (m, 2H), 4.20 - 4.09 (m, 2H), 4.07 - 4.02 (m, 1H), 3.52 (s, 2H), 3.26 - 3.23 (m, 2H), 3.21 (s, 1H), 3.10 - 3.05 (m, 1H), 2.95 - 2.88 (m, 1H), 2.73 - 2.65 (m, 1H), 2.40 - 2.35 (m, 1H), 2.05 - 2.00 (m, 1H), 1.72 (s, 3H), 1.51 - 1.40 (m, 2H), 1.32 (t, J = 6.4 Hz, 3H), 1.17 (s, 2H), 1.02 - 0.99 (m, 3H), 0.89 - 0.80 (m, 6H), 0.52 - 0.45 (m, 1H), 0.29 (s, 3H).

### Compound 16':

ESI-MS (m/z): 814.5 [M+H]⁺; LC-MS retention time RT = 1.86 min. HPLC retention time RT = 13.61 min.

¹H NMR (500 MHz, DMSO-d₆) δ 9.00 (d, J = 2.0 Hz, 1H), 8.64 (dt, J = 4.7, 1.4 Hz, 1H), 8.57 - 8.48 (m, 2H), 8.20 (d, J = 2.1 Hz, 1H), 7.90 (td, J = 7.7, 1.8 Hz, 1H), 7.82 (s, 1H), 7.77 - 7.70 (m, 2H), 7.56 (d, J = 8.7 Hz, 1H), 7.50 - 7.45 (m, 1H),5.55 (t, J = 9.2 Hz, 1H), 5.10 - 5.05 (m, 1H), 4.25 - 4.20 (m, 2H), 4.03 - 3.93 (m, 2H), 3.89 - 3.84 (m, 1H), 3.70 - 3.65 (m, 1H), 3.60 - 3.55 (m, 1H), 3.33 (s, 1H), 3.19 - 3.14 (m, 1H), 3.12 (s, 3H), 3.05 ( s, 1H), 2.77 (s, 1H), 2.38 - 2.32 (m, 1H), 2.15 - 2.10 (m, 1H), 1.80 (s, 2H), 1.57 - 1.45 (m, 2H), 1.24 (d, J = 6.3 Hz, 3H), 1.14 (t, J = 7.1 Hz, 3H), 1.09 - 1.06 (m, 4H), 0.94 (s, 3H), 0.88 (d, J = 6.4 Hz, 1H), 0.57 (t, J = 4.2 Hz, 1H), 0.53 (s, 3H).

### Example 17

### (1S,2S)-N-((63S,4S,Z)-11-ethyl-12-(2-((S)-1-methoxyethyl)-5-(pyridin-3-ylethynyl)pyridin-3-yl )-10,10-dimethyl-5,7-dioxo-61,62,63,64,65,66-hexahydro-11H-8-oxa-2(4,2)-thiazola-1(5,3)- indola-6(1,3)-pyridazinacyclonecaphane-4-yl)-2-methylcyclopropane-1-carboxamide

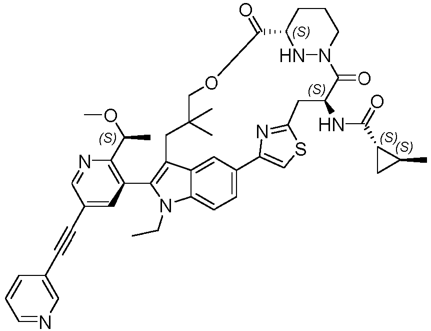

Example 17 is prepared by the following steps:

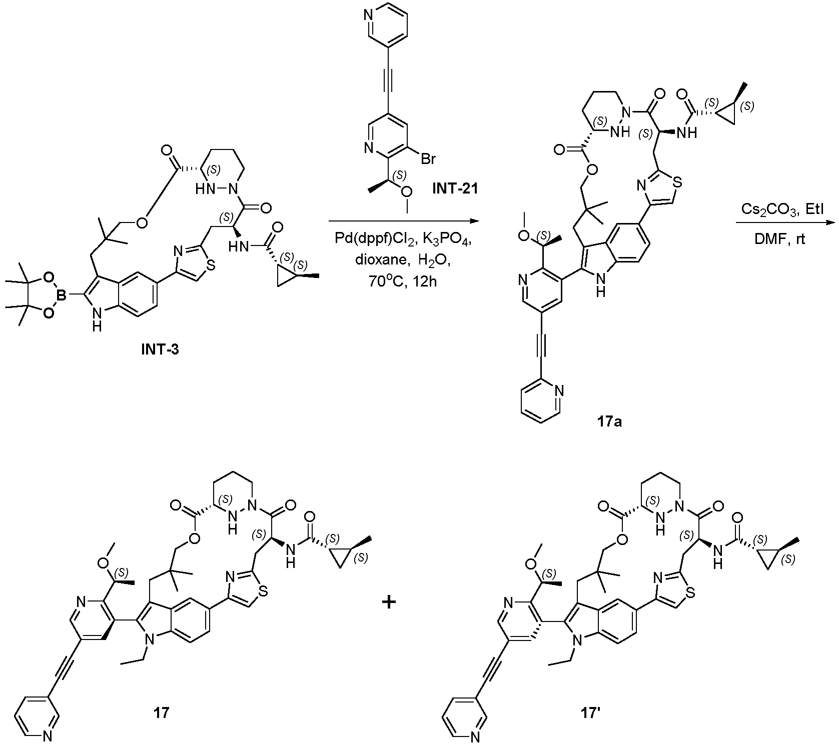

Replace **INT-15** in Example 10 with **INT-21,** and use similar methods and reaction steps to obtain compound 17 and epimer 17'. The absolute configurations drawn by the two compounds are based on empirical assumptions. In the existing analytical methods, 17 is a compound with relatively low polarity and relatively long retention time in LC-MS and HPLC, and 17' is compound with relatively high polarity and relatively short retention time in LC-MS and HPLC.

### Compound 17:

ESI-MS (m/z): 814.5 [M+H]⁺; LC-MS retention time RT = 1.91 min. HPLC retention time RT = 14.20 min.

¹H NMR (500 MHz, DMSO-d₆) δ 8.97 (d, J = 2.1 Hz, 1H), 8.83 (dd, J = 2.2, 0.9 Hz, 1H), 8.63 (dd, J = 4.9, 1.7 Hz, 1H), 8.56 - 8.48 (m, 2H), 8.05 (dd, J = 7.6, 2.0 Hz, 2H), 7.82 (s, 1H), 7.77 (dd, J = 8.7, 1.6 Hz, 1H), 7.60 (d, J = 8.7 Hz, 1H), 7.53 - 7.48 (m, 1H), 5.56 (t, J = 9.1 Hz, 1H), 5.10 - 5.05 (m, 1H), 4.40 - 4.29 (m, 2H), 4.28 - 4.14 (m, 2H), 4.12 - 4.07 (m, 1H), 3.59 (s, 2H), 3.32 (s, 1H), 3.28 (s, 3H), 3.18 - 3.13 (m, 1H), 3.00 - 2.95 (m, 1H), 2.80 - 2.74 (m, 1H), 2.45 - 2.40 (m, 1H), 2.12 - 2.07 (m, 1H), 1.79 (s, 2H), 1.56 - 1.47 (m, 2H), 1.38 (d, J = 6.1 Hz, 3H), 1.11 - 1.05 (m, 4H), 0.93 (s, 3H), 0.93 - 0.88 (m, 5H), 0.57 - 0.51 (m, 1H), 0.36 (s, 3H).

### Compound 17':

ESI-MS (m/z): 814.5 [M+H]⁺; LC-MS retention time RT = 1.86 min. HPLC retention time RT = 13.67 min.

¹H NMR (500 MHz, DMSO-d₆) δ 8.98 (d, J = 2.0 Hz, 1H), 8.82 (dd, J = 2.1, 0.9 Hz, 1H), 8.63 (dd, J = 4.9, 1.7 Hz, 1H), 8.58 - 8.50 (m, 2H), 8.18 (d, J = 2.1 Hz, 1H), 8.05 (dt, J = 8.0, 1.9 Hz, 1H), 7.82 (s, 1H), 7.75 (dd, J = 8.7 , 1.6 Hz, 1H), 7.56 (d, J = 8.7 Hz, 1H), 7.53 - 7.47 (m, 1H), 5.55 (t, J = 9.2 Hz, 1H), 5.10 - 5.05 (m, 1H), 4.29 - 4.13 (m, 2H), 4.00 - 3.95 (m, 2H), 3.90 - 3.85 (m, 1H), 3.70 - 3.65 (m, 1H), 3.58 - 3.53 (m, 1H), 3.32 (s, 1H) , 3.19 - 3.14 (m, 1H), 3.12 (s, 3H), 3.10 - 3.05 (m, 1H), 2.81 - 2.74 (m, 1H), 2.40 - 2.35 (m, 1H), 2.16 - 2.11 (m, 1H), 1.84 - 1.79 (m, 2H), 1.58 - 1.42 (m, 2H), 1.24 (d, J = 6.3 Hz, 3H), 1.13 (t, J = 7.2 Hz, 3H), 1.07 (d, J = 1.7 Hz, 4H), 0.94 (s, 3H), 0.88 (h, J = 3.7 Hz, 1H), 0.60 - 0.55 (m, 1H), 0.52 (s, 3H).

### Example 18

### (1r,2R,3S)-N-((6³S,4S,Z)-1¹-ethyl-1²-(2-((S)-1-methoxyethyl)-5-(((R)-1-methylpyrrolidin-3-yl)ethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

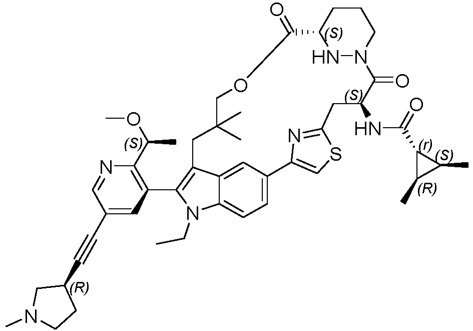

Example 18 was prepared by the following steps:

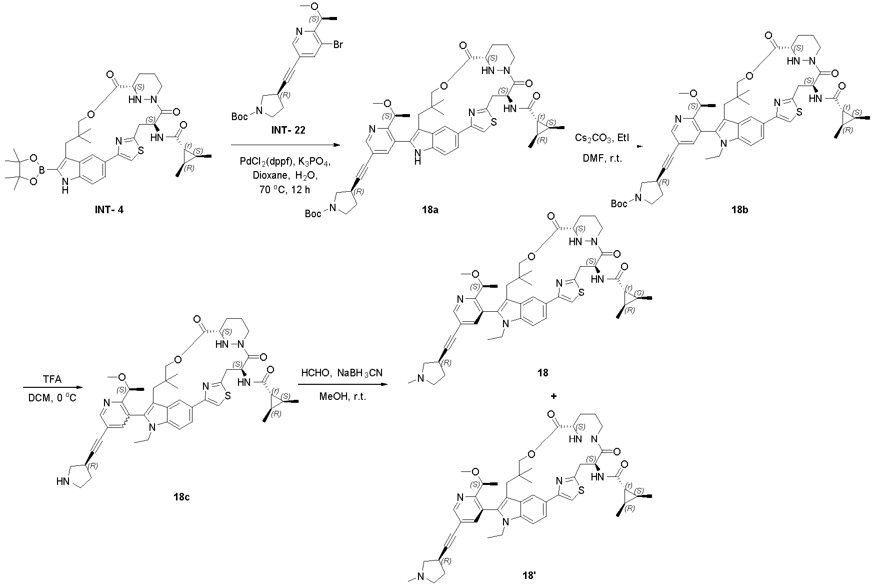

By replacing **INT-16** in the synthesis step of compound 11 with compound **INT-22,** compound **18** can be obtained using similar methods and reaction steps. **18** is a relatively less polar compound with relatively longer LC-MS and HPLC retention times. According to experience, the in vitro cell activity of compound **18** is much better than that of its epimer **18',** so the following examples will not further characterize the structure of the epimer.

### Compound 18:

ESI-MS (m/z): 834.7 [M+H]⁺; LC-MS retention time RT = 1.95 min. HPLC retention time RT = 14.85 min.

¹H NMR (500 MHz, DMSO) δ 8.74 (d, *J* = 2.0 Hz, 1H), 8.49 (d, *J* = 1.0 Hz, 1H), 8.39 (d, *J* = 9.0 Hz, 1H), 7.81 (s, 1H), 7.77 - 7.73 (m, 2H), 7.57 (d, *J* = 9.0 Hz, 1H), 5.55 (t, *J* = 9.5 Hz, 1H), 5.08 - 5.04 (m, 1H), 4.35 - 4.15 (m, 5H), 4.11 - 4.02 (m, 1H), 3.57 (s, 2H), 3.24 (s, 3H), 3.17 - 3.11 (m, 2H), 2.98 - 2.92 (m, 1H), 2.83 (t, *J* = 8.0 Hz, 1H), 2.79 - 2.70 (m, 1H), 2.40 - 2.35 (m, 1H), 2.25 (s, 3H), 2.24 - 2.19 (m, 1H), 2.10 - 2.05 (m, 1H), 1.91 - 1.83 (m, 2H), 1.82 - 1.76 (m, 2H), 1.56 - 1.47 (m, 2H), 1.34 (d, *J =* 6.0 Hz, 3H), 1.25 - 1.22 (m, 2H), 1.18 - 1.15 (m, 2H), 1.09 - 1.05 (m, 6H), 0.91 (s, 3H), 0.87 (t, *J=* 7.0 Hz, 3H), 0.33 (s, 3H).

### Example 19

### (1r,2R,3S)-N-((6³S,4S,Z)-1²-(5-((1,3-dimethylazetidin-3-yl)ethynyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

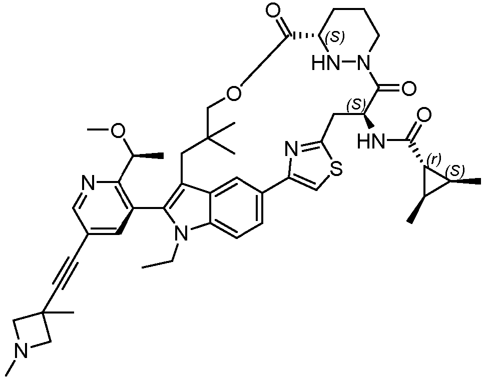

By replacing **INT-16** in the synthesis steps of compound **11** with compound **INT-23,** compound **19** can be obtained using similar methods and reaction steps. ESI-MS (m/z): 834.6 [M+H]⁺; LC-MS retention time RT=1.95 min. HPLC retention time RT = 14.62 min.

¹H NMR (500 MHz, DMSO-*d*₆) δ 8.76 (d, *J* = 2.0 Hz, 1H), 8.50 (d, *J* = 1.5 Hz, 1H), 8.39 (d, *J= 9.0* Hz, 1H), 7.82 (s, 1H), 7.78 (d, *J* = 2.0 Hz, 1H), 7.75 (dd, *J* = 8.5, 1.5 Hz, 1H), 7.58 (d, *J* = 9.0 Hz, 1H), 5.55 (t, *J* = 9.0 Hz, 1H), 5.10 - 5.01 (m, 1H), 4.35 - 4.16 (m, 4H), 4.11 - 4.02 (m, 1H), 3.57 (s, 2H), 3.30 - 3.28 (m, 2H), 3.25 (s, 3H), 3.22 - 3.19 (m, 2H), 3.18 - 3.11 (m, 1H), 3.00 - 2.93 (m, 1H), 2.79 - 2.72 (m, 1H), 2.41 - 2.35 (m, 1H), 2.24 (s, 3H), 2.11 - 2.05 (m, 1H), 2.03 - 1.95 (m, 1H), 1.83 - 1.74 (m, 2H), 1.56 (s, 3H), 1.54 - 1.43 (m, 2H), 1.35 (d, *J=* 6.0 Hz, 3H), 1.18 - 1.15 (m, 2H), 1.10 - 1.05 (m, 6H), 0.92 (s, 3H), 0.89 - 0.85 (m, 3H), 0.34 (s, 3H).

### Example 20

### (1S,2S)-N-((6³S,4S,Z)-1¹-ethyl-1²-(5-(3-((R)-2-(hydroxymethyl)morpholino)prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

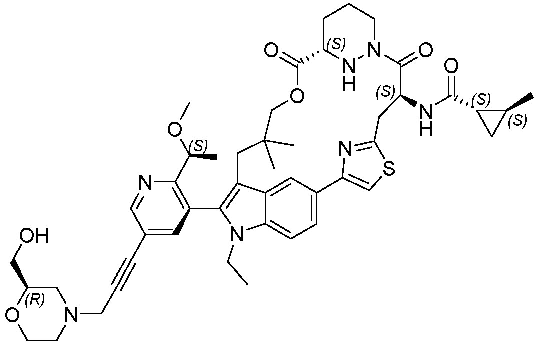

By replacing (R)-3-methylmorpholine hydrochloride in the synthesis step of compound **15** with (R)-morpholine-2-methanol hydrochloride, compound **20** can be obtained using a similar method and reaction steps. ESI-MS (m/z): 866.6 [M+H]⁺; LC-MS retention time RT=1.63 min.

### Example 21

### (1r,2R,3S)-N-((6³S,4S,Z)-1¹-ethyl-1²-(5-(3-((R)-3-fluoropyrrolidin-1-yl)prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

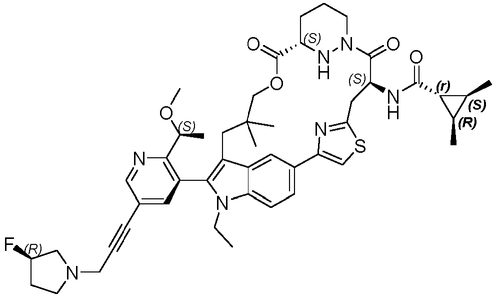

Compound **21** can be obtained by replacing (R)-3-methylmorpholine hydrochloride in the synthesis step of compound **15** with compound 3-(R)-fluoropyrrolidine and replacing **INT-19** with **INT-25,** using similar methods and reaction steps. ESI-MS (m/z): 852.6 [M+H]⁺; LC-MS retention time RT = 1.91 min.

¹H NMR (500 MHz, DMSO-*d*₆) δ 8.82 (d, *J* = 2.0 Hz, 1H), 8.51 - 8.49 (m, 1H), 8.39 (d, *J* = 9.0 Hz, 1H), 7.89 - 7.84 (m, 1H), 7.82 (s, 1H), 7.77 - 7.74 (m, 1H), 7.58 (d, *J* = 9.0 Hz, 1H), 5.56 (t, *J =* 9.1 Hz, 1H), 5.09 - 5.03 (m, 1H), 4.36 - 4.04 (m, 5H), 3.95 - 3.74 (m, 2H), 3.57 (s, 2H), 3.25 (s, 3H), 3.18 - 3.12 (m, 2H), 2.95 - 2.92 (m, 1H), 2.79 - 2.72 (m, 1H), 2.41 - 2.35 (m, 1H), 2.22 - 1.16 (m, 1H), 2.11- 2.04 (m, 1H), 1.86 - 1.70 (m, 3H), 1.55 - 1.48 (m, 1H), 1.35 (d, *J= 6.0* Hz, 3H), 1.26 - 1.22 (m, 2H), 1.17 - 1.15 (m, 2H), 1.10 - 1.05 (m, 6H), 1.04 - 0.97 (m, 2H), 0.92 - 0.86 (m, 6H), 0.72 - 0.63 (m, 2H), 0.34 (s, 3H).

### Example 22

### (1S,2S)-N-((6³S,4S,Z)-1¹-ethyl-1²-(2-((S)-1-methoxyethyl)-5-(pyrimidin-5-ylethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

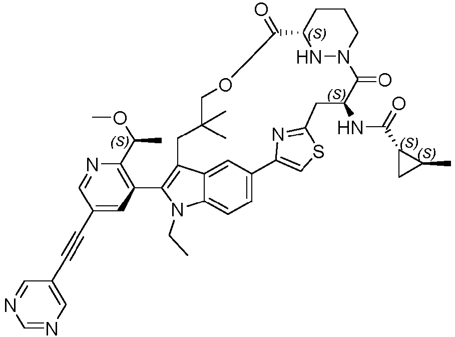

By replacing **INT-15** in the synthesis steps of compound **10** with compound **INT-24,** compound **22** can be obtained using similar methods and reaction steps. ESI-MS (m/z): 815.8 [M+H]⁺; LC-MS retention time RT=1.84 min. HPLC retention time RT = 13.73 min.

¹H NMR (500 MHz, DMSO-*d*₆) δ 9.23 - 9.22 (m, 1H), 9.09 - 9.07 (m, 2H), 8.99 - 8.98 (m, 1H), 8.53 - 8.50 (m, 2H), 8.08 - 8.06 (m, 1H), 7.81 (s, 1H), 7.76 (d, *J* = 8.6 Hz, 1H), 7.60 (d, *J =* 8.6 Hz, 1H), 5.56 (t, *J =* 9.1 Hz, 1H), 5.09 - 5.05 (m, 1H), 4.39 - 4.31 (m, 2H), 4.25 - 4.17 (m, 2H), 4.13 - 4.07 (m, 1H), 3.58 (s, 2H), 3.31 - 3.30 (m, 1H), 3.27 (s, 3H), 3.17 - 3.12 (m, 1H), 3.01 - 2.97 (m, 1H), 2.78 - 2.74 (m, 1H), 2.44 - 2.40 (m, 1H), 2.10 - 2.06 (m, 1H), 1.82 - 1.76 (m, 2H), 1.53 - 1.48 (m, 2H), 1.38 (d, *J= 6.0* Hz, 3H), 1.08 - 1.05 (m, 4H), 0.93 (s, 3H), 0.91 - 0.86 (m, 4H), 0.56 - 0.53 (m, 1H), 0.35 (s, 3H).

### Example 23

### (1S,2S)-N-((6³S,4S,Z)-1¹-ethyl-1²-(2-((S)-1-methoxyethyl)-5-(3-morpholinoprop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-phenylcyclopropane-1-carboxamide

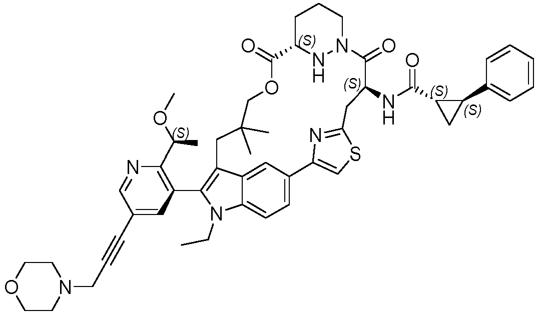

By replacing **INT-3b** in the synthesis step of compound **1** with compound (1S,2S)-2-phenylcyclopropylcarboxylic acid, compound **23** can be obtained using a similar method and reaction steps. ESI-MS (m/z): 898.7 [M+H]⁺; LC-MS retention time RT=1.92 min. HPLC retention time RT = 14.35 min.

¹H NMR (500 MHz, DMSO-*d*₆) δ 8.81 (d, *J =* 2.0 Hz, 1H), 8.70 (d, *J =* 9.0 Hz, 1H), 8.53 - 8.49 (m, 1H), 7.86 (d, *J =* 2.0 Hz, 1H), 7.83 (s, 1H), 7.78 - 7.75 (m, 1H), 7.61 - 7.57 (m, 1H), 7.33 - 7.28 (m, 2H), 7.22 - 7.17 (m, 1H), 7.16 - 7.12 (m, 2H), 5.61 (t, *J =* 9.0 Hz, 1H), 5.15 - 5.09 (m, 1H), 4.37 - 4.17 (m, 4H), 4.12 - 4.05 (m, 1H), 3.63 - 3.57 (m, 8H), 3.37 - 3.34 (m, 1H), 3.26 (s, 3H), 3.13 - 3.08 (m, 1H), 3.00 - 2.95 (m, 1H), 2.80 - 2.73 (m, 1H), 2.56 - 2.52 (m, 4H), 2.41 - 2.36 (m, 1H), 2.22 - 2.16 (m, 1H), 2.12 - 2.06 (m, 2H), 1.82 - 1.76 (m, 2H), 1.56 - 1.50 (m, 1H), 1.39 - 1.34 (m, 3H), 1.29 - 1.23 (m, 2H), 0.92 (s, 3H), 0.88 (t, *J* = 7.0 Hz, 3H), 0.34 (s, 3H).

### Example 24

### (1S,2S)-N-((6³S,4S,Z)-1¹-ethyl-1²-(2-((S)-1-methoxyethyl)-5-(3-morpholinoprop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-fluorocyclopropane-1-carboxamide

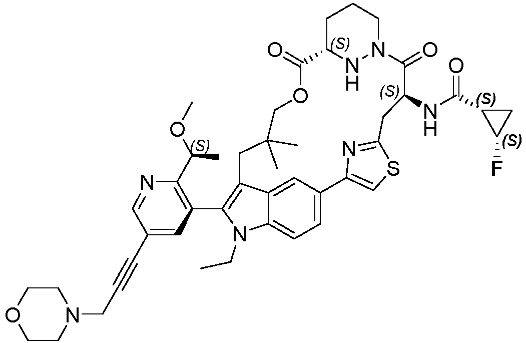

By replacing **INT-3b** in the synthesis step of compound 1 with compound (1S,2S)-2-fluorocyclopropanecarboxylic acid, compound **24** can be obtained using a similar method and reaction steps. ESI-MS (m/z): 840.7 [M+H]⁺; LC-MS retention time RT=1.69 min. HPLC retention time RT = 12.20 min.

¹H NMR (500 MHz, DMSO-*d*₆) δ 8.83 - 8.80 (m, 1H), 8.67 - 8.62 (m, 1H), 8.52 - 8.47 (m, 1H), 7.88 - 7.85 (m, 1H), 7.83 (s, 1H), 7.78 - 7.74 (m, 1H), 7.61 - 7.55 (m, 1H), 5.57 (t, *J* = 9.0 Hz, 1H), 5.14 - 5.07 (m, 1H), 4.96 - 4.76 (m, 1H), 4.36 - 4.18 (m, 4H), 4.12 - 4.04 (m, 1H), 3.64 - 3.55 (m, 8H), 3.26 (s, 3H), 3.20 - 3.13 (m, 1H), 3.02 - 2.93 (m, 1H), 2.81 - 2.73 (m, 1H), 2.57 - 2.53 (m, 4H), 2.40 - 2.35 (m, 1H), 2.12 - 2.06 (m, 1H), 1.96 - 1.89 (m, 1H), 1.84 - 1.75 (m, 2H), 1.55 - 1.44 (m, 3H), 1.35 (d, *J* = 6.0 Hz, 3H), 1.07 - 1.02 (m, 1H), 0.92 (s, 3H), 0.87 (t, *J =* 7.0 Hz, 3H), 0.34 (s, 3H).

### Example 25

### (1r,2R,3S)-N-((63S,4S,Z)-1¹-ethyl-1²-(5-(3-((S)-3-(hydroxymethyl)morpholino)prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

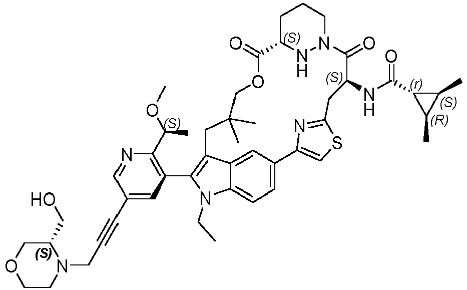

Compound **25** can be obtained by replacing (R)-3-methylmorpholine hydrochloride in the synthesis step of compound **15** with compound (S)-3-hydroxymethylmorpholine and replacing **INT-19** with **INT-25,** using similar methods and reaction steps. ESI-MS (m/z): 880.6 [M+H]⁺; LC-MS retention time RT = 1.68 min.

¹H NMR (500 MHz, DMSO-*d*₆) δ 8.81 (d, *J* = 2.0 Hz, 1H), 8.51 - 8.48 (m, 1H), 8.39 (d, *J =* 8.5 Hz, 1H), 7.85 (d, *J =* 2.0 Hz, 1H), 7.81 (s, 1H), 7.77 - 7.73 (m, 1H), 7.58 (d, *J* = 8.5 Hz, 1H), 5.55 (t, *J* = 9.0 Hz, 1H), 5.10 - 5.02 (m, 1H), 4.60 (t, *J* = 5.5 Hz, 1H), 4.38 - 4.04 (m, 5H), 3.83 - 3.78 (m, 2H), 3.76 - 3.71 (m, 2H), 3.62 - 3.56 (m, 3H), 3.25 (s, 3H), 3.17 - 3.11 (m, 2H), 2.99 - 2.93 (m, 1H), 2.79 - 2.68 (m, 2H), 2.64 - 2.59 (m, 2H), 2.42 - 2.35 (m, 1H), 2.11 - 2.04 (m, 1H), 1.83 - 1.75 (m, 2H), 1.58 - 1.44 (m, 2H), 1.36 (d, *J* = 6.0 Hz, 3H), 1.25 - 1.15 (m, 5H), 1.11 - 1.04 (m, 6H), 0.94 - 0.87 (m, 6H), 0.39 - 0.31 (s, 3H).

### Example 26

### N-((6³S,4S,Z)-1¹-ethyl-1²-(2-((S)-1-methoxyethyl)-5-(3-morpholinoprop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-1-(trifluoromethyl)cyclopropane-1-carboxamide

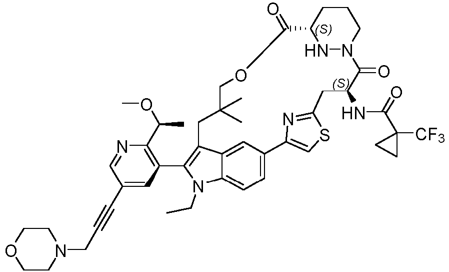

By replacing **INT-3b** in the synthesis step of compound **1** with compound 1-trifluoromethylcyclopropane-1-carboxylic acid, compound **26** can be obtained using similar methods and reaction steps. ESI-MS (m/z): 890.6 [M+H]⁺; LC-MS retention time RT=1.71 min. HPLC retention time RT = 14.19 min.

¹H NMR (500 MHz, DMSO-*d*₆) δ 8.81 (d, *J* = 2.0 Hz, 1H), 8.50 (d, *J* = 1.5 Hz, 1H), 8.11 (d, *J* = 8.5 Hz, 1H), 7.86 (d, *J* = 2.0 Hz, 1H), 7.83 (s, 1H), 7.76 (dd, *J* = 8.5, 1.5 Hz, 1H), 7.58 (d, *J* = 8.5 Hz, 1H), 5.58 - 5.48 (m, 1H), 5.15 - 5.09 (m, 1H), 4.39 - 4.26 (m, 2H), 4.25 - 4.16 (m, 2H), 4.15 - 4.04 (m, 1H), 3.64 - 3.59 (m, 4H), 3.59 - 3.54 (m, 4H), 3.45 - 3.39 (m, 1H), 3.30 - 3.28 (m, 1H), 3.26 (s, 3H), 3.01 - 2.91 (m, 1H), 2.80 - 2.73 (m, 1H), 2.57 - 2.52 (m, 4H), 2.41 - 2.34 (m, 1H), 2.14 - 2.05 (m, 1H), 1.86 - 1.73 (m, 2H), 1.57 - 1.43 (m, 2H), 1.39 - 1.30 (m, 6H), 0.93 (s, 3H), 0.87 (t, *J =* 7.0 Hz, 3H), 0.34 (s, 3H).

### Example 27

### (1S,2S)-N-((6³S,4S,Z)-1¹-ethyl-1²-(2-((S)-1-methoxyethyl)-5-(pyrazin-2-ylethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

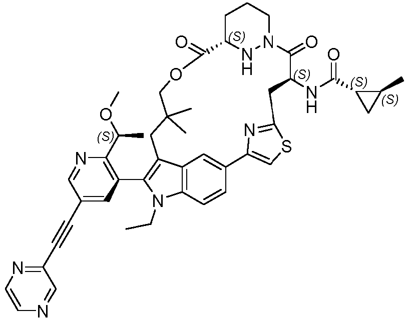

By replacing **INT-15** in the synthesis steps of compound **10** with compound **INT-26,** compound **27** can be obtained using similar methods and reaction steps. ESI-MS (m/z): 815.7 [M+H]⁺; LC-MS retention time RT=1.86 min. HPLC retention time RT = 13.80 min.

¹H NMR (500 MHz, DMSO-*d*₆) δ 9.03 (d, *J* = 2.0 Hz, 1H), 8.96 (d, *J=* 1.5 Hz, 1H), 8.74 - 8.73 (m, 1H), 8.69 (d, *J* = 2.0 Hz, 1H), 8.53 - 8.50 (m, 2H), 8.12 (d, *J* = 2.0 Hz, 1H), 7.82 (s, 1H), 7.77 (dd, *J* = 8.5, 1.5 Hz, 1H), 7.60 (d, *J* = 8.5 Hz, 1H), 5.56 (t, *J= 9.0* Hz, 1H), 5.09 - 5.05 (m, 1H), 4.39 - 4.30 (m, 2H), 4.26 - 4.13 (m, 2H), 4.13 - 4.06 (m, 1H), 3.59 (s, 2H), 3.32 - 3.29 (m, 1H), 3.28 (s, 3H), 3.17 - 3.12 (m, 1H), 3.00 - 2.98 (m, 1H), 2.79 - 2.73 (m, 1H), 2.46 - 2.43 (m, 1H), 2.10 - 2.07 (m, 1H), 1.82 - 1.75 (m, 2H), 1.54 - 1.48 (m, 2H), 1.39 (d, *J =* 6.0 Hz, 3H), 1.28 -1.24 (m, 1H), 1.07 - 1.06 (m, 4H), 0.93 (s, 3H), 0.90 (t, *J* = 7.0 Hz, 3H), 0.57 - 0.54 (m, 1H), 0.36 (s, 3H).

### Example 28

### (1S,2S)-N-((6³S,4S,Z)-1¹-ethyl-1²-(2-((S)-1-methoxyethyl)-5-(((R)-4-methylmorpholin-3-yl)ethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

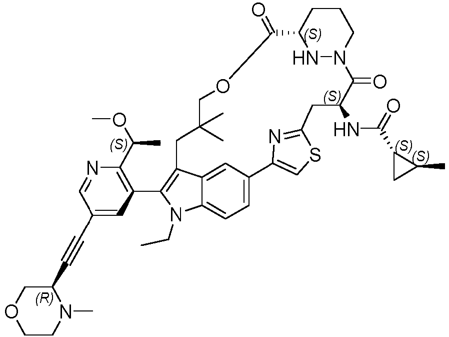

Example 28 was prepared by the following steps:

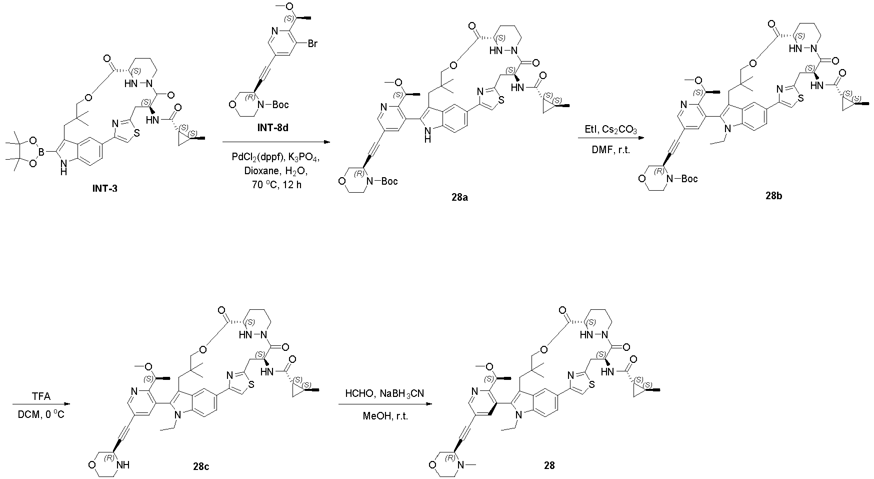

By replacing **INT-15** in the synthesis steps of compound 10 with compound INT-**8d,** compound **28** can be obtained using similar methods and reaction steps. ESI-MS (m/z): 836.7 [M+H]⁺; LC-MS retention time RT = 1.81 min. HPLC retention time RT = 13.37 min.

¹H NMR (500 MHz, DMSO) δ 8.81 (d, *J* = 2.0 Hz, 1H), 8.54 - 8.49 (m, 2H), 7.86 - 7.83 (m, 1H), 7.81 (s, 1H), 7.77 - 7.74 (m, 1H), 7.58 (d, *J* = 8.5 Hz, 1H), 5.56 (t, *J =* 9.5 Hz, 1H), 5.09 - 5.05 (m, 1H), 4.36 - 4.27 (m, 2H), 4.26 - 4.22 (m, 1H), 4.22 - 4.16 (m, 1H), 4.11 - 4.03 (m, 1H), 3.79 - 3.74 (m, 1H), 3.72 - 3.63 (m, 2H), 3.62 - 3.59 (m, 1H), 3.58 (s, 2H), 3.53 - 3.49 (m, 1H), 3.25 (s, 3H), 3.20 - 3.11 (m, 2H), 3.00 - 2.94 (m, 1H), 2.79 - 2.73 (m, 1H), 2.68 - 2.63 (m, 1H), 2.41 - 2.36 (m, 1H), 2.34 (s, 3H), 2.33 - 2.27 (m, 1H), 2.11 - 2.05 (m, 1H), 1.83 - 1.75 (m, 2H), 1.53 - 1.47 (m, 2H), 1.36 (d, *J =* 6.0 Hz, 3H), 1.10 - 1.05 (m, 4H), 0.92 (s, 3H), 0.90 - 0.83 (m, 4H), 0.57 - 0.52 (m, 1H), 0.35 (s, 3H).

### Example 29

### (1S,2S)-N-((63S,4S,Z)-1¹-ethyl-1²-(2-((S)-1-methoxyethyl)-5-((1-methylpiperidin-4-yl)ethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

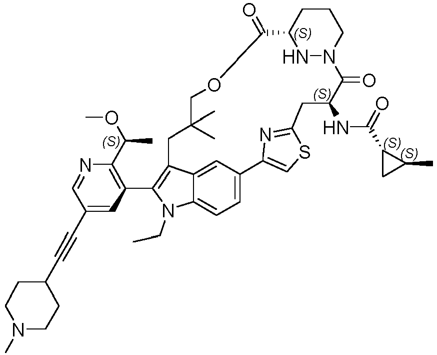

By replacing **INT-15** in the synthesis steps of compound **10** with compound **INT-27,** compound **29** can be obtained using similar methods and reaction steps. ESI-MS (m/z): 834.8 [M+H]⁺; LC-MS retention time RT = 1.90 min. HPLC retention time RT = 14.59 min.

¹H NMR (500 MHz, DMSO) δ 8.75 (d, *J* = 2.0 Hz, 1H), 8.53 - 8.48 (m, 2H), 7.80 (s, 1H), 7.76 - 7.73 (m, 2H), 7.57 (d, *J* = 8.5 Hz, 1H), 5.56 (t, *J* = 9.0 Hz, 1H), 5.09 - 5.05 (m, 1H), 4.36 - 4.29 (m, 1H), 4.27 - 4.16 (m, 4H), 4.11 - 4.02 (m, 1H), 3.57 (s, 2H), 3.24 (s, 3H), 3.17 - 3.11 (m, 2H), 2.99 - 2.93 (m, 1H), 2.79 - 2.74 (m, 1H), 2.64 - 2.61 (m, 2H), 2.39 - 2.35 (m, 1H), 2.15 (s, 3H), 2.10 - 2.03 (m, 2H), 1.90 - 1.85 (m, 3H), 1.82 - 1.78 (m, 2H), 1.67 - 1.62 (m, 2H), 1.51 - 1.47 (m, 2H), 1.34 (d, *J* = 6.0 Hz, 3H), 1.24 - 1.22 (m, 1H), 1.09 - 1.04 (m, 4H), 0.91 (s, 3H), 0.87 (t, *J* = 7.0 Hz, 3H), 0.56 - 0.53 (m, 1H), 0.33 (s, 3H).

### Example 30

### (1S,2S)-N-((6³S,4S,Z)-1¹-ethyl-1²-(2-((S)-1-methoxyethyl)-5-((2-methylisoindolin-4-yl)ethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

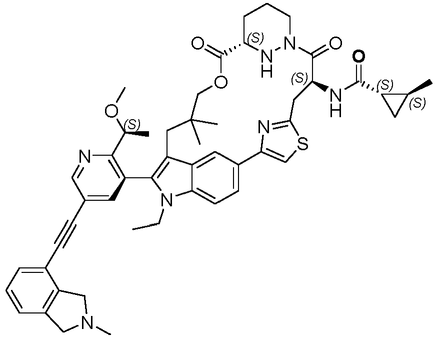

By replacing **INT-15** in the synthesis steps of compound **10** with compound **INT-28,** compound **30** can be obtained using similar methods and reaction steps. ESI-MS (m/z): 868.7 [M+H]⁺; LC-MS retention time RT=2.08 min.

¹H NMR (500 MHz, DMSO-*d*₆) δ 8.98 - 8.91 (m, 1H), 8.55 - 8.48 (m, 2H), 8.00 (d, *J* = 2.0 Hz, 1H), 7.85 - 7.80 (m, 1H), 7.79 - 7.74 (m, 1H), 7.64 - 7.57 (m, 1H), 7.44 - 7.38 (m, 1H), 7.34 - 7.24 (m, 2H), 5.56 (t, *J* = 9.0 Hz, 1H), 5.12 - 5.03 (m, 1H), 4.40 - 4.08 (m, 5H), 4.06 - 3.99 (m, 2H), 3.92 - 3.83 (m, 1H), 3.59 (s, 2H), 3.29 - 3.27 (m, 4H), 3.17 - 3.12 (m, 2H), 3.01 - 2.97 (m, 1H), 2.80 - 2.74 (m, 1H), 2.45 - 2.41 (m, 1H), 2.12 - 2.05 (m, 1H), 1.83 - 1.74 (m, 2H), 1.55 - 1.46 (m, 2H), 1.40 - 1.36 (m, 3H), 1.25- 1.22 (m, 1H), 1.20 - 1.15 (m, 2H), 1.10 - 1.03 (m, 4H), 0.95 - 0.85 (m, 7H), 0.58 - 0.52 (m, 1H), 0.36 (s, 3H).

### Example 31

### (1S,2S)-N-((6³S,4S,Z)-1¹-ethyl-1²-(5-((6-(ethylamino)pyridin-2-yl)ethynyl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

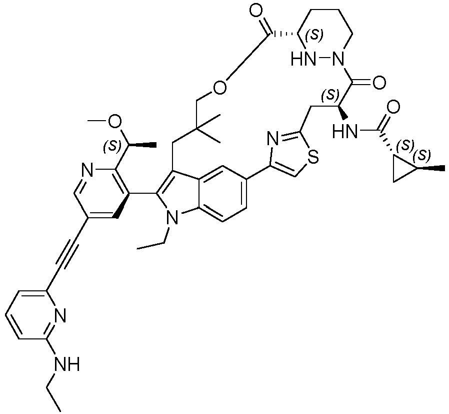

By replacing **INT-15** in the synthesis step of Example 10 with **INT-29,** compound 31 can be obtained using a similar method and reaction steps. ESI-MS (m/z): 857.7 [M+H]⁺; LC-MS retention time RT = 1.98 min. HPLC retention time RT = 15.11 min.

¹H NMR (500 MHz, DMSO) δ 8.93 (d, *J* = 2.0 Hz, 1H), 8.53 - 8.50 (m, 2H), 7.99 (d, *J=* 2.0 Hz, 1H), 7.81 (s, 1H), 7.78 - 7.74 (m, 1H), 7.59 (d, *J* = 8.5 Hz, 1H), 7.43 - 7.38 (m, 1H), 6.82 (d, *J=* 6.5 Hz, 1H), 6.74 - 6.69 (m, 1H), 6.52 - 6.49 (m, 1H), 5.56 (t, *J* = 9.5 Hz, 1H), 5.09 - 5.05 (m, 1H), 4.38 - 4.32 (m, 1H), 4.31 - 4.27 (m, 1H), 4.27 - 4.21 (m, 2H), 4.20 - 4.16 (m, 1H), 4.14 - 4.07 (m, 1H), 3.58 (s, 2H), 3.27 (s, 3H), 3.25 - 3.22 (m, 2H), 3.18 - 3.14 (m, 1H), 3.01 - 2.95 (m, 1H), 2.80 - 2.73 (m, 1H), 2.46 - 2.41 (m, 1H), 2.12 - 2.05 (m, 1H), 1.82 - 1.77 (m, 2H), 1.55 - 1.47 (m, 2H), 1.38 (d, *J= 6.0* Hz, 3H), 1.25 - 1.22 (m, 1H), 1.13 (t, *J=* 7.0 Hz, 3H), 1.08 - 1.05 (m, 4H), 0.93 (s, 3H), 0.90 (t, *J* = 7.0 Hz, 3H), 0.57 - 0.52 (m, 1H), 0.36 (s, 3H).

### Example 32

### (1r,2R,3S)-N-((6³S,4S,Z)-1¹-ethyl-1²-(5-((6-(ethylamino)pyridazin-3-yl)ethynyl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

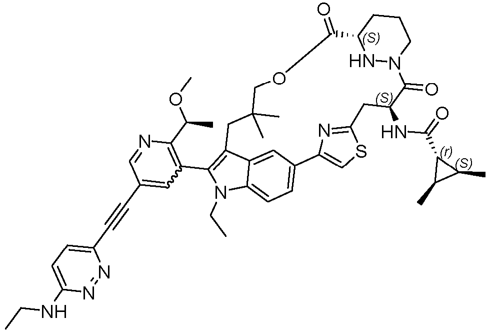

By replacing **INT-16** in the synthesis step of compound **11** with compound **INT-30,** compound **32+32'** can be obtained using similar methods and reaction steps. Neither thin layer chromatography nor existing preparative liquid chromatography can separate compound 32 and its epimer 32', so the cell activity of this compound is the activity of the racemic compound. ESI-MS (m/z): 872.6 [M+H]⁺; LC-MS retention time RT=1.81 min. HPLC retention time RT = 11.92 min.

### Example 33

### (1S,2S)-N-((6³S,4S,Z)-1¹-ethyl-1²-(5-(((R)-4-(2-hydroxyethyl)morpholin-3-yl)ethynyl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

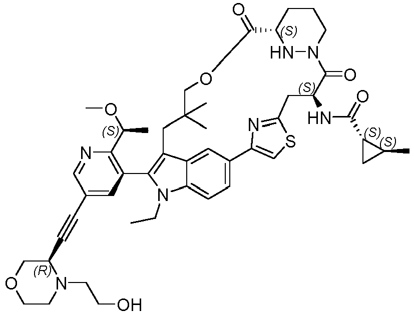

Example 33 was prepared by the following steps:

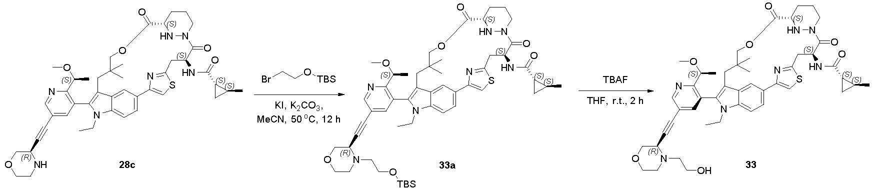

Step 1: Compound 28c (40 mg, 0.05 mmol) was dissolved in N,N-dimethylformamide (1 mL), and potassium carbonate (21 mg, 0.15 mmol), potassium iodide (25 mg, 0.15 mmol) and (2-bromoethoxy)-tert-butyldimethylsilane (24 mg, 0.10 mmol) were added to the reaction solution. The reaction solution was stirred at 50 °C for 8 hours. After the reaction was completed as detected by LCMS, water (10 mL) was added to the reaction system, and the mixture was extracted with dichloromethane (10 mL*2). The organic phases were combined and washed with saturated brine, dried over anhydrous sodium sulfate, filtered and concentrated. The residue was purified by silica gel column chromatography (dichloromethane/methanol = 20/1) to obtain compound **33a** (22 mg, yield 43%). ESI-MS (m/z): 980.7 [M+H]⁺;

Step 2: Compound 33a (22 mg, 0.02 mmol) was dissolved in tetrahydrofuran (1 mL), followed by the addition of tetrabutylammonium fluoride (0.1 mL, 1 M in THF). The reaction solution was stirred at room temperature for 2 hours. After the reaction was completed as detected by LCMS, saturated aqueous ammonium chloride solution (10 mL) was added to the reaction system, and the mixture was extracted with dichloromethane (10 mL*2). The organic phases were combined and washed with saturated brine, dried over anhydrous sodium sulfate, filtered and concentrated. The residue was purified by preparative liquid chromatography to obtain white solid compound **33** (1.0 mg, yield 7%). ESI-MS (m/z): 866.8 [M+H]⁺; LC-MS retention time RT = 1.64 min. HPLC retention time RT = 11.64 min.

¹H NMR (500 MHz, DMSO) δ 8.82 (d, *J =* 2.0 Hz, 1H), 8.55 - 8.51 (m, 2H), 7.97 (s, 1H), 7.81 (s, 1H), 7.76 - 7.72 (m, 1H), 7.54 (d, *J* = 8.5 Hz, 1H), 5.57 - 5.52 (m, 1H), 5.07 - 5.03 (m, 1H), 4.49 - 4.43 (m, 1H), 4.26 - 4.19 m, 2H), 3.98 - 3.92 (m, 2H), 3.86 - 3.82 (m, 1H), 3.79 - 3.75 (m, 2H), 3.73 - 3.69 (m, 1H), 3.68 - 3.64 (m, 1H), 3.56 - 3.52 (m, 2H), 3.18 - 3.14 (m, 3H), 3.09 (s, 2H), 3.06 - 3.01 (m, 1H), 2.65 - 2.63 (m, 1H), 2.59 - 2.55 (m, 1H), 2.38 - 2.36 (m, 1H), 2.34 - 2.29 (m, 1H), 2.14 - 2.09 (m, 1H), 1.82 - 1.78 (m, 2H), 1.58 - 1.54 (m, 3H), 1.34 - 1.28 (m, 4H), 1.22 (d, *J =* 6.0 Hz, 2H), 1.11 (t, *J* = 7.0 Hz, 3H), 1.07 (s, 3H), 0.96 - 0.91 (m, 5H), 0.88 - 0.86 (m, 1H), 0.57 - 0.54 (m, 1H), 0.50 (s, 3H).

### Example 34

### (1r,2R,3S)-N-((6³S,4S,Z)-1²-(5-((4-aminopyrimidin-5-yl)ethynyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

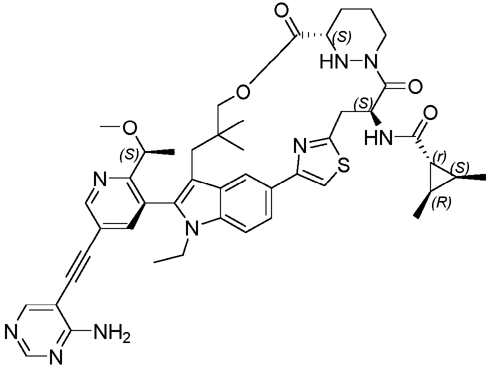

By replacing **INT-16** in the synthesis steps of compound **11** with compound **INT-31,** compound **34** can be obtained using similar methods and reaction steps. ESI-MS (m/z): 844.7 [M+H]⁺; LC-MS retention time RT=1.70 min.

¹H NMR (500 MHz, DMSO-*d*₆) δ 8.97 - 8.94 (m, 1H), 8.53 - 8.47 (m, 1H), 8.42 - 8.39 (m, 2H), 8.39 - 8.37 (m, 1H), 8.20 - 8.18 (m, 1H), 7.83 (s, 1H), 7.78 - 7.75 (m, 1H), 7.62 - 7.58 (m, 1H), 7.23 - 7.19 (m, 2H), 5.55 (t, *J* = 9.0 Hz, 1H), 5.10 - 5.00 (m, 1H), 4.39 - 4.07 (m, 5H), 3.58 (s, 2H), 3.3 - 3.30 (m, 1H), 3.27 (s, 3H), 3.18 - 3.11 (m, 1H), 3.04 - 2.96 (m, 1H), 2.80 - 2.71 (m, 1H), 2.47 - 2.41 (m, 1H), 2.12 - 2.04 (m, 1H), 1.83 - 1.73 (m, 2H), 1.55 - 1.48 (m, 1H), 1.38 (d, *J* = 6.0 Hz, 3H), 1.21 - 1.14 (m, 3H), 1.11 - 1.05 (m, 6H), 0.96 - 0.86 (m, 6H), 0.36 (s, 3H).

### Example 35

### (1S,2S)-N-((6³S,4S,Z)-1²-(5-((5-aminopyrazin-2-yl)ethynyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

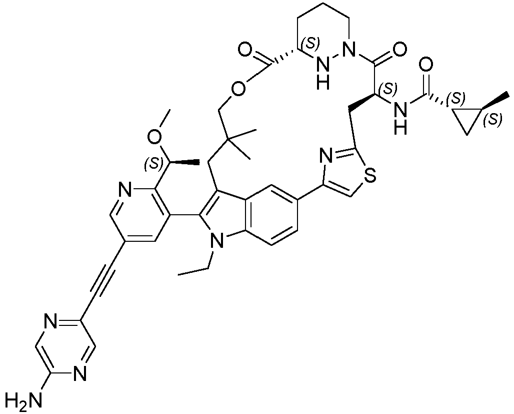

By replacing **INT-15** in the synthesis steps of compound **10** with compound **INT-32,** compound **35** can be obtained using similar methods and reaction steps. ESI-MS (m/z): 830.7 [M+H]⁺; LC-MS retention time RT = 1.70 min.

### Example 36

### (1r,2R,3S)-N-((6³S,4S,Z)-1¹-ethyl-1²-(2-((S)-1-methoxyethyl)-5-(3-(methyl(tetrahydro-2H-pyran-4-yl)amino)prop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

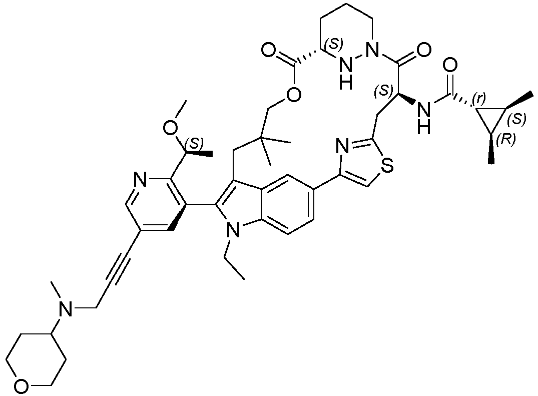

Example 36 was prepared by the following steps:

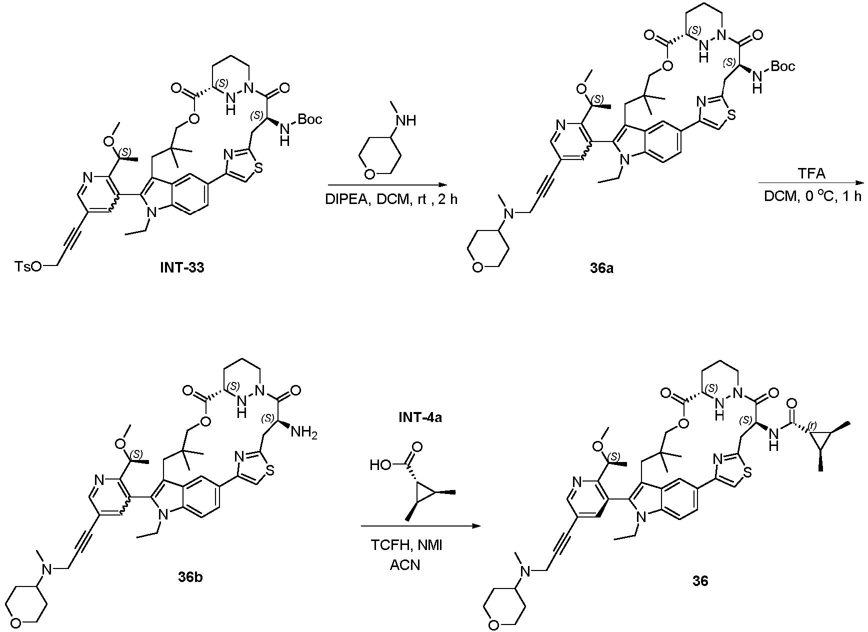

Step 1: Compound **INT-33** (90 mg, 0.10 mmol) was dissolved in dichloromethane (5 mL), and N-methyltetrahydro-2H-pyran-4-amine (55 mg, 0.48 mmol) and diisopropylethylamine (62 mg, 0.48 mmol) were added thereto. The reaction mixture was stirred at room temperature for 16 hours. The reaction was complete as determined by LCMS. Water (20 mL) was added to the reaction system, and the mixture was extracted with dichloromethane (20 mL*2). The organic phases were combined and washed with saturated brine, dried over anhydrous sodium sulfate, filtered and concentrated. The residue was purified by preparative thin layer chromatography (dichloromethane/methanol = 20/1) to obtain compound **36a** (60 mg, yield 71%). ESI-MS (m/z): 882.7 [M+H]⁺;

Step 2: Compound **36a** (50 mg, 0.06 mmol) was dissolved in dichloromethane (2 mL), and trifluoroacetic acid (0.5 mL) was added thereto. The reaction mixture was stirred at 0°C for 1 hours. LCMS detected that the reaction was complete. Saturated sodium bicarbonate solution (10 mL) was added to the reaction system under an ice bath, and the mixture was extracted with dichloromethane (20 mL*2). The organic phases were combined and washed with saturated brine, dried over anhydrous sodium sulfate, filtered and concentrated to obtain a crude product of compound **36b** (37 mg, yield 83%). ESI-MS (m/z): 782.8 [M+H]⁺;

Step 3: Compound **36b** (37 mg, 0.05 mmol) was dissolved in acetonitrile (2 mL), and **INT-4a** (5 mg, 0.05 mmol), N,N-diisopropylethylamine (30 mg, 0.24 mmol), N-methylimidazole (6 mg, 0.07 mmol) and (1-Cyano-2-ethoxy-2-oxoethylidenaminooxy)dimethylamino-morpholino-carbenium hexafluorophosphate (19 mg, 0.07 mmol) were added thereto. The reaction mixture was stirred in an ice bath for 1 hour. After the reaction was complete, water (20 mL) was added to the reaction system, and the mixture was extracted with dichloromethane (20 mL*2). The organic phases were combined and washed with saturated brine, dried over anhydrous sodium sulfate, filtered and concentrated. The residue was purified by preparative liquid chromatography to obtain white solid compound **36** (3.0 mg, yield 7%). ESI-MS (m/z): 878.6 [M+H]⁺; LC-MS retention time RT = 1.85 min. HPLC retention time RT = 13.67 min.

¹H NMR (500 MHz, DMSO-*d*₆) δ 8.80 (d, *J* = 2.0 Hz, 1H), 8.52 - 8.48 (m, 1H), 8.39 (d, *J* = 9.0 Hz, 1H), 7.85 - 7.80 (m, 2H), 7.77 - 7.73 (m, 1H), 7.58 (d, *J* = 8.5 Hz, 1H), 5.56 (t, *J* = 9.0 Hz, 1H), 5.10 - 5.04 (m, 1H), 4.39 - 4.02 (m, 5H), 3.91 - 3.84 (m, 2H), 3.70 - 3.65 (m, 2H), 3.58 (s, 2H), 3.30 - 3.29 (m, 2H), 3.25 (s, 3H), 3.18 - 3.11 (m, 2H), 2.98 - 2.91 (m, 1H), 2.79 - 2.71 (m, 1H), 2.42 - 2.35 (m, 2H), 2.32 (s, 3H), 2.14 - 2.02 (m, 2H), 1.82 - 1.74 (m, 4H), 1.55 - 1.46 (m, 1H), 1.43 - 1.32 (m, 5H), 1.18 - 1.14 (m, 2H), 1.10 - 1.04 (m, 6H), 0.93 - 0.84 (m, 6H), 0.35 (s, 3H).

### Example 37

### (1r,2R,3S)-N-((6³S,4S,Z)-12-(5-(3-((S)-3-(ethoxymethyl)morpholino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

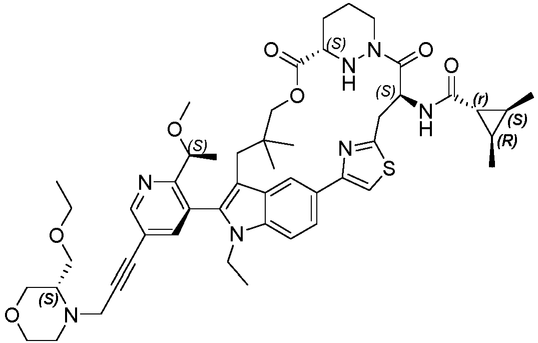

Example 37 was prepared by the following steps:

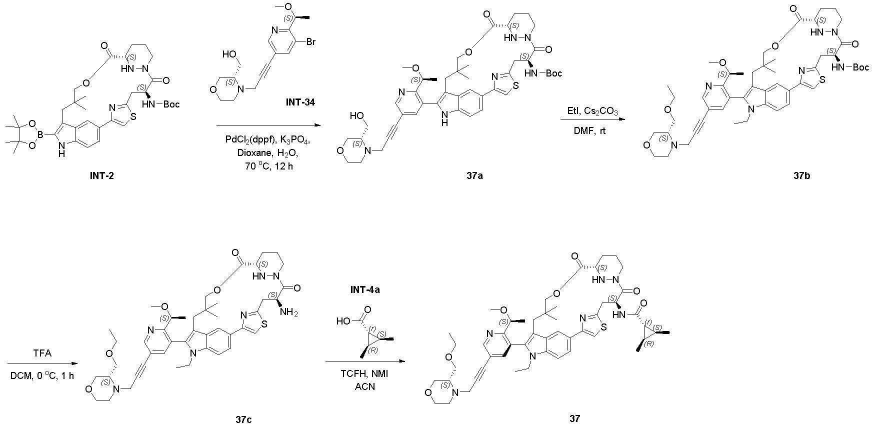

Step 1: Dissolve **INT-2** (350 mg, 0.51 mmol), **INT-34** (186 mg, 0.51 mmol), 1,1-bis(diphenylphosphine) ferrocene palladium dichloride (37 mg, 0.05 mmol), and potassium phosphate (214 mg, 1.08 mmol) in a mixed solution of 1,4-dioxane (5 mL) and water (1 mL). Stir the reaction solution at 70 °C overnight under a nitrogen atmosphere. LCMS detected that the reaction was complete. The reaction solution was filtered through celite, and the filtrate was concentrated. The residue was purified by preparative thin layer chromatography (dichloromethane/methanol = 20/1) to give a brown solid 37a (400 mg, yield 93%). ESI-MS (m/z): 856.7 [M+H]⁺;

Step 2: Compound 37a (300 mg, 0.35 mmol), cesium carbonate (228 mg, 0.70 mmol), and iodoethane (109 mg, 0.70 mmol) were dissolved in N,N-dimethylformamide (10 mL), and the reaction solution was stirred at room temperature overnight. The reaction was complete as determined by LCMS. Water (20 mL) was added to the reaction system, and the mixture was extracted with dichloromethane (20 mL*2). The organic phases were combined and washed with saturated brine, dried over anhydrous sodium sulfate, filtered and concentrated. The residue was purified by preparative thin layer chromatography (dichloromethane/methanol = 30/1) to give **37b** (280 mg, yield 88%). ESI-MS (m/z): 913.0 [M+H]⁺;

Step 3: Compound **37b** (280 mg, 0.31 mmol) was dissolved in dichloromethane (10 mL), and trifluoroacetic acid (5 mL) was added thereto. The reaction mixture was stirred at 0 °C for 1 hour. LCMS detected that the reaction was complete. Saturated sodium bicarbonate solution (30 mL) was added to the reaction system under an ice bath, and the mixture was extracted with dichloromethane (20 mL*2). The organic phases were combined and washed with saturated brine, dried over anhydrous sodium sulfate, filtered and concentrated to obtain a crude product of compound **37c** (220 mg, yield 88%). ESI-MS (m/z): 813.0 [M+H]⁺;

Step 3: Compound **37c** (40 mg, 0.05 mmol) was dissolved in acetonitrile (2 mL), and **INT-4a** (5 mg, 0.05 mmol), N,N-diisopropylethylamine (30 mg, 0.24 mmol), N-methylimidazole (6 mg, 0.07 mmol) and (1-Cyano-2-ethoxy-2-oxoethylidenaminooxy)dimethylamino-morpholino-carbenium hexafluorophosphate (19 mg, 0.07 mmol) were added thereto. The reaction mixture was stirred in an ice bath for 1 hour. After the reaction was complete, water (20 mL) was added to the reaction system, and the mixture was extracted with dichloromethane (20 mL*2). The organic phases were combined and washed with saturated brine, dried over anhydrous sodium sulfate, filtered and concentrated. The residue was purified by preparative liquid chromatography to obtain white solid compound **37** (7.0 mg, yield 15%). ESI-MS (m/z): 909.1 [M+H]+; LC-MS retention time RT = 1.94 min.

¹H NMR (500 MHz, DMSO-*d*₆) δ 8.80 (d, *J* = 2.0 Hz, 1H), 8.51 - 8.48 (m, 1H), 8.39 (d, *J =* 9.0 Hz, 1H), 7.87 - 7.80 (m, 2H), 7.77 - 7.73 (m, 1H), 7.58 (d, *J* = 8.5 Hz, 1H), 5.56 (t, *J* = 9.0 Hz, 1H), 5.10 - 5.03 (m, 1H), 4.40 - 4.02 (m, 5H), 3.85 - 3.78 (m, 1H), 3.77 - 3.67 (m, 3H), 3.59 - 3.51 (m, 3H), 3.23 - 3.19 (m, 3H), 3.18 - 3.11 (m, 2H), 2.98 - 2.92 (m, 1H), 2.79 - 2.56 (m, 5H), 2.41 - 2.35 (m, 1H), 2.11 - 1.97 (m, 2H), 1.82 - 1.74 (m, 2H), 1.57 - 1.42 (m, 1H), 1.36 (d, *J* = 6.0 Hz, 3H), 1.26 - 1.22 (m, 3H), 1.18 - 1.15 (m, 2H), 1.10 - 1.05 (m, 9H), 0.93 - 0.83 (m, 6H), 0.35 (s, 3H).

### Example 38

### (1r,2R,3S)-N-((6³S,4S,Z)-1¹-ethyl-1²-(5-(3-((R)-3-(hydroxymethyl)morpholino)prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

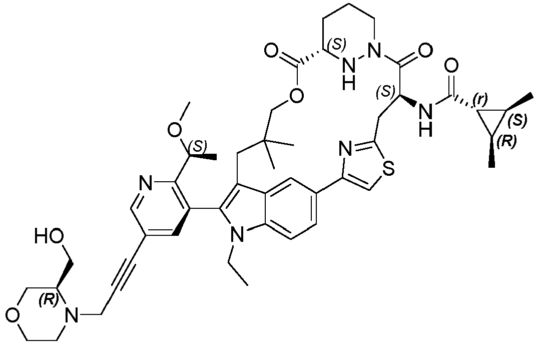

By replacing the N-methyltetrahydro-2H-pyran-4-amine in Example **36** with (R)-3-hydroxymethylmorpholine and following a similar method and reaction procedure, Compound **38** can be obtained. ESI-MS (m/z): 881.1 [M+H]⁺; LC-MS retention time RT = 1.67 min.

¹H NMR (500 MHz, DMSO-*d*₆) δ 8.81 (d, *J=* 2.0 Hz, 1H), 8.51 - 8.49 (m, 1H), 8.39 (d, *J* = 9.0 Hz, 1H), 7.85 (d, *J=* 2.0 Hz, 1H), 7.82 (s, 1H), 7.77 - 7.73 (m, 1H), 7.58 (d, *J* = 8.5 Hz, 1H), 5.56 (t, *J* = 9.0 Hz, 1H), 5.09 - 5.03 (m, 1H), 4.59 (t, *J* = 5.5 Hz, 1H), 4.38 - 4.03 (m, 5H), 3.87 - 3.66 (m, 4H), 3.63 - 3.55 (m, 3H), 3.51 - 3.43 (m, 1H), 3.31 - 3.28 (m, 2H), 3.25 (s, 3H), 3.22 - 3.07 (m, 2H), 2.99 - 2.93 (m, 1H), 2.78 - 2.68 (m, 2H), 2.64 - 2.57 (m, 1H), 2.56 - 2.52 (m, 1H), 2.42 - 2.35 (m, 1H), 2.11 - 2.04 (m, 1H), 1.83 - 1.74 (m, 2H), 1.57 - 1.46 (m, 1H), 1.36 (d, *J* = 6.0 Hz, 3H), 1.24 - 1.15 (m, 4H), 1.10 - 1.04 (m, 6H), 0.95 - 0.85 (m, 6H), 0.35 (s, 3H).

### Example 40

### (1r,2R,3S)-N-((6³S,4S,Z)-1¹-ethyl-1²-(5-(3-((S)-hexahydropyrazino[2,1-c][1,4]oxazin-8(1H)-yl)prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

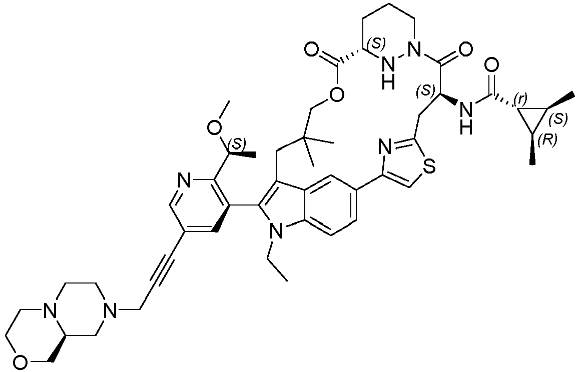

By replacing **INT-16** in Example **11** with **INT-35** and following a similar method and reaction procedure, Compound **40** can be obtained. ESI-MS (m/z): 906.3 [M+H]⁺; LC-MS retention time RT = 1.74 min; HPLC retention time RT = 12.56 min.

¹H NMR (500 MHz, DMSO-*d*₆) δ 8.81 (d, *J* = 2.0 Hz, 1H), 8.51 - 8.48 (m, 1H), 8.41 (d, *J* = 9.0 Hz, 1H), 7.85 (d, *J* = 2.0 Hz, 1H), 7.83 (s, 1H), 7.77 - 7.73 (m, 1H), 7.59 (d, *J* = 8.5 Hz, 1H), 5.55 (t, *J* = 9.0 Hz, 1H), 5.11 - 5.04 (m, 1H), 4.39 - 3.97 (m, 5H), 3.75 - 3.68 (m, 1H), 3.75 - 3.68 (m, 1H), 3.58 - 3.54 (m, 4H), 3.50 - 3.43 (m, 1H), 3.31 - 3.29 (m, 1H), 3.25 (s, 3H), 3.22 - 3.04 (m, 2H), 2.99 - 2.93 (m, 1H), 2.83 - 2.56 (m, 6H), 2.43 - 2.33 (m, 2H), 2.23 - 2.04 (m, 4H), 1.96 - 1.90 (m, 1H), 1.84 - 1.74 (m, 2H), 1.53 - 1.47 (m, 1H), 1.35 (d, *J= 6.0* Hz, 3H), 1.19 - 1.12 (m, 2H), 1.11 - 1.05 (m, 6H), 0.91 (s, 3H), 0.88 (t, *J* = 7.0 Hz, 3H), 0.34 (s, 3H).

### Example 41

### (1r,2R,3S)-N-((6³S,4S,Z)-1²-(5-(3-(4-acetylpiperazin-1-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

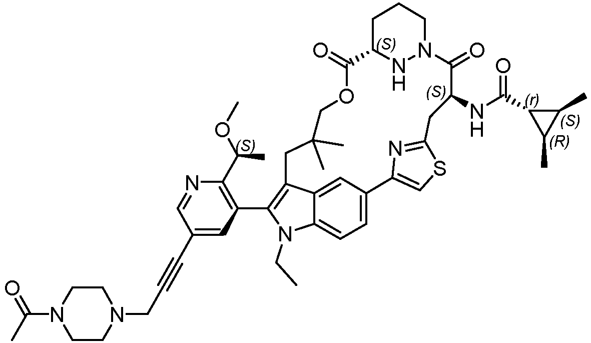

By replacing **INT-16** in the synthesis step of Compound **11** with **INT-36** and following a similar method and reaction procedure, Compound **41** can be obtained. ESI-MS (m/z): 891.3 [M+H]⁺; LC-MS retention time RT = 1.68 min; HPLC retention time RT = 12.18 min.

¹H NMR (500 MHz, DMSO-*d*₆) δ 8.81 (d, *J =* 2.0 Hz, 1H), 8.50 (d, *J =* 1.5 Hz, 1H), 8.41 (d, *J* = 9.0 Hz, 1H), 7.86 (d, *J =* 2.0 Hz, 1H), 7.82 (s, 1H), 7.75 (dd, *J =* 8.5*,* 1.5 Hz, 1H), 7.58 (d, *J =* 8.5 Hz, 1H), 5.55 (t, *J =* 9.0 Hz, 1H), 5.12 - 5.03 (m, 1H), 4.38 - 4.03 (m, 5H), 3.65 - 3.55 (m, 4H), 3.51 - 3.42 (m, 4H), 3.33 - 3.29 (m, 2H), 3.25 (s, 3H), 3.18 - 3.11 (m, 1H), 2.99 - 2.91 (m, 1H), 2.80 - 2.70 (m, 1H), 2.58 - 2.53 (m, 2H), 2.49 - 2.46 (m, 2H), 2.42 - 2.34 (m, 1H), 2.11 - 2.04 (m, 1H), 1.99 (s, 3H), 1.84 - 1.71 (m, 2H), 1.56 - 1.46 (m, 1H), 1.35 (d, *J* = 6.0 Hz, 3H), 1.19 - 1.14 (m, 2H), 1.10 - 1.04 (m, 6H), 0.93 - 0.83 (m, 6H), 0.34 (s, 3H).

### Example 42

### (1S,2S)-N-((6³S,4S,Z)-1¹-ethyl-1²-(5-((2-(ethylamino)pyrimidin-5-yl)ethynyl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

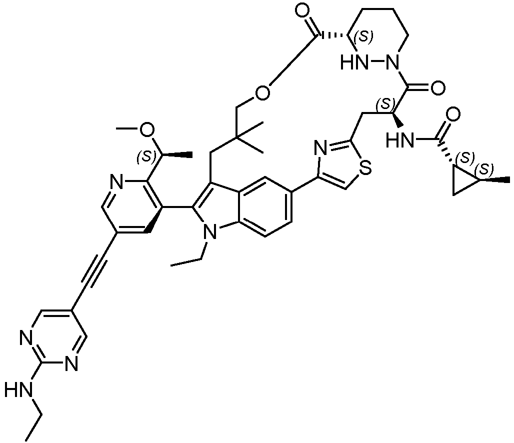

By replacing **INT-15** in Example **10** with **INT-37** and following a similar method and reaction procedure, Compound **42** can be obtained. ESI-MS (m/z): 859.5 [M+H]⁺; LC-MS retention time RT = 1.93 min; HPLC retention time RT = 14.59 min.

¹H NMR (500 MHz, DMSO) δ 8.87 (d, *J=* 2.0 Hz, 1H), 8.56 - 8.46 (m, 4H), 7.91 (d, *J* = 2.0 Hz, 1H), 7.81 (s, 1H), 7.79 - 7.74 (m, 2H), 7.59 (d, *J* = 8.5 Hz, 1H), 5.56 (t, *J =* 9.5 Hz, 1H), 5.10 - 5.05 (m, 1H), 4.38 - 4.05 (m, 6H), 3.58 (s, 2H), 3.26 (s, 3H), 3.17 - 3.10 (m, 2H), 3.00 - 2.95 (m, 1H), 2.80 - 2.71 (m, 1H), 2.44 - 2.38 (m, 1H), 2.11 - 2.06 (m, 1H), 1.84 - 1.73 (m, 2H), 1.54 - 1.47 (m, 2H), 1.37 (d, *J* = 6.0 Hz, 3H), 1.25 - 1.22 (m, 1H), 1.12 (t, *J* = 7.0 Hz, 3H), 1.09 - 1.03 (m, 4H), 0.93 (s, 3H), 0.88 (t, *J* = 7.0 Hz, 3H), 0.86 - 0.84 (m, 1H), 0.57 - 0.52 (m, 1H), 0.35 (s, 3H).

### Example 43

### (1S,2S)-N-((6³S,4S,Z)-1²-(5-(3-(1,1-dioxidothiomorpholino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

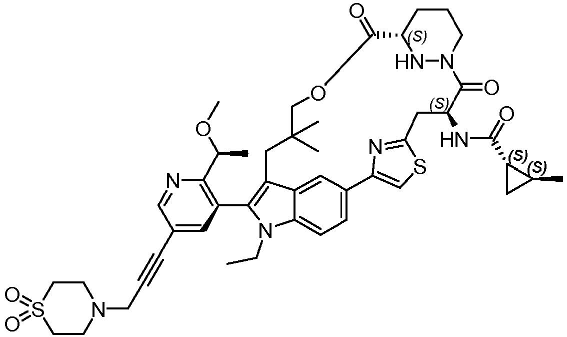

By replacing **INT-15** in Example **10** with **INT-38** and following a similar method and reaction procedure, Compound **43** can be obtained. ESI-MS (m/z): 884.8 [M+H]⁺; LC-MS retention time RT = 1.70 min; HPLC retention time RT = 12.44 min.

¹H NMR (500 MHz, DMSO-*d*₆) δ 8.81 (d, *J* = 2.0 Hz, 1H), 8.53 - 8.48 (m, 2H), 7.86 (d, *J =* 2.0 Hz, 1H), 7.80 (s, 1H), 7.75 (d, *J* = 8.5 Hz, 1H), 7.58 (d, *J* = 8.5 Hz, 1H), 5.56 (t, *J* = 9.0 Hz, 1H), 5.09 - 5.05 (m, 1H), 4.34 - 4.32 (m, 1H), 4.29 - 4.25 (m, 2H), 4.21 - 4.15 (m, 2H), 4.09 - 4.06 (m, 1H), 3.77 - 3.75 (m, 2H), 3.58 - 3.57 (m, 2H), 3.31 - 3.30 (m, 2H), 3.25 (s, 3H), 3.18 - 3.14 (m, 5H), 3.06 - 3.03 (m, 3H), 2.98 - 2.94 (m, 1H), 2.79 - 2.74 (m, 1H), 2.41 - 2.36 (m, 1H), 2.10 - 2.06 (m, 1H), 1.82 - 1.77 (m, 2H), 1.52 - 1.47 (m, 2H), 1.35 (d, *J =* 6.0 Hz, 3H), 1.08 - 1.05 (m, 4H), 0.91 (s, 3H), 0.90 - 0.86 (m, 4H), 0.57 - 0.53 (m, 1H), 0.34 (s, 3H).

### Example 44

### (1S,2S)-N-((6³S,4S,Z)-1¹-ethyl-1²-(2-((S)-1-methoxyethyl)-5-((6-methyl-5,6,7,8-tetrahydropyrido[4,3-d]pyrimidin-2-yl)ethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

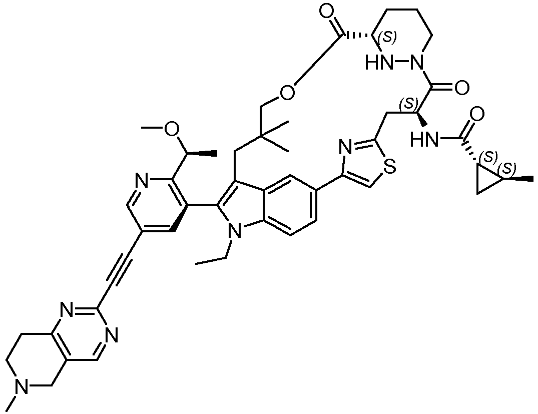

By replacing **INT-15** in Example **10** with **INT-39** and following a similar method and reaction procedure, Compound **44** can be obtained. ESI-MS (m/z): 883.7 [M+H]⁺; LC-MS retention time RT = 1.75 min; HPLC retention time RT = 12.64 min.

¹H NMR (500 MHz, DMSO-*d*₆) δ 9.02 (d, *J* = 2.0 Hz, 1H), 8.58 (s, 1H), 8.56 - 8.51 (m, 2H), 8.09 (d, *J* = 2.0 Hz, 1H), 7.82 (s, 1H), 7.79 - 7.75 (m, 1H), 7.60 (d, *J* = 8.5 Hz, 1H), 5.56 (t, *J* = 9.0 Hz, 1H), 5.12 - 5.06 (m, 1H), 4.39 - 4.29 (m, 2H), 4.27 - 4.17 (m, 2H), 4.14 - 4.07 (m, 1H), 3.60 - 3.54 (m, 4H), 3.28 (s, 3H), 3.17 - 3.11 (m, 1H), 3.01 - 2.96 (m, 1H), 2.93 - 2.89 (m, 2H), 2.78 - 2.71 (m, 3H), 2.46 - 2.42 (m, 1H), 2.38 (s, 3H), 2.12 - 2.05 (m, 1H), 1.83 - 1.76 (m, 2H), 1.54 - 1.47 (m, 2H), 1.38 (d, *J* = 6.0 Hz, 3H), 1.09 - 1.05 (m, 4H), 0.93 (s, 3H), 0.92 - 0.84 (m, 5H), 0.58 - 0.52 (m, 1H), 0.35 (s, 3H).

### Example 45

### (1S,2S)-N-((6³S,4S,Z)-1¹-ethyl-1²-(2-((S)-1-methoxyethyl)-5-(((S)-4-methyl-1,1-dioxidothiomorpholin-2-yl)ethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

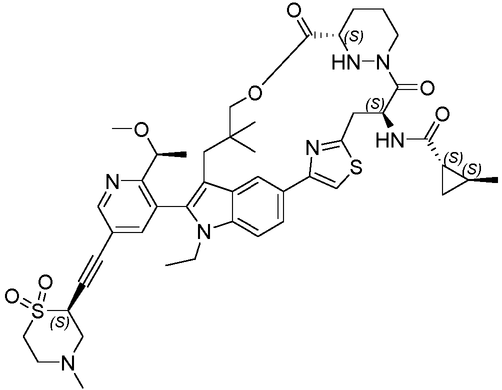

By replacing **INT-15** in Example **10** with **INT-40** and following a similar method and reaction procedure, Compound **45** can be obtained. ESI-MS (m/z): 883.7 [M+H]⁺; LC-MS retention time RT = 1.75 min; HPLC retention time RT = 12.64 min.

¹H NMR (500 MHz, DMSO-*d*₆) δ 8.87 - 8.80 (m, 1H), 8.54 (d, *J* = 9.0 Hz, 1H), 8.51 - 8.49 (m, 1H), 7.88 - 7.85 (m, 1H), 7.82 (s, 1H), 7.76 (dd, *J* = 8.5*,* 1.5 Hz, 1H), 7.59 (d, *J* = 8.5 Hz, 1H), 5.56 (t, *J* = 9.0 Hz, 1H), 5.12 - 5.07 (m, 1H), 4.35 - 4.18 (m, 5H), 4.11 - 4.04 (m, 1H), 3.59 - 3.55 (m, 2H), 3.48 - 3.41 (m, 2H), 3.26 (s, 3H), 3.20 - 3.12 (m, 4H), 3.01 - 2.95 (m, 1H), 2.89 - 2.83 (m, 1H), 2.79 - 2.73 (m, 1H), 2.47 - 2.46 (m, 2H), 2.40 - 2.35 (m, 1H), 2.11 - 2.06 (m, 1H), 2.03 - 1.96 (m, 1H), 1.82 - 1.75 (m, 2H), 1.55 - 1.46 (m, 3H), 1.36 (d, *J=* 6.0 Hz, 3H), 1.08 - 1.05 (m, 4H), 0.92 (s, 3H), 0.89 - 0.85 (m, 4H), 0.57 - 0.53 (m, 1H), 0.34 (s, 3H).

### Example 46

### (1S,2S)-N-((6³S,4S,Z)-1¹-ethyl-1²-(2-((S)-1-methoxyethyl)-5-((6-methyl-6,7-dihydro-5H-pyrrolo[3,4-b]pyridin-3-yl)ethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

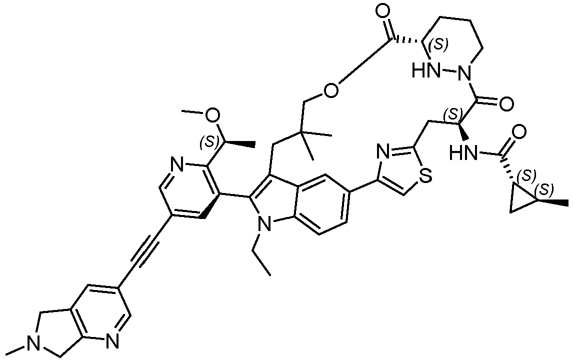

By replacing **INT-15** in Example **10** with **INT-41** and following a similar method and reaction procedure, Compound **46** can be obtained. ESI-MS (m/z): 869.3 [M+H]⁺; LC-MS retention time RT = 1.86 min; HPLC retention time RT = 13.81 min.

¹H NMR (500 MHz, DMSO-*d*₆) δ 8.94 (s, 1H), 8.59 (s, 1H), 8.53 - 8.49 (m, 2H), 8.01 (s, 1H), 7.89 (s, 1H), 7.81 (s, 1H), 7.76 (d, *J =* 8.5 Hz, 1H), 7.59 (d, *J* = 8.5 Hz, 1H), 5.57 (t, *J= 9.0* Hz, 1H), 5.09 - 5.05 (m, 1H), 4.37 - 4.30 (m, 2H), 4.26 - 4.19 (m, 2H), 4.12 - 4.08 (m, 1H), 3.89 - 3.85 (m, 4H), 3.58 (s, 2H), 3.27 (s, 3H), 3.18 - 3.12 (m, 2H), 3.01 - 2.97 (m, 1H), 2.79 - 2.74 (m, 1H), 2.44 - 2.41 (m, 1H), 2.10 - 2.07 (m, 1H), 1.82 - 1.77 (m, 2H), 1.53 - 1.48 (m, 2H), 1.40 - 1.36 (m, 3H), 1.25 - 1.23 (m, 2H), 1.08 - 1.05 (m, 4H), 0.93 (s, 3H), 0.91 - 0.86 (m, 5H), 0.56 - 0.53 (m, 1H), 0.36 (s, 3H).

### Example 47

### (1S,2S)-N-((6³S,4S,Z)-1¹-ethyl-1²-(2-((S)-1-methoxyethyl)-5-((1-methyl-1H-indol-7-yl)ethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

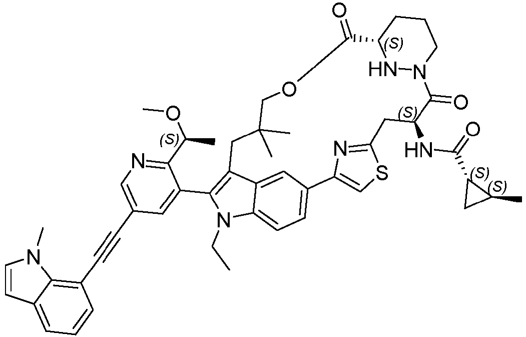

By replacing **INT-15** in Example **10** with **INT-42** and following a similar method and reaction procedure, Compound **47** can be obtained. ESI-MS (m/z): 867.0 [M+H]⁺; LC-MS retention time RT = 2.28 min; HPLC retention time RT = 17.46 min.

¹H NMR (500 MHz, DMSO-*d*₆) δ 8.98 (d, *J* = 2.0 Hz, 1H), 8.53 - 8.50 (m, 2H), 8.03 (d, *J =* 2.0 Hz, 1H), 7.82 (s, 1H), 7.78 - 7.75 (m, 1H), 7.65 (d, *J* =8.0 Hz, 1H), 7.60 (d, *J =* 8.5 Hz, 1H), 7.41 - 7.37 (m, 2H), 7.08 - 7.05 (m, 1H), 6.51 (d, *J* = 3.0 Hz, 1H), 5.57 (t, *J =* 9.0 Hz, 1H), 5.11 - 5.06 (m, 1H), 4.39 - 4.30 (m, 2H), 4.25 - 4.20 (m, 4H), 4.16 - 4.11 (m, 1H), 3.59 (s, 2H), 3.36 - 3.34 (m, 1H), 3.31 - 3.30 (m, 1H), 3.28 (s, 3H), 3.18 - 3.13 (m, 1H), 3.01 - 2.96 (m, 1H), 2.79 - 2.74 (m, 1H), 2.48 - 2.44 (m, 1H), 2.11 - 2.06 (m, 1H), 1.82 - 1.76 (m, 2H), 1.54 - 1.48 (m, 2H), 1.39 (d, *J=* 6.0 Hz, 3H), 1.07 (s, 4H), 0.94 - 0.89 (m, 6H), 0.88 - 0.85 (m, 1H), 0.57 - 0.53 (m, 1H), 0.38 (s, 3H).

### Example 48

### (1S,2S)-N-((6³S,4S,Z)-1¹-ethyl-1²-(2-((S)-1-methoxyethyl)-5-(piperidin-4-ylethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

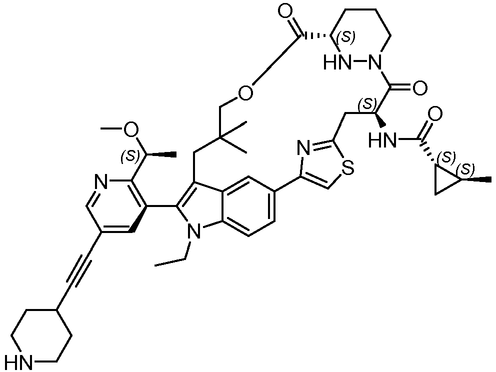

Example **48** was prepared by the following procedure:

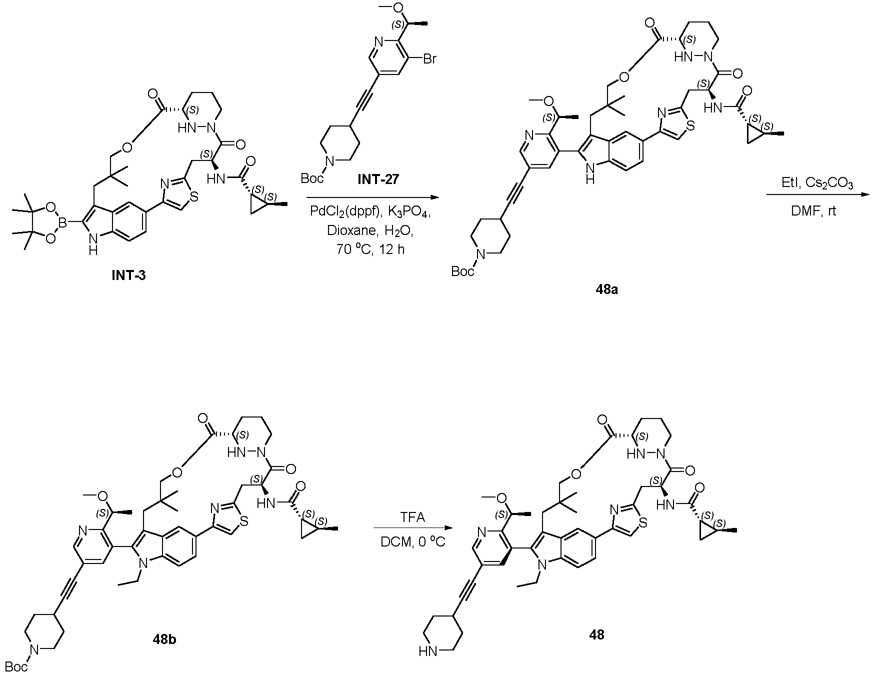

By replacing **INT-15** in the synthesis step of Compound **10** with **INT-27** and following a similar method and reaction procedure, Compound **48** can be obtained. ESI-MS (m/z): 820.5 [M+H]⁺; LC-MS retention time RT = 1.68 min; HPLC retention time RT = 12.19 min.

¹H NMR (500 MHz, DMSO) δ 8.75 (d, *J* = 2.0 Hz, 1H), 8.53 - 8.48 (m, 2H), 7.81 (s, 1H), 7.78 - 7.73 (m, 2H), 7.57 (d, *J* = 8.5 Hz, 1H), 5.58 - 5.53 (m, 1H), 5.10 - 5.05 (m, 1H), 4.36 - 4.28 (m, 2H), 4.26 (d, *J* = 6.0 Hz, 1H), 4.27 - 4.15 (m, 3H), 4.09 - 4.04 (m, 1H), 3.57 (s, 2H), 3.25 (s, 3H), 3.19 - 3.14 (m, 2H), 2.98 - 2.94 (m, 2H), 2.78 - 2.74 (m, 1H), 2.65 - 2.59 (m, 2H), 2.39 - 2.34 (m, 2H), 2.12 - 2.06 (m, 1H), 2.01 - 1.97 (m, 1H), 1.86 - 1.82 (m, 2H), 1.82 - 1.77 (m, 2H), 1.58 - 1.54 (m, 1H), 1.51 - 1.48 (m, 1H), 1.34 (d, *J* = 6.0 Hz, 3H), 1.27 - 1.20 (m, 4H), 1.06 (s, 3H), 0.91 (s, 3H), 0.89 - 0.85 (m, 3H), 0.56 - 0.53 (m, 1H), 0.33 (s, 3H).

### Example 49

### (1S,2S)-N-((6³S,4S,Z)-1¹-ethyl-1²-(2-((S)-1-methoxyethyl)-5-(3-(4-methyl-4-oxido-1,4-azaphosphinan-1-yl)prop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

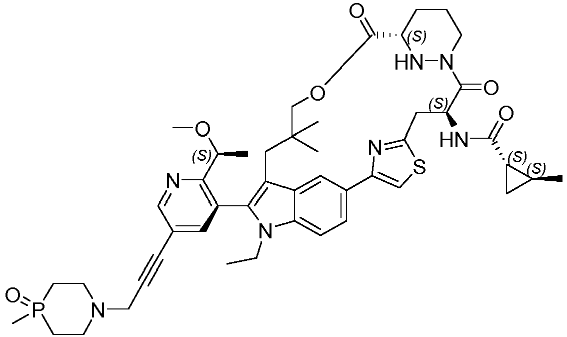

By replacing **INT-15** in Example **10** with **INT-43** and following a similar method and reaction procedure, Compound **49** can be obtained. ESI-MS (m/z): 882.5 [M+H]⁺; LC-MS retention time RT = 1.51 min; HPLC retention time RT = 10.62 min.

¹H NMR (500 MHz, DMSO) δ 8.81 (d, *J* = 2.0 Hz, 1H), 8.53 (d, *J* = 9.0 Hz, 1H), 8.49 (d, *J* = 1.5 Hz, 1H), 7.86 (d, *J* = 2.0 Hz, 1H), 7.81 (s, 1H), 7.77 - 7.73 (m, 1H), 7.58 (d, *J* = 9.0 Hz, 1H), 5.56 (t, *J* = 9.0 Hz, 1H), 5.11 - 5.06 (m, 1H), 4.37 - 4.30 (m, 1H), 4.29 - 4.22 (m, 2H), 4.12 - 4.03 (m, 1H), 4.12 - 4.03 (m, 1H), 3.66 (s, 2H), 3.57 (s, 2H), 3.32 - 3.29 (m, 1H), 3.25 (s, 3H), 3.17 - 3.13 (m, 1H), 3.00 - 2.90 (m, 3H), 2.78 - 2.70 (m, 3H), 2.40 - 2.35 (m, 1H), 2.12 - 2.04 (m, 1H), 1.93 - 1.83 (m, 3H), 1.83 - 1.76 (m, 3H), 1.54 - 1.47 (m, 2H), 1.44 - 1.40 (m, 3H), 1.35 (d, *J= 6.0* Hz, 3H), 1.09 - 1.02 (m, 4H), 0.91 (s, 3H), 0.89 - 0.83 (m, 4H), 0.57 - 0.52 (m, 1H), 0.33 (s, 3H).

### Example 50

### (1S,2S)-N-((6³S,4S,Z)-1¹-ethyl-1²-(2-((S)-1-methoxyethyl)-5-(3-(2-methyl-2,7-diazaspiro[3.5]nonan-7-yl)prop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

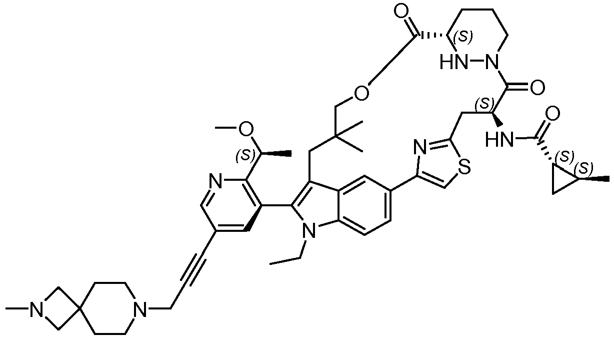

By replacing **INT-15** in Example **10** with **INT-44** and following a similar method and reaction procedure, Compound **50** can be obtained. ESI-MS (m/z): 889.6 [M+H]⁺; LC-MS retention time RT = 1.65 min; HPLC retention time RT = 12.62 min.

¹H NMR (500 MHz, DMSO) δ 8.78 (d, *J* = 2.0 Hz, 1H), 8.52 (d, *J* = 9.0 Hz, 1H), 8.49 (d, *J* = 1.5 Hz, 1H), 7.83 (d, *J* = 2.0 Hz, 1H), 7.81 (s, 1H), 7.77 - 7.73 (m, 1H), 7.58 (d, *J* = *9.0* Hz, 1H), 5.56 (t, *J* = 9.0 Hz, 1H), 5.11 - 5.06 (m, 1H), 4.37 - 4.30 (m, 1H), 4.28 - 4.22 (m, 2H), 4.21 - 4.13 (m, 2H), 4.11 - 4.03 (m, 1H), 3.60 - 3.54 (m, 2H), 3.50 (s, 2H), 3.25 (s, 3H), 3.17 - 3.11 (m, 2H), 2.98 - 2.93 (m, 4H), 2.78 - 2.72 (m, 1H), 2.65 - 2.61 (m, 1H), 2.44 - 2.40 (m, 3H), 2.37 - 2.35 (m, 1H), 2.24 (s, 3H), 2.09 - 2.04 (m, 1H), 1.82 - 1.76 (m, 2H), 1.70 - 1.64 (m, 4H), 1.56 - 1.47 (m, 3H), 1.35 (d, *J* = *6.0* Hz, 3H), 1.26 - 1.21 (m, 2H), 1.09 - 1.03 (m, 4H), 0.91 (s, 3H), 0.89 - 0.84 (m, 4H), 0.57 - 0.52 (m, 1H), 0.33 (s, 3H).

### Example 51

### (1S,2S)-N-((6³S,4S,Z)-1¹-ethyl-1²-(5-(((2S)-1-imino-4-methyl-1-oxido-1λ⁶-thiomorpholin-2-yl)ethynyl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

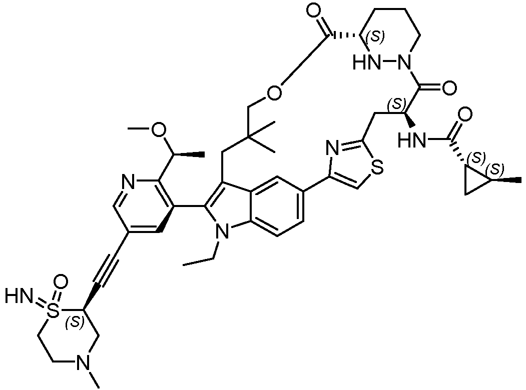

By replacing **INT-15** in Example **10** with **INT-46** and following a similar method and reaction procedure, Compound **51** can be obtained. ESI-MS (m/z): 883.5 [M+H]⁺; LC-MS retention time RT = 1.56 min; HPLC retention time RT = 11.07 min.

¹H NMR (500 MHz, DMSO-*d*₆) δ 8.87 - 8.79 (m, 1H), 8.57 - 8.48 (m, 2H), 7.93 - 7.88 (m, 1H), 7.81 (s, 1H), 7.76 (d, *J* = 8.5 Hz, 1H), 7.59 (d, *J = 9.0* Hz, 1H), 5.56 (t, *J* = 9.0 Hz, 1H), 5.13 - 5.06 (m, 1H), 4.36 - 4.17 (m, 4H), 4.10 - 4.02 (m, 2H), 4.00 (s, 1H), 3.57 (s, 2H), 3.32 - 3.30 (m, 2H), 3.29 - 3.24 (m, 4H), 3.17 - 2.94 (m, 6H), 2.81 - 2.73 (m, 2H), 2.45 (s, 3H), 2.41 - 2.36 (m, 1H), 2.11 - 2.05 (m, 1H), 1.83 - 1.76 (m, 2H), 1.54 - 1.47 (m, 2H), 1.36 (d, *J* = 6.0 Hz, 3H), 1.09 - 1.03 (m, 4H), 0.92 (s, 3H), 0.90 - 0.84 (m, 4H), 0.57 - 0.52 (m, 1H), 0.34 (s, 3H).

### Example 52

### (1r,2R,3S)-N-((6³S,4S,Z)-1¹-ethyl-1²-(2-((S)-1-methoxyethyl)-5-(3-(4-methylpiperazin-1-yl)prop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

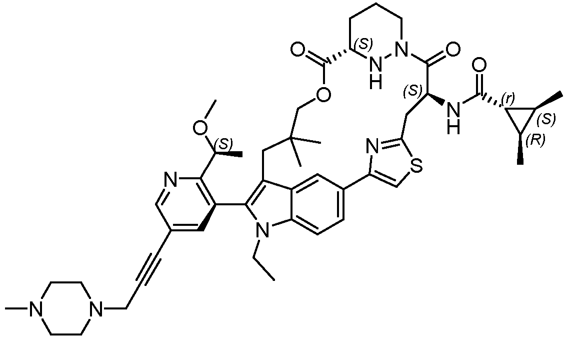

By replacing **INT-16** in the synthesis step of Compound **11** with **INT-45** and following a similar method and reaction procedure, Compound **52** can be obtained. ESI-MS (m/z): 863.6 [M+H]⁺; LC-MS retention time RT = 1.78 min; HPLC retention time RT = 12.82 min.

¹H NMR (500 MHz, DMSO-*d*₆) δ 8.80 (d, *J* = 2.0 Hz, 1H), 8.55 - 8.47 (m, 1H), 8.39 (d, *J* = 9.0 Hz, 1H), 7.87 - 7.78 (m, 2H), 7.75 (dd, *J* = 8.5, 1.5Hz, 1H), 7.58 (d, *J* = 8.5 Hz, 1H), 5.55 (t, *J* = 9.0 Hz, 1H), 5.12 - 5.00 (m, 1H), 4.35 - 4.17 (m, 4H), 4.12 - 4.04 (m, 1H), 3.59 - 3.55 (m, 4H), 3.25 (s, 3H), 3.17 - 3.13 (m, 1H), 2.99 - 2.93 (m, 1H), 2.79 - 2.71 (m, 2H), 2.59 - 2.54 (m, 4H), 2.40 - 2.36 (m, 2H), 2.16 (s, 3H), 2.09 - 2.06 (m, 1H), 1.82 - 1.75 (m, 2H), 1.55 - 1.45 (m, 2H), 1.35 (d, *J* = 6.0 Hz, 3H), 1.26 - 1.22 (m, 2H), 1.18 - 1.15 (m, 2H), 1.09 - 1.04 (m, 6H), 0.92 - 0.86 (m, 6H), 0.34 (s, 3H).

### Example 53

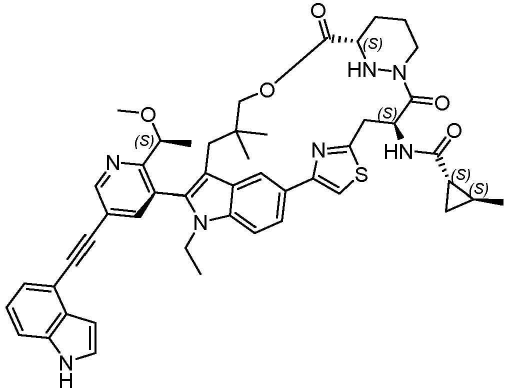

By replacing **INT-15** in the synthesis step of Compound **10** with **INT-47** and following a similar method and reaction procedure, Compound **53** can be obtained. ESI-MS (m/z): 852.7 [M+H]⁺; LC-MS retention time RT = 2.15 min; HPLC retention time RT = 16.48 min.

¹H NMR (500 MHz, DMSO-*d*₆) δ 11.42 (s, 1H), 9.03 (d, *J* = 2.0 Hz, 1H), 8.60 - 8.47 (m, 2H), 8.18 (d, *J* = 2.0 Hz, 1H), 7.82 (s, 1H), 7.78 (dd, *J* = *8.5,* 1.5 Hz, 1H), 7.67 (d, *J* = 8.5 Hz, 1H), 7.61 (d, *J* = 8.5 Hz, 1H), 7.45 (t, *J* = 3.0 Hz, 1H), 7.36 (d, *J* = 7.0 Hz, 1H), 7.07 (t, *J* = 8.0 Hz, 1H), 6.57 - 6.54 (m, 1H), 5.56 (t, *J* = 9.0 Hz, 1H), 5.12 - 5.05 (m, 1H), 4.42 - 4.32 (m, 2H), 4.27 - 4.13 (m, 3H), 3.60 (s, 2H), 3.36 - 3.34 (m, 1H), 3.29 (s, 3H), 3.17 - 3.12 (m, 1H), 3.05 - 3.00 (m, 1H), 2.80 - 2.74 (m, 1H), 2.47 - 2.43 (m, 1H), 2.12 - 2.05 (m, 1H), 1.82 - 1.75 (m, 2H), 1.54 - 1.47 (m, 2H), 1.40 (d, *J* = 6.0 Hz, 3H), 1.110 - 1.04 (m, 4H), 0.96 - 0.91 (m, 6H), 0.88 - 0.84 (m, 1H), 0.58 - 0.54 (m, 1H), 0.38 (s, 3H).

### Example 54

### N-((6³S,4S,Z)-1¹-ethyl-1²-(2-((S)-1-methoxyethyl)-5-(3-morpholinoprop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)cyclopropanecarboxamide

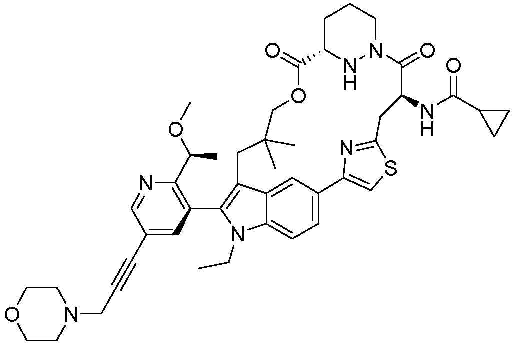

By replacing **INT-3b** in the synthesis step of Compound **1** with cyclopropanecarboxylic acid and following a similar method and reaction procedure, Compound **54** can be obtained. ESI-MS (m/z): 822.8 [M+H]⁺; LC-MS retention time RT = 1.73 min; HPLC retention time RT = 12.40 min.

¹H NMR (500 MHz, DMSO-*d*₆) δ 8.84 - 8.79 (m, 1H), 8.58 (d, *J* = 9.0 Hz, 1H), 8.52 - 8.47 (m, 1H), 7.88 - 7.85 (m, 1H), 7.82 (s, 1H), 7.76 (d, *J* = 8.5 Hz, 1H), 7.58 (d, *J* = 8.5 Hz, 1H), 5.57 (t, *J* = 9.0 Hz, 1H), 5.11 - 5.05 (m, 1H), 4.38 - 4.15 (m, 4H), 4.12 - 4.06 (m, 1H), 3.64 - 3.59 (m, 4H), 3.59 - 3.56 (m, 4H), 3.29 - 3.26 (m, 1H), 3.26 - 3.23 (m, 3H), 3.19 - 3.12 (m, 1H), 3.00 - 2.95 (m, 1H), 2.79 - 2.73 (m, 1H), 2.55 - 2.52 (m, 4H), 2.41 - 2.35 (m, 2H), 2.12 - 2.06 (m, 1H), 1.81 - 1.72 (m, 3H), 1.55 - 1.47 (m, 1H), 1.35 (d, *J* = 6.0 Hz, 3H), 0.92 (s, 3H), 0.88 (t, *J* = 7.0 Hz, 3H), 0.73 - 0.67 (m, 3H), 0.64 - 0.60 (m, 1H), 0.34 (s, 3H).

### Example 55

### (1S,2S)-N-((6³S,4S,Z)-1¹-ethyl-1²-(5-((1-(2-hydroxyethyl)piperidin-4-yl)ethynyl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

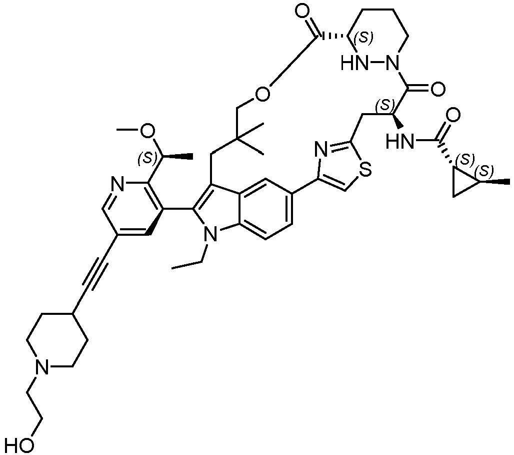

Example **55** was prepared via the following precedure:

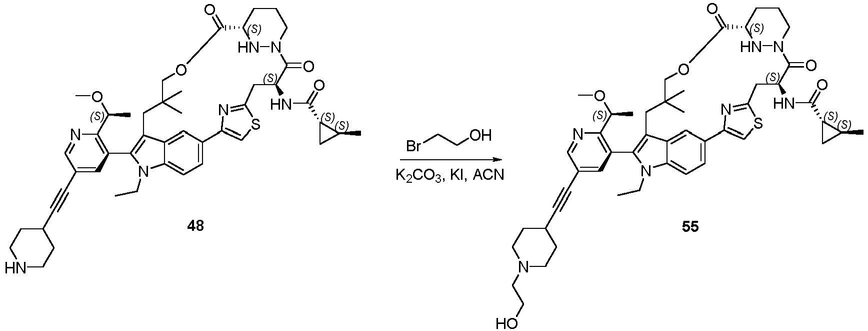

Step 1: Dissolve Compound **48** (60 mg, 0.073 mmol) in acetonitrile (2 mL), then sequentially add potassium carbonate (20 mg, 0.146 mmol), potassium iodide (24 mg, 0.146 mmol), and 2-bromoethanol (9 mg, 0.073 mmol). Stir the reaction mixture at room temperature for 8 hours. After LCMS confirms the completion of the reaction, add water (10 mL) to the reaction system, and extract with dichloromethane (10 mL × 2). Combine the organic phases, wash with saturated brine, dry over anhydrous sodium sulfate, filter, and concentrate. The residue is purified by preparative liquid chromatography to obtain Compound **55** (6 mg, yield 9.5%). ESI-MS (m/z): 864.7 [M+H]⁺; LC-MS retention time RT = 1.73 min.

¹H NMR (500 MHz, DMSO) δ 8.78 - 8.73 (m, 1H), 8.52 - 8.48 (m, 2H), 7.80 (s, 1H), 7.78 - 7.73 (m,2H), 7.57 (d, *J* = 8.5 Hz, 1H), 5.56 (t, *J* = 9.0 Hz, 1H), 5.10 - 5.05 (m, 1H), 4.36 - 4.15 (m, 5H), 4.11 - 4.02 (m, 1H), 3.57 (s, 2H), 3.50 - 3.45 (m, 2H), 3.24 (s, 3H), 3.19 - 3.11 (m, 1H), 3.00 - 2.94 (m, 1H), 2.80 - 2.72 (m, 3H), 2.71 - 2.63 (m, 2H), 2.42 - 2.34 (m, 3H), 2.23 - 2.14 (m, 2H), 2.10 - 2.05 (m, 1H), 1.89 - 1.85 (m, 2H), 1.82 - 1.75 (m, 2H), 1.67 - 1.59 (m, 2H), 1.56 - 1.47 (m, 2H), 1.35 (d, *J* = 6.0 Hz, 3H), 1.11 - 1.02 (m, 4H), 0.91 (s, 3H), 0.90 - 0.85 (m, 4H), 0.57 - 0.52 (m, 1H), 0.34 (s, 3H).

### Example 56

### (1S,2S)-N-((6³S,4S,Z)-1¹-ethyl-1²-(2-((S)-1-methoxyethyl)-5-((1-methyl-1H-indol-4-yl)ethynyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

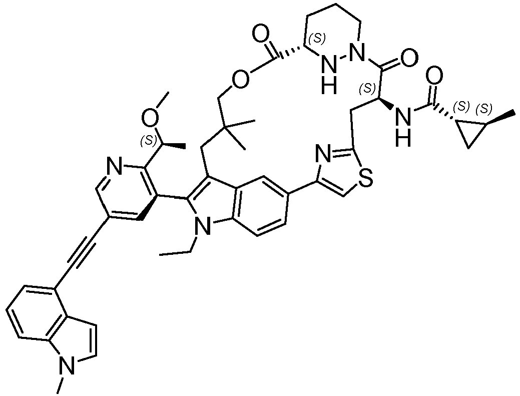

By replacing **INT-15** in the synthesis step of Compound **10** with **INT-48** and following a similar method and reaction procedure, Compound **56** can be obtained. ESI-MS (m/z): 865.7 [M+H]⁺; LC-MS retention time RT = 2.15 min; HPLC retention time RT = 16.44 min.

¹H NMR (500 MHz, DMSO-*d*₆) δ 8.99 (d, *J* = 2.5 Hz, 1H), 8.56 - 8.49 (m, 2H), 8.07 (d, *J* = 2.0 Hz, 1H), 7.82 (s, 1H), 7.77 (dd, *J* = 8.5, 2.0 Hz, 1H), 7.61-7.56 (m, 2H), 7.48 (d, *J* = 2.5 Hz, 1H), 7.33 (d, *J* = 7.0 Hz, 1H), 7.21 (t, *J* = 7.0 Hz, 1H), 6.73 (d, *J* = 2.0 Hz, 1H), 5.56 (t, *J* = 9.0 Hz, 1H), 5.10 - 5.05 (m, 1H), 4.38-4.29 (m, 2H), 4.26 - 4.10 (m, 3H), 3.84 (s, 3H), 3.59 (s, 2H), 3.37 - 3.33 (m, 1H), 3.28 (s, 3H), 3.17-3.12 (m, 1H), 3.03 - 2.97 (m, 1H), 2.79-2.73 (m, 1H), 2.49 - 2.35 (m, 1H), 2.10-2.07 (m, 1H), 1.85-1.76 (m, 2H), 1.57 - 1.46 (m, 2H), 1.39 (d, *J= 6.0* Hz, 3H), 1.10 - 1.04 (m, 4H), 0.97 - 0.90 (m, 6H), 0.89 - 0.84 (m, 1H), 0.58 - 0.52 (m, 1H), 0.38 (s, 3H).

### Example 57

### (1S,2S)-N-((6³S,4S,Z)-1¹-ethyl-1²-(5-(3-(1-imino-1-oxido-1l6-thiomorpholino)prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

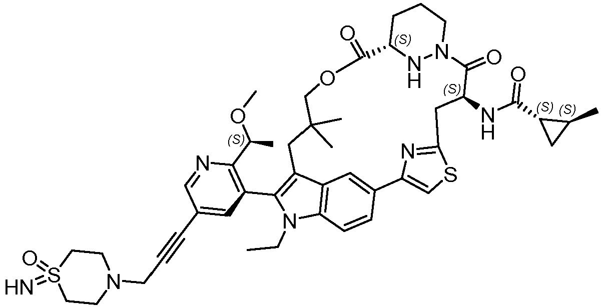

By replacing **INT-15** in the synthesis step of Compound **10** with **INT-48** and following a similar method and reaction procedure, Compound **57** can be obtained. ESI-MS (m/z): 883.7 [M+H]⁺; LC-MS retention time RT = 1.56 min; HPLC retention time RT = 10.93 min.

¹H NMR (500 MHz, DMSO-*d*₆) δ 8.81 (d, *J* = 2.0 Hz, 1H), 8.53 - 8.49 (m, 2H), 7.85 (d, *J* = 2.0 Hz, 1H), 7.81 (s, 1H), 7.76-7.74 (m, 1H), 7.59-7.57 (m, 1H), 5.56 (t, *J* = 9.0 Hz, 1H), 5.12 - 5.07 (m, 1H), 4.39 - 4.16 (m, 4H), 4.12 - 4.03 (m, 1H), 3.72 (s, 2H), 3.65 (s, 1H), 3.57 (s, 2H), 3.31 - 3.28 (m, 1H), 3.25 (s, 3H), 3.18 - 3.07 (m, 2H), 3.06 - 3.02 (m, 3H), 3.02 - 2.94 (m, 5H), 2.79 - 2.73 (m, 1H), 2.42 - 2.35 (m, 1H), 2.09 - 2.07 (m, 1H), 1.82 - 1.76 (m, 2H), 1.55 - 1.47 (m, 2H), 1.35 (d, *J* = 6.0 Hz, 3H), 1.07 - 1.06 (m, 4H), 0.91 (s, 3H), 0.89 - 0.86 (m, 4H), 0.56 - 0.54 (m, 1H), 0.34 (s, 3H).

### Example 58

### (1r,2R,3S)-N-((6³S,4S,Z)-1²-(5-(3-(dimethylamino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

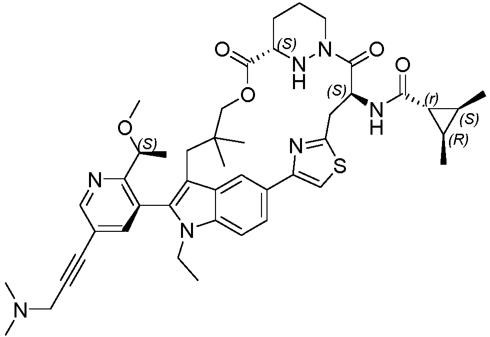

By replacing N-methyltetrahydro-2H-pyran-4-amine in the synthesis step of Compound **36** with dimethylamine and following a similar method and reaction procedure, Compound **58** can be obtained. ESI-MS (m/z): 808.6 [M+H]⁺; LC-MS retention time RT = 1.96 min; HPLC retention time RT = 14.23 min.

¹H NMR (500 MHz, DMSO-*d*₆) δ 8.81 (d, *J* = 2.0 Hz, 1H), 8.53 - 8.47 (m, 1H), 8.41 (d, *J* = 9.0 Hz, 1H), 7.88 - 7.81 (m, 2H), 7.76 (dd, *J* = 8.5, 1.5 Hz, 1H), 7.59 (d, *J* = 8.5Hz, 1H), 5.55 (t, *J* = 9.0 Hz, 1H), 5.13 - 5.04 (m, 1H), 4.36 - 4.07 (m, 5H), 3.57 (s, 2H), 3.51 (s, 2H), 3.32 - 3.29 (m, 1H), 3.25 (s, 3H), 3.17 - 3.11 (m, 1H), 2.99 - 2.93 (m, 1H), 2.80 - 2.72 (m, 1H), 2.41 - 2.33 (m, 2H), 2.26 (s, 6H), 2.11 - 2.03 (m, 1H), 1.82 - 1.74 (m, 2H), 1.56 - 1.46 (m, 1H), 1.35 (d, *J* = 6.0Hz, 3H), 1.17 - 1.14 (m, 2H), 1.09 - 1.05 (m, 6H), 0.91 - 0.83 (m, 6H), 0.34 (s, 3H).

### Example 59

### (1S,2S)-N-((6³S,4S,Z)-1¹-ethyl-1²-(2-((S)-1-methoxyethyl)-5-(3-(piperidin-1-yl)prop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

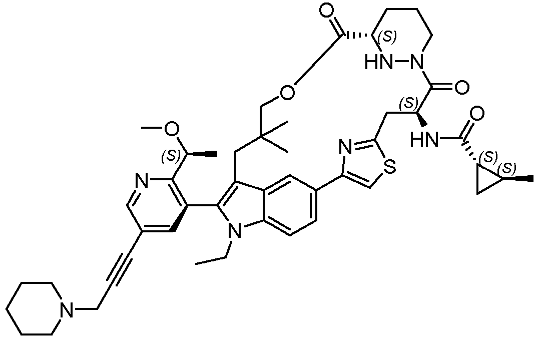

By replacing **INT-15** in Example **10** with **INT-49** and following a similar method and reaction procedure, Compound **59** can be obtained. ESI-MS (m/z): 835.1 [M+H]⁺; LC-MS retention time RT = 2.06 min.

¹H NMR (500 MHz, DMSO) δ 8.80 (d, *J* = 2.0 Hz, 1H), 8.52 (d, *J* = 9.0 Hz, 1H), 8.50 (d, *J* = 1.0 Hz, 1H), 7.84 (d, *J* = 2.0 Hz, 1H), 7.81 (s, 1H), 7.77 - 7.73 (m, 1H), 7.58 (d, *J* = 8.5 Hz, 1H), 5.56 (t, *J* = 9.5 Hz, 1H), 5.11 - 5.06 (m, 1H), 4.36 - 4.16 (m, 5H), 4.12 - 4.03 (m, 1H), 3.57 (s, 2H), 3.51 (s, 2H), 3.25 (s, 3H), 3.17 - 3.08 (m, 2H), 2.99 - 2.93 (m, 1H), 2.78 - 2.72 (m, 1H), 2.40 - 2.34 (m, 2H), 2.10 - 2.05 (m, 1H), 1.82 - 1.73 (m, 2H), 1.56 - 1.47 (m, 7H), 1.40 - 1.36 (m, 2H), 1.35 (d, *J* = 6.0 Hz, 3H), 1.10 - 1.02 (m, 5H), 0.91 (s, 3H), 0.89 - 0.85 (m, 4H), 0.58 - 0.52 (m, 1H), 0.33 (s, 3H).

### Example 60

### (1S,2S)-N-((6³S,4S,Z)-1¹-ethyl-1²-(2-((S)-1-methoxyethyl)-5-(3-(4-methylpiperazin-1-yl)prop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

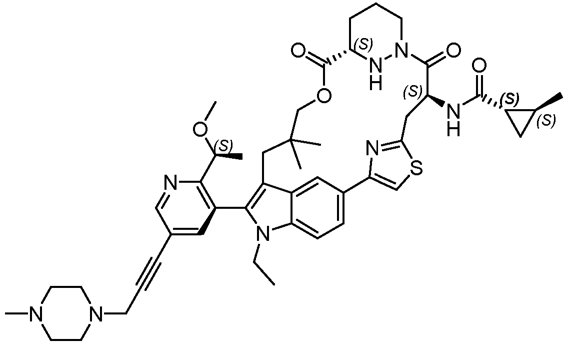

By replacing **INT-15** in the synthesis step of Compound **10** with **INT-45** and following a similar method and reaction procedure, Compound **60** can be obtained. ESI-MS (m/z): 849.9 [M+H]⁺; LC-MS retention time RT = 1.70 min.

¹H NMR (500 MHz, DMSO-*d*₆) δ 8.81 (d, *J* = 2.0 Hz, 1H), 8.57 - 8.48 (m, 2H), 7.85 (d, *J* = 2.0 Hz, 1H), 7.82 (s, 1H), 7.76 (dd, *J* = 8.5, 1.5 Hz, 1H), 7.59 (d, *J* = 8.5 Hz, 1H), 5.56 (t, *J* = 9.0 Hz, 1H), 5.15 - 5.01 (m, 1H), 4.38 - 4.16 (m, 4H), 4.12 - 4.04 (m, 1H), 3.67 - 3.53 (m, 4H), 3.33 - 3.29 (m, 4H), 3.26 (s, 3H), 3.17 - 3.11 (m, 1H), 3.00 - 2.93 (m, 1H), 2.80 - 2.72 (m, 1H), 2.66 - 2.55 (m, 4H), 2.40 - 2.35 (m, 2H), 2.34 - 2.24 (m, 3H), 2.11 - 2.03 (m, 1H), 1.83 - 1.72 (m, 2H), 1.55 - 1.45 (m, 2H), 1.35 (d, *J* = 6.0 Hz, 3H), 1.09 - 1.02 (m, 4H), 0.92 (s, 3H), 0.89 - 0.85 (m, 3H), 0.58 - 0.52 (m, 1H), 0.33 (s, 3H).

### Example 61

### (1S,2S)-N-((6³S,4S,Z)-1²-(5-(3-(dimethylamino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

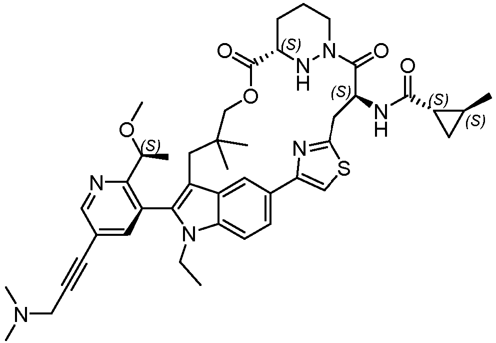

By replacing (R)-3-methylmorpholine hydrochloride in the synthesis step of Compound **15** with dimethylamine and following a similar method and reaction procedure, Compound **61** can be obtained. ESI-MS (m/z): 794.8 [M+H]⁺; LC-MS retention time RT = 1.83 min; HPLC retention time RT = 13.71 min.

¹H NMR (500 MHz, DMSO-*d*₆) δ 8.81 (d, *J* = 2.0 Hz, 1H), 8.55 - 8.49 (m, 2H), 7.84 (d, *J* = 2.0 Hz, 1H), 7.81 (s, 1H), 7.78 - 7.74 (m, 1H), 7.58 (d, *J* = 8.5 Hz, 1H), 5.56 (t, *J* = 9.0 Hz, 1H), 5.12 - 5.06 (m, 1H), 4.35 - 4.06 (m, 5H), 3.57 (s, 2H), 3.50 (s, 2H), 3.33 - 3.30 (m, 1H), 3.25 (s, 3H), 3.18 - 3.11 (m, 1H), 2.99 - 2.93 (m, 1H), 2.80 - 2.73 (m, 1H), 2.42 - 2.35 (m, 1H), 2.25 (s, 6H), 2.11 - 2.05 (m, 1H), 1.83 - 1.74 (m, 2H), 1.55 - 1.47 (m, 2H), 1.35 (d, *J* = 6.0 Hz, 3H), 1.09 - 1.04 (m, 4H), 0.93 - 0.84 (m, 7H), 0.57 - 0.52 (m, 1H), 0.34 (s, 3H).

### Biological screening and results of RAS inhibitors

### Test Example 1: In vitro cell proliferation inhibition test

Due to the diversity of RAS mutations and in order to evaluate the activity of compounds in different RAS mutant cell lines, we selected KRAS^{WT}, KRAS^{G12C}, KRAS^{G12D}), KRAS^{G12V} and BRAF mutant cell lines (see the table below) for in vitro activity evaluation and screening of compounds .

| **Cell Line** | **Histotype** | **Mutant** |
|---|---|---|
| NCI-H358 | Lung; Bronchiole | KRAS (p.G12C) |
| MIA PaCa-2 | Pancreas | KRAS (p.G12C) |
| LS513 | Large intestine; Cecum | KRAS (p.G12D) |
| AsPC-1 | Pancreas | KRAS (p.G12D) |
| HCC1588 | Lung | KRAS (p.G12D; BRAF (p.E204L) |
| SW480 | Large intestine; Colon | KRAS (p.G12V) |
| NCI-H727 | Lung; Bronchus | KRAS (p.G12V) |
| NCI-H520 | Lung | KRASWT; |
| HT-29 | Colon | KRAS^{WT}; BRAF (p.V600E) |

### Experimental protocol: CellTiter-Glo^{®} Cell Luminescent Viability Assay (Promega)

According to the doubling time of different cell lines, different numbers of cells (1000-5000 cells/well) were inoculated into 96-well plates containing 180 µl of the corresponding medium, and cultured overnight in a 37°C cell incubator containing 5% CO₂. On the second day, the compound to be tested was pre-diluted 3-fold with the medium, the highest concentration was 100 µM, and there were 10 concentration gradients in total; then 20 µl of the medium containing different concentrations of the compound was added to the cells in the 96-well plate to ensure the final concentration of the compound to be up to 10 µM, with 10 concentration gradients of 3-fold dilutions. After co-incubating cells and compounds for 72 hours, the 96-well plate was taken out of the incubator, placed at room temperature for 30 minutes, and then 25 µl CellTiter-Glo^{®} Reagent was added to each well to mix well, and incubated at room temperature for 10 minutes, and then 100 µl of the sample was transferred to a white 96-well plate (OptiPlateTM-96, PerkinElmer), the fluorescent signal value was read using a multi-functional microplate reader (SpectraMax^{®} i3x, Molecular devices). Subsequently, the signal value was standardized, and the four-parameter fitting regression equation was used for curve fitting to calculate the half maximal inhibitory concentration (IC50) of the compound on the cell line.

**Table 3: Antiproliferative activity of compounds of the present invention on KRAS cell mutants**

| Example | IC₅₀ (nM) | | Example | IC₅₀ (nM) | | Example | IC₅₀ (nM) | |
|---|---|---|---|---|---|---|---|---|
| | AsPC-1 (G12D) | SW480 (G12V) | | AsPC-1 (G12D) | SW480 (G12V) | | AsPC-1 (G12D) | SW480 (G12V) |
| 1 | 0.92 | 1.02 | 14 | <0.1 | NT | 36 | 0.73 | 0.54 |
| 1' | 582 | 343 | 14' | 57.1 | 0.2 | 37 | 1.01 | 0.68 |
| 2 | 0.25 | 0.33 | 15 | 1.7 | 2.2 | 38 | 0.07 | 0.18 |
| 2' | 172 | 74 | 15' | 1875 | 1065 | 40 | 1.35 | 1.5 |
| 3 | 3.5 | 2.2 | 16 | 38.8 | 28.8 | 41 | 0.56 | 0.65 |
| 3' | 292 | 2060 | 16' | 591 | 584 | 42 | 22.2 | 6.9 |
| 4 | 4.9 | 2.9 | 17 | 37.8 | 30.2 | 43 | 0.42 | 0.68 |
| 4' | 523 | 813 | 17' | 700 | 474 | 44 | 16.8 | 6.3 |
| 5 | NT | 24.6 | 18 | 3.4 | 0.42 | 45 | 5.3 | 7.4 |
| 5' | 3218 | 2017 | 19 | 2.2 | 1.5 | 46 | 19.5 | 11.4 |
| 6 | 14 | 18.5 | 20 | 2.4 | 8.9 | 47 | 456 | 116 |
| 6' | 3958 | 1834 | 21 | 0.82 | 1.1 | 48 | 5.4 | 1.7 |
| 7 | 5.1 | 4.6 | 22 | 6.5 | 19.2 | 49 | 7.5 | 9.2 |
| 7' | 2643 | 830 | 23 | 1.6 | 3.8 | 50 | 1.2 | 15.2 |
| 8 | 17.9 | 18.9 | 24 | 24.2 | 58.4 | 51 | 4.5 | 5.4 |
| 8' | 3873 | 2033 | 25 | 0.35 | 0.51 | 52 | 0.24 | 0.30 |
| 9 | 4.5 | 31.6 | 26 | 20.8 | 34.6 | 53 | 458 | 500 |
| 9' | 1356 | 963 | 27 | 4.6 | 14.5 | 54 | 17.3 | 35 |
| 10 | 14 | 19.8 | 28 | 1.9 | 2.5 | 55 | 3 | 23.3 |
| 10' | 4224 | 2889 | 29 | 1.2 | 2.8 | 56 | 291 | 487 |
| 11 | 1.7 | NT | 30 | 8.2 | 84.2 | 57 | 1.9 | 2.0 |
| 11' | NT | NT | 31 | 78 | 100 | 58 | 0.7 | 0.24 |
| 12 | 11.8 | 33.9 | 32+32' | 15.3 | 55.4 | 59 | 0.96 | 1.76 |
| 12' | 442.9 | 1790 | 33 | 2.9 | 6.5 | 60 | 1.71 | 1.55 |
| 13 | 5.7 | NT | 34 | 1.3 | 0.75 | 61 | 5.0 | 1.58 |
| 13' | 2825 | 7586 | 35 | 56 | 37 | | | |

| | | | | | | | | |
|---|---|---|---|---|---|---|---|---|
| * NT means not tested | | | | | | | | |

## Claims

1. A compound with a structure of formula (I) or a pharmaceutically acceptable salt, isotopic derivative, or stereoisomer thereof: 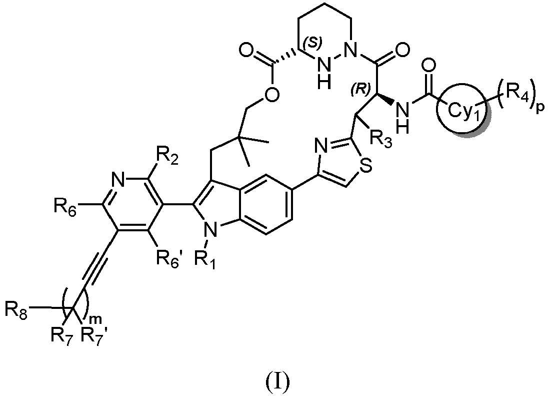 wherein:
R₁ represents C₁-C₆ alkyl, -(C₁-C₆ alkylene)-(C₃-C₈ cycloalkyl) or -(C₁-C₆ alkylene)-(3-8 membered heterocycloalkyl);
R₂ represents halogen, cyano, C₁-C₆ alkyl, -(C₀-C₆ alkylene)-(C₃-C₈ cycloalkyl), or -(C₀-C₆ alkylene)-(3-8 membered heterocycloalkyl), which may optionally be substituted by 0, 1 or 2 of the following substituents: -ORa, -SRa or - NRaRa';
R₃ represents hydrogen, -O (C₀-C₆ alkylene) Ra, -S (C₀-C₆ alkylene) Ra, -N (C₀-C₆ alkylene) Ra (C₀-C₆ alkylene) Ra', -O (C₂-C₆ alkylene) R_{L}, -S (C₂-C₆ alkylene) R_{L}, -N (C₂-C₆ alkylene) R_{L} (C₂-C₆ alkylene) R_{L}', or -N( C₂-C₆ alkylene) Ra (C₂-C₆ alkylene) R_{L}, wherein, R_{L}, R_{L}' each independently represents -ORa, -SRa, or NRaRa';
Cy₁ represents C₃-C₁₂ cycloalkyl or 3-12 membered heterocycloalkyl;
R₄ represents hydrogen, halogen, oxo, C₁-C₆ alkyl, -(C₀-C₆ alkylene) (C₃-C₆) cycloalkyl, -(C₀-C₆ alkylene) (3-8 membered) heterocycloalkyl, -(C₀-C₆ alkylene) ORa, -(C₀-C₆ alkylene) SRa, -(C₀-C₆ alkylene) NRaRa', -(C₀-C₆ alkylene) CORa, -(C₀-C₆ alkylene) COORa, -(C₀-C₆ alkylene) CONRaRa', -(C₀-C₆ alkylene) NRaCORa', -(C₀-C₆ alkylene) OCONRaRa', -(C₀-C₆ alkylene) NRaCONRaRa', -(C₀-C₆ alkylene) SORa, -(C₀-C₆ alkylene) S(O)₂Ra, -(C₀-C₆ alkylene) NRaS(O)₂Ra', -(C₀-C₆ alkylene) CN, - (C₀-C₆ alkylene) (C₆-C₁₀ aryl) or -(C₀-C₆ alkylene) (5-12 membered heteroaryl); wherein, R₄ on the two C atoms of Cy₁ together with the C atoms connected thereto and the atoms between the two C atoms can form a 3-8 membered ring, and the 3-8 membered ring can optionally contain 0 or 1 , 2 or 3 heteroatoms selected from N, O or S; or Cy₁ and two R4 on the same C atom together with the C atom connected thereto can form a 3-8 membered ring, and the 3-8 membered ring can optionally contain 0, 1, 2 or 3 heteroatoms selected from N, O or S;
R₈ represents-Cy₂-(R₅)_{q} or-NR₉R₉', wherein,
Cy₂ represents C₃-C₁₂ cycloalkyl, 3-12 membered heterocycloalkyl, C₆-C₁₀ aryl or 5-12 membered heteroaryl;
R₅ represents hydrogen, halogen, oxo, C₁-C₆ alkyl, -(C₀-C₆ alkylene) ORa, -(C₀-C₆ alkylene) SRa, -(C₀-C₆ alkylene) NRaRa', - (C₀-C₆ alkylene) CORa, -(C₀-C₆ alkylene) COORa, -(C₀-C₆ alkylene) CONRaRa', -(C₀-C₆ alkylene) NRaCORa', -(C₀-C₆ alkylene) OCONRaRa', -(C₀-C₆ alkylene) NRaCONRaRa', -(C₀-C₆ alkylene)SORa, -(C₀-C₆ alkylene)S(O)₂Ra or -(C₀-C₆ alkylene) NRaS(O)₂Ra', or R₅ on two C atoms of Cy₂ together with the C atoms connected thereto and the atoms between the two C atoms can form a 3-8 membered ring, and the 3-8 membered ring can optionally contain 0, 1, 2 or 3 heteroatoms selected from N, O or S; or Cy₂ and two R₅ on the same C atom together with the C atom connected thereto can form a 3-8 membered ring, and the 3-8 membered ring optionally may contain 0, 1, 2 or 3 heteroatoms selected from N, O or S; or at least one atom on the Cy₂ ring is replaced by S(=O)(=NRa) or S(=O)₂ substitution;
R₉ and R₉' each independently represent C₁-C₆ alkyl, C₃-C₈ cycloalkyl, 3-8 membered heterocycloalkyl, C₆-C₁₀ aryl or 5-12 membered heteroaryl which may be optionally substituted by q R₅;
R₆ and R₆' each independently represent hydrogen, halogen, C₁-C₆ alkyl, C₃-C₈ cycloalkyl or -(C₀-C₆ alkylene)CN;
R₇ and R₇' each independently represent hydrogen, halogen, C₁-C₆ alkyl, C₃-C₈ cycloalkyl, 3-8 membered heterocycloalkyl; or R₇ and R₇' form a 3-8 membered ring, which may optionally contain 0, 1, 2 or 3 heteroatoms selected from N, O, S;
wherein, p and q independently represent 0, 1, 2, 3 or 4;
m represents 0, 1, 2 or 3;
Ra and Ra' independently represent hydrogen, C₁-C₆ alkyl, and C₃-C₈ cycloalkyl; wherein, if Ra and Ra' are connected to the same N atom, the Ra and Ra' can be connected to the common N atom to form a 4-8 membered ring, which optionally may contain 0, 1, 2 or 3 heteroatoms selected from N, O or S;
each of the alkyl, cycloalkyl, heterocycloalkyl and alkylene can be independently substituted by 0, 1, 2, 3, 4, 5 or 6 halogen atoms.

2. The compound with the structure of formula (I) or a pharmaceutically acceptable salt, isotopic derivative, stereoisomer thereof according to claim 1, wherein, R₁ represents C₁-C₆ alkyl, preferably C₁-C₃ alkyl.

3. The compound having the structure of formula (I) or a pharmaceutically acceptable salt, isotopic derivative, stereoisomer thereof according to any one of the preceding claims, wherein, R₂ represents C₁-C₆ alkyl, which can optionally be substituted by 0, 1 or 2 -ORa substituents; more preferably, R₂ represents 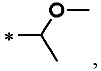 preferably 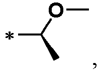 wherein, * represents the site where R₂ is linked to the linking site in formula (I).

4. The compound having the structure of formula (I) or a pharmaceutically acceptable salt, isotopic derivative, stereoisomer thereof according to any one of the preceding claims, wherein, R₃ represents -O(C₁-C₆) alkyl , -O (C₀-C₆ alkylene) (C₃-C₈) cycloalkyl, -O (C₀-C₆ alkylene) (3-8 membered) heterocycloalkyl, -O (C₂-C₆ alkylene )R_{L} or hydrogen.

5. The compound having the structure of formula (I) or a pharmaceutically acceptable salt, isotopic derivative, or stereoisomer thereof according to any one of the preceding claims, wherein Cy₁ represents C₃-C₈ cycloalkyl or 3-8 membered heterocycloalkyl.

6. The compound having the structure of formula (I) or a pharmaceutically acceptable salt, isotopic derivative, stereoisomer thereof according to any one of the preceding claims, wherein R₄ represents hydrogen, halogen, C₁-C₆ alkyl, -(C₀-C₆ alkylene) CONRaRa', -(C₀-C₆ alkylene) NRaCORa', -(C₀-C₆ alkylene) OCONRaRa', - (C₀-C₆ alkylene) CN, -(C₀ -C₆ alkylene) (5-12 membered heteroaryl), or R₄ on the two C atoms of Cy₁ together with the C atoms connected thereto and the atoms between the two C atoms can form a 3-8 member ring, and the 3-8 membered ring optionally can contain 0, 1, 2 or 3 heteroatoms selected from N, O or S; or two R₄ on the same C atom of Cy₁ together with **the C atom** connected thereto may form a 3-8 membered ring, and the 3-8 membered ring may optionally contain 0, 1, 2 or 3 heteroatoms selected from N, O or S.

7. The compound having the structure of formula (I) or a pharmaceutically acceptable salt, isotopic derivative, stereoisomer thereof according to any one of the preceding claims, wherein R₄ represents hydrogen, halogen, C₁-C₆ alkyl, -(C₀-C₆ alkylene) CONRaRa', -(C₀-C₆ alkylene) (5-12 membered heteroaryl), or R₄ on the two C atoms of Cy1 together with the C atoms connected thereto and the atoms between the two C atoms can form a 3-8 membered ring, and the 3-8 membered ring optionally can contain 0, 1, 2 or 3 heteroatoms selected from N, O or S; or two R₄ on the same C atom of Cy₁ together with the C atom **connected thereto** can form a 3-8 membered ring, and the 3-8 membered ring optionally can contain 0, 1, 2 or 3 heteroatoms selected from N, O or S.

8. The compound having the structure of formula (I) or a pharmaceutically acceptable salt, isotopic derivative, stereoisomer thereof according to any of the preceding claims, wherein Cy₂ represents a 3-8 membered heterocycloalkyl or a 5-12 membered heteroaryl.

9. The compound having the structure of formula (I) or a pharmaceutically acceptable salt, isotopic derivative, or stereoisomer thereof according to any one of the preceding claims, wherein R₅ represents hydrogen, halogen, C₁-C₆ alkyl , -(C₀-C₆ alkylene) ORa, -(C₀-C₆ alkylene) NRaRa', -(C₀-C₆ alkylene) CORa, -(C₀-C₆ alkylene) COORa, -(C0-C6 alkylene) CONRaRa', -(C₀-C₆ alkylene) NRaCORa'; or R₅ on the two C atoms of Cy₂ together with the C atoms connected thereto and the atoms between the two C atoms can form 3-8 membered ring, and the 3-8 membered ring optionally can contain 0, 1, 2 or 3 heteroatoms selected from N, O or S; or the two R₅ on the same C atom of Cy₂ and the C atom connected thereto can form a 3-8 membered ring, and the 3-8 membered ring optionally can contain 0, 1, 2 or 3 heteroatoms selected from N, O or S; Or at least one atom on the Cy₂ ring is replaced by S(=O)(=NRa) or S(=O)₂.

10. The compound having the structure of formula (I) or a pharmaceutically acceptable salt, isotopic derivative, or stereoisomer thereof according to any one of the preceding claims, wherein R₅ represents hydrogen, halogen, C₁-C₆ alkyl , -(C₀-C₆ alkylene) ORa, -(C₀-C₆ alkylene) NRaRa'; or R₅ on the two C atoms of Cy2 together with the C atoms connected thereto and the atoms between the two C atoms can form a 3-8 membered ring, and the 3-8 membered ring optionally can contain 0, 1, 2 or 3 heteroatoms selected from N, O or S; or two R₅ on the same C atom of Cy₂ is and the C atom connected thereto can form a 3-8 membered ring, and the 3-8 membered ring optionally can contain 0, 1, 2 or 3 selected from N, O or S of heteroatoms.

11. The compound having the structure of formula (I) or a pharmaceutically acceptable salt, isotopic derivative, or stereoisomer thereof according to any one of the preceding claims, wherein at least one of R₉ and R₉' represents C₁-C₆ alkyl substituted by q R₅.

12. The compound having the structure of formula (I) or a pharmaceutically acceptable salt, isotopic derivative, or stereoisomer thereof according to any one of the preceding claims, wherein R₆ and R₆' each independently represent hydrogen or C₁ -C₆ alkyl; more preferably, R₆ and R₆' each independently represent hydrogen or methyl.

13. The compound having the structure of formula (I) or a pharmaceutically acceptable salt, isotopic derivative, or stereoisomer thereof according to any of the preceding claims, wherein R₇ and R₇' each independently represent hydrogen, C₁ -C₆ alkyl; or R₇, R₇' form a 3-8 membered ring with the C atom connected thereto, and the ring may optionally contain 0, 1, 2 or 3 heteroatoms selected from N, O, S; more preferably, R₇ represents hydrogen.

14. The compound having the structure of formula (I) or a pharmaceutically acceptable salt, isotopic derivative, or stereoisomer thereof according to any one of the preceding claims, wherein m, p, and q are each independently preferably 0, 1 or 2.

15. The compound having the structure of formula (I) or pharmaceutically acceptable salt, isotopic derivative, stereoisomer thereof according to any one of the preceding claims, wherein the structure of -Cy₁-(R₄)p in formula (I) is selected from the following: 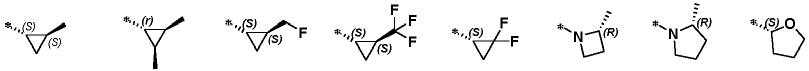 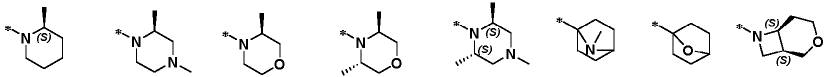 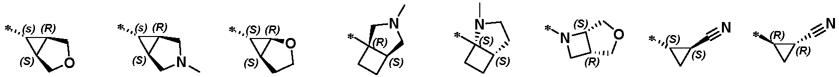 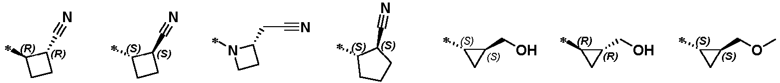 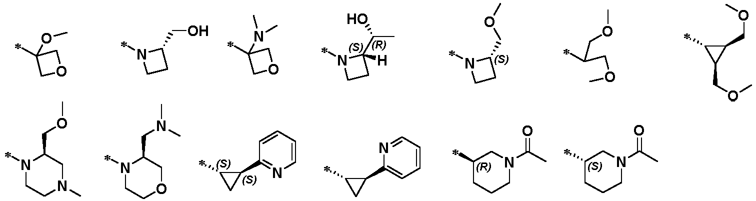 wherein, * represents the site where -Cy₁-(R₄)p is linked to the linking site in formula (I).

16. The compound having the structure of formula (I) or pharmaceutically acceptable salt, isotopic derivative, stereoisomer thereof according to any one of the preceding claims, wherein the structure of -Cy₁-(R₄)p in formula (I) is selected from the following:

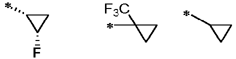

17. The compound having the structure of formula (I) or pharmaceutically acceptable salt, isotopic derivative, stereoisomer thereof according to any one of the preceding claims, wherein the structure of -Cy₂-(R₅)q in formula (I) is selected from the following: 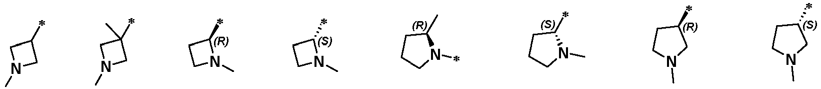 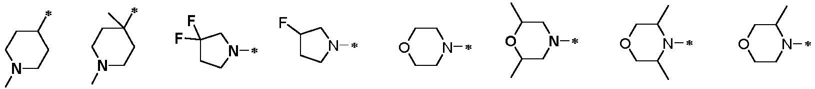 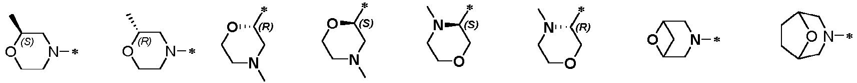 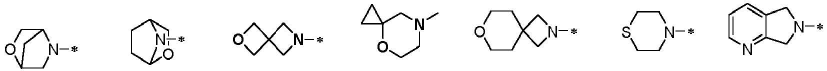 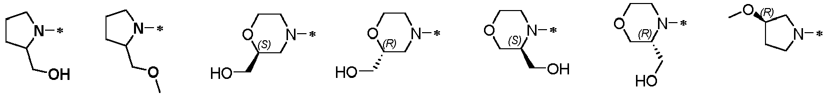 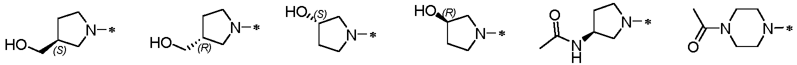 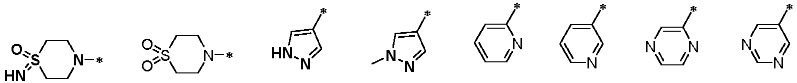 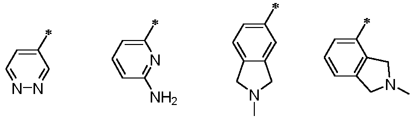 wherein, * represents the site where -Cy₂-(R₅)_{q} is linked to the linking site in formula (I).

18. The compound having the structure of formula (I) or pharmaceutically acceptable salt, isotopic derivative, stereoisomer thereof according to any one of claims 1-16, wherein the structure of -Cy₂-(R₅)q in formula (I) is selected from the following: 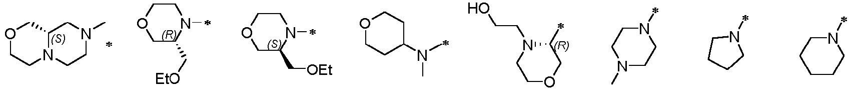 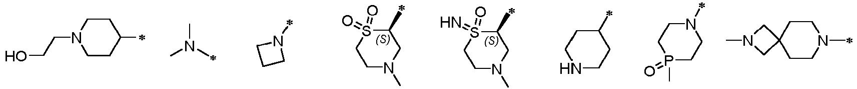 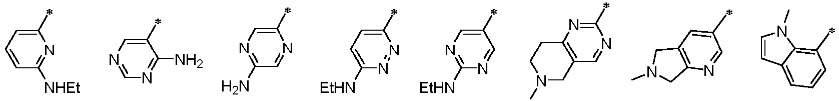 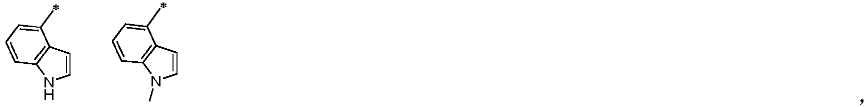 wherein, * represents the site where -Cy₂-(R₅)_{q} is linked to the linking site in formula (I).

19. The compound having the structure of formula (I) or its pharmaceutically acceptable salt, isotopic derivative, stereoisomer according to any one of the preceding claims, wherein the compound of formula (I) is the structure of formula (II) :

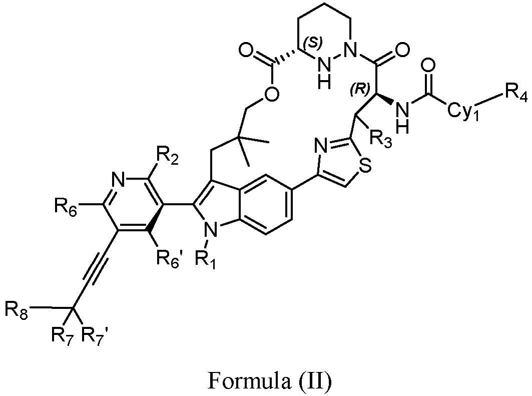

20. A compound having the structure: 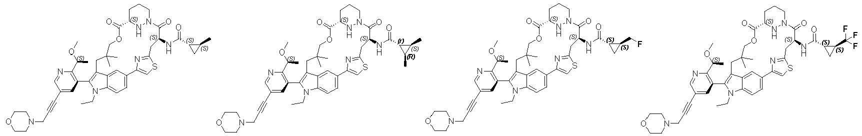 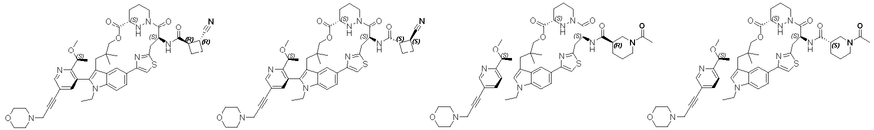 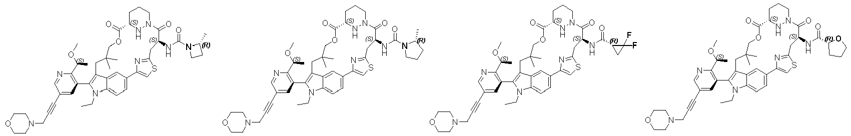 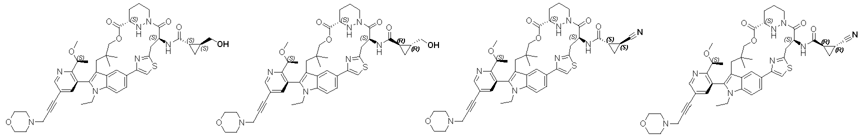 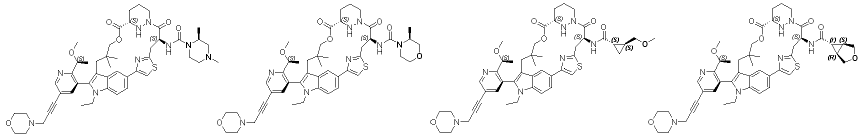 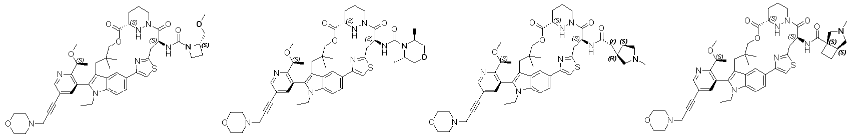 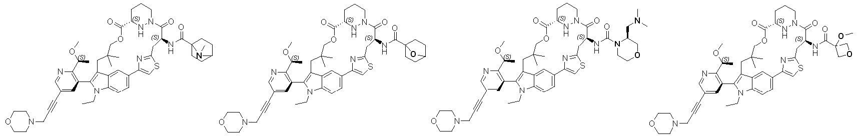 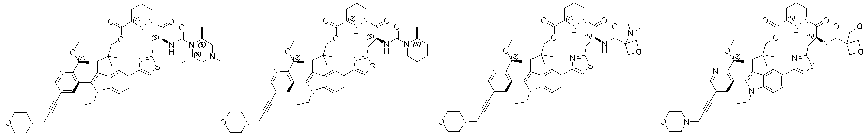 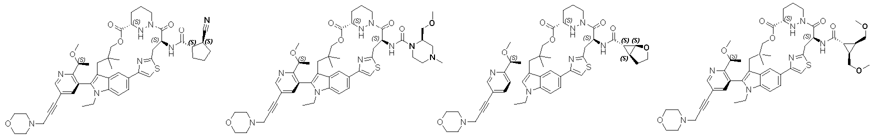 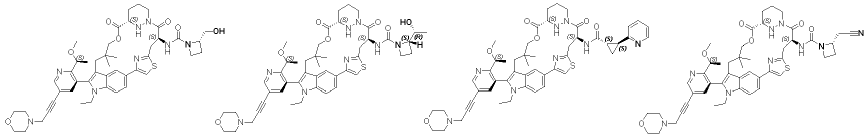 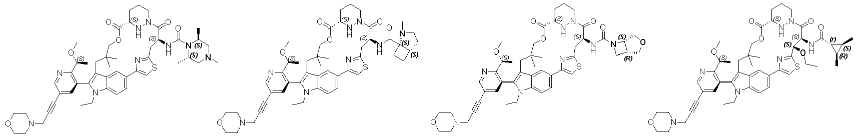 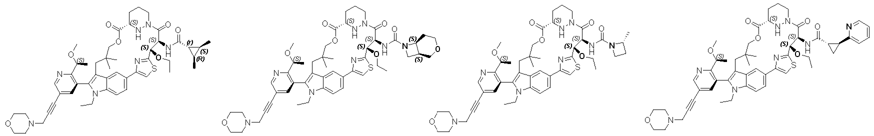 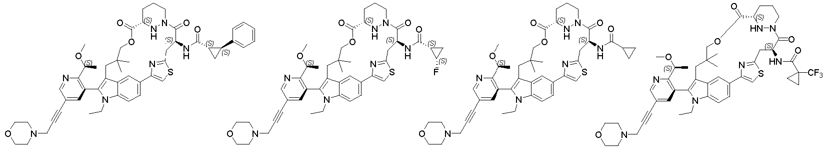 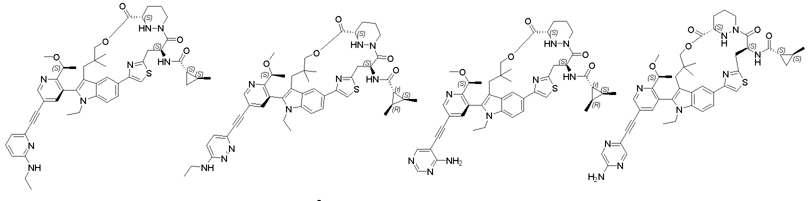 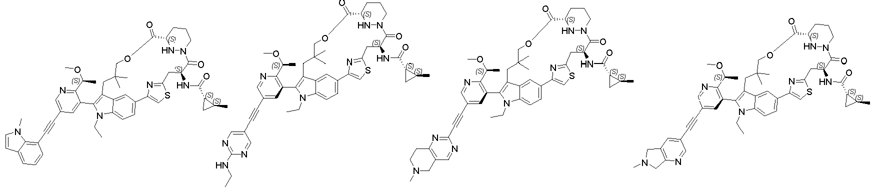 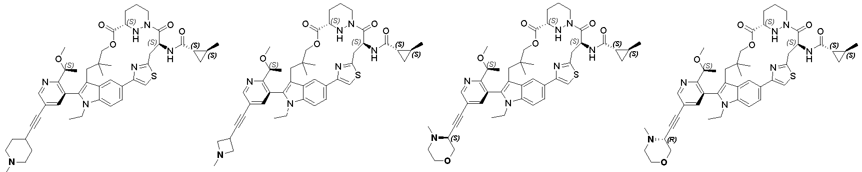 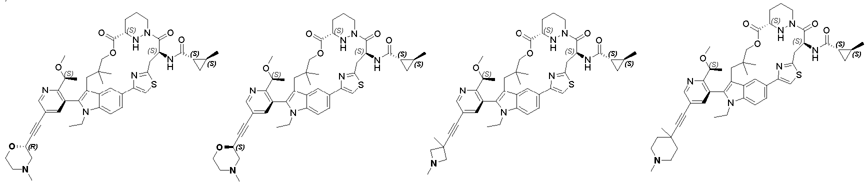 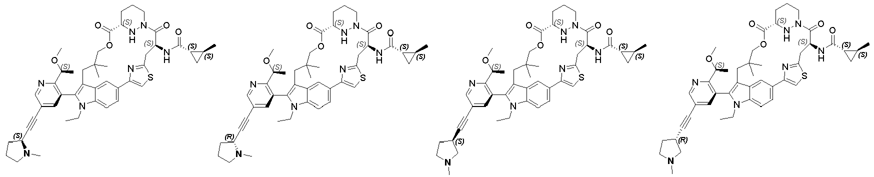 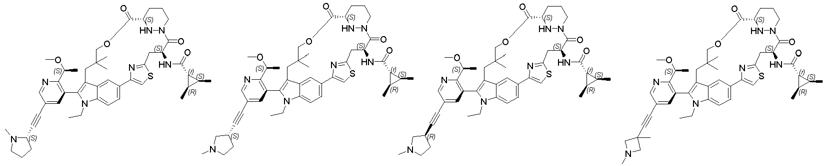 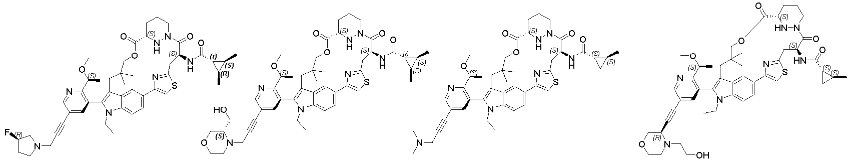 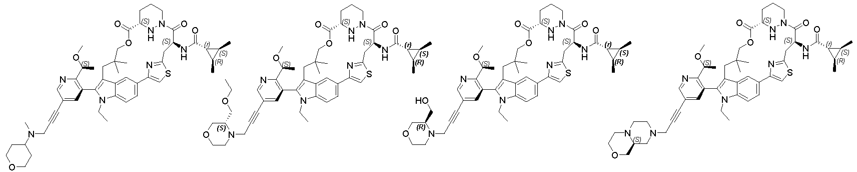 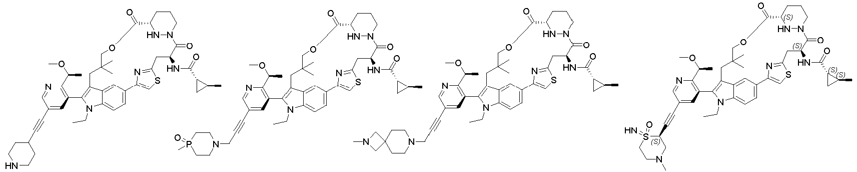 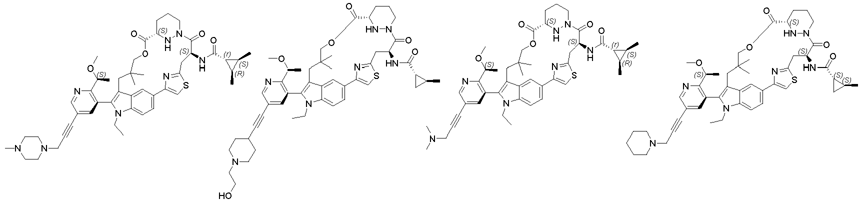 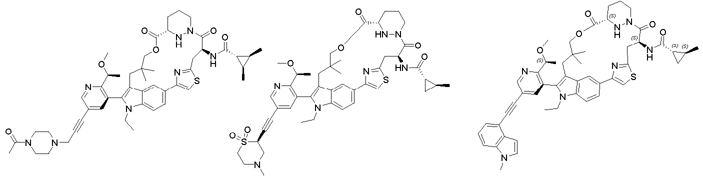 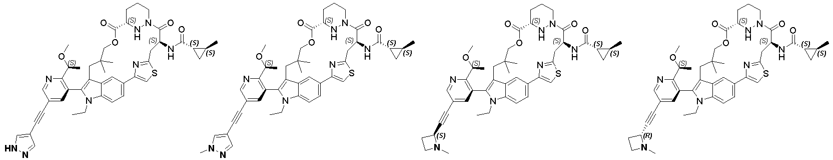 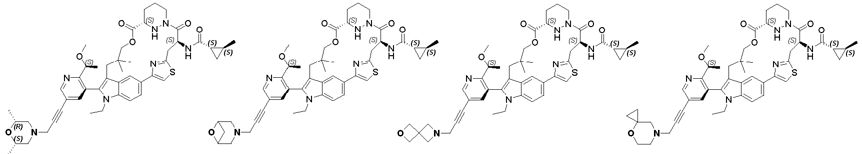 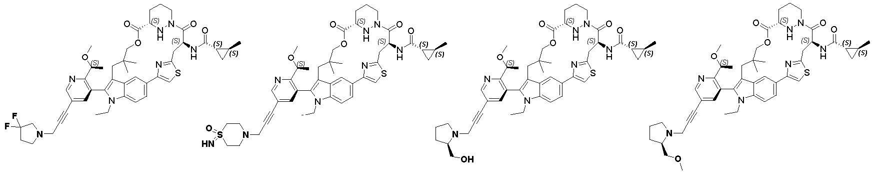 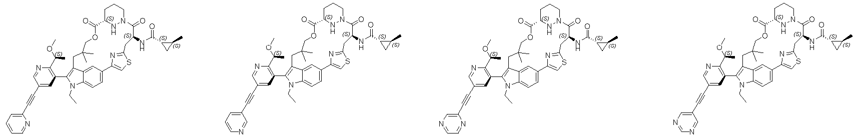 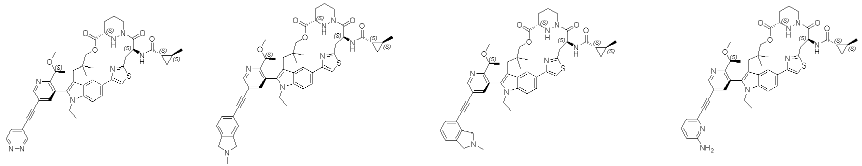 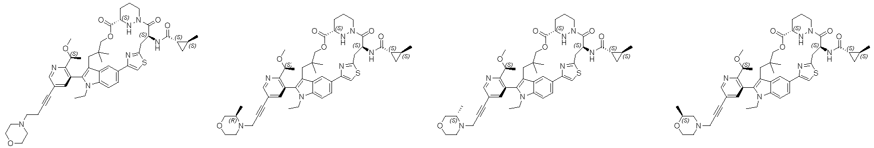 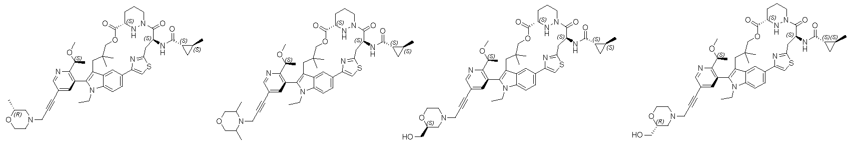 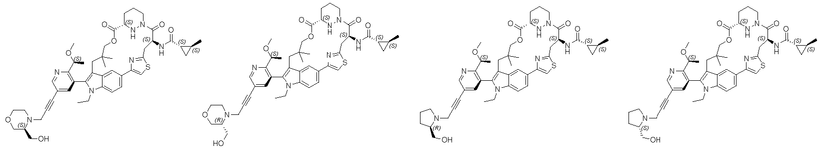 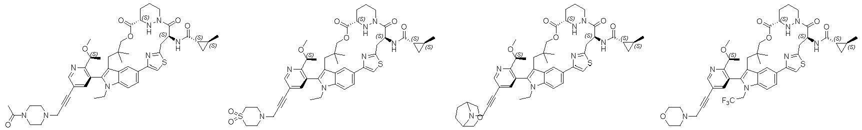 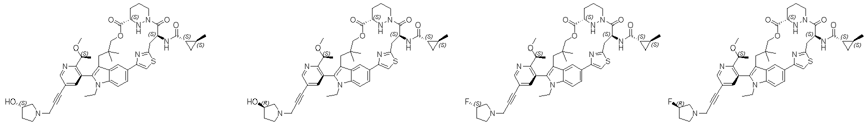 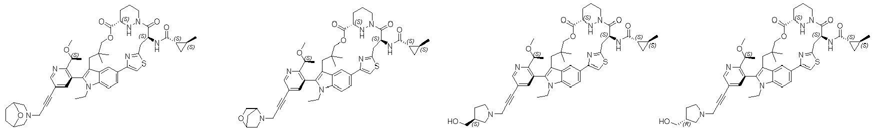 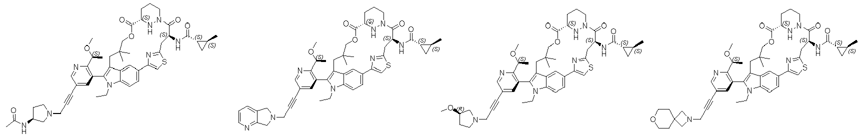 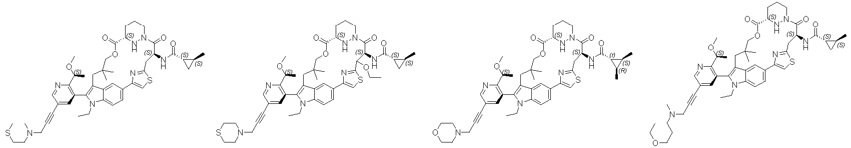 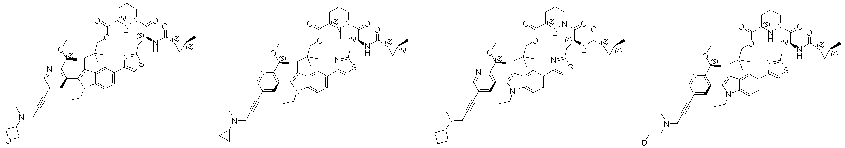 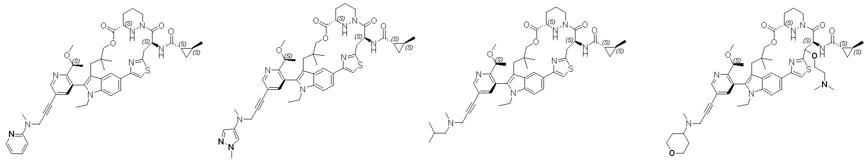 _{∘}

21. A pharmaceutical composition comprising the compound or a pharmaceutically acceptable salt, isotopic derivative, or stereoisomer thereof of any one of the preceding claims.

22. Use of the compound or a pharmaceutically acceptable salt, isotopic derivative, stereoisomer thereof according to any one of claims 1-20 and the pharmaceutical composition according to claim 18 in the preparation of medicine for preventing and/or treating cancer, tumor, inflammatory disease, autoimmune disease or immune mediated disease.
